# EUROPEAN PATENT SPECIFICATION

(11) **EP 2 734 624 B1**
(45) Date of publication and mention of the grant of the patent: **14.03.2018**
(21) Application number: 12721788.3
(22) Date of filing: 02.05.2012
(51) Int. Cl.: C12N 15/82, C07K 14/435, A01H 5/00, A01H 5/10

(54) **DOWN REGULATING GENE EXPRESSION IN INSECT PESTS**
HERUNTERREGULIERUNG DER GENEXPRESSION BEI INSEKTENSCHÄDLINGEN
DÉRÉGULATION DE L'EXPRESSION GÉNIQUE CHEZ DES INSECTES NUISIBLES

(30) Priority: 18.07.2011 US 201161508826 P; 20.04.2012 WO PCT/EP2012/057332
(43) Date of publication of application: 28.05.2014
(73) Proprietor: Devgen NV, 9052 Gent-Zwijnaarde (BE)
(72) Inventor: BEGHYN, Myriam, B-9870 Zulte (BE); BOGAERT, Thierry, B-8500 Kortrijk (BE); FELDMAN, Pascale, B-9030 Gent-Mariakerke (BE); RAEMAEKERS, Romaan, B-9840 De Pinte (BE)
(74) Representative: Syngenta International AG
(86) International application number: PCT/EP2012/058037
(87) International publication number: WO 2013/010690

(56) References cited:
- WO-A2-2009/091864
- DATABASE EMBL [Online] 21 October 2005 (2005-10-21), "NADBB46TR Aedes aegypti infected with Dengue virus Pool library Aedes aegypti cDNA clone NADBB46, mRNA sequence.", XP002681238, retrieved from EBI accession no. EM_EST:DV392288 Database accession no. DV392288
- DATABASE EMBL [Online] 18 November 2009 (2009-11-18), "DPO109.CR_F14 Dpo10-EAdult-MGFB-JH-Norm (DPO10) Dendroctonus ponderosae cDNA clone DPO109_F14 3', mRNA sequence.", XP002684133, retrieved from EBI accession no. EM_NEW:GT410548 Database accession no. GT410548
- WHYARD S ET AL: "Ingested double-stranded RNAs can act as species-specific insecticides", INSECT BIOCHEMISTRY AND MOLECULAR BIOLOGY, ELSEVIER SCIENCE LTD, GB, vol. 39, no. 11, 1 November 2009 (2009-11-01), pages 824-832, XP026777046, ISSN: 0965-1748, DOI: 10.1016/J.IBMB.2009.09.007 [retrieved on 2009-10-06]

## Description

### Field of the invention

The present invention relates generally to genetic control of infestation by insect pest species, particularly prevention and/or control of pest infestation of plants. More specifically, the invention relates to down-regulation of expression of target genes in insect pest species by interfering ribonucleic acid (RNA) molecules. Compositions and combinations containing the interfering RNA molecules of the invention for use in topical applications, for example in the form of insecticides, are also provided.

### Background to the invention

There exists an abundance of insect pest species that can infect or infest a wide variety of environments and host organisms. Insect pests include a variety of species from the insect Orders *Hemiptera* (true bugs), *Coleoptera* (beetles), *Siphonaptera* (fleas), *Dichyoptera* (cockroaches and mantids), *Lepidoptera* (moths and butterflies), *Orthoptera* (e.g. grasshoppers) and *Diptera* (true flies). Pest infestation can lead to significant damage. Insect pests that infest plant species are particularly problematic in agriculture as they can cause serious damage to crops and significantly reduce plant yields. A wide variety of different types of plant are susceptible to pest infestation including commercial crops such as rice, cotton, soybean, potato and corn.

Traditionally, infestation with insect pests has been prevented or controlled through the use of chemical pesticides. However, these chemicals are not always suitable for use in the treatment of crops as they can be toxic to other species and can cause significant environmental damage. Over more recent decades, researchers have developed more environmentally-friendly methods of controlling pest infestation. For example, microorganisms such as *Bacillus thuringiensis* bacteria that naturally express proteins toxic to insect pests have been used. Scientists have also isolated the genes encoding these insecticidal proteins and used them to generate transgenic crops resistant to insect pests e.g. corn and cotton plants genetically engineered to produce proteins of the Cry family. Although bacterial toxins have been highly successful in controlling certain types of pest, they are not effective against all pest species. Researchers have therefore looked for other more targeted approaches to pest control and in particular to RNA interference or 'gene silencing' as a means to control pests at the genetic level.

RNA interference or 'RNAi' is a process whereby the expression of genes in the context of a cell or whole organism is down-regulated in a sequence-specific manner. RNAi is now a well-established technique in the art for inhibiting or down-regulating gene expression in a wide variety of organisms including pest organisms such as fungi, nematodes and insects. Furthermore, previous studies have shown that down-regulation of target genes in insect pest species can be used as a means to control pest infestation.

WO2007/074405 describes methods of inhibiting expression of target genes in invertebrate pests including Colorado potato beetle. Furthermore, WO2009/091864 describes compositions and methods for the suppression of target genes from insect pest species including pests from the *Lygus* genus.

Although the use of RNAi for down-regulating gene expression in pest species is known in the art, the success of this technique for use as a pest control measure depends on selection of the most appropriate target genes, namely those wherein loss of function results in significant disruption of an essential biological process and/or death of the organism. The present invention is thus directed towards the down-regulation of particular target genes in insect pests as a means to achieve more effective prevention and/or control of insect pest infestation, particularly of plants.

### Summary of the invention

The current inventors sought to identify improved means for preventing and/or controlling insect pest infestation using genetic approaches. In particular, they investigated the use of RNAi to down-regulate genes in such a way as to impair the ability of the insect pest to survive, grow, colonize specific environments and/or infest host organisms and thus limit the damage caused by the pest. Therefore, in accordance with one aspect of the invention, there is provided an interfering ribonucleic acid (RNA or double stranded RNA) that functions upon uptake by an insect pest species to down-regulate expression of a target gene in said insect pest,
wherein the RNA comprises at least one silencing element wherein the silencing element is a region of double-stranded RNA comprising annealed complementary strands, one strand of which comprises or consists of a sequence of at least 30 contiguous nucleotides that is at least 85%, 90%, 95%, 98% or 99% identical to SEQ ID NO 389, or the complement thereof.
In a particular aspect of the invention, interfering RNA molecules of the current invention comprise at least one double-stranded region, typically the silencing element of the interfering RNA, comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene.

In certain embodiments, the present invention relates to an interfering RNA molecule which comprises at least one double-stranded region, typically the silencing element of the interfering RNA molecule, comprising a sense RNA strand annealed by complementary basepairing to an antisense RNA strand wherein the sense strand of the dsRNA molecule comprises a sequence of at least 30, 35, 40, 45, 50, 55, 60, 70, 80, 90, 100, 110, 125, 150, 175, 200, 225, 250, 300, 350, 400, 450, 500, 550, 600, 700, 800, 900 1000, 1100, 1200, 1300, 1400, 1500, 2000 or 3000 contiguous nucleotides, that is at least 85%, 90%, 95%, 98%, 99% or 100% complementary to a sequence of nucleotides located within the RNA transcript of a target gene.
In one embodiment, the target gene encodes the Rpn12 protein (e.g. an insect orthologue of the CG4157 Dm protein) said target gene being represented by SEQ ID NO. 389. In a preferred embodiment, the insect orthologue has at least 85%, 90%, 92%, 94%, 96%, 98%, 99% or 100% amino acid sequence identity to SEQ ID NO.393.

In accordance with a second aspect of the invention, there is provided a composition for preventing and/or controlling insect pest infestation comprising at least one interfering ribonucleic acid (RNA) and at least one suitable carrier, excipient or diluent, wherein the interfering RNA functions upon uptake by the pest to down-regulate the expression of a target gene within said pest,
wherein the RNA comprises at least one silencing element wherein the silencing element is a region of double-stranded RNA comprising annealed complementary strands, one strand of which comprises or consists of a sequence of at least 30 contiguous nucleotides that is at least 85% identical to SEQ ID NO 389, or the complement thereof.
The composition of the invention may be used for the prevention and/or control of pest infestation. In certain embodiments, the composition may be used as a pesticide for a plant or for propagation or reproductive material of a plant. In a further aspect, provided herein is a combination for preventing and/or controlling pest infestation comprising the composition of the invention and at least one other active agent.
In a further aspect, provided herein is a method for down-regulating expression of a target gene in an insect pest species in order to prevent and/or control pest infestation, comprising contacting said pest species with an effective amount of at least one interfering ribonucleic acid (RNA), wherein the interfering RNA functions upon uptake by the pest to down-regulate the expression of a target gene within said pest, wherein the RNA comprises at least one silencing element wherein the silencing element is a region of double-stranded RNA comprising annealed complementary strands, one strand of which comprises or consists of a sequence of at least 30 contiguous nucleotides that is at least 85%, 90%, 95%, 98% or 99% identical to SEQ ID NO 389, or the complement thereof, or.

In accordance with a further aspect of the invention, there is provided an isolated polynucleotide selected from the group consisting of:
(i) a polynucleotide which comprises at least 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by SEQ ID NO 389, or the complement thereof, and
wherein said polynucleotide is no longer than 10000, 9000, 8000, 7000, 6000, 5000, 4000, 3000, 2000 or 1500 nucleotides.
In a particular aspect of the invention, the isolated polynucleotide is part of an interfering RNA molecule, typically part of the silencing element, comprising at least one double-stranded region comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene. More particularly, the isolated polynucleotide is cloned in a DNA construct in a sense and antisense orientation so that the upon transcription of the sense and antisense polynucleotide a dsRNA molecule is formed, which functions upon uptake by a pest to inhibit or down-regulate the expression of a target gene within said pest.
In one aspect the present disclosure relates to an isolated polynucleotide that is cloned in a DNA construct in a sense and antisense orientation so that the upon transcription of the sense and antisense polynucleotide a dsRNA molecule is formed, which functions upon uptake by an insect to inhibit or down-regulate the expression of a target gene.

According to other embodiments, the present invention relates to an isolated polynucleotide that is cloned in a DNA construct in a sense and antisense orientation so that the upon transcription of the sense and antisense polynucleotide a dsRNA molecule is formed, which functions upon uptake by an insect to inhibit or down-regulate the expression of a target gene that encodes an insect ribosomal protein.

Preferably, the methods of the invention find practical application in the prevention and/or control of insect pest infestation, in particular, control of pest infestation of crop plants such as but not limited to cotton, potato, rice, strawberries, alfalfa, soy, tomato, canola, sunflower, sorghum, pearl millet, corn, eggplant, pepper and tobacco. In addition, the interfering RNA of the invention may be introduced into the plants to be protected by routine genetic engineering techniques.

### Brief description of the Tables and Figures

**Table 1** *Lygus hesperus* novel targets identified from first screen.
**Table 1B** *Lygus hesperus* novel targets in Lh594 pathway.
**Table 1C** *Lygus hesperus* novel targets identified from second round screen.
**Table 2** Polynucleotide sequences of target genes identified in *Lygus hesperus.*
**Table 3** Amino acid sequences of target genes identified in *Lygus hesperus.*
**Table 4** dsRNAs (sense strand represented by equivalent DNA sequence) corresponding to *Lygus hesperus* target genes and primers for producing the dsRNAs.
**Table 5** *Lygus hesperus* targets ranking according to dose response curves (DRCs) and compared to bench mark targets Lh423 & Lh105.
**Table 6** *Lygus hesperus* targets from second round screen-ranking according to DRCs and compared to bench mark targets Lh423 & Lh594.
**Table 7** Polynucleotide sequences of target genes identified in Colorado potato beetle (CPB).
**Table 8** Amino acid sequences of target genes identified in CPB.
**Table 9** dsRNAs (sense strand represented by equivalent DNA sequence) corresponding to CPB target genes and primers for producing the dsRNAs.
**Table 10** Polynucleotide sequences of target genes identified in brown plant hopper (BPH).
**Table 11** Amino acid sequences of target genes identified in BPH.
**Table 12** dsRNAs (sense strand represented by equivalent DNA sequence) corresponding to BPH target genes and primers for producing the dsRNAs.
**Table 13** Primers used for amplification of aphid cDNAs, based on pea aphid genomic sequence.
**Table 14** Polynucleotide sequences of target genes identified in aphids.
**Table 15** Amino acid sequences of target genes identified in aphids.
**Table 16** dsRNAs (sense strand represented by equivalent DNA sequence) corresponding to aphid target genes and primers for producing the dsRNAs.
**Table 17** Degenerate primers used for amplification of CPB Ld594 cDNA
**Table 18** Degenerate primers used for amplification of BPH cDNAs
**Table 19:** *Leptinotarsa decemlineata* novel targets from the screen.
**Table 20:** *Nilaparvata lugens* novel identified target.
**Table 21:** *Acyrthosiphon pisum* novel identified targets.
**Figure 1****:** Plates Lh001_009 second confirmation assay. Dark bars: mortality at day 3 to 6, light bars: mortality at day 6 to 8. Candidate clones are named using the "Lygxxx" screening codes and the "Lhxxx" target nomenclature codes.
**Figure 2****:** Plates Lh010_020 second confirmation assay. Dark bars: mortality at day 3 to 6, light bars: mortality at day 6 to 8. Candidate clones are named using the "Lygxxx" screening codes and the "Lhxxx" target nomenclature codes.
**Figure 3****:** Mortality analysis of *Lygus* novel targets from plates Lh001 to Lh009, expressed as % mortality over a 10 day period. Controls are indicated in dotted lines. Positive control: Lh423 dsRNA (RpL19). Negative controls: GFP dsRNA and diet only (Control).
**Figure 4****:** Mortality analysis of *Lygus* novel targets from plates Lh010 to Lh020, expressed as % mortality over a 10 day period. Controls are indicated in dotted lines. Positive control: Lh423 (RpL19). Negative controls: GFP and diet only (Control).
**Figures 5 to 9** *Lygus hesperus* novel targets - dose response curves at concentrations of purified synthetic dsRNA ranging from 0.4 to 0.025 µg/µl (in the figure, the unit "µg/µl" is not displayed). GFP dsRNA and milliQ water were used negative controls. dsRNA of targets were produced using the primers as described in the example section 1.1.
**Figure 10** Lh594 dose response curve, at dsRNA concentrations ranging from 0.05 to 0.001 µg/µl. GFP dsRNA and milliQ water were used negative controls.
**Figure 11** **A** dsRNA activity in *Lygus hesperus* bioassay in absence of tRNA. Lh594 (5µg/µl); positive control: Lh423 (5µg/µl); negative controls: GFP dsRNA (5µg/µl) and milliQ water; **B** Identification of Lh594 limit of activity using decreasing concentration of dsRNA (from 5 µg to 0.25 µg). Negative controls: GFP dsRNA (5µg/µl) and milliQ water.
**Figure 12** Plates Lh010 to Lh020 second confirmation assay of second screen targets. Dark bars: mortality at day 4 to 8, light bars: mortality at day 4 to 6. Candidate clones are named using the "Lygxxx" screening codes and the "Lhxxx" target nomenclature codes.
**Figure 13** Assay results for *Lygus* troponin pathway targets, tested at 0.5 µg/µl fixed.
**Figures 14 A-B** *Lygus hesperus* novel targets from troponin pathway - dose response curves at concentrations of purified synthetic dsRNA ranging from 0.4 to 0.025 µg/µl (in the figure, the unit "µg/µl" is not always displayed). GFP dsRNA and milliQ water were used as negative controls.
**Figures 15 A-D** *Lygus hesperus* novel targets of second screen targets - dose response curves at concentrations of purified synthetic dsRNA ranging from 0.5 to 0.05 µg/µl. GFP dsRNA and milliQ water were used as negative controls.
**Figure 16** Survival analysis of CPB larvae treated with 1 µg dsRNA Ld594, Ld619 and Ld620. Positive controls included 1 µg dsRNA of bench mark targets Ld513 and Ld049. Negative controls included milliQ water and FP.
**Figure 17** Effects of Ld594, Ld619 and Ld620 dsRNAs on pupation of CPB 4^{th} instar larvae, compared to untreated control (**UTC**)**.** Bugs were fed 1 µg dsRNA dispensed in potato leaf disks, then were allowed to feed on untreated potato leaves (**A**) for 4 days before being placed on vermiculite. To assess the effect of the dsRNA, dead insects were excavated from the vermiculite (because of the strong effects induced by Ld594 dsRNA, no pupae could be recovered from the vermiculite and therefore, no image is available for this target dsRNA) (**B**)**.**
**Figure 18** Effect of CPB Ld594, 619 & 620 dsRNAs on survival and fitness of CPB adults. Assessments were performed on days 4, 6, 7, 8, 11 and 13. Control MQ: milliQ water.
**Figure 19** Activity of dsRNA from NI594 pathway in brown plant hopper. DsRNAs were tested at 0.5 µg/µl in presence of 0.1% CHAPSO. Positive control: NI537 dsRNA (0.5 µg/µl), negative controls: GFP dsRNA (0.5 µg/µl) and diet alone.
**Figure 20** Activity of dsRNA from Ap594, Ap423, Ap537 and Ap560 on *A. pisum.* DsRNAs were tested at 0.5 µg/µl in presence of 5 µg/µl tRNA. Negative control: GFP dsRNA (0.5 µg/µl).
**Figure 21** Mortality percentages of *L. decemlineata* larvae on artificial diet treated with dsRNA. Ld583, Ld584, Ld586 & Ld588 represent target clones. Positive control: Ld513; negative control: FP.

### Detailed description of the invention

The present inventors have discovered that down-regulating the expression of particular target genes in insect pest species by RNAi can be used to effectively prevent and/or control infestation by said insect pest.
As used herein, the term "control" of pest infestation refers to any effect on a pest that serves to limit and/or reduce either the numbers of pest organisms and/or the damage caused by the pest. Preferred target genes are therefore essential genes that control or regulate one or more essential biological functions within the insect pest, for example, cell division, reproduction, energy metabolism, digestion, neurological function and the like. Down-regulation of these essential genes by RNAi techniques can lead to death of the insect, or otherwise significantly retard growth and development or impair the ability of the pest to colonize an environment or infest host organisms.
The present inventors have now identified superior target genes of insect pest species belonging to the *Lygus, Leptinotarsa, Nilaparvata* and *Acyrthosiphum* genus, which targets are envisaged for use singly or in combination as an effective means for RNAi-mediated control of insect infestation, for example of agronomically important crops. Orthologues of these newly identified target genes can be used in other insect species to control pest infestation of the corresponding relevant crops.
More specifically, the present inventors describe here that genes encoding for proteins of the troponin/myofilament complex form excellent target genes for suppression by the RNA inhibition machinery. One of these target genes encoded the insect troponin I protein (wings up A) which is an orthologue of the Drosophila CG7178 protein. This protein is involved in muscular contraction and belongs to a physiological pathway that was not yet fully explored for (insect) pest control through RNA inhibition. Moreover, since this protein complex is animal specific, no plant genes homologues or orthologues are known, reducing the risk of off-type plant phenotypes when expressing target dsRNA in plants. In addition, in Drosophila, troponin I is described as a haplo-insufficient gene, displaying a mutant phenotype in the heterozygote state. Such genes are particularly susceptible to reduced mRNA expression levels and as such can be considered as ideal RNAi targets.
Further interesting target genes in this troponin/myofilament complex are listed below.

| **Annotation ID** | **Cytology** | **Dm identifier** |
|---|---|---|
| up | upheld | CG7107 |
| Tm1 | tropomyosin 1 | CG4898 |
| Tm2 | tropomyosin 2 | CG4843 |
| Mhc | myosin heavy chain | CG 17927 |
| Mlc-c | myosin light chain cytoplasmic | CG3201 |
| sqh | spaghetti squash | CG3595 |
| zip | zipper | CG 15792 |

Thus, according to one embodiment the present invention relates to an interfering ribonucleic acid (RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of at least 30 contiguous nucleotides that is at least 85%, 90%, 95%, 98% or 99% identical to SEQ ID NO 389, or the complement thereof.

In a preferred embodiment, the target gene encodes an insect regulatory particle non-ATPase 12 protein.
Thus, in one aspect, the invention provides an interfering ribonucleic acid (RNA) that functions upon uptake by an insect pest species to down-regulate expression of a target gene in said insect pest. As used herein, a "target gene" comprises any gene in the insect pest which one intends to down-regulate. In a preferred embodiment, the target gene is down-regulated so as to control pest infestation, for example by disrupting an essential biological process occurring in the pest, or by decreasing the pathogenicity of the pest. Preferred target genes therefore include but are not limited to those that play key roles in regulating feeding, survival, growth, development, reproduction, infestation and infectivity. According to one embodiment, the target gene is such that when its expression is down-regulated or inhibited, the insect pest is killed. According to another embodiment, the target gene is such that when its expression is down-regulated or inhibited, growth of the pest is prevented or retarded or stunted or delayed or impeded, pest reproduction is prevented, or transition through the life cycles of the pest is prevented. According to yet another embodiment of the invention, the target gene is such that when its expression is down-regulated or inhibited, the damage caused by the pest and/or the ability of the pest to infect or infest environments, surfaces and/or plant or crop species is reduced; or the pest stops feeding from its natural food resources such as plants and plant products. The terms "infest" and "infect" or "infestation" and "infection" are generally used interchangeably throughout.
The target genes may be expressed in all or some of the cells of the insect pest. Furthermore, the target genes may only be expressed by the insect pest at a particular stage of its life-cycle, for example, the mature adult phase, immature nymph or larval phase or egg phase.
As used herein "pest" species are preferably insect species that cause infection or infestation, preferably of plants. The insect species may comprise and species belonging to the Orders *Coleoptera, Lepidoptera, Diptera, Dichyoptera, Orthoptera, Hemiptera,* or *Siphonaptera.*
Preferred plant pathogenic insects according to the invention are plant pest are selected from the group consisting of *Leptinotarsa* spp. (e.g. *L. decemlineata* (Colorado potato beetle), *L. juncta* (false potato beetle), or *L. texana* (Texan false potato beetle)); *Nilaparvata* spp. (e.g. *N. lugens (brown planthopper)); Laodelphax* spp. (e.g. *L. striatellus* (small brown planthopper)); *Nephotettix* spp. (e.g. *N. virescens* or *N. cincticeps* (green leafhopper), or *N.nigropictus* (rice leafhopper)); *Sogatella* spp. (e.g. *S*. *furcifera* (white-backed planthopper)); *Chilo* spp. (e.g. *C. suppressalis* (rice striped stem borer), *C*. *auricilius* (gold-fringed stem borer), or *C*. *polychrysus* (dark-headed stem borer)); *Sesamia* spp. (e.g. *S*. *inferens* (pink rice borer)); *Tryporyza* spp. (e.g. *T. innotata* (white rice borer), or *T. incertulas* (yellow rice borer)); *Anthonomus* spp. (e.g. *A. grandis* (boll weevil)); *Phaedon* spp. (e.g. *P*. *cochleariae* (mustard leaf beetle)); *Epilachna* spp. (e.g. *E. varivetis* (mexican bean beetle)); *Tribolium* spp. (e.g. *T. castaneum* (red floor beetle)); *Diabrotica* spp. (e.g. *D*. *virgifera virgifera* (western corn rootworm), *D. barberi* (northern corn rootworm), *D*. *undecimpunctata* howardi (southern corn rootworm), *D*. *virgifera zeae* (Mexican corn rootworm); *Ostrinia* spp. (e.g. *O*. *nubilalis* (European corn borer)); *Anaphothrips* spp. (e.g. *A. obscrurus* (grass thrips)); *Pectinophora* spp. (e.g. *P. gossypiella* (pink bollworm)); *Heliothis* spp. (e.g. *H. virescens* (tobacco budworm)); *Trialeurodes* spp. (e.g. *T. abutiloneus* (banded-winged whitefly) *T. vaporariorum* (greenhouse whitefly)); *Bemisia* spp. (e.g. B. *argentifoli*i (silverleaf whitefly)); *Aphis* spp. (e.g. *A. gossypii* (cotton aphid)); *Lygus* spp. (e.g. *L*. *lineolaris* (tarnished plant bug) or *L. hesperus* (western tarnished plant bug)); *Euschistus* spp. (e.g. *E*. *conspersus* (consperse stink bug)); *Chlorochroa* spp. (e.g. *C*. *sayi* (Say stinkbug)); *Nezara* spp. (e.g. *N. viridula* (southern green stinkbug)); *Thrips* spp. (e.g. *T. tabaci* (onion thrips)); *Frankliniella* spp. (e.g. *F. fusca* (tobacco thrips), or *F. occidentalis* (western flower thrips)); *Acheta* spp. (e.g. *A. domesticus* (house cricket)); *Myzus* spp. (e.g. *M. persicae* (green peach aphid)); *Macrosiphum spp. (e.g. M. euphorbiae* (potato aphid)); *Blissus* spp. (e.g. *B*. *leucopterus leucopterus* (chinch bug)); *Acrosternum* spp. (e.g. *A. hilare* (green stink bug)); *Chilotraea* spp. (e.g. *C*. *polychrysa* (rice stalk borer)); *Lissorhoptrus* spp. (e.g. *L. oryzophilus* (rice water weevil)); *Rhopalosiphum* spp. (e.g. *R*. *maidis* (corn leaf aphid)); and *Anuraphis* spp. (e.g. *A. maidiradicis* (corn root aphid)).

According to a more specific embodiment, the invention is applicable for species belonging to the family of Chrysomelidae or leaf beatles. Chrysomelid beetles such Colorado potato Beetles, Flea Beetles, Corn Rootworms and Curculionids such as Alfalfa Weevils are particularly important pests. Specific Leptinotarsa species to control according to the invention include Colorado Potato Beetle (*Leptinotarsa decemlineata* (Say) and False Potato Beetle (*Leptinotarsa juncta* (Say). CPB is a (serious) pest on our domestic potato, other cultivated and wild tuber bearing and non-tuber bearing potato species and other Solanaceous (nightshades) plant species incuding the crop species tomato, eggplant, peppers, tobacco (Nicotiana species including ornamentals), ground cherry, rice, corn or cotton; and the weed/herb species, horse nettle, common nightshade, thorn apple, henbane and buffalo burr. Corn rootworms include species found in the genus Diabrotica (e.g., *D*. *undecimpunctata undecimpunctata, D. undecimpunctata howardii, D. longicornis, D. virgifera* and *D*. *balteata*)*.* Corn rootworms cause extensive damage to corn and curcubits.

According to a more specific embodiment, the invention is applicable for species belonging to the order of Hemipterans (family of Aphidoidea), such as *Myzus persicae* (green peach aphid, *Aphis fabae* (Black Bean Aphid), *Acyrthosiphum pisum* (Pea Aphid), *Brevicoryne brassicae* (Cabbage Aphid), *Sitobion avenae* (Grain Aphid), *Cavariella aegopodii* (Carrot Aphid), *Aphis craccivora* (Groundnut Aphid), *Aphis gossypii* (Cotton Aphid), *Toxoptera aurantii* (Black Citrus Aphid), *Cavariella spp* (Willow Aphid), *Chaitophorus spp* (Willow Leaf Aphids), *Cinara spp.* (Black Pine Aphids), *Drepanosiphum platanoides* (Sycamore Aphid) *Elatobium spp* (Spruce Aphids) which cause damage to plants such as Prunus trees, particularly peach, apricot and plum; trees that are mainly cultured for wood production such as willows and poplars, to row crops such as corn, cotton, soy, wheat and rice, to vegetable crops of the families Solanaceae, Chenopodiaceae, Compositae, Cruciferae, and Cucurbitaceae, including but not limited to, artichoke, asparagus, bean, beets, broccoli, Brussels sprouts, cabbage, carrot, cauliflower, cantaloupe, celery, corn, cucumber, fennel, kale, kohlrabi, turnip, eggplant, lettuce, mustard, okra, parsley, parsnip, pea, pepper, potato, radish, spinach, squash, tomato, turnip, watercress, and watermelon; or field crops such as, but not limited to, tobacco, sugar beet, and sunflower; a flower crop or other ornamental plant such as pine trees and conifers. Other Hemipterans belong to *Nilaparvata ssp* (eg. *N. lugens, Sogatella furcifera*) and cause damage to rice plants. Other Hemipterans belong to *Lygus ssp* (eg. *Lygus hesperus, Lygus rugulipennis, Lygus lineolaris, Lygus sully*) and other species of plant-feeding insects in the family of the Miridae, and cause damage to cotton, potato plants, strawberries, cotton, alfalfa, canola, peach, plums, grape, lettuce, eggplant, onion, green beans. As well as several Mediterranean trees and several ornamental trees such as elm tree (*Ulmus spp.*) pine nut (*Pinus Pinea*) London plane tree (*Platanus Acerifolia*), white redbud (*Malus alba*). Other Hemipterans belong to the family of the Pentatomoidea, they are commonly referred to as shield bugs, chust bugs, and stink bugs (eg; the brown marmorated stink bug (*Halyomorpha halys*), the Consperse stink bug (*Euschistus conspersus*), southern green stink bug (*Nezara viridula*), forest bug (*Pentatoma rufipes*), harlequin bug (*Murgantia histrionica*), rice stink bug (*Oebalus pugnax*)) and cause damage to fruits including apples, peaches, figs, mulberries, citrus fruits and persimmons, blackberry, and vegetables including sweetcorn, tomatoes, soy beans, lima beans and green peppers, cabbage, cauliflower, turnips, horseradish, collards, mustard, Brussels sprouts, potato, egg plant, okra, beans, asparagus, beets, weeds, fruit trees and field crops such as field corn and soy bean. Stink bugs are also a pest of grasses, sorghum and rice.
A plant to be used in the methods of the invention, or a transgenic plant according to the invention encompasses any plant, but is preferably a plant that is susceptible to infestation by a plant pathogenic insect.
Accordingly, the present invention extends to plants and to methods as described herein wherein the plant is chosen from the following group of plants (or crops): alfalfa, apple, apricot, artichoke, asparagus, avocado, banana, barley, beans, beet, blackberry, blueberry, broccoli, Brussels sprouts, cabbage, canola, carrot, cassava, cauliflower, a cereal, celery, cherry, citrus, clementine, coffee, corn, cotton, cucumber, eggplant, endive, eucalyptus, figs, grape, grapefruit, groundnuts, ground cherry, kiwifruit, lettuce, leek, lemon, lime, pine, maize, mango, melon, millet, mushroom, nut oat, okra, onion, orange, an ornamental plant or flower or tree, papaya, parsley, pea, peach, peanut, peat, pepper, persimmon, pineapple, plantain, plum, pomegranate, potato, pumpkin, radicchio, radish, rapeseed, raspberry, rice, rye, sorghum, soy, soybean, spinach, strawberry, sugar beet, sugarcane, sunflower, sweet potato, tangerine, tea, tobacco, tomato, a vine, watermelon, wheat, yams and zucchini.
In specific embodiments, the present invention provides target genes which encode proteins involved in the function of a wings up A (troponin I), a mitochondrial cytochrome c oxidase subunit II protein, or one of the ribosomal proteins as specified in Table 1.
In preferred embodiments, the present invention provides target genes selected from the group of genes (i) having a nucleotide sequence comprising SEQ ID NO 389, or the complement thereof, or having a nucleotide sequence so that, when the two sequences are optimally aligned and compared, is at least 85%, 90%, 95%, 98% or 99% identical SEQ ID NO 389, or the complement thereof;
and wherein the nucleotide sequence of said gene is no longer than 10000, 9000, 8000, 7000, 6000, 5000, 4000, 3000, 2000 or 1500 nucleotides.
As used herein, the term "having" has the same meaning as "comprising".
As used herein, the term "sequence identity" is used to describe the sequence relationship between two or more nucleotide or amino acid sequences. The percentage of "sequence identity" between two sequences is determined by comparing two optimally aligned sequences over a comparison window (a defined number of positions), wherein the portion of the sequence in the comparison window may comprise additions or deletions (*i.e*. gaps) as compared to the reference sequence in order to achieve optimal alignment. The percentage sequence identity is calculated by determining the number of positions at which the identical nucleotide base or amino acid residue occurs in both sequences to yield the number of 'matched' positions, dividing the number of matched positions by the total number of positions in the comparison window and multiplying the result by 100. Methods and software for determining sequence identity are available in the art and include the Blast software and GAP analysis. For nucleic acids, the percent identity is calculated preferably by the BlastN alignment tool whereby the percent identity is calculated over the entire length of the query nucleotide sequence.
A person skilled in the art will recognise that homologues or orthologues (homologues existing in different species) of the target genes represented by SEQ ID NO 389 can be identified. These pest homologues and/or orthologues are also disclosed. Preferred homologues and/or orthologues are genes similar in nucleotide sequence to such a degree that when the two genes are optimally aligned and compared, the homologue and/or orthologue has a sequence that is at least 85%, more preferably at least 90% or 95%, and most preferably at least about 99% identical to SEQ ID NO 389 or the complement thereof. Other homologues are genes which are alleles of a gene comprising a sequence as represented by SEQ ID NO 389. Further preferred homologues are genes comprising at least one single nucleotide polymorphism (SNP) compared to a gene comprising a sequence as represented by SEQ ID NO 389.
The 'interfering ribonucleic acid (RNA)' of the current disclosure are any type of RNA molecule capable of down-regulating or 'silencing' expression of a target gene, including but not limited to sense RNA, antisense RNA, short interfering RNA (siRNA), microRNA (miRNA), double-stranded RNA (dsRNA), hairpin RNA (RNA) and the like. Methods to assay for functional interfering RNA molecules are well known in the art and are disclosed elsewhere herein.
The interfering RNA molecules of the current invention effect sequence-specific down-regulation of expression of a target gene by binding to a target nucleotide sequence within the target gene. Binding occurs as a result of base pairing between complementary regions of the interfering RNA and the target nucleotide sequence. As used herein, the term 'silencing element' refers to the portion or region of the interfering RNA comprising or consisting of a sequence of nucleotides which is complementary to a target nucleotide sequence within the target gene, and which functions as the active portion of the interfering RNA to direct down-regulation of expression of said target gene. In one embodiment of the invention, the silencing element comprises or consists of a sequence of at least 30 contiguous nucleotides complementary to a target nucleotide sequence within the target gene.
As used herein, "expression of a target gene" refers to the transcription and accumulation of the RNA transcript encoded by a target gene and/or translation of the mRNA into protein. The term 'down-regulate' is intended to refer to any of the methods known in the art by which interfering RNA molecules reduce the level of primary RNA transcripts, mRNA or protein produced from a target gene. In certain embodiments, down-regulation refers to a situation whereby the level of RNA or protein produced from a gene is reduced by at least 10%, preferably by at least 33%, more preferably by at least 50%, yet more preferably by at least 80%. In particularly preferred embodiments, down-regulation refers to a reduction in the level of RNA or protein produced from a gene by at least 80%, preferably by at least 90%, more preferably by at least 95%, and most preferably by at least 99% within cells of the insect pest as compared with an appropriate control insect pest which has for example, not been exposed to an interfering RNA or has been exposed to a control interfering RNA molecule. Methods for detecting reductions in RNA or protein levels are well known in the art and include RNA solution hybridization, Northern hybridization, reverse transcription (e.g. quantitative RT-PCR analysis), microarray analysis, antibody binding, enzyme-linked immunosorbent assay (ELISA) and Western blotting. In another embodiment of the invention, down-regulation refers to a reduction in RNA or protein levels sufficient to result in a detectable change in a phenotype of the pest as compared with an appropriate pest control, for example, cell death, cessation of growth, or the like. Down-regulation can thus be measured by phenotypic analysis of the insect pest using techniques routine in the art.
In a preferred aspect of the disclosure, the interfering RNA down-regulates gene expression by RNA interference or RNAi. RNAi is a process of sequence-specific gene regulation typically mediated by double-stranded RNA molecules such as short interfering RNAs (siRNAs). siRNAs comprise a sense RNA strand annealed by complementary basepairing to an antisense RNA strand. The sense strand or 'guide strand' of the siRNA molecule comprises a sequence of nucleotides complementary to a sequence of nucleotides located within the RNA transcript of the target gene. The sense strand of the siRNA is therefore able to anneal to the RNA transcript via Watson-Crick-type basepairing and target the RNA for degradation within a cellular complex known as the RNAi-induced silencing complex or RISC. Thus, in the context of preferred interfering RNA molecules of the current invention, the silencing element as referred to herein may be a double-stranded region comprising annealed complementary strands, at least one strand of which comprises or consists of a sequence of nucleotides which is complementary or at least partially complementary to a target nucleotide sequence within a target gene. In one embodiment the double-stranded region has a length of at least 30, 35, 40, 50, 55, 60, 70, 80, 90, 100, 125, 150, 175, 200, 300, 400, 500, 600, 700, 800, 900, 1000, 1100, 1200, 1300, 1400, 1500, 2000 or 3000 base pairs.
Longer double-stranded RNA (dsRNA) molecules comprising one or more functional double-stranded silencing elements as described elsewhere herein, and capable of RNAi-mediated gene silencing are also contemplated within the scope of the current invention. Such longer dsRNA molecules comprise at least 80, 200, 300, 350, 400, 450, 500, 550, 600, 700, 800, 900, 1000, 1100, 1200, 1300, 1400, 1500, 2000 or 3000 base pairs. These dsRNA molecules may serve as precursors for the active siRNA molecules that direct the RNA transcript to the RISC complex for subsequent degradation. dsRNA molecules present in the environment surrounding an organism or the cells thereof may be taken up by the organism and processed by an enzyme called Dicer to yield siRNA molecules. Alternatively, the dsRNA may be produced *in vivo i.e.* transcribed from a polynucleotide or polynucleotides encoding the same present within a cell, for instance a bacterial cell or a plant cell, and subsequently processed by Dicer either within the host cell or preferably within the insect pest cells following uptake of the longer precursor dsRNA. The dsRNA may be formed from two separate (sense and antisense) RNA strands that anneal by virtue of complementary basepairing. Alternatively, the dsRNA may be a single strand that is capable of folding back on itself to form a hairpin RNA (RNA) or stem-loop structure. In the case of a RNA, the double-stranded region or 'stem' is formed from two regions or segments of the RNA that are essentially inverted repeats of one another and possess sufficient complementarity to allow the formation of a double-stranded region. One or more functional double-stranded silencing elements may be present in this 'stem region' of the molecule. The inverted repeat regions are typically separated by a region or segment of the RNA known as the 'loop' region. This region can comprise any nucleotide sequence conferring enough flexibility to allow self-pairing to occur between the flanking complementary regions of the RNA. In general, the loop region is substantially single-stranded and acts as a spacer element between the inverted repeats.
All the interfering RNA molecules of the invention effect sequence-specific down-regulation of expression of a target gene by binding to a target nucleotide sequence within the target gene. Binding occurs as a result of complementary base pairing between the silencing element of the interfering RNA and the target nucleotide sequence. The interfering RNA molecules of the invention comprise at least one or at least two silencing elements. In one embodiment of the current invention, the target nucleotide sequence comprises a sequence of nucleotides as represented by the RNA transcript of the target gene, or a fragment thereof wherein the fragment is preferably at least 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 nucleotides. In a preferred embodiment of the current invention, the target nucleotide sequence comprises a sequence of nucleotides equivalent to the RNA transcript encoded by any of the polynucleotides selected from the group consisting of (i) a polynucleotide which comprises at least 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100 or 1115 contiguous nucleotides of a nucleotide sequence as represented by SEQ ID NO 389, or the complement thereof. In a more preferred embodiment of the above, said polynucleotide is no longer than 10000, 9000, 8000, 7000, 6000, 5000, 4000, 3000, 2000 or 1500 nucleotides.
Preferably, the interfering RNA molecules of the current invention comprise at least one double-stranded region, typically the silencing element of the interfering RNA, comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene.
The silencing element, or at least one strand thereof wherein the silencing element is double-stranded, may be fully complementary or partially complementary to the target nucleotide sequence of the target gene. As used herein, the term "fully complementary" means that all the bases of the nucleotide sequence of the silencing element are complementary to or 'match' the bases of the target nucleotide sequence. The term "at least partially complementary" means that there is less than a 100% match between the bases of the silencing element and the bases of the target nucleotide sequence. The skilled person will understand that the silencing element need only be at least partially complementary to the target nucleotide sequence in order to mediate down-regulation of expression of the target gene. It is known in the art that RNA sequences with insertions, deletions and mismatches relative to the target sequence can still be effective at RNAi. According to the current invention, it is preferred that the silencing element and the target nucleotide sequence of the target gene share at least 85% sequence identity, preferably at least 90% or 95% sequence identity, or more preferably at least 97% or 98% sequence identity and still more preferably at least 99% sequence identity. Alternatively, the silencing element may comprise 1, 2 or 3 mismatches as compared with the target nucleotide sequence over every length of 24 partially complementary nucleotides.
It will be appreciated by the person skilled in the art that the degree of complementarity shared between the silencing element and the target nucleotide sequence may vary depending on the target gene to be down-regulated or depending on the insect pest species in which gene expression is to be controlled.
In another embodiment of the current invention, the silencing element comprises a sequence of nucleotides that is the RNA equivalent of any of the polynucleotides selected from the group consisting of a polynucleotide which comprises at least 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100 or 1115 contiguous nucleotides of a nucleotide sequence as represented by SEQ ID NO 389, or the complement thereof, wherein said polynucleotide is no longer than 10000, 9000, 8000, 7000, 6000, 5000, 4000, 3000, 2000 or 1500 nucleotides. It will be appreciated that in such embodiments the silencing element may comprise or consist of a region of double-stranded RNA comprising annealed complementary strands, one strand of which, the sense strand, comprises a sequence of nucleotides at least partially complementary to a target nucleotide sequence within a target gene.
The target nucleotide sequence may be selected from any suitable region or nucleotide sequence of the target gene or RNA transcript thereof. For example, the target nucleotide sequence may be located within the 5'UTR or 3'UTR of the target gene or RNA transcript or within exonic or intronic regions of the gene.
The skilled person will be aware of methods of identifying the most suitable target nucleotide sequences within the context of the full-length target gene. For example, multiple silencing elements targeting different regions of the target gene can be synthesised and tested. Alternatively, digestion of the RNA transcript with enzymes such as RNAse H can be used to determine sites on the RNA that are in a conformation susceptible to gene silencing. Target sites may also be identified using *in silico* approaches, for example, the use of computer algorithms designed to predict the efficacy of gene silencing based on targeting different sites within the full-length gene.
The interfering RNAs of the current invention may comprise one silencing element or multiple silencing elements, wherein each silencing element comprises or consists of a sequence of nucleotides which is at least partially complementary to a target nucleotide sequence within a target gene and that functions upon uptake by an insect pest species to down-regulate expression of said target gene. Concatemeric RNA constructs of this type are described in WO2006/046148. In the context of the present invention, the term 'multiple' means at least two, at least three, at least four, etc and up to at least 10, 15, 20 or at least 30. In one embodiment, the interfering RNA comprises multiple copies of a single silencing element *i.e.* repeats of a silencing element that binds to a particular target nucleotide sequence within a specific target gene. In another embodiment, the silencing elements within the interfering RNA comprise or consist of different sequences of nucleotides complementary to different target nucleotide sequences. It should be clear that combinations of multiple copies of the same silencing element combined with silencing elements binding to different target nucleotide sequences are within the scope of the current invention.
The different target nucleotide sequences may originate from a single target gene in an insect pest species in order to achieve improved down-regulation of a specific target gene in an insect pest species. In this case, the silencing elements may be combined in the interfering RNA in the original order in which the target nucleotide sequences occur in the target gene, or the silencing elements may be scrambled and combined randomly in any rank order in the context of the interfering RNA as compared with the order of the target nucleotide sequences in the target gene.
Alternatively, the different target nucleotide sequences are representing a single target gene but originating from different insect pest species.
Alternatively, the different target nucleotide sequences may originate from different target genes. If the interfering RNA is for use in preventing and/or controlling pest infestation, it is preferred that the different target genes are chosen from the group of genes regulating essential biological functions of insect pest species, including but not limited to survival, growth, development, reproduction and pathogenicity. The target genes may regulate the same or different biological pathways or processes. In one embodiment, at least one of the silencing elements comprises or consists of a sequence of nucleotides which is at least partially complementary to a target nucleotide sequence within a target gene wherein the target gene is selected from the group of genes as described earlier.
In a further embodiment of the invention, the different genes targeted by the different silencing elements originate from the same insect pest species. This approach is designed to achieve enhanced attack against a single insect pest species. In particular, the different target genes may be expressed differentially in the different stages of the insect's life cycle, for example, the mature adult, immature larval and egg stages. The interfering RNA of the invention may thus be used to prevent and/or control insect pest infestation at more than one stage of the insect's life cycle.
In an alternative embodiment of the invention, the different genes targeted by the different silencing elements originate from different insect pest species. The interfering RNA of the invention can thus be used to prevent and/or control infestation by more than one insect pest species simultaneously. The silencing elements may be arranged as one contiguous region of the interfering RNA or may be separated by the presence of linker sequences. The linker sequence may comprise a short random nucleotide sequence that is not complementary to any target nucleotide sequences or target genes. In one embodiment, the linker is a conditionally self-cleaving RNA sequence, preferably a pH-sensitive linker or a hydrophobic-sensitive linker. In one embodiment, the linker comprises a sequence of nucleotides equivalent to an intronic sequence. Linker sequences of the current invention may range in length from about 1 base pair to about 10000 base pairs, provided that the linker does not impair the ability of the interfering RNA to down-regulate the expression of target gene(s).
In addition to the silencing element(s) and any linker sequences, the interfering RNA of the invention may comprise at least one additional polynucleotide sequence. In different embodiments of the invention, the additional sequence is chosen from (i) a sequence capable of protecting the interfering RNA against RNA processing, (ii) a sequence affecting the stability of the interfering RNA, (iii) a sequence allowing protein binding, for example to facilitate uptake of the interfering RNA by cells of the insect pest species, (iv) a sequence facilitating large-scale production of the interfering RNA, (v) a sequence which is an aptamer that binds to a receptor or to a molecule on the surface of the insect pest cells to facilitate uptake, or (v) a sequence that catalyses processing of the interfering RNA within the insect pest cells and thereby enhances the efficacy of the interfering RNA. Structures for enhancing the stability of RNA molecules are well known in the art and are described further in WO2006/046148.
The length of the interfering RNA of the invention needs to be sufficient for uptake by the cells of an insect pest species and down-regulation of target genes within the pest as described elsewhere herein. However, the upper limit on length may be dependent on (i) the requirement for the interfering RNA to be taken up by cells of the pest and (ii) the requirement for the interfering RNA to be processed in the cells of the pest to mediate gene silencing via the RNAi pathway. The length may also be dictated by the method of production and the formulation for delivery of the interfering RNA to cells. Preferably, the interfering RNA of the current disclosure will be between 21 and 10000 nucleotides in length, preferably between 50 and 5000 nucleotides or between 100 and 2500 nucleotides, more preferably between 80 and 2000 nucleotides in length.
The interfering RNA may contain DNA bases, non-natural bases or non-natural backbone linkages or modifications of the sugar-phosphate backbone, for example to enhance stability during storage or enhance resistance to degradation by nucleases. Furthermore, the interfering RNA may be produced chemically or enzymatically by one skilled in the art through manual or automated reactions. Alternatively, the interfering RNA may be transcribed from a polynucleotide encoding the same. Thus, provided herein is an isolated polynucleotide encoding any of the interfering RNAs of the current invention.
Also provided herein is an isolated polynucleotide selected from the group consisting of (i) a polynucleotide which comprises at least 30, 35, 40, 45, 50, 55, 60, 70, 80, 90, 100, 110, 125, 150, 175, 200, 225, 250, 300, 350, 400, 450, 500, 550, 600, 700, 800, 900, 1000, 1100, 1200, 1300, 1400, 1500, 2000 or 3000 contiguous nucleotides of a nucleotide sequence as represented by SEQ ID NO 389, or the complement thereof, and wherein said polynucleotide is no longer than 10000, 9000, 8000, 7000, 6000, 5000, 4000, 3000, 2000 or 1500 nucleotides.
In preferred embodiments, the isolated polynucleotide is part of an interfering RNA molecule, typically part of the silencing element, comprising at least one double-stranded region comprising a sense RNA strand annealed by complementary basepairing to an antisense RNA strand wherein the sense strand of the dsRNA molecule comprises a sequence of nucleotides complementary to a sequence of nucleotides located within the RNA transcript of the target gene. The sense strand of the dsRNA is therefore able to anneal to the RNA transcript and target the RNA for degradation within the RNAi-induced silencing complex or RISC.
The polynucleotides of the invention may be inserted via routine molecular cloning techniques into DNA constructs or vectors known in the art. Therefore, according to one embodiment, a DNA construct comprising any of the polynucleotides of the current invention is provided. Preferably, provided herein is a DNA construct comprising a polynucleotide encoding at least one of the interfering RNAs of the current invention. The DNA construct may be a recombinant DNA vector, for example a bacterial, viral or yeast vector. In a preferred embodiment of the invention, the DNA construct is an expression construct and the polynucleotide is operably linked to at least one regulatory sequence capable of driving expression of the polynucleotide sequence. The term 'regulatory sequence' is to be taken in a broad context and is intended to refer to any nucleotide sequence capable of effecting expression of polynucleotides to which it is operably linked including but not limited to promoters, enhancers and other naturally-occurring or synthetic transcriptional activator elements. The regulatory sequence may be located at the 5' or 3' end of the polynucleotide sequence. The term 'operably linked' refers to a functional linkage between the regulatory sequence and the polynucleotide sequence such that the regulatory sequence drives expression of the polynucleotide. Operably linked elements may be contiguous or non-contiguous.
Preferably, the regulatory sequence is a promoter selected from the group comprising but not limited to constitutive promoters, inducible promoters, tissue-specific promoters and growth/developmental stage-specific promoters. In one embodiment, the polynucleotide is placed under the control of a strong constitutive promoter such as any selected from the group comprising the CaMV35S promoter, doubled CaMV35S promoter, ubiquitin promoter, actin promoter, rubisco promoter, GOS2 promoter, Figwort mosaic virus 34S promoter.
Optionally, one or more transcription termination sequences may be incorporated in the expression construct of the invention. The term 'transcription termination sequence' encompasses a control sequence at the end of a transcriptional unit, which signals termination of transcription, 3' processing and poly-adenylation of a primary transcript. Additional regulatory sequences including but not limited to transcriptional or translational enhancers may be incorporated in the expression construct, for instance as with the double enhanced CaMV35S promoter.
The present invention also encompasses a method for generating any of the interfering RNAs of the invention comprising the steps of (i) contacting a polynucleotide encoding said interfering RNA or a DNA construct comprising the same with cell-free components; or (ii) introducing (e.g. by transformation, transfection or injection) a polynucleotide encoding said interfering RNA or a DNA construct comprising the same into a cell.
The invention thus also relates to any double stranded ribonucleotide produced from the expression of a polynucleotide described herein.
Accordingly, also provided herein is a host cell transformed with any of the polynucleotides described herein. Further encompassed by the present invention are host cells comprising any of the interfering RNA's of the current invention, any of the polynucleotides of the current invention or a DNA construct comprising the same. The host cell may be a prokaryotic cell including but not limited to gram-positive and gram-negative bacterial cells, or an eukaryotic cell including but not limited to yeast cells or plant cells. Preferably, said host cell is a bacterial cell or a plant cell. The bacterial cell can be chosen from the group comprising, but not limited to, Gram positive and Gram negative cells comprising *Escherichia* spp. (e.g. *E. coli*), *Bacillus* spp. (e.g. *B. thuringiensis*), *Rhizobium* spp., *Lactobacillus* spp., *Lactococcus* spp., *Pseudomonas* spp. and *Agrobacterium* spp.. The polynucleotide or DNA construct of the invention may exist or be maintained in the host cell as an extra-chromosomal element or may be stably incorporated into the genome of the host cell. Characteristics of particular interest in selecting a host cell for the purposes of the current invention include the ease with which the polynucleotide or DNA construct encoding the interfering RNA can be introduced into the host, the availability of compatible expression systems, the efficiency of expression, and the stability of the interfering RNA in the host.

Preferably, the interfering RNAs of the invention are expressed in a plant host cells. Preferred plants of interest include but are not limited to cotton, potato, rice, tomato, canola, soy, sunflower, sorghum, pearl millet, corn, alfalfa, strawberries, eggplant, pepper and tobacco.
In situations wherein the interfering RNA is expressed within a host cell and/or is used to prevent and/or control pest infestation of a host organism, it is preferred that the interfering RNA does not exhibit significant 'off-target' effects *i.e.* the interfering RNA does not affect expression of genes within the host. Preferably, the silencing element does not exhibit significant complementarity with nucleotide sequences other than the intended target nucleotide sequence of the target gene. In one embodiment of the invention, the silencing element shows less than 30%, more preferably less than 20%, more preferably less than 10% and even more preferably less than 5% sequence identity with any gene of the host cell or organism. If genomic sequence data is available for the host organism, one can cross-check identity with the silencing element using standard bioinformatics tools. In one embodiment, there is no sequence identity between the silencing element and a gene from the host cell or host organism over a region of 17, more preferably over a region of 18 or 19 and most preferably over a region of 20 or 21 contiguous nucleotides.
In the practical application of the invention, the interfering RNAs of the invention may be used for the prevention and/or control of any insect pest belonging to the Orders *Coleoptera, Lepidoptera, Diptera, Dichyoptera, Orthoptera, Hemiptera and Siphonaptera.*

Furthermore, in accordance with another aspect of the invention, there is provided herein a composition for preventing and/or controlling insect pest infestation comprising at least one interfering ribonucleic acid (RNA) and optionally at least one suitable carrier, excipient or diluent, wherein the interfering RNA functions upon uptake by the pest to down-regulate the expression of a target gene within said pest. The interfering RNA may be any of those as disclosed elsewhere herein. Preferably, the interfering RNA comprises or consists of at least one silencing element and said silencing element is a region of double-stranded RNA comprising annealed complementary strands, one strand of which (the sense strand) comprises a sequence of nucleotides which is at least partially complementary to a target nucleotide sequence within a target gene. The 'target gene' may be any of the pest target genes as disclosed elsewhere herein including but not limited to genes involved in regulating pest survival, growth, development, reproduction and pathogenicity. Alternatively, the composition comprises at least one host cell comprising at least one interfering RNA molecule or DNA construct encoding the same and optionally at least one suitable carrier, excipient or diluent, wherein the interfering RNA functions upon uptake of the host cell by the insect pest to down-regulate the expression of a target gene within said pest.
In the practical application of the invention, the composition may be used for the prevention and/or control of any insect pest belonging to the Orders *Coleoptera, Lepidoptera, Diptera, Dichyoptera, Orthoptera, Hemiptera and Siphonaptera.* The composition may therefore be in any suitable form for application to insect pests or for application to substrates and/or organisms, in particular plants, susceptible to infestation by said insect pest. In one embodiment, the composition is for use in preventing and/or controlling pest infestation of plants or propagation or reproductive material of plants and is thus directed towards insect pest species that infest plants. The composition of the present invention is particularly effective when the insect pest belongs to the category of 'chewing' insects that cause considerable damage to plants by eating plant tissues such as roots, leaves, flowers, buds, twigs and the like. Examples from this large insect category include beetles and their larvae.
The composition of the invention may be used to control insect pests at all stages of their life cycle, for example, the mature adult stage, the larval and egg stages.
In the context of the composition of the invention, the interfering RNA may be produced from a DNA construct, in particular an expression construct as described elsewhere herein, comprising a polynucleotide encoding the same. In preferred embodiments, the interfering RNA may be produced inside a host cell or organism engineered to express said interfering RNA from a polynucleotide encoding the same.

Suitable host organisms for use in the compositions of the current invention include but are not limited to microorganisms that are known to colonize the environment on and/or around plants or crops of interest *i.e.* plants or crops susceptible to infestation by insect pest species. Such microorganisms include but are not limited to those that occupy the phylloplane (the surface of plant leaves) and/or the rhizosphere (the soil surrounding plant roots). These microorganisms are selected so as to be capable of successfully competing with any wild-type organisms present in the plant environment. Suitable microorganisms for use as hosts include various species of bacteria, algae and fungi. It is clear that the chosen microorganisms must not be toxic to plants. Such compositions applied to plants susceptible of infestation by insect pest species will be ingested by the insect pests feeding on the treated plants.
Host organisms that do not naturally colonize plants and/or their environment are also within the scope of the current invention. Such organisms may serve only as a means to generate the interfering RNA of the composition. For example, in one embodiment, the interfering RNA is fermented/produced in a bacterial host and the bacteria are subsequently inactivated/killed. The resulting bacteria may be processed and used as an insecticidal spray in the same manner that *Bacillus thuringiensis* strains have been used as an insecticide for a spray application. In certain embodiments, a bacterial extract or lysate may be suitably purified to leave a substantially pure interfering RNA containing extract, which is subsequently formulated into one of the compositions of the invention. Standard extraction/purification techniques would be known by a person skilled in the art.
Compositions of the invention may be in any suitable physical form for application to insects. For example, the composition may be in solid form (powder, pellet or a bait), liquid form (including a form administered as a spray insecticide) or gel form. In a specific embodiment, the composition may be a coating, paste or powder that can be applied to a substrate in order to protect said substrate from infestation by insects. In this embodiment, the composition can be used to protect any substrate or material that is susceptible to infestation by or damage caused by an insect.

The nature of the excipients and the physical form of the composition may vary depending on the nature of the substrate that it is desired to treat. For example, the composition may be a liquid that is brushed or sprayed onto or imprinted into the material or substrate to be treated, or a coating or powder that is applied to the material or substrate to be treated.
In one embodiment, the composition is in the form of a bait. The bait is designed to lure the insect to come into contact with the composition. Upon coming into contact therewith, the composition is then internalised by the insect, by ingestion for example and mediates RNAi to thus kill the insect. Said bait may comprise a food substance, such as a protein based food, for example fish meal. Boric acid may also be used as a bait. The bait may depend on the species being targeted. An attractant may also be used. The attractant may be a pheromone, such as a male or female pheremone for example. As an example, the pheromones referred to in the book "Insect Pheremones and their use in Pest Management" (Howse et al, Chapman and Hall, 1998) may be used in the invention. The attractant acts to lure the insect to the bait, and may be targeted for a particular insect or may attract a whole range of insects. The bait may be in any suitable form, such as a solid, paste, pellet or powdered form.
The bait may also be carried away by the insect back to the colony. The bait may then act as a food source for other members of the colony, thus providing an effective control of a large number of insects and potentially an entire insect pest colony. This is an advantage associated with use of the double stranded RNA of the invention, because the delayed action of the RNAi mediated effects on the pests allows the bait to be carried back to the colony, thus delivering maximal impact in terms of exposure to the insects.
Additionally, compositions which come into contact with the insects may remain on the cuticle of the insect. When cleaning, either an individual insect cleaning itself or insects cleaning one another, the compositions may be ingested and can thus mediate their effects in the insect. This requires that the composition is sufficiently stable such that the interfering RNA remains intact and capable of mediating RNAi even when exposed to external environmental conditions for a length of time, which may be a period of days for example.
The baits may be provided in a suitable "housing" or "trap". Such housings and traps are commercially available and existing traps may be adapted to include the compositions of the invention. Any housing or trap which may attract an insect to enter it is included within the scope of the invention. The housing or trap may be box-shaped for example, and may be provided in preformed condition or may be formed of foldable cardboard for example. Suitable materials for a housing or trap include plastics and cardboard, particularly corrugated cardboard. Suitable dimensions for such a housing or trap are, for example, 7-15 cm wide, 15-20 cm long and 1-5 cm high. The inside surfaces of the traps may be lined with a sticky substance in order to restrict movement of the insect once inside the trap. The housing or trap may contain a suitable trough inside which can hold the bait in place. A trap is distinguished from a housing because the insect can not readily leave a trap following entry, whereas a housing acts as a "feeding station" which provides the insect with a preferred environment in which they can feed and feel safe from predators.

Accordingly, in a further aspect the invention provides a housing or trap for insects which contains a composition of the invention, which may incorporate any of the features of the composition described herein.
In a further alternative embodiment, the composition may be provided in the form of a spray. Thus, a human user can spray the pest directly with the composition. The composition is then internalized by the insect, from where it can mediate RNA interference, thus controlling the insect. The spray is preferably a pressurized/aerosolized spray or a pump spray. The particles may be of suitable size such that they adhere to the insect, for example to the exoskeleton, and may be absorbed therefrom. Particle size may be measured by known means, such as by use of a Mastersizer, which is a commercially available device.

In a still further embodiment, the carrier is an electrostatically charged powder or particle which adheres to the insect. Suitable powders and particles which are capable of adhering to an insect and thus delivering the RNA constructs of the invention are described in detail in WO 94/00980 and WO 97/33472.
Alternatively, the carrier may comprise magnetic particles which adhere to the insect cuticle. Suitable magnetic particles which are capable of adhering to an insect and thus delivering the RNA constructs of the invention are described in detail in WO 00/01236.
In a still further embodiment, the carrier of the composition comprises metallic particles which are initially unmagnetised but which are capable of becoming magnetically polarised when subjected to the electrical field provided by the insect body. This mode of action is described in detail in WO 2004/049807.
Preferably, the composition incorporates a carrier which increases the uptake of the interfering RNA into the insect pest. Such a carrier may be a lipid-based carrier, preferably comprising one or more of, oil-in water emulsions, micelles, cholesterol, lipopolyamines and liposomes. Other agents which promote uptake of the constructs of the invention are well known to those of skill in the art and include polycations, dextrans and (tris) cationic lipids, such as CS096, CS102 etc. Commercially available liposomes include LIPOFECTIN® and CELLFECTIN® etc. A number of suitable carriers are listed under the heading "Transfection promoting agent" in WO 03/004644 and each of the examples provided.
In a further preferred embodiment, the carrier is a nucleic acid condensing agent. Preferably, the nucleic acid condensing agent comprises spermidine or protamine sulphate or a derivative thereof. Wherein the composition of the invention is for use in preventing and/or controlling pest infestation of a plant, the composition can contain an agriculturally suitable carrier. Such a carrier may be any material that the plant to be treated can tolerate, which does not cause undue damage to the environment or other organisms therein and, which allows the interfering RNA to remain effective against the insect pest species. In particular, the compositions of the invention may be formulated for delivery to plants in accordance with routine agricultural practices used in the bioinsecticide industry. The composition may contain further components capable of performing other functions including but not limited to (i) enhancement or promotion of uptake of the interfering RNA by cells of the pest and (ii) stabilization of the active components of the composition. Specific examples of such further components contained in the composition comprising the interfering RNA, are yeast tRNA or yeast total RNA.
The compositions may be formulated for direct application or as a concentration of a primary composition that requires dilution prior to use. Alternatively, the composition may be supplied as kit comprising the interfering RNA or the host cell comprising or expressing the same in one container and the suitable diluent or carrier for the RNA or host cell in a separate container. In the practical application of the invention, the composition may be applied to a plant or any part of a plant at any stage of the plant's development. In one embodiment, the composition is applied to the aerial parts of a plant, for example during cultivation of plant crops in a field. In a further embodiment, the composition is applied to the seeds of a plant either while they are in storage or once they are planted in the soil. It is generally important to obtain good control of pests in the early stages of plant growth as this is the time when the plant can be most severely damaged by pest species.
The composition may be applied to the environment of an insect pest by various techniques including but not limited to spraying, atomizing, dusting, scattering, pouring, coating of seeds, seed treatment, introduction into the soil, and introduction into irrigation water. In the treatment of plants susceptible to pest infestation, the composition may be delivered to the plant or part of a plant before the appearance of the pest (for the purposes of prevention), or once signs of pest infestation begin to appear (for the purposes of pest control).
In a further embodiment of the invention, the compositions of the invention may be formulated so as to contain at least one further active agent. Thus, the composition may be provided as a "kit-of-parts" comprising the interfering RNA containing composition in one container and one or more suitable active ingredients, for example a chemical or biological pesticide, in a separate container. Alternatively, the compositions may be provided as a mixture which are stable and to be used in conjunction with one another.
Suitable active ingredients which may act in a complementary manner to the interfering RNA molecules of the present invention include, but are not limited to the following: Chlorpyrifos, Allethrin, Resmethrin, Tetrabromoethyl, Dimethol-cyclopropane carboxylic acid (which are generally included in liquid compostions); and Hydramethylnon, Avermectin, Chlorpyrifos, Sulfuramid, Hydroprene, Fipronil (GABA receptor), Isopropylphenyl methyl carbamate, Indoxacarb (PARA), Noviflumuron (Chitinsynthesis inhibitor), Imiprothrin (PARA), Abamectin (Glutamate-gated Chloride channel), Imidacloprid (Acethylcholin receptor) (which are generally included in bait compositions).
In a preferred embodiment, the active ingredient is known to be a preferred insecticide in terms of health and environmental considerations, such as for instance Hydramethylnon and Avermectin. In a further embodiment of the invention, the composition is formulated so as to contain at least one further agronomical agent, for example a herbicide or an additional pesticide. As used herein, a 'second pesticide' or 'additional pesticide' refers to a pesticide other than the first or original interfering RNA molecule of the composition. Alternatively, the composition of the invention may be delivered in combination with at least one other agronomical agent, for a example a herbicide or a second pesticide. In one embodiment, the composition is provided in combination with a herbicide selected from any known in the art, for instance glyphosate, imidazolinone, sulphonylurea and bromoxynil. In a further embodiment, the composition is provided in combination with at least one additional pesticide. The additional pesticide may be selected from any pesticides known in the art and/or may comprise an interfering ribonucleic acid that functions upon uptake by a pest to down-regulate expression of a target gene in said pest species. In one embodiment, the target pest is an insect pest species and the interfering RNA is selected from any of the interfering RNAs as described herein. In a further embodiment, the additional pesticide comprises an interfering RNA that functions to down-regulate expression of a known gene in any target pest species, not limited to insect pests. The original interfering RNA molecule of the composition and the second or additional pesticide(s) may target the same insect pest species or may be intended to target different insect pest species. For example, the original interfering RNA and the second pesticide may target different species of insect pest or may target different families or classes of pest organisms, for example, fungi or nematodes or insects. It will be apparent to one skilled in the art how to test combinations of interfering RNA molecules and other agronomical agents for synergistic effects. In a preferred embodiment, the composition contains a first interfering RNA molecule described elsewhere herein and one or more additional pesticides, each toxic to the same insect pest, wherein the one or more additional pesticides are selected from a patatin, a *Bacillus thuringiensis* insecticidal protein, a *Xenorhabdus* insecticidal protein, a *Photorhabdus* insecticidal protein, a *Bacillus laterosporous* insecticidal protein, a *Bacillus spaericus* insecticidal protein, and a lignin, and wherein said *Bacillus thuringiensis* insecticidal protein is selected from the group consisting of a Cry1Ab, a Cry1C, a Cry2Aa, a Cry3, a TIC851, a CryET70, a Cry22, a VIP, a TIC901, a TIC1201, a TIC407, a TIC417, a binary insecticidal protein selected from CryET33 and CryET34, CryET80 and CryET76, TIC100 and TIC101, and PS149B1, and insecticidal chimeras of any of the preceding insecticidal proteins.
The different components of the combinations described herein may be administered, for example to a host organism susceptible to infestation by pest, in any order. The components may be delivered simultaneously or sequentially to the area or organism to be treated.
Also provided herein is a method for preventing and/or controlling pest infestation, comprising contacting an insect pest species with an effective amount of at least one interfering RNA wherein the RNA functions upon uptake by said pest to down-regulate expression of an essential pest target gene. The essential target gene may be any pest gene involved in the regulation of an essential biological process required by the pest to initiate or maintain infestation including but not limited to survival, growth, development, reproduction and pathogenicity. In particular, the target gene may be any of the pest genes as described elsewhere herein.
Furthermore, there is provided herein a method for preventing and/or controlling insect pest infestation in a field of crop plants, said method comprising expressing in said plants an effective amount of an interfering RNA as described herein.
Wherein the method is for the control of pest infestation, the phrase 'effective amount' extends to the quantity or concentration of interfering RNA required to produce a phenotypic effect on the pest such that the numbers of pest organisms infesting a host organism are reduced and/or the amount of damage caused by the pest is reduced. In one embodiment, the phenotypic effect is death of the pest and the interfering RNA is used to achieve at least 20%, 30%, 40%, preferably at least 50%, 60%, 70%, more preferably at least 80% or 90% pest mortality as compared to control insect pests. In a further embodiment, the phenotypic effects include but are not limited to stunting of pest growth, cessation of feeding and reduced egg-laying. The total numbers of pest organisms infesting a host organism may thus be reduced by at least 20%, 30%, 40%, preferably at least 50%, 60%, 70%, more preferably at least 80% or 90% as compared with control pests. Alternatively, the damage caused by the insect pest may be reduced by at least 20%, 30%, 40%, preferably at least 50%, 60%, 70%, more preferably at least 80% or 90% as compared with control insect pests. Hence, the method of the invention can be used to achieve at least 20%, 30%, 40%, preferably at least 50%, 60%, 70%, more preferably at least 80% or 90% pest control.
As used herein, the term 'plant' may include any reproductive or propagation material for a plant. Reference to a plant may also include plant cells, plant protoplasts, plant tissue cultures, plant calli, plant clumps and plant cells that are intact in plants or parts of plants such as embryos, pollen, ovules, seeds, leaves, flowers, branches, fruit, kernels, ears, cobs, husks, stalks, roots, root tips and the like.
Also provided herein is the use of the interfering ribonucleic acid (RNA) as described herein or the DNA construct as described herein for preventing and/or controlling insect pest infestation, preferably insect pest infestation of plants.
The invention will be further understood with reference to the following non-limiting examples.

### Examples

### Example 1 Identification of target genes in insect pest species

### 1.1. Lygus hesperus normalized cDNA library and preparation of dsRNAs in multiwell plates for the screening assays

Nucleic acids were isolated from *Lygus hesperus* nymphs of different life stages, including freshly hatched nymphs 2, 4, 6 and 9 days old nymphs and adults. A cDNA library was prepared using the SMARTer™ PCR cDNA Synthesis Kit, following the manufacturer's instructions (Clontech Cat. No 634925). The cDNA library was normalized using the Trimmer kit (Evrogen Cat No NK001) and cloned in the PCR4-TOPO vector (Invitrogen). The normalization of the clones introduced M2 adapters (Trimmer Kit, Evrogen, SEQ ID NO 92: AAGCAGTGGTATCAACGCAG), oppositely oriented at each end of the clones. The recombinant vector constructs were transformed into cells of *Escherichia coli* strain TOP10 (Invitrogen). The transformed cells were subsequently diluted and plated so as to obtain single colonies or clones. The clones were checked to ensure that clone redundancy for the library did not exceed 5%. Single clones were picked in liquid LB (Luria-broth) media, in 96-deep-well plates, and grown overnight at 37°C. The plates also included positive (Lh423) and negative (FP) control clones.

To generate the dsRNA, sense and antisense DNA fragments, containing T7 promoter sequence, were generated by PCR. In brief, per clone, 1µl of bacterial suspension was dispensed in multiwell PCR plates containing REDTaq® (Sigma Cat No D4309) and primers oGCC2738 (SEQ ID NO 93: AAGCAGTGGTATCAACGCAG) and oGCC2739 (SEQ ID NO 94: GCGTAATACGACTCACTATAGGAAGCAGTGGTATCAACGCAG) based on the M2 and the T7-M2 sequences respectively. The PCR reaction was followed by *in vitro* transcription, where per clone, 6µl PCR product were added to 9µl RiboMAX™ Large Scale RNA Production System-T7 (Promega Cat No P1300) and incubated overnight at 37°C. The final dsRNA solution was diluted 2 times in *L*. *hesperus* sucrose diet, containing 15 % sucrose and 5µg/µl yeast tRNA (Invitrogen Cat No 15401-029) and used for screening. The dsRNA corresponding to the positive Lh423 control clone is SEQ ID NO 101 and to the negative FP control clone is SEQ ID NO 104 (see **Table 4**)**.**

### 1.2. Screen for novel and potent Lygus hesperus target genes using a dsRNA expression cDNA library

A new screening assay for potent *Lygus hesperus* targets has been developed. The assay set-up was as follows: each well of a 96-well plate houses a one-day-old *L. hesperus* nymph exposed to a parafilm sachet containing sucrose diet which includes either test dsRNA or control dsRNA in the presence of tRNA. Each plate contained dsRNA from 90 different clones, 3 x Lh423 (positive control) and 3 x FP (fluorescent protein; negative control). Each clone (test dsRNA) was replicated over three plates. After three days exposure, the nymphal survival number was recorded and the diet replaced with fresh rearing (complex) diet in absence of dsRNA. The mortality was assessed at days 4, 6 and 8. An identical set up was used for the first and second round confirmation assays, with 8 and 20 insects respectively, with one nymph per well.

The assay system was validated using dsRNA corresponding to Lh423 target as the positive control and a fluorescent protein dsRNA as the negative control: over 90% were true positives and under 5% were false positives, respectively.

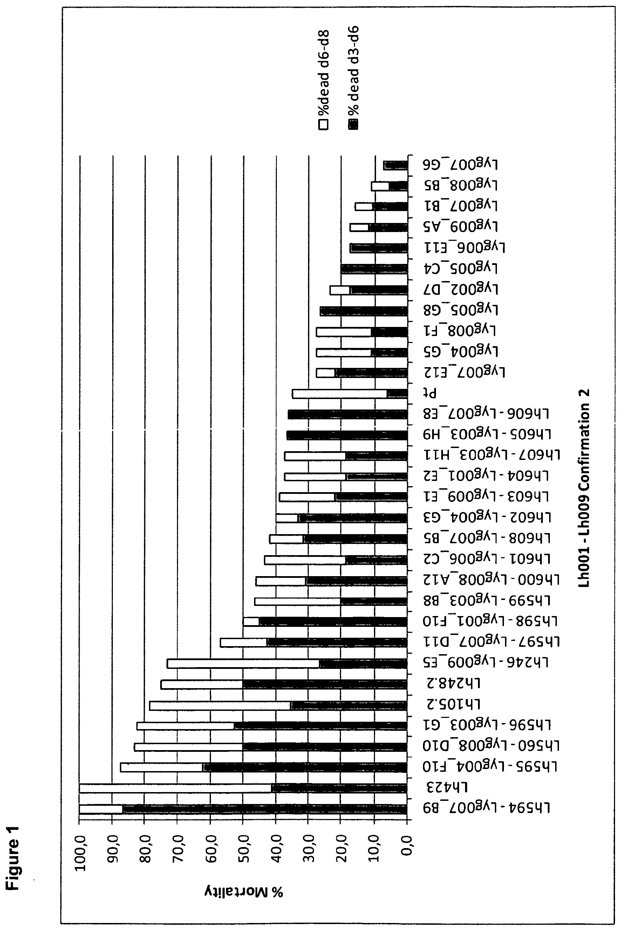

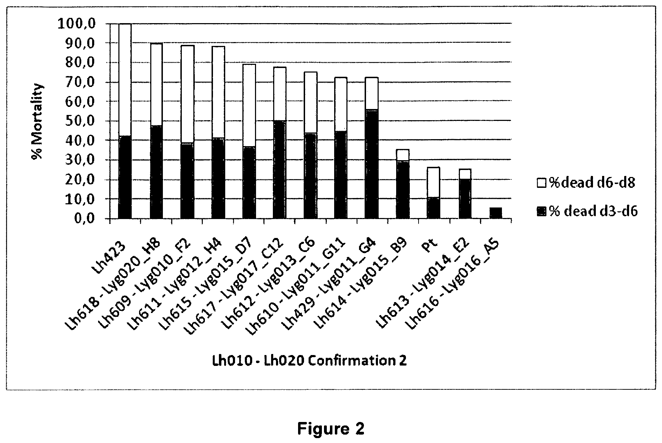

Twenty 96 well-plates, named Lh001 to Lh020 (see bottom line in **Figures 1** **&** **2**), containing 1800 individual clones have been tested. 205 candidates were identified and tested in a first confirmation assay. Setting the threshold at showing ≥ 50% mortality, 41 independent clones were identified and progressed to a second round of confirmation. In the assay, the clones were compared to the positive controls Lh423 (RpL19) and Lh105.2 (Sec23) and the negative control Pt (encoding a coral fluorescent protein). The dsRNA corresponding to the positive (Lh423) control clone is SEQ ID NO 101, to the positive Lh105.2 control clone is SEQ ID NO 102 and to the negative (Pt) control clone is SEQ ID NO 104 (see **Table 4**)**.**

Second round confirmation assays, testing 20 insects / test dsRNA, were initiated for all the test dsRNAs displaying ≥ 50% mortality in the first confirmation (Figures 1 and 2). Candidate targets corresponding to the confirmed test dsRNAs were named with an "Lhxxx number" (see **Table 1**)**.** Using the same cut-off at ≥ 50% mortality, 15 targets were confirmed in the first screen.

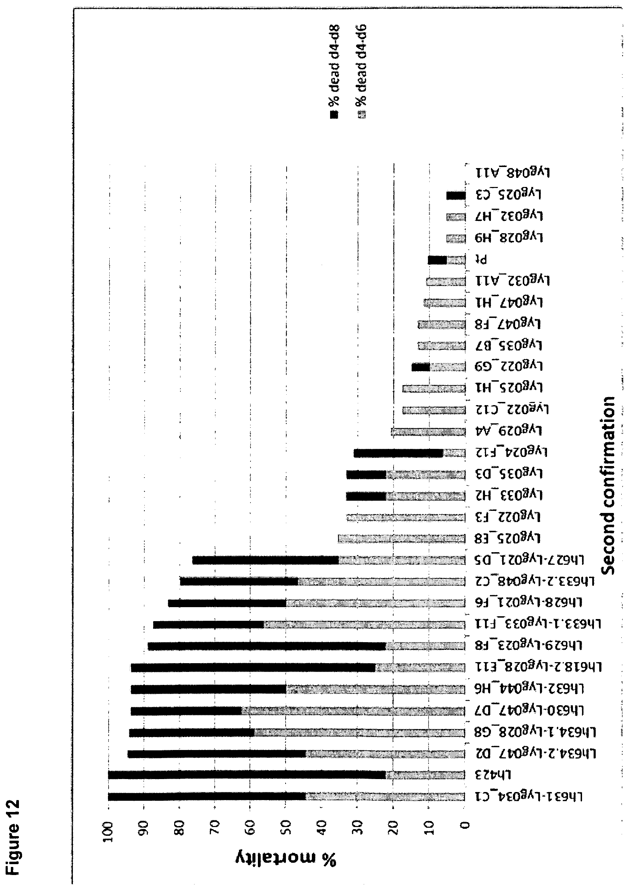

A second screen for identifying more *Lygus hesperus* targets was performed. The results of the second round confirmation assays are represented in **Figure 12****.** Using the same cut-off at ≥ 50% mortality, several targets were confirmed in the second screen (see **Table 1 C**)**.**

### 1.3. Identification of Lygus targets

In parallel to the confirmation insect assays, the inserts corresponding to the positive clones were sequenced and BlastX searches against both *Drosophila* and *Tribolium* protein databases were used to confirm the identity of the targets. **Table 1** provides a summary of the bio-informatics analysis and current annotation of the novel identified *L. hesperus* target sequences.

Fifteen novel *L. hesperus* targets were identified in the first screen and 11 novel *L. Hesperus* targets were identified in the second screen. All targets exhibit high potency against *L. hesperus* nymphs indicating that the cDNAs encoding double-stranded RNAs contained therein are essential for pest survival and thus represent target genes of interest for the purposes of pest control. The DNA sequences and deduced amino acid sequences of these target genes were therefore determined and are provided in **Tables 2 and 3** respectively.

Lh594, the *Lygus hesperus* orthologue of *Drosophila* troponin I, involved in muscle contraction - and therefore absent in plants-, represents a novel class of target belonging to an animal specific physiological pathway not yet explored for GM-RNAi. In the fruit fly, troponin I is described as a haplo-insufficient gene, displaying a mutant phenotype in the heterozygote state. Such genes may be particularly susceptible to reduced mRNA expression levels and as such can be considered as ideal RNAi targets.

In this Lh594 pathway, eight targets were selected (see **Table 1B**). For each target, up to 4 pairs of degenerated PCR primers were designed based on the alignments of the sequences of various insects, including bee, *Tribolium* and aphid. The primers are being used to amplify fragments from *Lygus hesperus* targets. The DNA sequences and deduced amino acid sequences of these target genes were determined and are provided in **Tables 2 and 3** respectively.

**Table 1: Lygus hesperus novel targets ranked in % mortality according to the second confirmation assay results (first screen).**

| **Target ID** | **rank 2nd confirmati on** | **Best Drosophila hit** | **NAME** | **SYMBOL** |
|---|---|---|---|---|
| Lh594 | 1 | CG7178 | wings up A (troponin I) | wupA |
| Lh618 | 2 | CG2168 | ribosomal protein S3A | RpS3A |
| Lh609 | 3 | CG4087 | ribosomal protein LP1 | RpLP1 |
| Lh595 | 4 | - | no *Drosophila* hit found, *Lygus* specific target/sequence | |
| Lh611 | 5 | CG6779 | ribosomal protein S3 | RpS3 |
| Lh560 | 6 | CG 10423 | ribosomal protein S27 | RpS27 |
| Lh596 | 7 | - | no *Drosophila* hit found, *Lygus* specific target/sequence | RpL34b |
| Lh615 | 8 | CG11522 | ribosomal protein L6 | RpL6 |
| Lh617 | 9 | CG7283 | ribosomal protein L10Ab | RpL10Ab |
| Lh612 | 10 | CG 13389 | ribosomal protein S13 | RpS13 |
| Lh246 | 11 | CG3195 | ribosomal protein L12 | RpL12 |
| Lh429 | 12 | CG8900 | ribosomal protein S18 | RpS18 |
| Lh610 | 13 | CG5502 | ribosomal protein L4 | RpL4 |
| Lh597 | 14 | no hit found | | |
| Lh598 | 15 | CG34069 | mitochondrial cytochrome c oxidase subunit II | mt:Coll |
| Lh614 | - | CG7610 | ATP synthase-y chain | ATPsyn-y |

**Table 1B: Lygus hesperus novel targets in Lh594 pathway**

| **Target ID** | **Best Drosophila hit(s)** | **NAME** | **SYMBOL** |
|---|---|---|---|
| Lh619 | CG7107 | troponin T (upheld) | up |
| Lh620 | CG17927 | myosin heavy chain | Mhc |
| Lh621 | CG4843 | tropomyosin2 (Tm2) | Tm2 |
| Lh622 | CG3201 | myosin light chain cytoplasmic | Mlc-c |
| Lh623 | CG3595 | spaghetti squash | sqh |
| Lh624 | CG15792 | zipper | zip |
| Lh625 | *CG2981,CG7930,CG9 073,CG6514,CG12408 | troponin C | |
| Lh626 | *CG9073,CG7930,CG2 981,CG12408,CG6514 | troponin C | |

| | | | |
|---|---|---|---|
| *unclear : multiple hits in family - ranked according e-value | | | |

**Table 1C: Lygus hesperus novel targets ranked in % mortality according to the second confirmation assay results (second screen).**

| **Target ID** | **rank 2nd confirmation** | **Best Drosophila hit** | **NAME** | **SYMBOL** |
|---|---|---|---|---|
| **Lh631** | 1 | CG6846 | Ribosomal protein L26 | RpL26 |
| **Lh634.2** | 2 | CG 12775 | Ribosomal protein L21 | RpL21 |
| **Lh634.1** | 3 | CG 12775 | Ribosomal protein L21 | RpL21 |
| **Lh630** | 4 | CG11271 | Ribosomal protein S12 | RpS12 |
| **Lh632** | 5 | CG2998 | Ribosomal protein S28b | RpS28b |
| **Lh618.2** | 6 | CG2168 | Ribosomal protein S3A | RpS3A |
| **Lh629** | 7 | CG4651 | Ribosomal protein L13 | RpL13 |
| **Lh633.2** | 8 | CG 17521 | Ribosomal protein L10 | RpL10 |
| **Lh628** | 9 | CG 17489 | Ribosomal protein L5 | RpL5 |
| **Lh633** | 10 | CG 17521 | Ribosomal protein L10 | RpL10 |
| **Lh627** | 11 | CG2033 | Ribosomal protein S15Aa | RpS15A |

### 1.4. Full length cDNA cloning by RACE (rapid amplification of cDNA ends)

In order to clone full length cDNA, starting from a known clone of internal fragment from the most potent targets, the 5'/3' RACE kit was used (Roche, Cat. No. 1 734 792; based on Sambrook, J. & Russell, D.M). The standard protocol, described in the Instruction Manual, was followed. Briefly, for a 5' RACE, a target sequence specific antisense primer was designed on the known sequence and used for a first strand cDNA synthesis, using *Lygus* RNA as template. A tail was added to the first strand cDNA and used as an anchor for the second strand synthesis and amplification of an unknown end portion of the transcript. For a 3' RACE, an oligo dT anchor primer was used for the first strand cDNA synthesis. For the 5' and 3' RACEs, nested primers, specific to the target sequence were used in a second PCR reaction. The PCR fragments were analysed on agarose gel, purified, cloned and sequenced for confirmation.

Full length cDNA sequences corresponding to the targets were assembled in VectorNTi, a fully integrated sequence analysis software package for DNA sequence analysis (Invitrogen).

### Example 2 In vitro production of double-stranded RNAs for gene silencing

### 2.2. Production of dsRNAs corresponding to the partial sequences of the Lygus hesperus target genes

Double-stranded RNA was synthesized in milligram quantities. First, two separate 5' T7 RNA polymerase promoter templates (a sense template and an antisense template) were generated by PCR. PCRs were designed and carried out so as to produce sense and antisense template polynucleotides, each having the T7 promoter in a different orientation relative to the target sequence to be transcribed.

For each of the target genes, the sense template was generated using a target-specific T7 forward primer and a target-specific reverse primer. The antisense templates were generated using target-specific forward primers and target-specific T7 reverse primers. The sequences of the respective primers for amplifying the sense and antisense templates via PCR for each of the target genes are provided in **Table 4.** The PCR products were analysed by agarose gel electrophoresis and purified. The resultant T7 sense and antisense templates were mixed and transcribed by the addition of T7 RNA polymerase. The single-stranded RNAs produced by transcription from the templates were allowed to anneal, were treated with DNase and RNase, and were purified by precipitation. The sense strand of the resulting dsRNA produced from each of the target genes is provided in **Table 4.**

### 2.2. Survival analysis assays for novel Lygus hesperus targets

To enable ranking according to potency, in vitro dsRNAs corresponding to the novel targets were synthesized and applied to *L. hesperus* in 10 days survival analysis bioassays. Briefly, one day old *L*. *hesperus* nymphs were placed in 96 well-plates with sucrose seals containing 0.5µg/µl target dsRNA, supplemented with 5µg/µl yeast tRNA. The plates were incubated for 3 days under standard *Lygus* rearing conditions. At day 3, 6 and 8, the diet seals were refreshed with seals containing *Lygus* diet only. Lh423 (RpL19) was used as positive control and GFP dsRNA and sucrose diet were used as negative controls.

The results from the survival analyses confirmed the data from the first and second confirmation assays. Lh594 was established as a highly potent target, with activity and speed-to-kill stronger than the strong control Lh423.

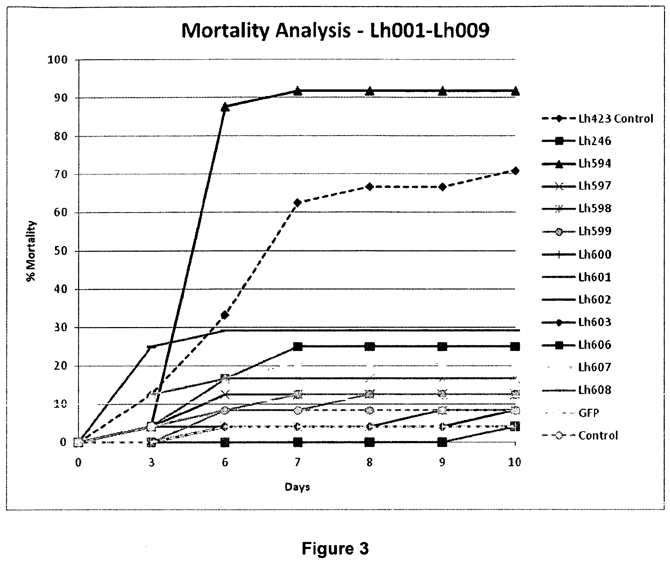

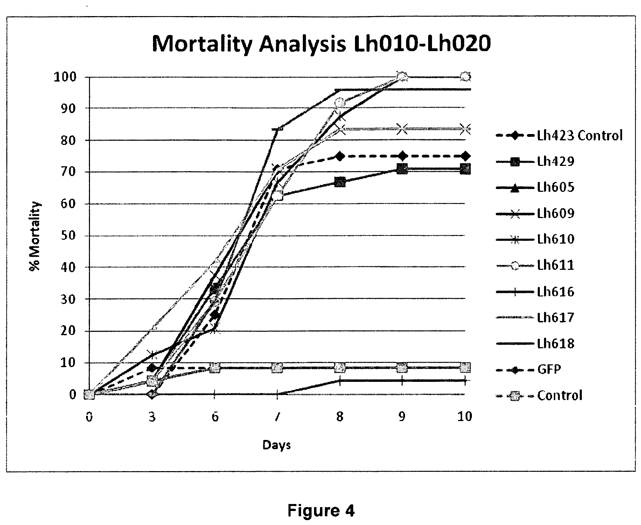

So far, the *Lygus* screen for novel targets identified new targets with activities higher or in the range of the positive control Lh423, these include Lh429, Lh594, Lh609, Lh610, Lh611, Lh617 and Lh618. The mortality induced by these targets is show in the **Figures 3** **and** **4****.**

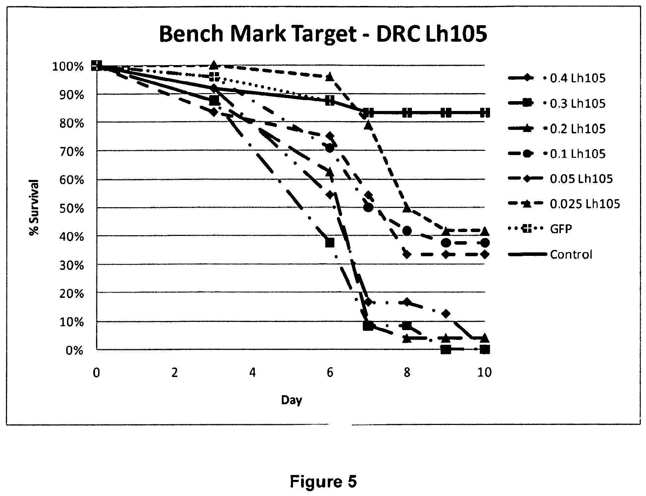

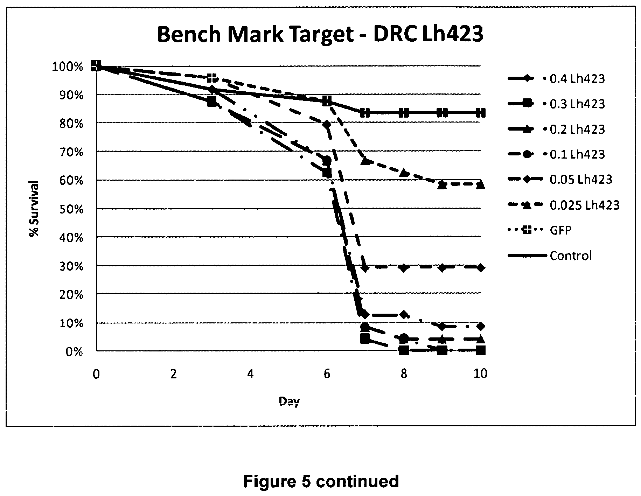

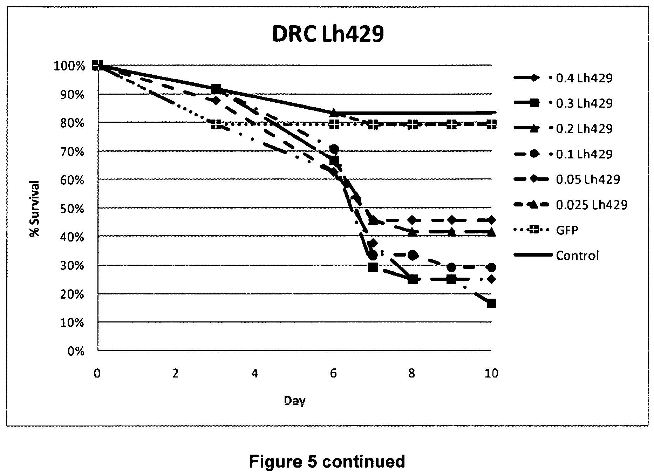

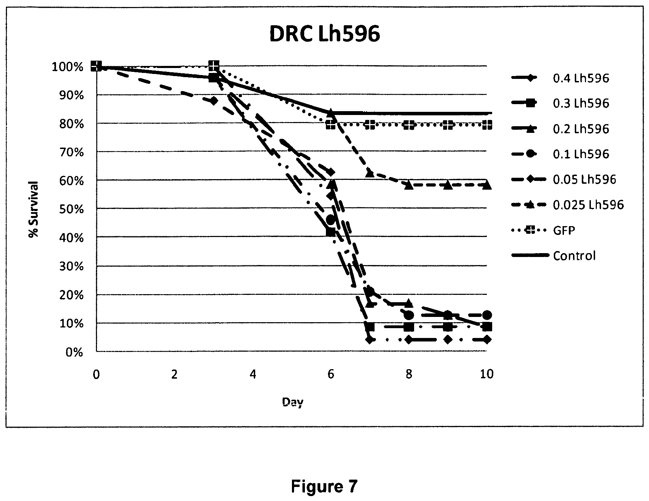

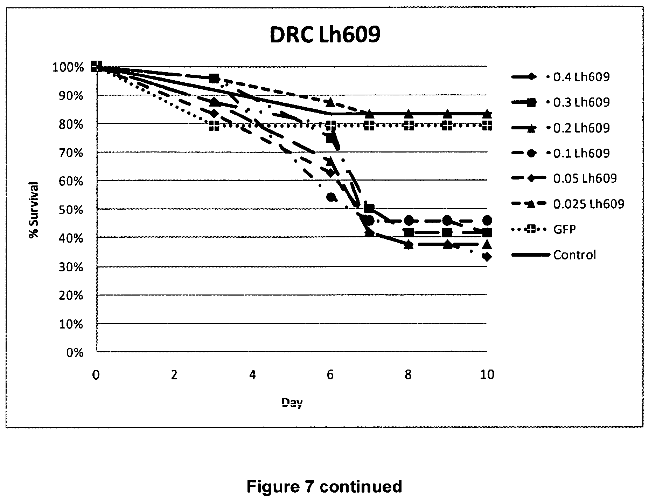

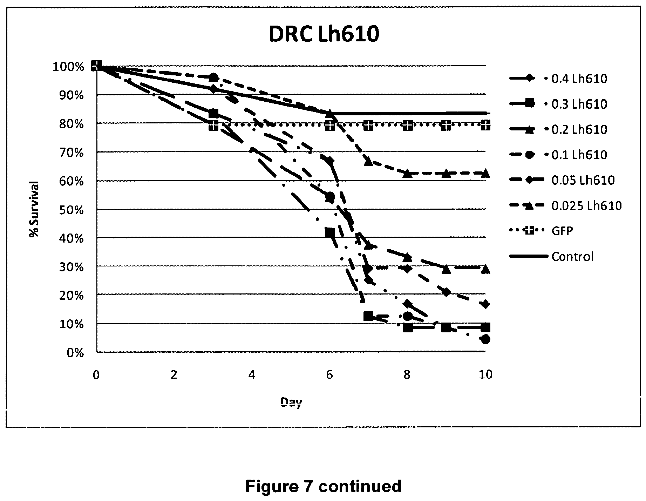

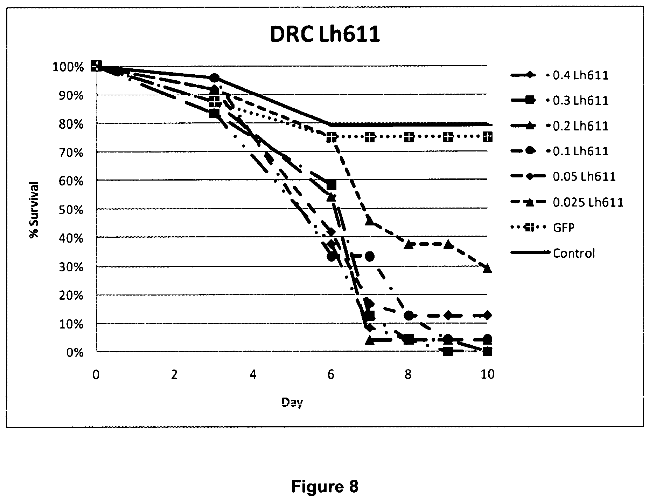

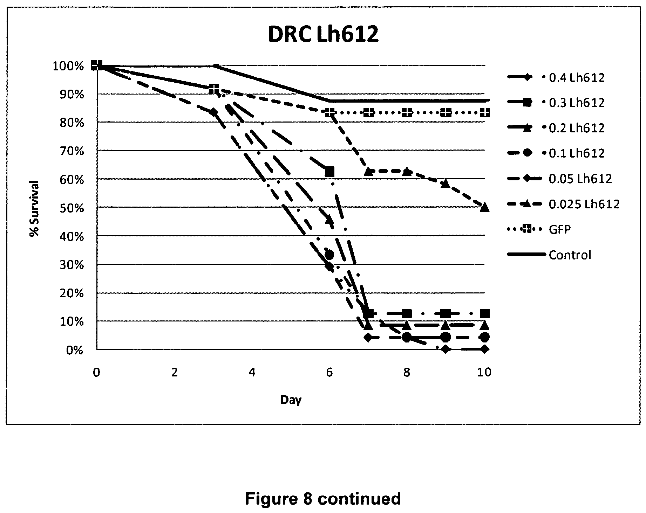

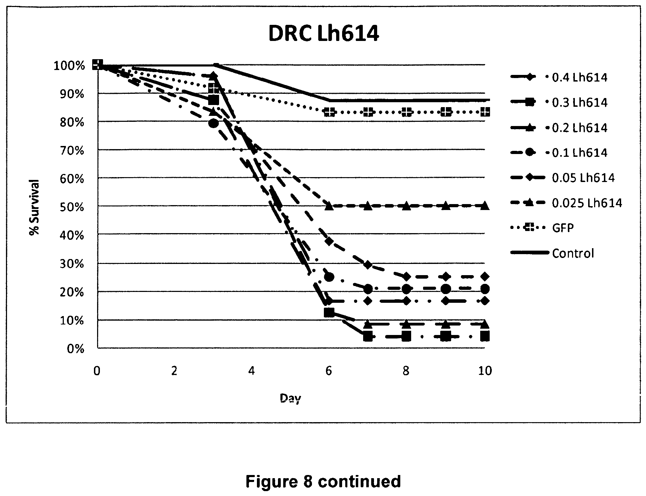

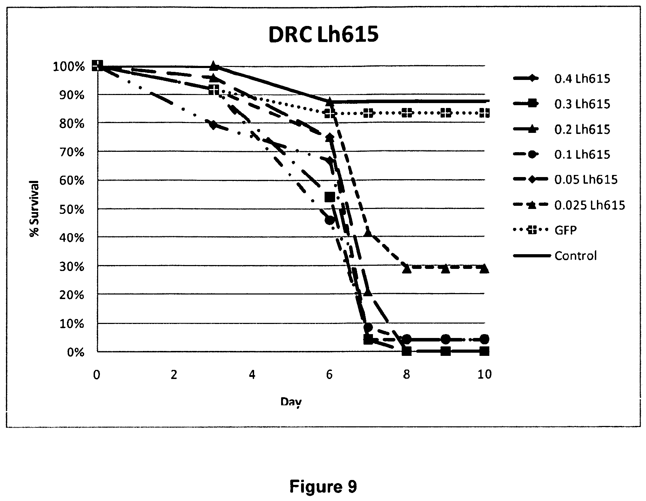

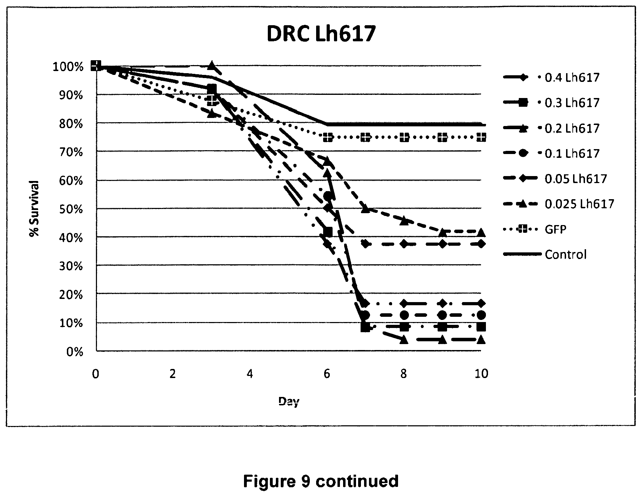

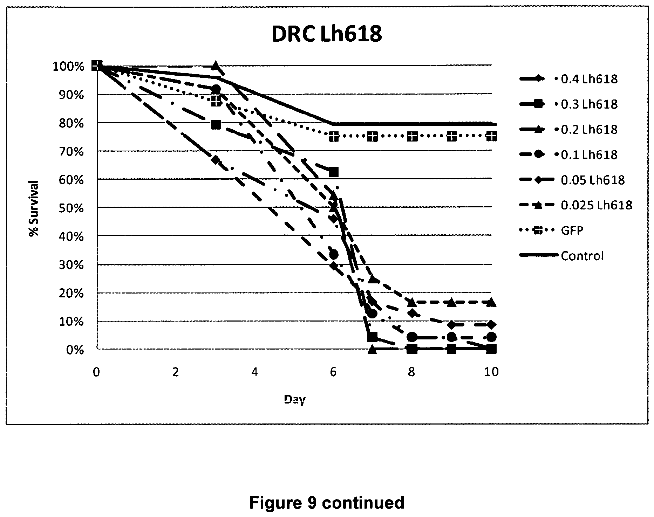

To allow a more precise ranking of the targets according to their activity, dose response concentration analyses were made. The novel targets were tested in *in vitro* assays, with concentrations ranging from 0.4 to 0.025 µg/µl. Per condition, 24 one day old nymphs were tested in the 96 well-plate set-up, in sucrose diet supplemented with dsRNA and tRNA carrier. The results are presented as % survival over a 10 day experiment (**Figures 5 to 9**) and are summarized in **Table 5.**

Based on the concentration curve analyses, the targets were ranked by comparison to the bench mark controls Lh423 and Lh105 (**Table 5**)**.**

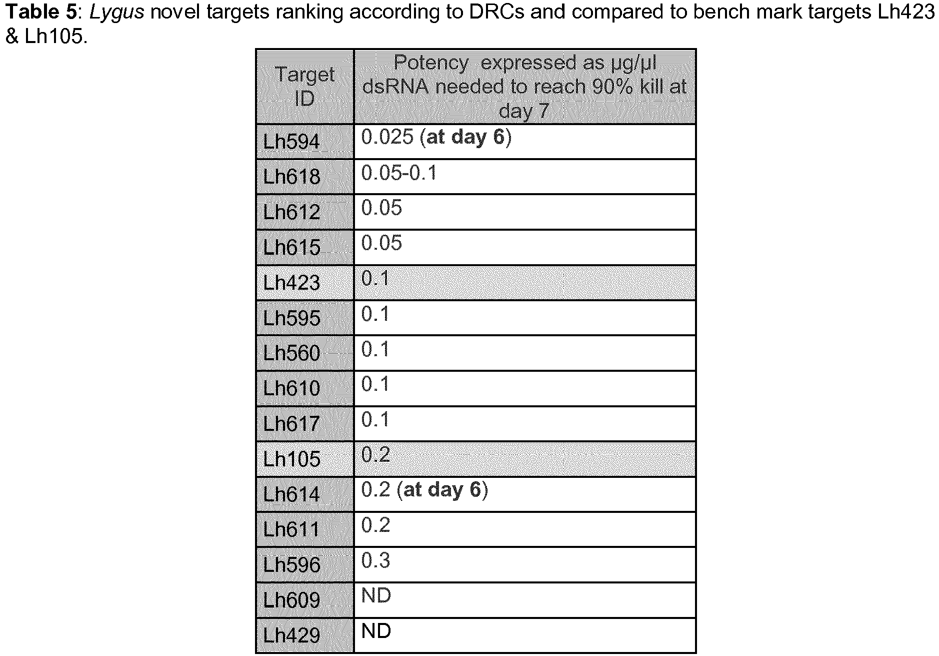

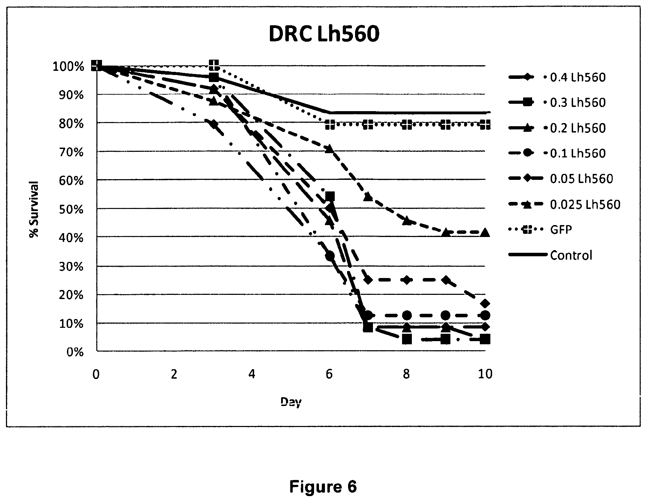

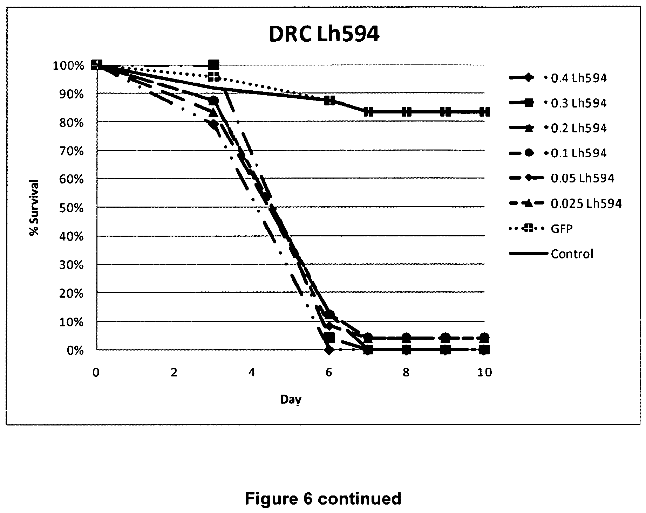

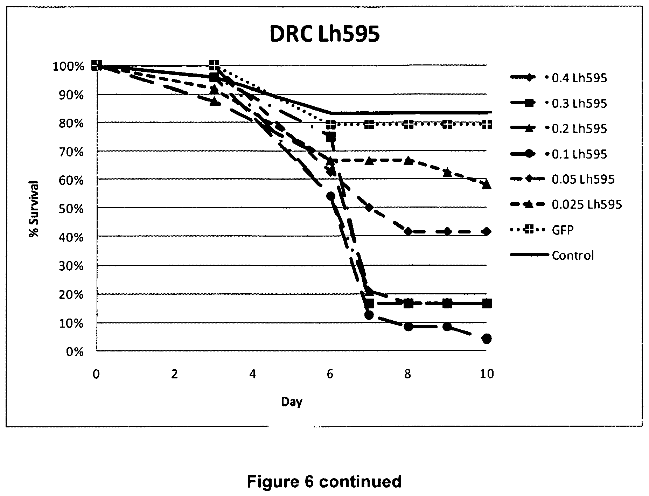

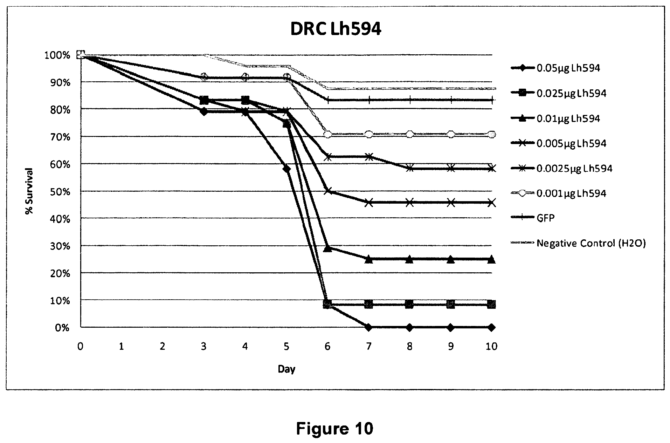

The potency of Lh594 was further confirmed. This target effect is clearly observed at least one day before the other targets and the bench mark positive control Lh105 and Lh423. Because Lh594 was highly potent, the LD50 was not reached in the standard DRC experiment, with concentration ranging from 0.4 to 0.025µg/µl dsRNA (**Figure 6**), the Lh594 experiment was therefore repeated, including lower concentrations ranging from 0.05 to 0.001 µg/µl dsRNA (**Figure 10**). In conclusion, Lh594 activity was observed at concentration as low as 0.0025 µg/µl and about 90% kill (corresponding to about 10 % survival) was obtained at day 6 with 0.025 µg dsRNA.

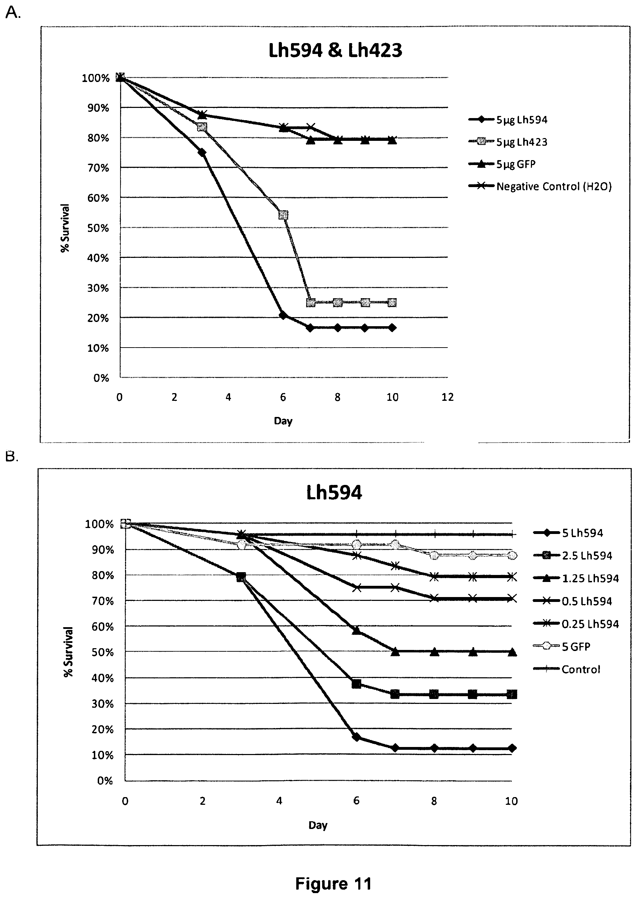

To further explore the potency of Lh594 and the role of tRNA carrier in the RNAi response in *Lygus hesperus,* additional *in vitro* feeding assays were set up in the absence of carrier tRNA. Lh594, Lh423 **(**bench mark control) and GFP (negative control) dsRNAs were produced *in vitro,* using the standard method. The dsRNAs were purified and tested at 5 µg/µl in the absence of tRNA (**Figure 11** **A**)**.**

In absence of tRNA, targets Lh594 and Lh423, induced high lethality in *Lygus* nymphs. The results from this experiment have been since reproduced. Target dsRNA was able to induce RNAi-by-feeding effects in *Lygus* nymphs in the absence of tRNA.

To investigate the activity of dsRNA at lower concentrations in the absence of carrier tRNA, additional experiments were set up, using decreasing amounts of dsRNA (**Figure 11** **B**)**.**

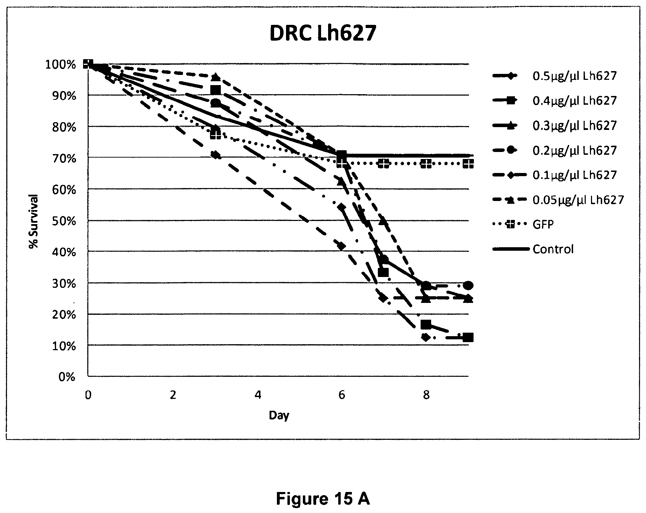

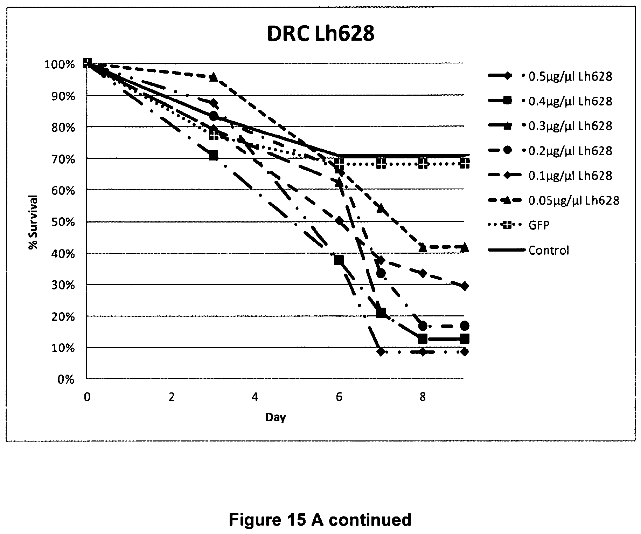

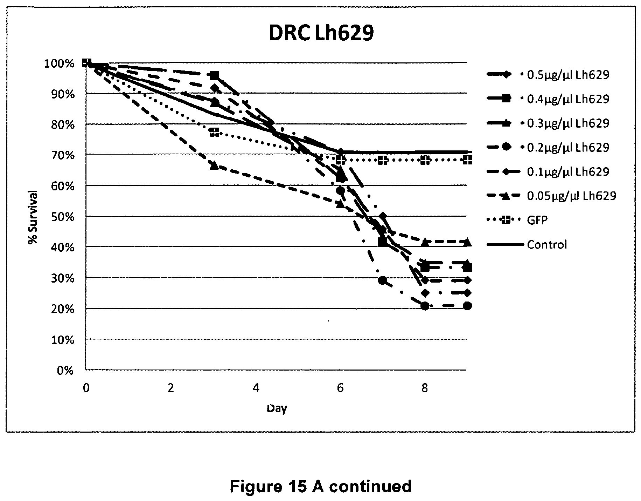

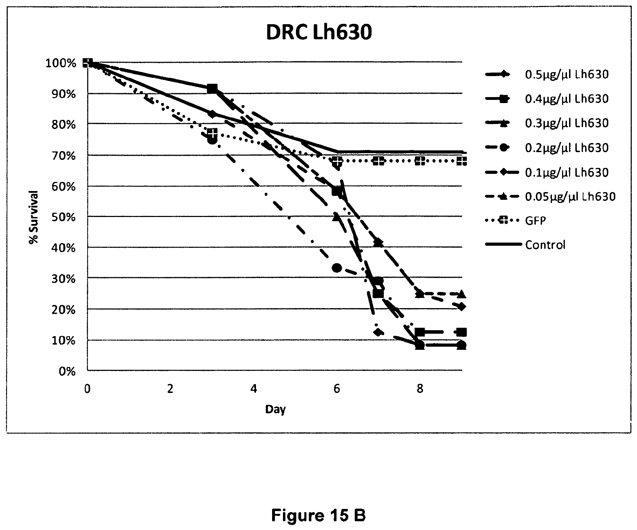

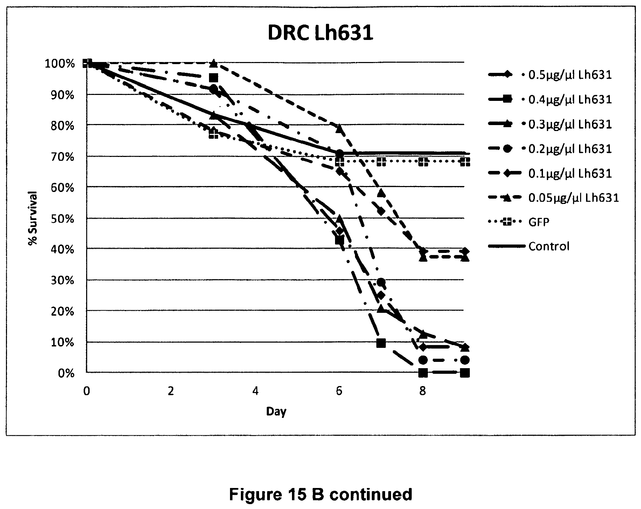

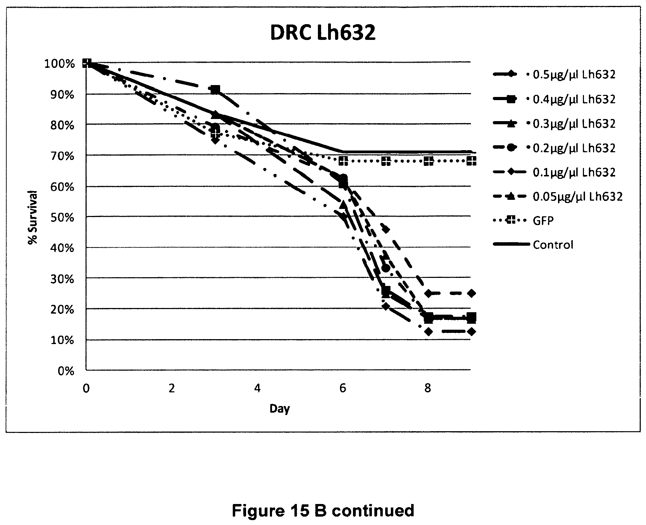

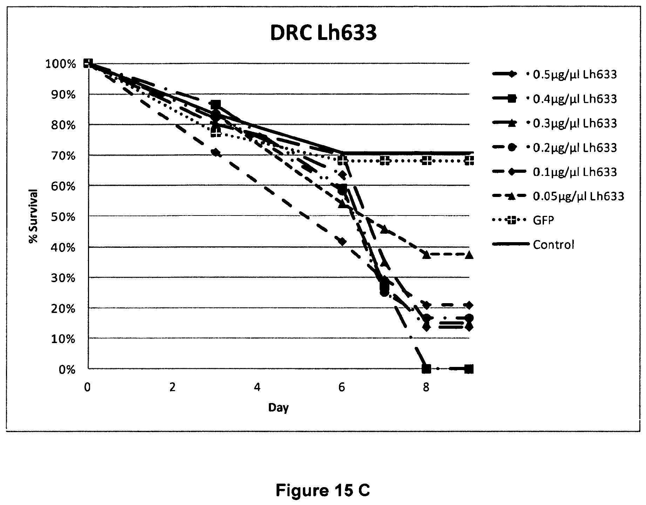

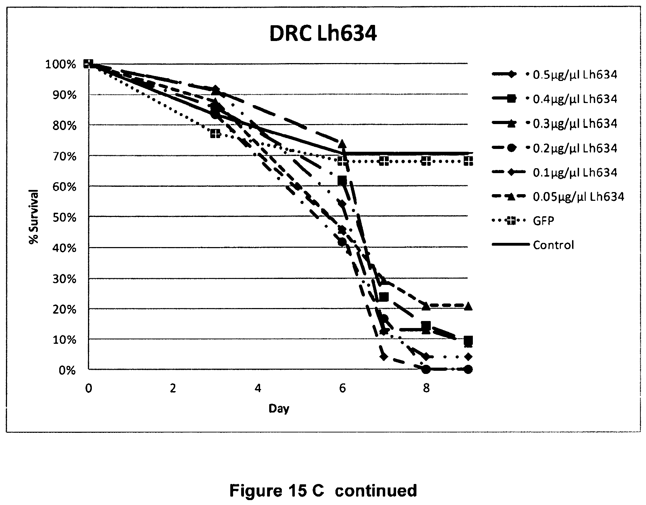

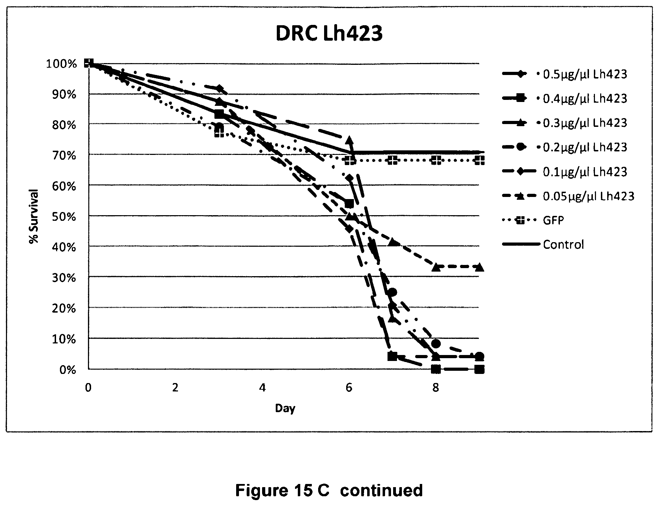

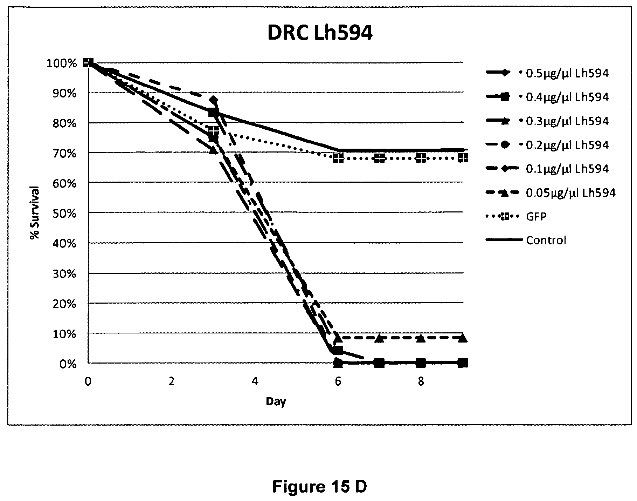

A similar approach was followed for the *Lygus* targets that were identified in the second screen. To allow a ranking of the targets according to their activity, dose response concentration analyses were made. The novel targets were tested in *in vitro* assays, with concentrations ranging from 0.5 to 0.05 µg/µl. Per condition, 24 one day old nymphs were tested in the 96 well-plate set-up, in sucrose diet supplemented with dsRNA and tRNA carrier. The results are presented as % survival over a 9 day experiment **(****Figures 15 A-D**)**.** Lh594 and Lh423 have been included in the assay as a reference targets. The results are summarized in **Table 6.** Based on the concentration curve analyses, the targets were ranked by comparison to the bench mark control Lh423.

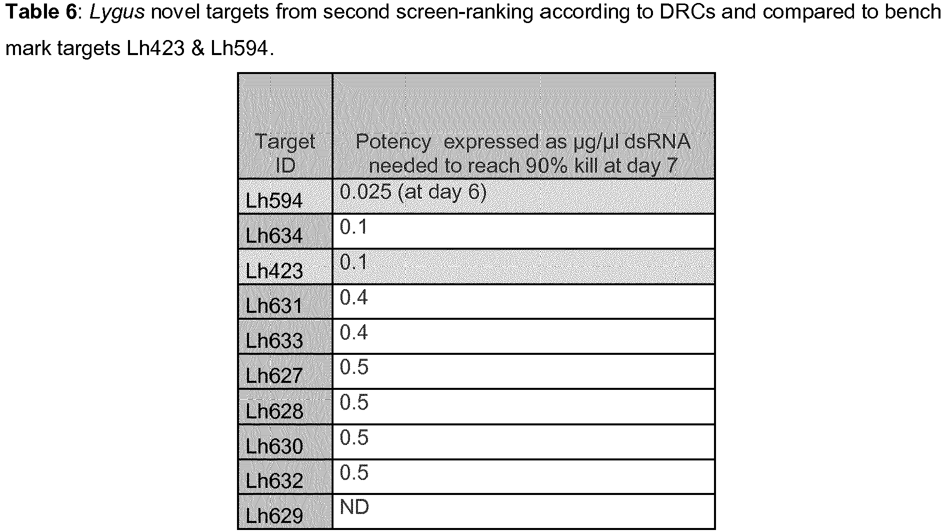

### Example 3 Troponin pathway screen

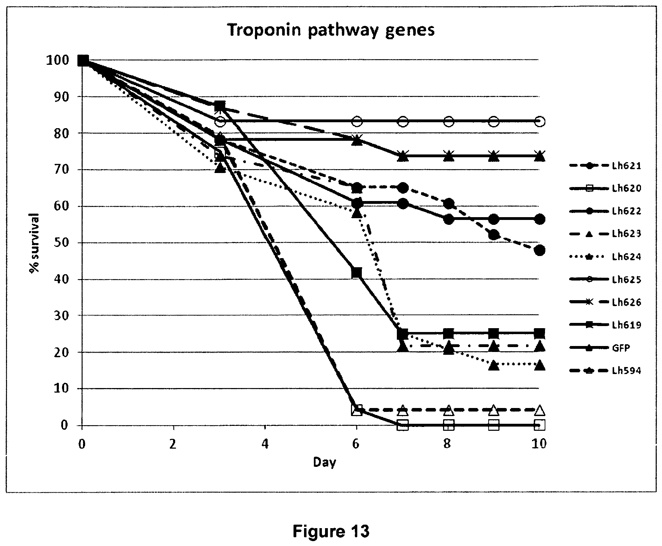

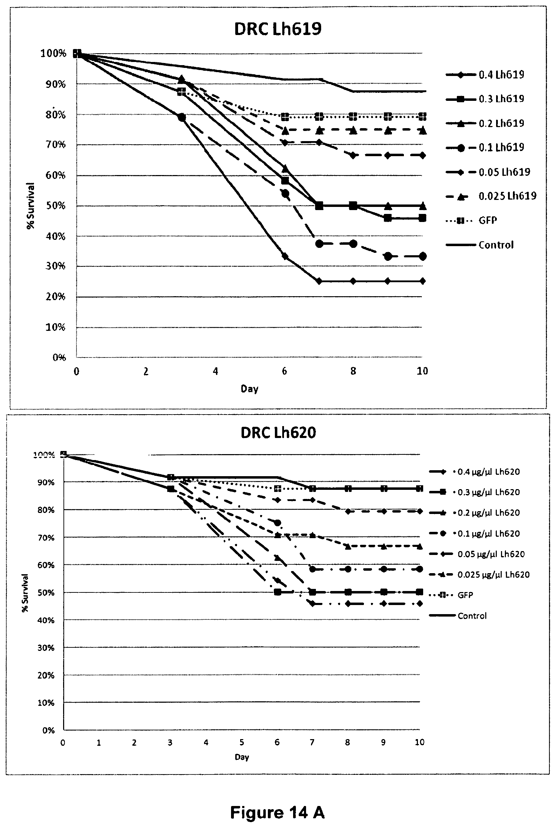

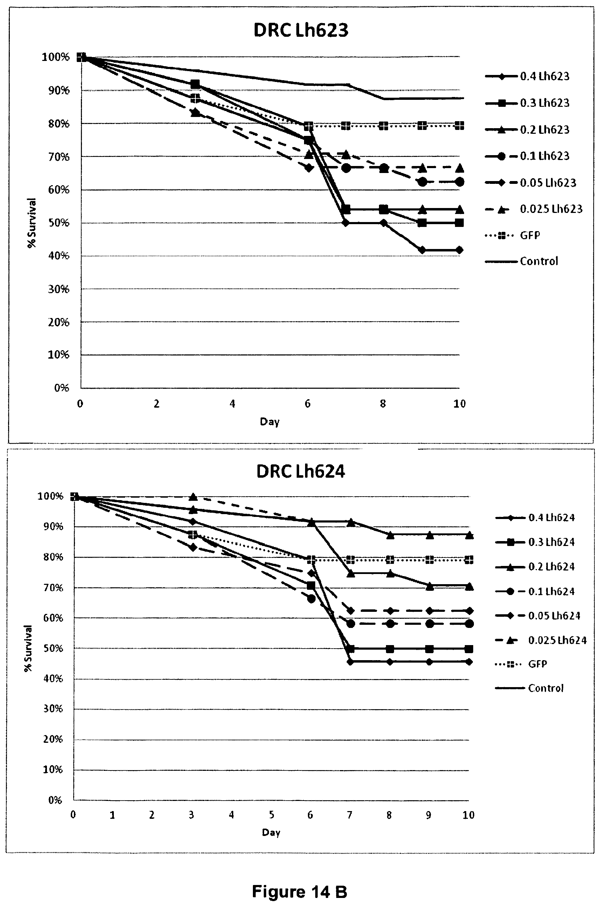

To enable testing of the Troponin pathway targets, in vitro produced dsRNAs corresponding to Lh619, Lh620, Lh621, Lh622, Lh622, Lh623, Lh624, Lh625 and Lh626 were synthesized and applied to *L. hesperus* in 10 days survival analysis bioassays. Briefly, one day old *L. hesperus* nymphs were placed in 96 well-plates with sucrose seals containing 0.5µg/µl target dsRNA, supplemented with 5 µg/µl yeast tRNA. The plates were incubated for 3 days under standard *Lygus* rearing conditions. At day 3, 6 and 8, the diet seals were refreshed with seals containing *Lygus* diet only. Lh594 (Troponin I) was used as positive control and GFP dsRNA and sucrose diet were used as negative controls (**Figure 13**)**.** Four targets were then included in dose response curve analyses in an *in vitro* assay, with concentrations ranging from 0.4 to 0.025 µg/µl. Per condition, 24 one day old nymphs were tested in the 96 well-plate set-up, in sucrose diet supplemented with dsRNA and tRNA carrier. The results are presented as % survival over a 10 day experiment (**Figures 14 A-B**)**.**

### Example 4 Identification of target genes in Leptinotarsa decemlineata

### 4.1. Leptinotarsa decemlineata normalized cDNA library and preparation of dsRNAs in multiwell plates for the screening assays

Nucleic acids were isolated from *Leptinotarsa decemlineata* larvae of different stages. A cDNA library was prepared using the SMARTer™ PCR cDNA Synthesis Kit, following the manufacturer's instructions (Clontech Cat. No 634925). The cDNA library was normalized using the Trimmer kit (Evrogen Cat No NK001) and cloned in the PCR®-BLUNTII-TOPO® vector (Invitrogen). The normalization of the clones introduced M2 adapters (Trimmer Kit, Evrogen, SEQ ID NO 92: AAGCAGTGGTATCAACGCAG), oppositely oriented at each end of the clones. The recombinant vector constructs were transformed into cells of *Escherichia coli* strain TOP10 (Invitrogen). The transformed cells were subsequently diluted and plated so as to obtain single colonies or clones. The clones were checked to ensure that clone redundancy for the library did not exceed 5%. Single clones were inoculated into liquid LB (Luria-broth) media, in 96-well plates, and grown overnight at 37°C. The plates also included positive (Ld513) and negative (FP) control clones.

To generate the dsRNA, sense and antisense DNA fragments, containing T7 promoter sequence, were generated by PCR. In brief, per clone, 1 µl of bacterial suspension was dispensed in multiwell PCR plates containing REDTaq® (Sigma Cat No D4309) and primers oGCC2738 (SEQ ID NO 93: AAGCAGTGGTATCAACGCAG) and oGCC2739 (SEQ ID NO 94: GCGTAATACGACTCACTATAGGAAGCAGTGGTATCAACGCAG) based on the M2 and the T7-M2 sequences, respectively. The PCR reaction was followed by *in vitro* transcription, where, per clone, 6 µl PCR product was used in a 20 µl reaction volume containing the transcription reagents provided by the RiboMAX™ Large Scale RNA Production System - T7 kit (Promega Cat No P1300) and incubated overnight at 37°C. The final dsRNA solution was diluted in sterile Milli-Q water and used for screening. The dsRNA corresponding to the positive Ld513 control clone is SEQ ID NO 400 (see **Table 9**) and to the negative FP control clone is SEQ ID NO 104 (see **Table 4**)**.**

### 4.2. Screen for novel and potent Leptinotarsa decemlineata target genes using a dsRNA expression cDNA library

Each well of a 48-well plate contained 0.5 mL artificial diet pretreated with a topical overlay of 25 µl (or 1 µg) of the test or control dsRNA. One L2 larva was placed in each well and 3 larvae were tested per clone. CPB survival numbers were assessed at days 4, 7 and 10.

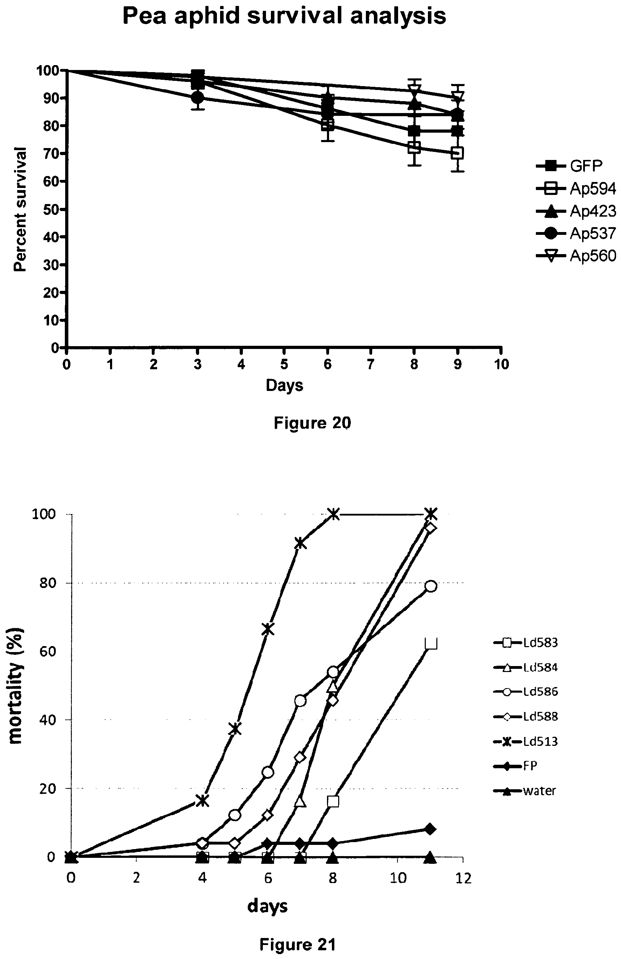

In a second bioassay, CPB larvae were fed on diet treated with topically applied test dsRNA generated from clones derived from a normalized cDNA library. One larva was placed in a well of a 48-well multiplate containing 0.5 mL diet pretreated with a topical overlay of 25 µL of a 40 ng/µL dsRNA solution. A total of twenty-four larvae were tested per treatment (clone). The number of surviving insects were assessed at days 4, 5, 6, 7, 8 & 11. The larval mortality percentage was calculated relative to day 0 (start of assay) (see **Figure 21****).**

### 4.3. Identification of L. decemlineata beetle targets

The new target sequences from the screen in 5.2. and the target sequences corresponding to the troponin pathway targets, orthologuous to the *Lygus* Lh594, Lh619 and Lh620 sequences, have been identified in *L. decemlineata.* The primers which provided relevant cDNA fragment for Ld594 are listed in **Table 17.The** cDNA sequences and deduced amino acid sequences of these target genes were determined and are provided in **Tables 7 and 9** respectively.

### 4.4. Production of dsRNAs corresponding to the partial sequences of the L. decemlineata target genes

dsRNA was synthesized using the primers as provided in **Table 9.** The sense strand of the resulting dsRNA produced from the target genes is provided in **Table 9.**

### 4.5. Survival analysis assays for novel L. decemlineata targets

### Early larval assay

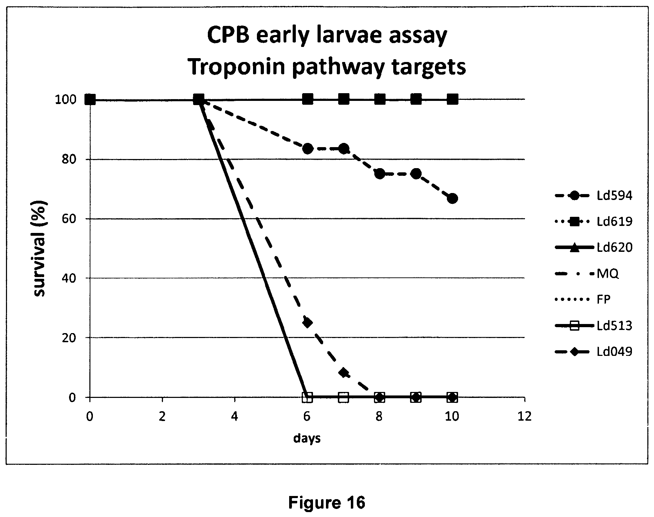

Synthetic dsRNAs were produced for the 3 targets, Ld594, Ld619 and Ld620, and were tested in a feeding assay on CPB larvae (see **Figure 16**)**.** A 10 day assay was performed in 48 well plates, on artificial diet (based on Gelman et al, J Ins Sc,1:7, 1-10: Artificial diets for rearing the Colorado Potato Beetle), supplemented with 1 µg dsRNA /well, with 12 larvae per condition.

A clear effect on the development of the larvae could be observed. A second assay was set up to investigate the effect of these dsRNAs during the course of pupation and metamorphosis (see pupation assay underneath).

### Pupation assay

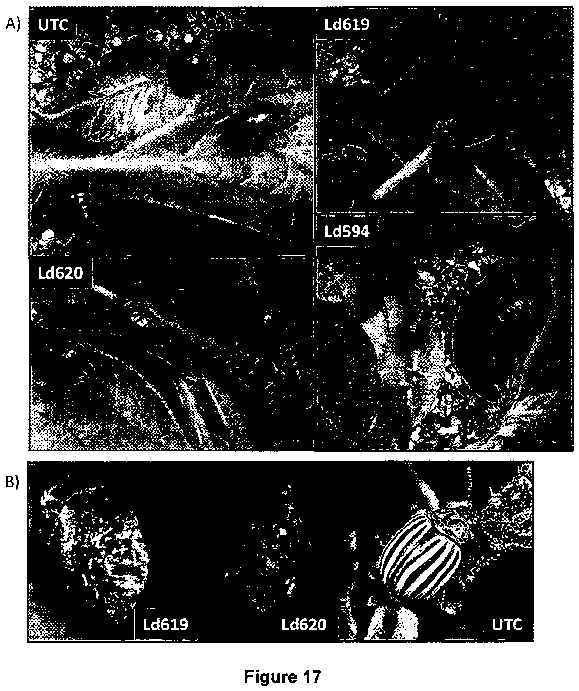

A CPB pupation assay was set up to investigate the effect of RNAi knock-down of Ld594, Ld619 and Ld620 during pupation and metamorphosis. Fourth instar larvae were fed 1 µg *in vitro* synthesized dsRNA dispensed on a potato leaf disk and were then transferred to a box containing untreated fresh potato leaves. Four days later the surviving insects were placed on vermiculite to allow pupation. Lh594 treated insects were slow, smaller and mostly were unable to go through pupation. The hatching of the pupa was assessed at the end of the experiment. For the untreated control 24 larvae pupated and all hatched into healthy adults. For Ld620, a decrease in numbers of larvae progressing into pupation was observed. For the three targets tested, no larvae progressed into healthy pupae and none emerged into adult. Dead insects recovered from the vermiculite showed various degrees of malformations (**Figure 17**)**.**

Ld594, Ld619 and Ld620, first appeared as not lethal targets in the CPB larval assay, although a reduction of vitality was clearly observed in the dsRNA treated insects. On the other hand, in the pupation assay, all 3 targets induced strong effects and inhibited the entry in pupation and/or metamorphosis.

### Adult assay

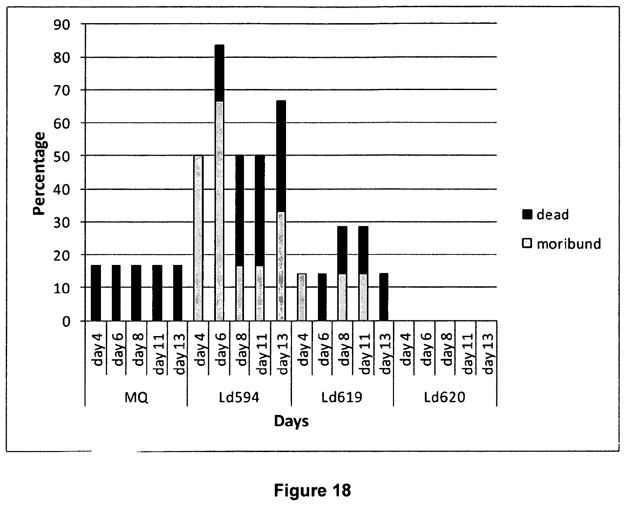

To assess activity of Ld594, Ld619 and Ld620 in CPB adults, a leaf disc assay was set up. A potato leaf disc (1.7 cm diameter) was painted with dsRNA or controls and was placed in a 3.5 cm Petri dish with one adult beetle. The next day a fresh treated leaf disc was provided to the insects. On the third day, the adults were transferred to a box containing enough fresh, untreated potato leaves to sustain the survival of the untreated controls. Per treatment, 6 adults were tested and the numbers of survivors and moribund insects were counted at regular intervals from day 6 to day 13. The insects were considered moribund if they were unable to right themselves after being placed on their back. Despite the relatively high level of background in the negative control in this particular assay, clear effects were observed for the insects that had been exposed to Ld594 or Ld619 dsRNAs (Figure 18).

### Example 5 Identification of target genes in Nilaparvata lugens

### 5.1 Identification of Nilaparvata lugens targets

New target sequences, corresponding to Troponin pathway targets and named NI594 (Troponin I), NI619 (Troponin T) and NI626 (Troponin C) have been identified in brown plant hopper, *Nilaparvata lugens.* Orthologous sequences of the *Lygus* genes, named NI594 (Troponin I), NI619 (Troponin T) and NI625/626 (Troponin C), were cloned through degenerated primer PCR, using BPH cDNA as template. In addition, full length cDNA was identified for NI594, using RACE (see above for method). AmpliTaq Gold PCR system (Applied Biosystems) was used following the manufacters' instructions and with standard conditions for the degenerate primer PCR reactions, typically as follows: 1 cycle with 10 minutes at 95°C, followed by 40 cycles with 30 seconds at 95°C, 1 minute at 50°C and 1 minute at 72°C, followed by 10 minutes at 72°C. To increase the rate of success, up to 10 different degenerated primers, forward and reverse, were designed, based on alignments of orthologous sequences in other species, and used in various combinations. PCR fragments obtained were purified from the gel by gel extraction kit (Qiagen Cat. No 28706) and cloned into a TOPO TA vector (Invitrogen). The clones were sequenced and the consensus sequences were used in Blast searches against various available insect sequence databases to confirm the relevance of the insert. The degenerated primers that resulted in successful amplification are listed in **Table 18.**

The DNA sequences and deduced amino acid sequences of these target genes and one other target gene (NI537) were determined and are provided in **Tables 10 and 11** respectively.

### 5.2 Production of dsRNAs corresponding to the partial sequences of the Nilaparvata lugens target genes

dsRNA was synthesized using the primers as provided in **Table 12.** The sense strand of the resulting dsRNA produced from each of the target genes is provided in **Table 12.**

### 5.3 Survival analysis assays for novel Nilaparvata lugens targets

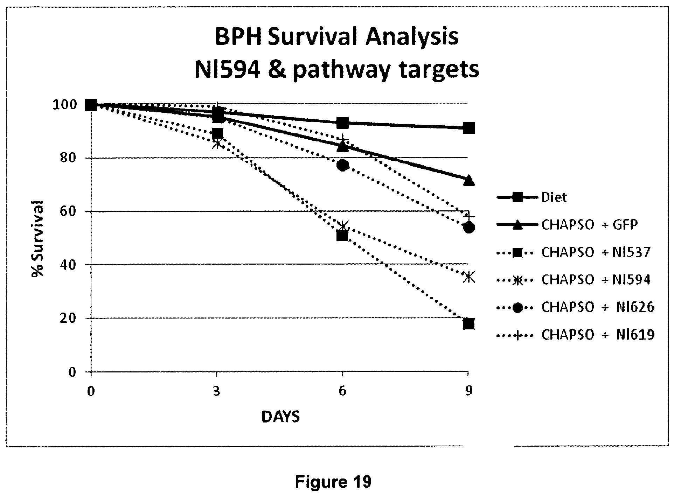

dsRNAs were synthesized and tested in the previously optimized BPH RNAi-by-feeding assays, in the presence of the zwitterionic detergent, CHAPSO, at 0.1% final concentration. The dsRNAs were tested at 0.5 µg/µl final concentration. NI537, a potent target in the BPH assays was used as bench mark target in the assay. The insect survival was assessed over the course of 9 days. The results of the bioassay showed that in BPH NI594, NI619 and NI626 were also potent RNAi targets in BPH (**Figure 19**)**.**

### Example 6 Identification of target genes in Acyrthosiphon pisum

### 6.1 Identification of Acyrthosiphon pisum targets

New target sequences have been identified in aphids and were named Ap423, Ap537, Ap560 and Ap594, following the same nomenclature: "Apxxx", where "Ap" corresponds to *Acyrthosiphon pisum* and "xxx" to the ID of the target. Primers were designed based on public domain gene prediction in AphidBase (ref: http://www.aphidbase.com/) (**Table 13**)**.**

The DNA sequences and deduced amino acid sequences of these target genes were determined and are provided in **Tables 14 and 15** respectively.

### 6.2 Production of dsRNAs corresponding to the partial sequences of the aphid target genes

dsRNA was synthesized using the primers as provided in **Table 16.** The sense strand of the resulting dsRNA produced from each of the target genes is provided in **Table 16.**

### 6.3 Survival analysis assays for novel aphid targets

RNAi-by-feeding was tested in *Acyrthosiphon pisum* (pea aphid) with 4 targets Ap594, Ap423, Ap560, Ap537. The sequences were amplified by PCR using primers, designed on public domain sequence information (http://www.aphidbase.com), and cDNA prepared from aphids. The synthetic dsRNAs were prepared and tested at a final concentration of 0.5 µg/µl in presence of 5 µg/µl yeast tRNA in a sucrose diet. Ten neonate pea aphid nymphs were placed in a small Petri dish (32 mm). Fifty µl diet (with tRNA and dsRNA) was pipetted on top of the first layer of parafilm. A second layer of parafilm covered the diet and created a feeding sachet where the aphids could feed. Per target five replicates of 10 neonate nymphs were set-up. GFP dsRNA was used as a negative control. The diet was refreshed on day 4 and 7 of the assays and survival was assessed (**Figure 20**)**.**

**Table 2**

| **Target ID** | **cDNA Sequence (sense strand) 5' → 3'** |
|---|---|
| Lh594 | SEQ ID NO 1 |
| Lh609 | SEQ ID NO 3 |
| Lh610 | SEQ ID NO 5 |
| Lh610 (b) | SEQ ID NO 139 |
| Lh611 | SEQ ID NO 7 |
| Lh611 (b) | SEQ ID NO 140 |
| Lh617 | SEQ ID NO 9 |
| Lh618 | SEQ ID NO 11 |
| Lh618 (b) | SEQ ID NO 141 |
| Lh429 | SEQ ID NO 13 |
| Lh423 | SEQ ID NO 95 |
| Lh105.2 | SEQ ID NO 96 |
| Lh560 | SEQ ID NO 15 |
| Lh615 | SEQ ID NO 17 |
| Lh612 | SEQ ID NO 19 |
| Lh246 | SEQ ID NO 21 |
| Lh597 | SEQ ID NO 23 |
| Lh598 | SEQ ID NO 25 |
| Lh619 | SEQ ID NO 121 |
| Lh619 (b) | SEQ ID NO 142 |
| Lh619 (c) | SEQ ID NO 143 |
| Lh620 | SEQ ID NO 122 |
| Lh620 (b) | SEQ ID NO 144 |
| Lh620 (c) | SEQ ID NO 145 |
| Lh621 | SEQ ID NO 123 |
| Lh622 | SEQ ID NO 124 |
| Lh623 | SEQ ID NO 125 |
| Lh623 (b) | SEQ ID NO 146 |
| Lh624 | SEQ ID NO 126 |
| Lh624 (b) | SEQ ID NO 147 |
| Lh625 | SEQ ID NO 127 |
| Lh625 (b) | SEQ ID NO 148 |
| Lh626 | SEQ ID NO 128 |
| Lh626 (b) | SEQ ID NO 149 |
| Lh614 | SEQ ID NO 129 |
| Lh627 | SEQ ID NO 150 |
| Lh628 | SEQ ID NO 152 |
| Lh629 | SEQ ID NO 154 |
| Lh630 | SEQ ID NO 156 |
| Lh631 | SEQ ID NO 158 |
| Lh632 | SEQ ID NO 160 |
| Lh633.1 | SEQ ID NO 162 |
| Lh633.2 | SEQ ID NO 163 |
| Lh634.1 | SEQ ID NO 165 |
| Lh634.2 | SEQ ID NO 167 |
| Lh595.1 | SEQ ID NO 168 |
| Lh595.2 | SEQ ID NO 170 |
| Lh596 | SEQ ID NO 172 |

**Table 3**

| **Target ID** | **Corresponding amino acid sequence of cDNA clone as represented in Table 2** |
|---|---|
| Lh594 | SEQ ID NO 79 |
| Lh609 | SEQ ID NO 80 |
| Lh610 | SEQ ID NO 81 |
| Lh610 (b) | SEQ ID NO 326 |
| Lh611 | SEQ ID NO 82 |
| Lh611 (b) | SEQ ID NO 327 |
| Lh617 | SEQ ID NO 83 |
| Lh618 | SEQ ID NO 84 |
| Lh618 (b) | SEQ ID NO 328 |
| Lh429 | SEQ ID NO 85 |
| Lh429 (b) | SEQ ID NO 329 |
| Lh423 | SEQ ID NO 99 |
| Lh105.2 | SEQ ID NO 100 |
| Lh560 | SEQ ID NO 86 |
| Lh615 | SEQ ID NO 87 |
| Lh612 | SEQ ID NO 88 |
| Lh246 | SEQ ID NO 89 |
| Lh597 | SEQ ID NO 90 |
| Lh598 | SEQ ID NO 91 |
| Lh619 | SEQ ID NO 330 |
| Lh620 | SEQ ID NO 331 |
| Lh621 | SEQ ID NO 332 |
| Lh622 | SEQ ID NO 333 |
| Lh623 | SEQ ID NO 334 |
| Lh624 | SEQ ID NO 335 |
| Lh625 | SEQ ID NO 336 |
| Lh626 | SEQ ID NO 337 |
| Lh614 | SEQ ID NO 338 |
| Lh627 | SEQ ID NO 339 |
| Lh628 | SEQ ID NO 340 |
| Lh629 | SEQ ID NO 341 |
| Lh630 | SEQ ID NO 342 |
| Lh631 | SEQ ID NO 343 |
| Lh632 | SEQ ID NO 344 |
| Lh633.1 | SEQ ID NO 345 |
| Lh633.2 | SEQ ID NO 346 |
| Lh634.1 | SEQ ID NO 347 |
| Lh634.2 | SEQ ID NO 348 |

**Table 4**

| **Target ID** | **Primers Forward 5' → 3'** | **Primers Reverse 5' → 3'** | **dsRNA: sense strand represented by equivalent DNA Sequence 5' → 3'** |
|---|---|---|---|
| Lh594 | SEQ ID NO 27 | SEQ ID NO 28 | SEQ ID NO 2 |
| | SEQ ID NO 29 | SEQ ID NO 30 | |
| Lh609 | SEQ ID NO 31 | SEQ ID NO 32 | SEQ ID NO 4 |
| | SEQ ID NO 33 | SEQ ID NO 34 | |
| Lh610 | SEQ ID NO 35 | SEQ ID NO 36 | SEQ ID NO 6 |
| | SEQ ID NO 37 | SEQ ID NO 38 | |
| Lh611 | SEQ ID NO 39 | SEQ ID NO 40 | SEQ ID NO 8 |
| | SEQ ID NO 41 | SEQ ID NO 42 | |
| Lh617 | SEQ ID NO 43 | SEQ ID NO 44 | SEQ ID NO 10 |
| | SEQ ID NO 45 | SEQ ID NO 46 | |
| Lh618 | SEQ ID NO 47 | SEQ ID NO 48 | SEQ ID NO 12 |
| | SEQ ID NO 49 | SEQ ID NO 50 | |
| Lh429 | SEQ ID NO 51 | SEQ ID NO 52 | SEQ ID NO 14 |
| | SEQ ID NO 53 | SEQ ID NO 54 | |
| Lh423 | SEQ ID NO 105 | SEQ ID NO 106 | SEQ ID NO 101 |
| | SEQ ID NO 107 | SEQ ID NO 108 | |
| Lh105.2 | SEQ ID NO 109 | SEQ ID NO 110 | SEQ ID NO 102 |
| | SEQ ID NO 111 | SEQ ID NO 112 | |
| GFP | SEQ ID NO 113 | SEQ ID NO 114 | SEQ ID NO 103 |
| | SEQ ID NO 115 | SEQ ID NO 116 | |
| Pt | SEQ ID NO 117 | SEQ ID NO 118 | SEQ ID NO 104 |
| | SEQ ID NO 119 | SEQ ID NO 120 | |
| Lh560 | SEQ ID NO 55 | SEQ ID NO 56 | SEQ ID NO 16 |
| | SEQ ID NO 57 | SEQ ID NO 58 | |
| Lh615 | SEQ ID NO 59 | SEQ ID NO 60 | SEQ ID NO 18 |
| | SEQ ID NO 61 | SEQ ID NO 62 | |
| Lh612 | SEQ ID NO 63 | SEQ ID NO 64 | SEQ ID NO 20 |
| | SEQ ID NO 65 | SEQ ID NO 66 | |
| Lh246 | SEQ ID NO 67 | SEQ ID NO 68 | SEQ ID NO 22 |
| | SEQ ID NO 69 | SEQ ID NO 70 | |
| Lh597 | SEQ ID NO 71 | SEQ ID NO 72 | SEQ ID NO 24 |
| | SEQ ID NO 73 | SEQ ID NO 74 | |
| Lh598 | SEQ ID NO 75 | SEQ ID NO 76 | SEQ ID NO 26 |
| | SEQ ID NO 77 | SEQ ID NO 78 | |
| Lh619 | SEQ ID NO 206 | SEQ ID NO 207 | SEQ ID NO 130 |
| | SEQ ID NO 208 | SEQ ID NO 209 | |
| Lh620 | SEQ ID NO 210 | SEQ ID NO 211 | SEQ ID NO 131 |
| | SEQ ID NO 212 | SEQ ID NO 213 | |
| Lh621 | SEQ ID NO 214 | SEQ ID NO 215 | SEQ ID NO 132 |
| | SEQ ID NO 216 | SEQ ID NO 217 | |
| Lh622 | SEQ ID NO 218 | SEQ ID NO 219 | SEQ ID NO 133 |
| | SEQ ID NO 220 | SEQ ID NO 221 | |
| Lh623 | SEQ ID NO 222 | SEQ ID NO 223 | SEQ ID NO 134 |
| | SEQ ID NO 224 | SEQ ID NO 225 | |
| Lh624 | SEQ ID NO 226 | SEQ ID NO 227 | SEQ ID NO 135 |
| | SEQ ID NO 228 | SEQ ID NO 229 | |
| Lh625 | SEQ ID NO 230 | SEQ ID NO 231 | SEQ ID NO 136 |
| | SEQ ID NO 232 | SEQ ID NO 233 | |
| Lh626 | SEQ ID NO 234 | SEQ ID NO 235 | SEQ ID NO 137 |
| | SEQ ID NO 236 | SEQ ID NO 237 | |
| Lh614 | SEQ ID NO 238 | SEQ ID NO 239 | SEQ ID NO 138 |
| | SEQ ID NO 240 | SEQ ID NO 241 | |
| Lh627 | SEQ ID NO 242 | SEQ ID NO 243 | SEQ ID NO 151 |
| | SEQ ID NO 244 | SEQ ID NO 245 | |
| Lh628 | SEQ ID NO 246 | SEQ ID NO 247 | SEQ ID NO 153 |
| | SEQ ID NO 248 | SEQ ID NO 249 | |
| Lh629 | SEQ ID NO 250 | SEQ ID NO 251 | SEQ ID NO 155 |
| | SEQ ID NO 25 | SEQ ID NO 253 | |
| Lh630 | SEQ ID NO 254 | SEQ ID NO 255 | SEQ ID NO 157 |
| | SEQ ID NO 256 | SEQ ID NO 257 | |
| Lh631 | SEQ ID NO 258 | SEQ ID NO 259 | SEQ ID NO 159 |
| | SEQ ID NO 260 | SEQ ID NO 261 | |
| Lh632 | SEQ ID NO 262 | SEQ ID NO 263 | SEQ ID NO 161 |
| | SEQ ID NO 264 | SEQ ID NO 265 | |
| Lh633.2 | SEQ ID NO 266 | SEQ ID NO 267 | SEQ ID NO 164 |
| | SEQ ID NO 268 | SEQ ID NO 269 | |
| Lh634.1 | SEQ ID NO 270 | SEQ ID NO 271 | SEQ ID NO 166 |
| | SEQ ID NO 272 | SEQ ID NO 273 | |
| Lh595 | SEQ ID NO 274 | SEQ ID NO 275 | SEQ ID NO 169 |
| | SEQ ID NO 276 | SEQ ID NO 277 | |
| Lh596 | SEQ ID NO 278 | SEQ ID NO 279 | SEQ ID NO 173 |
| | SEQ ID NO 280 | SEQ ID NO 281 | |

**Table 7**

| **Target ID** | **cDNA sequence (sense strand) 5' → 3'** |
|---|---|
| Ld594 | SEQ ID NO 174 |
| Ld594(b) | SEQ ID NO 404 |
| Ld619 | SEQ ID NO 176 |
| Ld620 | SEQ ID NO 178 |
| Ld583 | SEQ ID NO 386 |
| Ld584 | SEQ ID NO 387 |
| Ld586 | SEQ ID NO 388 |
| Ld588 | SEQ ID NO 389 |
| Ld513 | SEQ ID NO 394 |

**Table 8**

| **Target ID** | **Corresponding amino acid sequence of cDNA clone as represented in Table 9** |
|---|---|
| Ld594 | SEQ ID NO 349 |
| Ld594(b) | SEQ ID NO 405 |
| Ld619 | SEQ ID NO 350 |
| Ld620 | SEQ ID NO 351 |
| Ld583 | SEQ ID NO 390 |
| Ld584 | SEQ ID NO 391 |
| Ld586 | SEQ ID NO 392 |
| Ld588 | SEQ ID NO 393 |
| Ld513 | SEQ ID NO 395 |

**Table 9**

| **Target ID** | **Primers Forward 5' → 3'** | **Primers Reverse 5' → 3'** | **dsRNA: sense strand represented by equivalent DNA Sequence 5' → 3'** |
|---|---|---|---|
| Ld594 | SEQ ID NO 282 | SEQ ID NO 283 | SEQ ID NO 175 |
| | SEQ ID NO 284 | SEQ ID NO 285 | |
| Ld619 | SEQ ID NO 286 | SEQ ID NO 287 | SEQ ID NO 177 |
| | SEQ ID NO 288 | SEQ ID NO 289 | |
| Ld620 | SEQ ID NO 290 | SEQ ID NO 291 | SEQ ID NO 179 |
| | SEQ ID NO 292 | SEQ ID NO 293 | |
| Ld513 | SEQ ID NO 396 | SEQ ID NO 397 | SEQ ID NO 400 |
| | SEQ ID NO 398 | SEQ ID NO 399 | |

**Table 10**

| **Target ID** | **cDNA Sequence (sense strand) 5' → 3'** |
|---|---|
| NI594 | SEQ ID NO 180 |
| NI619 | SEQ ID NO 182 |
| NI626 | SEQ ID NO 184 |
| NI537 | SEQ ID NO 186 |

**Table 11**

| **Target ID** | **Corresponding amino acid sequence of cDNA clone as represented in Table 12** |
|---|---|
| NI594 | SEQ ID NO 352 |
| NI619 | SEQ ID NO 353 |
| NI626 | SEQ ID NO 354 |
| NI537 | SEQ ID NO 355 |

**Table 12**

| **Target ID** | **Primers Forward 5' → 3'** | **Primers Reverse 5' → 3'** | **dsRNA: sense strand represented by equivalent DNA Sequence 5' → 3'** |
|---|---|---|---|
| NI594 | SEQ ID NO 294 | SEQ ID NO 295 | SEQ ID NO 181 |
| | SEQ ID NO 296 | SEQ ID NO 297 | |
| NI619 | SEQ ID NO 298 | SEQ ID NO 299 | SEQ ID NO 183 |
| | SEQ ID NO 300 | SEQ ID NO 301 | |
| NI626 | SEQ ID NO 302 | SEQ ID NO 303 | SEQ ID NO 185 |
| | SEQ ID NO 304 | SEQ ID NO 305 | |
| NI537 | SEQ ID NO 306 | SEQ ID NO 307 | SEQ ID NO 187 |
| | SEQ ID NO 308 | SEQ ID NO 309 | |

**Table 13**

| **Target** | **Fw primer sequence** | **Reverse primer sequence** |
|---|---|---|
| Ap594 | SEQ ID NO 369 | SEQ ID NO 370 |
| Ap423 | SEQ ID NO 371 | SEQ ID NO 372 |
| Ap537 | SEQ ID NO 373 | SEQ ID NO 374 |
| Ap560 | SEQ ID NO 375 | SEQ ID NO 376 |

**Table 14**

| **Target ID** | **cDNA Sequence (sense strand) 5' → 3'** |
|---|---|
| Ap594 | SEQ ID NO 188 |
| Ap423 | SEQ ID NO 200 |
| Ap537 | SEQ ID NO 202 |
| Ap560 | SEQ ID NO 204 |

**Table 15**

| **Target ID** | **Corresponding amino acid sequence of cDNA clone as represented in Table 16** |
|---|---|
| Ap594 | SEQ ID NO 356 |
| Ap423 | SEQ ID NO 357 |
| Ap537 | SEQ ID NO 358 |
| Ap560 | SEQ ID NO 359 |

**Table 16**

| **Target ID** | **Primers Forward 5' → 3'** | **Primers Reverse 5' → 3'** | **dsRNA: sense strand represented by equivalent DNA sequence 5' → 3'** |
|---|---|---|---|
| Ap594 | SEQ ID NO 310 | SEQ ID NO 311 | SEQ ID NO 189 |
| | SEQ ID NO 312 | SEQ ID NO 313 | |
| Ap423 | SEQ ID NO 314 | SEQ ID NO 315 | SEQ ID NO 201 |
| | SEQ ID NO 316 | SEQ ID NO 317 | |
| Ap537 | SEQ ID NO 318 | SEQ ID NO 319 | SEQ ID NO 203 |
| | SEQ ID NO 320 | SEQ ID NO 321 | |
| Ap560 | SEQ ID NO 322 | SEQ ID NO 323 | SEQ ID NO 205 |
| | SEQ ID NO 324 | SEQ ID NO 325 | |

**Table 17**

| **Target** | **Forward primer** | **Reverse primer** |
|---|---|---|
| Ld594 | SEQ ID NO 377 | SEQ ID NO 378 |

**Table 18**

| **Target** | **Forward primer** | **Reverse primer** |
|---|---|---|
| NI594 | seq id no 379 | seq id no 380 |
| NI619 | seq id no 381 | seq id no 382 |
| NI626 | seq id no 383 | seq id no 384 |

**Table 19**

| **Target ID** | **Best Drosophila hit** | **NAME** | **SYMBOL** |
|---|---|---|---|
| **Ld583** | CG4759 | Ribosomal protein L27 | RpL27 |
| **Ld584** | CG 17331 | Proteasome, beta-type subunit | |
| **Ld586** | CG13704 | unknown | |
| **Ld588** | CG4157 | Rpn12 | |

**Table 20**

| **Target ID** | **Best Drosophila hit** | **NAME** | **SYMBOL** |
|---|---|---|---|
| **NI594** | CG7178 | wings up A (troponin I) | wupA |
| **NI619** | CG7107 | troponin T (upheld) | up |
| **NI626** | *CG9073, CG7930, CG2981, CG12408, CG6514, CG2981, CG7930, CG9073, CG6514, CG12408 | troponin C | |
| **NI537** | CG32744 | Ubiquitin-5E; protein modification process | |

| | | | |
|---|---|---|---|
| *unclear : multiple hits in family | | | |

**Table 21**

| **Target ID** | **Best Drosophila hit** | **NAME** | **SYMBOL** |
|---|---|---|---|
| **Ap594** | CG7178 | wings up A (troponin I) | wupA |
| **Ap423** | CG2746 | ribosomal protein L19 | RpL19 |
| **Ap537** | CG32744 | Ubiquitin-5E; protein modification process | |
| **Ap560** | CG10423 | ribosomal protein S27 | RpS27 |

It will be appreciated by persons skilled in the art that numerous variations and/or modifications may be made to the above mentioned assays. Those skilled in the art will recognize, or be able to ascertain using no more than routine experimentation, many equivalents to the specific examples. The present example, therefore, is to be considered in all respects as illustrative and not restrictive.

## Claims

1. An interfering ribonucleic acid (RNA) that functions upon uptake by an insect pest species to down-regulate expression of a target gene in said insect pest, wherein the interfering RNA comprises at least one silencing element wherein said silencing element is a region of double-stranded RNA comprising annealed complementary strands, one strand of which comprises or consists of a sequence of at least 30 contiguous nucleotides that is at least 85% complementary to SEQ ID NO 389, or the complement thereof.

2. The interfering RNA of claim 1 wherein the RNA comprises at least two silencing elements, wherein each silencing element comprises or consists of a sequence of nucleotides which is complementary to a target nucleotide sequence within a target gene.

3. The interfering RNA of any of claims 1-2 wherein said insect pest species is a plant pest.

4. The interfering RNA of claim 3 wherein said plant pest is an insect pest species selected from the insect species belonging to the orders *Coleoptera, Hemiptera, Lepidoptera, Diptera, Dichyoptera, Orthoptera, and Siphonaptera.*

5. The interfering RNA of claim 4 wherein said insect plant pest is selected from the group consisting of *Leptinotarsa* spp. (e.g. *L. decemlineata* (Colorado potato beetle), *L. juncta* (false potato beetle), or *L. texana* (Texan false potato beetle)); *Nilaparvata* spp. (e.g. *N. lugens (brown planthopper));* Lygus spp. (e.g. L. lineolaris (tarnished plant bug) or L. hesperus (western tarnished plant bug)); Myzus spp. (e.g. M. persicae (green peach aphid)); *Diabrotica spp. (e.g. D. virgifera virgifera (western corn rootworm), D. barberi (northern corn rootworm), D. undecimpunctata howardi (southern corn rootworm), D. virgifera zeae (Mexican corn rootworm).*

6. The interfering RNA of any of claims 1-5 wherein the target gene encodes the Regulatory particle non-ATPase 12 protein.

7. The interfering RNA of any of claims 1-6 wherein down-regulating expression of said target gene causes decreased growth, development, reproduction, or survival of said pest as compared with pest species exposed to an interfering ribonucleic acid targeting a non-essential gene or an interfering ribonucleic acid that does not down-regulate any genes within said pest species.

8. A polynucleotide comprising a sequence of nucleotides encoding the interfering RNA of any of claims 1-7.

9. A DNA construct comprising the polynucleotide of claim 8.

10. The DNA construct of claim 9 which is an expression construct, wherein said nucleotide sequence encoding said interfering RNA is operably linked to at least one regulatory sequence capable of driving expression of said nucleotide sequence.

11. A host cell comprising an interfering RNA of any of claims 1-7, the polynucleotide of claim 8 or the DNA construct of claim 9 or 10.

12. The host cell of claim 11 which is a prokaryotic or a eukaryotic cell.

13. The host cell of claim 12 wherein the host cell is a bacterial cell.

14. A composition for preventing and/or controlling insect pest infestation comprising at least one interfering ribonucleic acid (RNA) according to any of claims 1-7 and at least one suitable carrier, excipient or diluent.

15. The composition comprising a host cell expressing or capable of expressing the interfering RNA of any of claims 1-7.

16. The composition of claim 15 wherein the host cell is a bacterial cell.

17. The composition of any of claims 14-16 wherein the composition is in solid, liquid or gel form.

18. The composition of any of claims 14-17 wherein the composition is formulated as an insecticidal spray.

19. The composition of claim 18 wherein the spray is a pressurized/aerosolized spray or a pump spray.

20. The composition of any of claims 14-19 wherein the composition further comprises at least one pesticidal agent selected from the group consisting of a chemical insecticide, a patatin, a *Bacillus thuringiensis* insecticidal protein, a *Xenorhabdus* insecticidal protein, a *Photorhabdus* insecticidal protein, a *Bacillus laterosporus* insecticidal protein, and a *Bacillus sphaericus* insecticidal protein.

21. The composition of claim 20 wherein said *Bacillus thuringiensis* insecticidal protein is selected from the group consisting of a Cry1, a Cry3, a TIC851, a CryET170, a Cry22, a TIC901, a TIC201, a TIC407, a TIC417, a binary insecticidal protein CryET80 and CryET76, a binary insecticidal protein TIC100 and TIC101, a combination of an insecticidal protein ET29 or ET37 with an insecticidal protein TIC810 or TIC812, and a binary insecticidal protein PS149B1.

22. A housing or trap for an insect pest which contains a composition as defined in any of claims 14-21.

23. Use of a composition according to any of claims 14-21 for preventing and/or controlling pest infestation.

24. Use of a composition according to any of claims 14-21 as a pesticide for a plant or for propagation of reproductive material of a plant.

25. A combination for preventing and/or controlling pest infestation comprising the composition of any of claims 14-21 and at least one other active agent.

26. The combination of claim 25 for preventing and/or controlling pest infestation of a plant wherein said other active agent comprises a herbicide.

27. The combination of claim 26 wherein the herbicide is selected from a glyphosate-insensitive version of a 5-enolpyruvylshikimate-3-phosphate synthase (EPSPS), a catabolic enzyme that is able to break down dicamba such as dicamba monoxygenase or a phosphinothricin acetyl transferase gene that is able to catabolize glufosinate-ammonium.

28. A method for down-regulating expression of a target gene in an insect pest species in order to prevent and/or control pest infestation, comprising contacting said pest species with an effective amount of at least one interfering ribonucleic acid (RNA) according to any of claims 1-7.

29. The method of claim 28 wherein down-regulation of expression of said target gene in said insect pest species is used to obtain at least 20% pest control or at least 20% pest mortality as compared to control insect pests contacted with an interfering ribonucleic acid (RNA) targeting a non-essential pest gene or a target gene not expressed in said pest.

30. The method of claim 28 or 29 wherein the method is used to prevent and/or control pest infestation of a plant.

31. The method of claim 30 wherein the plant is chosen from the group comprising cotton, potato, rice, canola, sunflower, sorghum, pearl millet, corn, strawberries, soy, alfalfa, tomato, eggplant, pepper and tobacco.

32. Use of the interfering ribonucleic acid (RNA) of any of claims 1-7, the DNA construct of claim 9 or claim 10, or the composition of any of claims 14-21 for preventing and/or controlling insect pest infestation.

## Patentansprüche

1. Interferierende Ribonukleinsäure (RNA), die bei der Aufnahme durch eine Insektenschädlingsart derart wirkt, dass sie die Expression eines Zielgens in dem Insektenschädling abwärts reguliert, wobei die interferierende RNA mindestens ein Silencing-Element umfasst, wobei das Silencing-Element eine Region mit doppelsträngiger RNA ist, die hybridisierte komplementäre Stränge umfasst, von denen ein Strang eine Sequenz von mindestens 30 zusammenhängenden Nukleotiden, die zu SEQ ID NO: 389, oder deren Komplement zumindest 85% komplementär ist, umfasst oder daraus besteht.

2. Interferierende RNA nach Anspruch 1, wobei die RNA mindestens zwei Silencing-Elemente umfasst, wobei jedes Silencing-Element eine Sequenz von Nukleotiden umfasst oder daraus besteht, die komplementär zu einer Zielnukleotidsequenz innerhalb eines Zielgens ist.

3. Interferierende RNA nach einem der Ansprüche 1 bis 2, wobei die Insektenschädlingsart ein Pflanzenschädling ist.

4. Interferierende RNA nach Anspruch 3, wobei der Pflanzenschädling eine Insektenschädlingsart ist, die aus den Insektenarten ausgewählt ist, die zu den Ordnungen *Coleoptera, Hemiptera, Lepidoptera, Diptera, Dichyoptera, Orthoptera* und *Siphonaptera* gehören.

5. Interferierende RNA nach Anspruch 4, wobei der Insektenpflanzenschädling aus der Gruppe ausgewählt ist, die aus *Leptinotarsa* spp. (z.B. *L. decemlineata* (Colorado-Kartoffelkäfer), *L. juncta* (falscher Kartoffelkäfer) oder *L. texana* (texanischer falscher Kartoffelkäfer)); *Nilaparvata* spp. (z.B. *N. lugens* (braune Reiszikade)); *Lygus* spp. (z.B. *L. lineolaris* (tarnished plant bug) oder *L. hesperus* (western tarnished plant bug)); *Myzus* spp. (z.B. *M. persicae* (grüne Pfirsichblattlaus)); *Diabrotica* spp. (z.B. *D. virgifera virgifera* (westlicher Maiswurzelbohrer), *D. barberi* (nördlicher Maiswurzelbohrer), *D. undecimpunctata howardi* (südlicher Maiswurzelbohrer), *D. virgifera zeae* (mexikanischer Maiswurzelbohrer)) besteht.

6. Interferierende RNA nach einem der Ansprüche 1 bis 5, wobei das Zielgen das Regulatory-Particle-Non-ATPase-12-Protein codiert.

7. Interferierende RNA nach einem der Ansprüche 1 bis 6, wobei die abwärts regulierende Expression des Zielgens ein(e) verminderte(s) Wachstum, Entwicklung, Reproduktion oder Überleben des Schädlings im Vergleich zu Schädlingsarten verursacht, die einer interferierenden Ribonukleinsäure ausgesetzt sind, die auf ein nicht-essentielles Gen zielen, oder einer interferierenden Ribonukleinsäure, die keine Gene innerhalb der Schädlingsarten abwärts reguliert.

8. Polynukleotid, umfassend eine Sequenz von Nukleotiden, die für die interferierende RNA nach einem der Ansprüche 1 bis 7 codieren.

9. DNA-Konstrukt, umfassend das Polynukleotid nach Anspruch 8.

10. DNA-Konstrukt nach Anspruch 9, bei dem es sich um ein Expressionskonstrukt handelt, wobei die Nukleotidsequenz, die die interferierende RNA codiert, funktionsfähig an mindestens eine regulatorische Sequenz gebunden ist, die die Expression der Nukleotidsequenz steuern kann.

11. Wirtszelle, umfassend eine interferierende RNA nach einem der Ansprüche 1 bis 7, das Polynukleotid nach Anspruch 8 oder das DNA-Konstrukt nach Anspruch 9 oder 10.

12. Wirtszelle nach Anspruch 11, bei der es sich um eine prokaryotische oder eine eukaryotische Zelle handelt.

13. Wirtszelle nach Anspruch 12, wobei es sich bei der Wirtszelle um eine Bakterienzelle handelt.

14. Zusammensetzung zur Prävention und/oder Bekämpfung von Insektenschädlingsbefall, umfassend mindestens eine interferierende Ribonukleinsäure (RNA) nach einem der Ansprüche 1 bis 7 und mindestens einen geeigneten Träger, einen geeigneten Exzipienten oder ein geeignetes Verdünnungsmittel.

15. Zusammensetzung, umfassend eine Wirtszelle, die die interferierende RNA nach einem der Ansprüche 1 bis 7 exprimiert oder exprimieren kann.

16. Zusammensetzung nach Anspruch 15, wobei die Wirtszelle eine Bakterienzelle ist.

17. Zusammensetzung nach einem der Ansprüche 14 bis 16, wobei die Zusammensetzung in fester, flüssiger oder Gelform vorliegt.

18. Zusammensetzung nach einem der Ansprüche 14 bis 17, wobei die Zusammensetzung als Insektizidspray formuliert ist.

19. Zusammensetzung nach Anspruch 18, wobei das Spray ein unter Druck gesetztes/aerosolisiertes Spray oder ein Pumpspray ist.

20. Zusammensetzung nach einem der Ansprüche 14 bis 19, wobei die Zusammensetzung zudem mindestens ein Pestizidmittel umfasst, das aus der Gruppe ausgewählt ist, die aus einem chemischen Insektizid, einem Patatin, einem Insektizidprotein von *Bacillus thuringiensis,* einem Insektizidprotein von *Xenorhabdus,* einem Insektizidprotein von *Photorhabdus,* einem Insektizidprotein von *Bacillus laterosporus* und einem Insektizidprotein von *Bacillus sphaericus* besteht.

21. Zusammensetzung nach Anspruch 20, wobei das Insektizidprotein von *Bacillus thuringiensis* aus der Gruppe ausgewählt ist, die aus einem Cry1, einem Cry3, einem TIC851, einem CryET170, einem Cry22, einem TIC901, einem TIC201, einem TIC407, einem TIC417, einem binären Insektizidprotein CryET80 und CryET76, einem binären Insektizidprotein TIC100 und TIC101, einer Kombination aus einem Insektizidprotein ET29 oder ET37 mit einem Insektizidprotein TIC810 oder TIC812 und einem binären Insektizidprotein PS149B1 besteht.

22. Gehäuse oder Falle für einen Insektenschädling, das/die eine Zusammensetzung nach einem der Ansprüche 14 bis 21 enthält.

23. Verwendung einer Zusammensetzung nach einem der Ansprüche 14 bis 21 zur Prävention und/oder Bekämpfung von Schädlingsbefall.

24. Verwendung einer Zusammensetzung nach einem der Ansprüche 14 bis 21 als Pestizid für eine Pflanze oder zur Vermehrung von Fortpflanzungsmaterial einer Pflanze.

25. Kombination zur Prävention und/oder Bekämpfung von Schädlingsbefall, umfassend die Zusammensetzung nach einem der Ansprüche 14 bis 21 und mindestens einen anderen Wirkstoff.

26. Kombination nach Anspruch 25 zur Prävention und/oder Bekämpfung von Schädlingsbefall einer Pflanze, wobei der andere Wirkstoff ein Herbizid umfasst.

27. Kombination nach Anspruch 26, wobei das Herbizid aus einer Glyphosat-unempfindlichen Version einer 5-Enolpyruvylshikimat-3-phosphatsynthase (EPSPS) ausgewählt ist, einem katabolischen Enzym, das Dicamba, wie Dicambamonoxygenase, abbauen kann, oder einem Phosphinothricin-Acetyltransferase-Gen, das Glufosinat-Ammonium katabolisieren kann.

28. Verfahren zur Abwärtsregulierung der Expression eines Zielgens in einer Insektenschädlingsart, um den Schädlingsbefall zu verhindern und/oder zu bekämpfen, umfassend das Inkontaktbringen der Schädlingsart mit einer wirksamen Menge von mindestens einer interferierenden Ribonukleinsäure (RNA) nach einem der Ansprüche 1 bis 7.

29. Verfahren nach Anspruch 28, wobei die Abwärtsregulierung der Expression des Zielgens in der Insektenschädlingsart verwendet wird, um mindestens 20% Schädlingsbekämpfung oder mindestens 20% Schädlingsmortalität zu erhalten, verglichen mit Kontroll-Insektenschädlingen, die mit einer interferierenden Ribonukleinsäure (RNA) in Kontakt gebracht werden, die auf ein nicht-essentielles Schädlings-Gen oder ein Ziel-Gen, das nicht in dem Schädling exprimiert wird, zielt.

30. Verfahren nach einem der Ansprüche 28 oder 29, wobei das Verfahren verwendet wird, um den Schädlingsbefall einer Pflanze zu verhindern und/oder zu kontrollieren.

31. Verfahren nach Anspruch 30, wobei die Pflanze aus der Gruppe ausgewählt ist, die aus Baumwolle, Kartoffel, Reis, Raps, Sonnenblume, Sorghum, Perlhirse, Mais, Erdbeeren, Soja, Luzerne, Tomate, Aubergine, Paprika und Tabak besteht.

32. Verwendung der interferierenden Ribonukleinsäure (RNA) nach einem der Ansprüche 1 bis 7, des DNA-Konstrukts nach Anspruch 9 oder Anspruch 10 oder der Zusammensetzung nach einem der Ansprüche 14 bis 21 zur Prävention und/oder Bekämpfung von Insektenschädlingsbefall.

## Revendications

1. Acide ribonucléique (ARN) interférent qui fonctionne dès l'absorption par une espèce d'insecte ravageur pour réguler à la baisse l'expression d'un gène cible dans ledit insecte ravageur, l'ARN interférent comprenant au moins un élément de silençage, ledit élément de silençage étant une région d'ARN en double brin comprenant des brins complémentaires annelés dont un brin comprend une séquence, ou est constitué par celle-ci, d'au moins 30 nucléotides contigus qui est complémentaire, au moins à 85%, à la SEQ ID n° : 389 ou le complément de celle-ci.

2. ARN interférent de la revendication 1, dans lequel l'ARN comprend au moins deux éléments de silençage, dans lequel chaque élément de silençage comprend une séquence de nucléotides, ou est constitué par celle-ci, qui est complémentaire à une séquence nucléotidique cible au sein d'un gène cible.

3. ARN interférent de l'une quelconque des revendications 1 à 2, dans lequel ladite espèce d'insectes ravageurs est un ravageur des plantes.

4. ARN interférent de la revendication 3, dans lequel ledit ravageur des plantes est une espèce d'insectes ravageurs choisie parmi les espèces d'insectes appartenant aux ordres des Coléoptères, Hémiptères, Lépidoptères, Diptères, Dichyoptères, Orthoptères et Siphonaptères.

5. ARN interférent de la revendication 4, dans lequel ledit insecte ravageur des plantes est choisi dans le groupe constitué par les *Leptinotarsa* spp. (à titre d'exemple, *L. decemlineata* (doryphore de la pomme de terre), *L. juncta* (faux doryphore de la pomme de terre), ou *L. texana* (faux doryphore de la pomme de terre du Texas)) ; *Nilaparvata* spp. (à titre d'exemple, *N. lugens* (cicadelle brune)) ; *Lygus* spp. (à titre d'exemple, *L. lineolaris* (punaise terne) ou *L. hesperus* (punaise terne occidentale)) ; *Myzus* spp. (à titre d'exemple, *M. persicae* (puceron vert du pêcher)) ; *Diabrotica spp*. *(*à titre d'exemple, *D. virgifera virgifera* (chrysomèle occidentale des racines du maïs), *D. barberi* (chrysomèle des racines du maïs du Nord), *D. undecimpunctata howardi* (chrysomèle des racines du maïs du Sud), *D. virgifera* zeae (chrysomèle des racines du maïs du Mexique).

6. ARN interférent de l'une quelconque des revendications 1 à 5, dans lequel le gène cible code pour la protéine à particule régulatrice non-ATPasique 12.

7. ARN interférent de l'une quelconque des revendications 1 à 6, dans lequel une régulation à la baisse de l'expression dudit gène cible cause une diminution de la croissance, du développement, de la reproduction ou de la survie dudit ravageur, en comparaison avec des espèces de ravageurs exposées à un acide ribonucléique interférent ciblant un gène non essentiel ou un acide ribonucléique interférent qui ne régule à la baisse aucun gène au sein desdites espèces de ravageurs.

8. Polynucléotide comprenant une séquence de nucléotides codant pour l'ARN interférent de l'une quelconque des revendications 1 à 7.

9. Construit d'ADN comprenant le polynucléotide de la revendication 8.

10. Construit d'ADN de la revendication 9 qui est un construit d'expression dans lequel ladite séquence nucléotidique codant pour ledit ARN interférent est liée, de manière opérationnelle, à au moins une séquence de régulation étant capable de conduire l'expression de ladite séquence nucléotidique.

11. Cellule hôte comprenant un ARN interférent de l'une quelconque des revendications 1 à 7, le polynucléotide de la revendication 8, ou le construit d'ADN des revendications 9 ou 10.

12. Cellule hôte de la revendication 11 qui est une cellule procaryote ou eucaryote.

13. Cellule hôte de la revendication 12, la cellule hôte étant une cellule bactérienne.

14. Composition destinée à prévenir et/ou à lutter contre une infestation par insectes ravageurs, comprenant au moins un acide ribonucléique (ARN) interférent, selon l'une quelconque des revendications 1 à 7, et au moins un transporteur, un excipient ou un diluant approprié.

15. Composition comprenant une cellule hôte exprimant l'ARN interférent de l'une quelconque des revendications 1 à 7 ou étant capable de l'exprimer.

16. Composition de la revendication 15, dans laquelle la cellule hôte est une cellule bactérienne.

17. Composition de l'une quelconque des revendications 14 à 16, la composition étant sous une forme solide, liquide ou de gel.

18. Composition de l'une quelconque des revendications 14 à 17, la composition étant formulée sous forme de pulvérisateur insecticide.

19. Composition de la revendication 18, dans laquelle le pulvérisateur est un pulvérisateur pressurisé/de type aérosol ou un pulvérisateur à pompe.

20. Composition de l'une quelconque des revendications 14 à 19, la composition comprenant en outre au moins un agent pesticide, choisi dans le groupe constitué par un insecticide chimique, une patatine, une protéine insecticide de *Bacillus thuringiensis,* une protéine insecticide de *Xenorhabdus,* une protéine insecticide de *Photorhabdus,* une protéine insecticide de *Bacillus laterosporus,* ainsi qu'une protéine insecticide de *Bacillus sphaericus.*

21. Composition de la revendication 20, dans laquelle ladite protéine insecticide de *Bacillus thuringiensis* est choisie dans le groupe constitué par une Cry1, une Cry3, une TIC851, une CryET170, une Cry22, une TIC901, une TIC201, une TIC407, une TIC417, une protéine insecticide binaire CryET80 et CryET76, une protéine insecticide binaire TIC100 et TIC101, une combinaison d'une protéine insecticide ET29 ou ET37 avec une protéine insecticide TIC810 ou TIC812, ainsi qu'une protéine insecticide binaire PS149B1.

22. Logement ou piège pour insectes ravageurs qui contient une composition telle que définie dans l'une quelconque des revendications 14 à 21.

23. Utilisation d'une composition, selon l'une quelconque des revendications 14 à 21, pour prévenir et/ou lutter contre une infestation par ravageurs.

24. Utilisation d'une composition, selon l'une quelconque des revendications 14 à 21, comme pesticide pour une plante ou pour la propagation d'un matériel de reproduction d'une plante.

25. Combinaison destinée à prévenir et/ou à lutter contre une infestation par ravageurs comprenant la composition de l'une quelconque des revendications 14 à 21 et au moins un autre agent actif.

26. Combinaison de la revendication 25 destinée à prévenir et/ou à lutter contre une infestation par ravageurs d'une plante, dans laquelle ledit autre agent actif comprend un herbicide.

27. Combinaison de la revendication 26, dans laquelle l'herbicide est choisi parmi une version insensible au glyphosate d'une 5-énolpyruvylshikimate-3-phosphate synthase (EPSPS), une enzyme catabolique qui est capable de briser le dicamba telle qu'une dicamba monoxygénase, ou un gène de phosphinothricine acétyl transférase, qui est capable de cataboliser le glufosinate d'ammonium.

28. Procédé destiné à réguler à la baisse l'expression d'un gène cible chez une espèce d'insectes ravageurs dans le but de prévenir et/ou de lutter contre une infestation par ravageurs, comprenant la mise en contact de ladite espèce de ravageurs avec une quantité efficace d'au moins un acide ribonucléique (ARN) interférent selon l'une quelconque des revendications 1 à 7.

29. Procédé de la revendication 28, dans lequel une régulation à la baisse de l'expression dudit gène cible dans ladite espèce d'insectes ravageurs est utilisée pour obtenir au moins 20% de contrôle des ravageurs ou au moins 20% de mortalité des ravageurs, en comparaison avec des insectes ravageurs témoins mis en contact avec un acide ribonucléique (ARN) interférent ciblant un gène non essentiel de ravageur ou un gène cible n'étant pas exprimé dans ledit ravageur.

30. Procédé des revendications 28 ou 29, le procédé étant utilisé pour prévenir et/ou pour lutter contre l'infestation par ravageurs d'une plante.

31. Procédé de la revendication 30, dans lequel la plante est choisie dans le groupe comprenant le coton, la pomme de terre, le riz, le colza canola, le tournesol, le sorgho, le millet perlé, le maïs, les fraises, le soja, la luzerne, la tomate, l'aubergine, le poivre et le tabac.

32. Utilisation de l'acide ribonucléique (ARN) interférent de l'une quelconque des revendications 1 à 7, du construit d'ADN de la revendication 9 ou la revendication 10, ou de la composition de l'une quelconque des revendications 14 à 21 pour prévenir et/ou pour lutter contre une infestation par insectes ravageurs.


### SEQUENCE LISTING

<110> DEVGEN NV
<120> DOWN-REGULATING GENE EXPRESSION IN INSECT PESTS
<130> NLW/P122274WO01
<160> 405
<170> PatentIn version 3.5
<210> 1
   <211> 1096
   <212> DNA
   <213> Lygus hesperus
<400> 1
   gcgatctaag gcaggtggca gacagctcga tgacggcagt gggccaagca ataatggata 60
   gtcattcata gcaccccagc tttactaagc tctgccgtag tgttggattg ggagcggata 120
   caattcacca cagaacagct atgacatgat acgcagtccg aataccctca taaaggacta 180
   gtctgcaggt ttaacgatcg cgtagcagtg tatcacgcag agtacatggg gagtgactgt 240
   gtgaacctgc tgggtacatc atcacccctc tccttcttca gttatataag acacagtccc 300
   taaaggacac cagcaaaaat ggcggatgat gaggcgaaga aggccaaaca ggccgaaatc 360
   gagaggaagc gcgctgaagt gcgcaagagg atggaggaag cctctaaggc gaagaaagcc 420
   aagaagggtt tcatgacccc ggaaaggaag aagaaactcc gactcctgct gaggaaaaaa 480
   gccgctgagg aactgaagaa ggagcaggaa cgcaaagcag ctgagaggag gcgaacgatt 540
   gaggagcgct gcgggcaaat tgccgacgtc gacaacgcca atgaagcaac cttgaagaaa 600
   ctctgcacag actaccataa gcgaattgac gctctggaga ggagtaaaat tgacatcgaa 660
   ttcgaagtgg agagacgtga ccttgagatc gccgacctca acagccaggt caacgacctc 720
   cgtggtaaat tcgtcaaacc taccttgaaa aaggtttcca agtacgaaaa caaattcgcc 780
   aagctccaga agaaggctgc cgagttcaac ttcagaaacc aactcaaggt cgtcaaaaag 840
   aaagaattca ccctggaaga agaagacaaa gagccgaaga aatcggaaaa ggcggagtgg 900
   cagaagaaat gaagggaaaa caagcacacc atctcacaaa ataaaataaa cgaaaatctt 960
   tcacacgttt accaatttta taacacggtc ctcacaaatt atgttcctta aataatttgt 1020
   ataatccatc ctcgcactac aatcaatatt aatatttaaa tacaaaacca aaaaaaaaaa 1080
   aaaaaaaaaa aaaaaa 1096
<210> 2
   <211> 491
   <212> DNA
   <213> Lygus hesperus
<400> 2
   caaacaggcc gaaatcgaga ggaagcgcgc tgaagtgcgc aagaggatgg aggaagcctc 60
   taaggcgaag aaagccaaga agggtttcat gaccccggaa aggaagaaga aactccgact 120
   cctgctgagg aaaaaagccg ctgaggaact gaagaaggag caggaacgca aagcagctga 180
   gaggaggcga acgattgagg agcgctgcgg gcaaattgcc gacgtcgaca acgccaatga 240
   agcaaccttg aagaaactct gcacagacta ccataagcga attgacgctc tggagaggag 300
   taaaattgac atcgaattcg aagtggagag acgtgacctt gagatcgccg acctcaacag 360
   ccaggtcaac gacctccgtg gtaaattcgt caaacctacc ttgaaaaagg tttccaagta 420
   cgaaaacaaa ttcgccaagc tccagaagaa ggctgccgag ttcaacttca gaaaccaact 480
   caaggtcgtc a 491
<210> 3
   <211> 431
   <212> DNA
   <213> Lygus hesperus
<400> 3
   atgggcatca tgtcgaaagc tgaactcgct tgtgtttact ccgctctcat cctcatcgac 60
   gacgatgtcg ccgtgacggg tgagaagatt caaaccatcc tgaaggctgc cagtgtcgac 120
   atcgagccgt actggcccgg tctgttcgcc aaggccctcg agggtatcaa ccccaaagac 180
   ctcatctcct ccattggaag cggagttggt gctggagcgc cggctgtcgg tggagctgca 240
   cctgccgccg ctgctgcccc tgccgctgag gctaagaagg aagagaagaa gaaggtcgaa 300
   agcgatccag aatccgatga tgacatgggc ttcggtcttt tcgactaaga gcattccaca 360
   gcgggttctc atttgttttt aagattttct tttaaaaaat aaaacttcca aaaaaaaaaa 420
   aaaaaaaaaa g 431
<210> 4
   <211> 332
   <212> DNA
   <213> Lygus hesperus
<400> 4
   gggcatcatg tcgaaagctg aactcgcttg tgtttactcc gctctcatcc tcatcgacga 60
   cgatgtcgcc gtgacgggtg agaagattca aaccatcctg aaggctgcca gtgtcgacat 120
   cgagccgtac tggcccggtc tgttcgccaa ggccctcgag ggtatcaacc ccaaagacct 180
   catctcctcc attggaagcg gagttggtgc tggagcgccg gctgtcggtg gagctgcacc 240
   tgccgccgct gctgcccctg ccgctgaggc taagaaggaa gagaagaaga aggtcgaaag 300
   cgatccagaa tccgatgatg acatgggctt cg 332
<210> 5
   <211> 468
   <212> DNA
   <213> Lygus hesperus
<400> 5
   atgggggcag gtcttctcca taaccataga ttatcttcgt gtatcgtgtc gggctttcgg      60
   ctgaggtcct aattagtaaa taatgattcc gcctacgtcg cggcctcagg tcactgtcta     120
   cagtgacaaa aatgaggcca ccgggactct cctcaacctc ccggctgtct tcaacgcccc     180
   cattcgcccc gatgttgtga acttcgttca ccaaaatgtc gctaaaaacc acaggcagcc     240
   ctactgtgtc tccgctcaag ctggtcatca gacttcagct gagtcctggg gtaccggtcg     300
   tgctgtggct cgtatccccc gtgttcgcgg aggtggtact caccgctcag gtcagggtgc     360
   ttttggcaac atgtgtcgcg gcggtaggat gttcgctccc actcgcccat ggcgtcgttg     420
   gcaccgcaaa atcaacgtta accaaaaaaa aaaaaaaaaa aaaaaaaa                  468
<210> 6
   <211> 429
   <212> DNA
   <213> Lygus hesperus
<400> 6
   gggcaggtct tctccataac catagattat cttcgtgtat cgtgtcgggc tttcggctga      60
   ggtcctaatt agtaaataat gattccgcct acgtcgcggc ctcaggtcac tgtctacagt     120
   gacaaaaatg aggccaccgg gactctcctc aacctcccgg ctgtcttcaa cgcccccatt     180
   cgccccgatg ttgtgaactt cgttcaccaa aatgtcgcta aaaaccacag gcagccctac     240
   tgtgtctccg ctcaagctgg tcatcagact tcagctgagt cctggggtac cggtcgtgct     300
   gtggctcgta tcccccgtgt tcgcggaggt ggtactcacc gctcaggtca gggtgctttt     360
   ggcaacatgt gtcgcggcgg taggatgttc gctcccactc gcccatggcg tcgttggcac     420
   cgcaaaatc                                                             429
<210> 7
   <211> 523
   <212> DNA
   <213> Lygus hesperus
<400> 7
   atgggatctc tatgctgaaa aggtcgccac cagaggtttg tgtgctattg cacaagctga       60
   atccctccgt tacaaactca ttggcggtct tgctgtccga ggggcttgct atggtgtcct      120
   tcgcttcatc atggaaaatg gtgccaaggg ttgcgaagtc gtagtatctg gaaaactgcg      180
   tggtcagaga gccaagtcaa tgaagttcgt ggatggtttg atgatccaca gtggggatcc      240
   ctgtaacgaa tatgttgata ctgctacccg acatgtgctc cttagacaag gtgtcctggg      300
   aataaaggtg aagattatgt tgccgtggga cgttaccggc aaaaatgggc cgaagaaccc      360
   tcttcccgac cacgtcagcg ttctcttacc taaggaggag ctaccaaatt tggccgttag      420
   tgtgcctgga tccgacatca aaccaaagcc tgaagtacca gcacccgctt tgtgaatata      480
   aacttctttt ttgtaaaaaa aaaaaaaaaa aaaaaaaaaa aaa                        523
<210> 8
   <211> 431
   <212> DNA
   <213> Lygus hesperus
<400> 8
   attgcacaag ctgaatccct ccgttacaaa ctcattggcg gtcttgctgt ccgaggggct       60
   tgctatggtg tccttcgctt catcatggaa aatggtgcca agggttgcga agtcgtagta      120
   tctggaaaac tgcgtggtca gagagccaag tcaatgaagt tcgtggatgg tttgatgatc      180
   cacagtgggg atccctgtaa cgaatatgtt gatactgcta cccgacatgt gctccttaga      240
   caaggtgtcc tgggaataaa ggtgaagatt atgttgccgt gggacgttac cggcaaaaat      300
   gggccgaaga accctcttcc cgaccacgtc agcgttctct tacctaagga ggagctacca      360
   aatttggccg ttagtgtgcc tggatccgac atcaaaccaa agcctgaagt accagcaccc      420
   gctttgtgaa t                                                           431
<210> 9
   <211> 823
   <212> DNA
   <213> Lygus hesperus
<400> 9
   catggggaca ctctcttttt cttcatcgcg tggctcgctg ccgtgtggtt agggagtttc      60
   ctactttaat tttttagtgt aattcatctt caaaatgacg tcgaaggttt ctcgtgagac     120
   cctctacgag tgcatcaatg gagtcatcca gtcctcccag gagaagaaga ggaacttcgt     180
   ggagactgtg gagatccaga tcggtctgaa gaactacgat ccccagaagg acaagcgttt     240
   ctcgggaact gtcaagctga agcacattcc aaggcctaaa atgcaggttt gcatcctcgg     300
   agatcaacag cattgcgacg aggccaaagc caacaacgtg ccctacatgg acgtcgaggc     360
   tctgaagaag ctcaacaaaa acaagaagct cgtcaagaaa ttggccaaga aatacgacgc     420
   tttcctcgcc tcagaagccc tcatcaagca gatccccagg ctcctcggac ccggtctcaa     480
   caaggcgggc aagttccctg gtctcctctc tcaccaggag tccatgatga tgaagatcga     540
   cgaagtcaag gccaccatca agttccaaat gaagaaggtg ttgtgcctct cagtggctgt     600
   cggtcacgtc ggcatgactg ctgatgagct cgtccagaac gtgcacttgt cggtcaactt     660
   cctcgtttcg ctcctcaaga agcactggca gaacgtcagg tctctccacg tcaaatccac     720
   gatgggacct ccccagaggc tttactaaac atcttgtttt ttacttttga cgaataaaat     780
   tcgttttatt ctcgaaaaaa aaaaaaaaaa aaaaaaaaaa aaa                       823
<210> 10
   <211> 607
   <212> DNA
   <213> Lygus hesperus
<400> 10
   ccctctacga gtgcatcaat ggagtcatcc agtcctccca ggagaagaag aggaacttcg      60
   tggagactgt ggagatccag atcggtctga agaactacga tccccagaag gacaagcgtt     120
   tctcgggaac tgtcaagctg aagcacattc caaggcctaa aatgcaggtt tgcatcctcg     180
   gagatcaaca gcattgcgac gaggccaaag ccaacaacgt gccctacatg gacgtcgagg     240
   ctctgaagaa gctcaacaaa aacaagaagc tcgtcaagaa attggccaag aaatacgacg     300
   ctttcctcgc ctcagaagcc ctcatcaagc agatccccag gctcctcgga cccggtctca     360
   acaaggcggg caagttccct ggtctcctct ctcaccagga gtccatgatg atgaagatcg     420
   acgaagtcaa ggccaccatc aagttccaaa tgaagaaggt gttgtgcctc tcagtggctg     480
   tcggtcacgt cggcatgact gctgatgagc tcgtccagaa cgtgcacttg tcggtcaact     540
   tcctcgtttc gctcctcaag aagcactggc agaacgtcag gtctctccac gtcaaatcca     600
   cgatggg                                                               607
<210> 11
   <211> 435
   <212> DNA
   <213> Lygus hesperus
<400> 11
   atgggaccaa taaagatcaa ctttcccaga gaaagacttg ctatgcccag cataatcagg      60
   tccgagaaat ccgcaaaaag atggttaaaa acatcagtga cagcatttcc agctgtgatt     120
   tgaggagtgt tgtgaacaag ctgatcccag actccatcgc taaagatata gaaaagaatt     180
   gccaaggaat ctacccactc cacgatgtgt acattcggaa ggtgaaggtg ttgaagaagc     240
   cgaggttcga gctcagcaag ctccttgagc ttcacgtcga tggcaaaggg atcgacgaac     300
   ccggcgcgaa agtgacgagg actgacgctt acgagcctcc agttcaagag tctgtctaag     360
   taaacatttt atataaagtt aacaaaaaat aaaggtgtct cgcctgacta aaaaaaaaaa     420
   aaaaaaaaaa aaaaa                                                       435
<210> 12
   <211> 353
   <212> DNA
   <213> Lygus hesperus
<400> 12
   ccaataaaga tcaactttcc cagagaaaga cttgctatgc ccagcataat caggtccgag      60
   aaatccgcaa aaagatggtt aaaaacatca gtgacagcat ttccagctgt gatttgagga     120
   gtgttgtgaa caagctgatc ccagactcca tcgctaaaga tatagaaaag aattgccaag     180
   gaatctaccc actccacgat gtgtacattc ggaaggtgaa ggtgttgaag aagccgaggt     240
   tcgagctcag caagctcctt gagcttcacg tcgatggcaa agggatcgac gaacccggcg     300
   cgaaagtgac gaggactgac gcttacgagc ctccagttca agagtctgtc taa            353
<210> 13
   <211> 474
   <212> DNA
   <213> Lygus hesperus
<400> 13
   catgggtacg aatatcgacg gtaaaagaaa ggtgatgttc gccatgaccg ccatcaaagg 60
   tgtcggcaga cggtacgcca acattgtcct caagaaggcc gatgtcaact tggacaagag 120
   ggccggcgaa tgctccgaag aagaagttga aaagatcgtt accatcatgc aaaaccctag 180
   gcaatacaaa attcccaact ggttcctcaa cagacaaaaa gacaccgtcg agggcaaata 240
   ctctcagttg acttcctccc tgctggattc caagctccgt gacgaccttg agcgactcaa 300
   gaagatcagg gcccacagag gcatgaggca ctactggggt ttgagggtgc gtggtcaaca 360
   cacgaagacc accggaagga gaggacgaac tgttggtgtg tccaagaaga agtaatttta 420
   atttcctaat aaattggttt tttcaaaaaa aaaaaaaaaa aaaaaaaaaa aaaa 474
<210> 14
   <211> 332
   <212> DNA
   <213> Lygus hesperus
<400> 14
   gaaaggtgat gttcgccatg accgccatca aaggtgtcgg cagacggtac gccaacattg 60
   tcctcaagaa ggccgatgtc aacttggaca agagggccgg cgaatgctcc gaagaagaag 120
   ttgaaaagat cgttaccatc atgcaaaacc ctaggcaata caaaattccc aactggttcc 180
   tcaacagaca aaaagacacc gtcgagggca aatactctca gttgacttcc tccctgctgg 240
   attccaagct ccgtgacgac cttgagcgac tcaagaagat cagggcccac agaggcatga 300
   ggcactactg ggtttgaggg gtgcgtggtc aa 332
<210> 15
   <211> 440
   <212> DNA
   <213> Lygus hesperus
<400> 15
   gtgagttctt ctgttgatta gtttttcctt ccctgaaatt atttcgttga agttaatttg 60
   gattaccctg aaagaatccg ctgctttttc tctcgctaaa aatcttttac acccgtcacc 120
   acggccccct gtgggcaggc acaagctgaa gcacctgccc gtgcacccta actcgcactt 180
   catggacgtc aactgccctg ggtgttataa aatcccaacg gtgttctccc ccgcccagaa 240
   cgacttcggc tgctggacct gttccaccat cctctgcctg cccacagggg gccgtgccga 300
   cctcaccaaa agatgctcgt ttaggagaaa tcaacattat tattcttggt gggaacactt 360
   attttttttg taattaaatt tcaaactaca aaataacttt tccgaaaaac actacaaaaa 420
   aaattaaaaa caaaaaaaaa 440
<210> 16
   <211> 324
   <212> DNA
   <213> Lygus hesperus
<400> 16
   cttccctgaa attatttcgt tgaagttaat ttggattacc ctgaaagaat ccgctgcttt       60
   ttctctcgct aaaaatcttt tacacccgtc accacggccc cctgtgggca ggcacaagct      120
   gaagcacctg cccgtgcacc ctaactcgca cttcatggac gtcaactgcc ctgggtgtta      180
   taaaatccca acggtgttct cccccgccca gaacgacttc ggctgctgga cctgttccac      240
   catcctctgc ctgcccacag ggggccgtgc cgacctcacc aaaagatgct cgtttaggag       300
   aaatcaacat tattattctt ggtg                                             324
<210> 17
   <211> 357
   <212> DNA
   <213> Lygus hesperus
<400> 17
   atgggttcaa gagagttaaa gccaagaggg ccaagaagga cgacggtgag atatttgccg       60
   ctaaaaagga agtctacaag ccctctgagc agaggaaagc agaccagaaa aacattgaca      120
   aacagaccct gaaagccatc aagcgactca agggagacgc ttgcctcatg aggaaatacc      180
   tttgcaccat gttcggattc aggagcagtc aatatcccca ccgtatgaag ttttaatatg      240
   ttttcagcca ataaataagt gaaagtttct cttttttatt actacagact caaattttta      300
   ttttctgaaa attattaaaa attcttaatg gcaaaaaaaa aaaaaaaaaa aaaaaaa         357
<210> 18
   <211> 223
   <212> DNA
   <213> Lygus hesperus
<400> 18
   gttcaagaga gttaaagcca agagggccaa gaaggacgac ggtgagatat ttgccgctaa       60
   aaaggaagtc tacaagccct ctgagcagag gaaagcagac cagaaaaaca ttgacaaaca      120
   gaccctgaaa gccatcaagc gactcaaggg agacgcttgc ctcatgagga aatacctttg      180
   caccatgttc ggattcagga gcagtcaata tccccaccgt atg                        223
<210> 19
   <211> 632
   <212> DNA
   <213> Lygus hesperus
<400> 19
   atgggacctt ttttccgtgt gtctggctta ggcctcgcgt gttcttgtat ttttacggga      60
   aatttagtga aaaagtgtaa atttaacgcg taaaaatggg tcgtatgcac gcacctggta     120
   agggtatttc ccagtcagct ctcccctatc gtcgtagcgt cccaacatgg ctgaagctca     180
   ctcctgacga cgtcaaggat cagattttca aactcaccaa gaaaggactg actccatctc     240
   agatcggtgt catcctcagg gattctcacg gtgtggctca agtcagattc gtcaccgggt     300
   cgaagatcct caggatcatg aaagccatcg gcctcgctcc tgacctccca gaggacctct     360
   acttcctcat caaaaaagcc gttgctatca ggaaacatct tgaaagaaat aggaaagaca     420
   aagactctaa attcggactt atccccgtcg agtccaggat ccacaggttg gcaagatact     480
   acaaaaccaa gggcaccctt ccacccacct ggaaatacga gtccagcacc gcctctgctc     540
   tggtggcttg aatattcaac tttttatttg tctactgttt aattaatata atgtgattta     600
   gcaaaaaaaa aaaaaaaaaa aaaaaaaaaa aa                                   632
<210> 20
   <211> 457
   <212> DNA
   <213> Lygus hesperus
<400> 20
   gggtcgtatg cacgcacctg gtaagggtat ttcccagtca gctctcccct atcgtcgtag      60
   cgtcccaaca tggctgaagc tcactcctga cgacgtcaag gatcagattt tcaaactcac     120
   caagaaagga ctgactccat ctcagatcgg tgtcatcctc agggattctc acggtgtggc     180
   tcaagtcaga ttcgtcaccg ggtcgaagat cctcaggatc atgaaagcca tcggcctcgc     240
   tcctgacctc ccagaggacc tctacttcct catcaaaaaa gccgttgcta tcaggaaaca     300
   tcttgaaaga aataggaaag acaaagactc taaattcgga cttatccccg tcgagtccag     360
   gatccacagg ttggcaagat actacaaaac caagggcacc cttccaccca cctggaaata     420
   cgagtccagc accgcctctg ctctggtggc ttgaata                              457
<210> 21
   <211> 407
   <212> DNA
   <213> Lygus hesperus
<400> 21
   atgggaccgt ttgcctcaca atccagaaca gacaggctgc catatccgtc gtcccctctg        60
   cagcctccct cgtaatcaag gccctcaaag agcccccgag ggacaggaag aagaacaaga       120
   acatcaaaca cgacggtaac ctgagtatgg atgacattct cggaattgcc aaaaccatga       180
   ggccgaggtc gatgtccagg aaactggaag gaaccgtcaa ggaaatcctt gggacagctc       240
   agtctgtcgg atgcacgatc gaaggccgag ctccccacga cgtcatcgac tccatcaaca       300
   acggcgaaat ggaaatccct gacgaataaa ctgttcatga gtttatggat tttatataaa       360
   aaataaaaag ttgaaaaatc caaaaaaaaa aaaaaaaaag aaaaaaa                     407
<210> 22
   <211> 302
   <212> DNA
   <213> Lygus hesperus
<400> 22
   accgtttgcc tcacaatcca gaacagacag gctgccatat ccgtcgtccc ctctgcagcc        60
   tccctcgtaa tcaaggccct caaagagccc ccgagggaca ggaagaagaa caagaacatc       120
   aaacacgacg gtaacctgag tatggatgac attctcggaa ttgccaaaac catgaggccg       180
   aggtcgatgt ccaggaaact ggaaggaacc gtcaaggaaa tccttgggac agctcagtct       240
   gtcggatgca cgatcgaagg ccgagctccc cacgacgtca tcgactccat caacaacggc       300
   ga                                                                      302
<210> 23
   <211> 794
   <212> DNA
   <213> Lygus hesperus
<400> 23
   catggggagt caatttggat ctatcgccag atgaagatgt ctcctgccgt gttcgctgtt      60
   ctgctggtac tttcagcttc ccaggtcttg ggagatgatg catccaagtt ccaacacgag     120
   gaaatcatgg aagtcctcag ctcggtcaac aaaaccgtca acaaattgta cgacttgatg     180
   tccacgcaga aggaaagaga tattgacttt atcgagaaga aaatggatga gacgtaccag     240
   caactcagga acaagaggga ggcgccggct gagaaccctg aagccattga caagatccaa     300
   aacgcgttca aaagctttca agacggcgtc aaggacttcg tcaagtccgc ttcttcctcg     360
   gacctctaca agaaggttca ggaaatcggc gaggacctgt agaacaaagg caaagagctc     420
   ggagagaagc tgcaagaaac catcaataac gccagaacga aaaactcaga cgagaagaag     480
   gactaaactg aggattttga ctctgcacaa acgcccgttg gtgtttaaac gtatttctta     540
   cgtttattat catcggggtt catgaaatca aaaatacacc atcgcatacc acctcgaaaa     600
   gaacataata tatgtgaaaa gacaagaaaa ggtgttcaat tgtgtcttta actggtggtt     660
   atcacgattc acatgaaata ctactaagaa aacccaaaaa ccgtcatgaa acccgaagta     720
   tgcttctgta ttacctaatt gtgctgataa ttcttaataa aatattatac tgagaaaaaa     780
   aaaaaaaaaa aaaa                                                       794
<210> 24
   <211> 278
   <212> DNA
   <213> Lygus hesperus
<400> 24
   ttctgctggt actttcagct tcccaggtct tgggagatga tgcatccaag ttccaacacg      60
   aggaaatcat ggaagtcctc agctcggtca acaaaaccgt caacaaattg tacgacttga     120
   tgtccacgca gaaggaaaga gatattgact ttatcgagaa gaaaatggat gagacgtacc     180
   agcaactcag gaacaagagg gaggcgccgg ctgagaaccc tgaagccatt gacaagatcc     240
   aaaacgcgtt caaaagcttt caagacggcg tcaaggac                             278
<210> 25
   <211> 437
   <212> DNA
   <213> Lygus hesperus
<400> 25
   atgggatcca ataataacca ttaaggcaat tggacatcaa tgatactgaa catatgaata 60
   ttcagatatc aaaaatatcg aaatagaatc atatataaaa ccaactaacg cattagaaaa 120
   taacgaattc cgattacttg aagtagacaa tcgaatcgta ttacctataa aatcaactat 180
   ccgaattcta gttacatcat ctgatgtaat tcattcatga accatcccaa gtttgggaat 240
   caaaattgat ggcacaccag gacgattaaa tcaagggaga ataaacataa accgaccagg 300
   actaatatat gggcaatgtt ctgaaatttg tggagcaaac cacagattta taccaatcgt 360
   aattgaaaga gtttcaatta atcaatttat aaactgatta aattcaaaat aaaaaaaaaa 420
   aaaaaaaaaa aaaaaaa 437
<210> 26
   <211> 327
   <212> DNA
   <213> Lygus hesperus
<400> 26
   aacgcagagt acatgggatc caataataac cattaaggca attggacatc aatgatactg 60
   aacatatgaa tattcagata tcaaaaatat cgaaatagaa tcatatataa aaccaactaa 120
   cgcattagaa aataacgaat tccgattact tgaagtagac aatcgaatcg tattacctat 180
   aaaatcaact atccgaattc tagttacatc atctgatgta attcattcat gaaccatccc 240
   aagtttggga atcaaaattg atggcacacc aggacgatta aatcaaggga gaataaacat 300
   aaaccgacca ggactaatat atgggca 327
<210> 27
   <211> 42
   <212> DNA
   <213> Artificial
<220>
   <223> Primer
<400> 27
   gcgtaatacg actcactata ggcaaacagg ccgaaatcga ga 42
<210> 28
   <211> 23
   <212> DNA
   <213> Artificial
<220>
   <223> Primer
<400> 28
   tgacgacctt gagttggttt ctg 23
<210> 29
   <211> 20
   <212> DNA
   <213> Artificial
<220>
   <223> Primer
<400> 29
   caaacaggcc gaaatcgaga 20
<210> 30
   <211> 45
   <212> DNA
   <213> Artificial
<220>
   <223> Primer
<400> 30
   gcgtaatacg actcactata ggtgacgacc ttgagttggt ttctg 45
<210> 31
   <211> 42
   <212> DNA
   <213> Artificial
<220>
   <223> Primer
<400> 31
   gcgtaatacg actcactata gggggcatca tgtcgaaagc tg 42
<210> 32
   <211> 20
   <212> DNA
   <213> Artificial
<220>
   <223> Primer
<400> 32
   cgaagcccat gtcatcatcg 20
<210> 33
   <211> 20
   <212> DNA
   <213> Artificial
<220>
   <223> Primer
<400> 33
   gggcatcatg tcgaaagctg 20
<210> 34
   <211> 42
   <212> DNA
   <213> Artificial
<220>
   <223> Primer
<400> 34
   gcgtaatacg actcactata ggcgaagccc atgtcatcat cg 42
<210> 35
   <211> 44
   <212> DNA
   <213> Artificial
<220>
   <223> Primer
<400> 35
   gcgtaatacg actcactata gggggcaggt cttctccata acca 44
<210> 36
   <211> 20
   <212> DNA
   <213> Artificial
<220>
   <223> Primer
<400> 36
   gattttgcgg tgccaacgac 20
<210> 37
   <211> 22
   <212> DNA
   <213> Artificial
<220>
   <223> Primer
<400> 37
   gggcaggtct tctccataac ca 22
<210> 38
   <211> 42
   <212> DNA
   <213> Artificial
<220>
   <223> Primer
<400> 38
   gcgtaatacg actcactata gggattttgc ggtgccaacg ac 42
<210> 39
   <211> 44
   <212> DNA
   <213> Artificial
<220>
   <223> Primer
<400> 39
   gcgtaatacg actcactata ggattgcaca agctgaatcc ctcc 44
<210> 40
   <211> 20
   <212> DNA
   <213> Artificial
<220>
   <223> Primer
<400> 40
   attcacaaag cgggtgctgg 20
<210> 41
   <211> 22
   <212> DNA
   <213> Artificial
<220>
   <223> Primer
<400> 41
   attgcacaag ctgaatccct cc 22
<210> 42
   <211> 42
   <212> DNA
   <213> Artificial
<220>
   <223> Primer
<400> 42
   gcgtaatacg actcactata ggattcacaa agcgggtgct gg 42
<210> 43
   <211> 44
   <212> DNA
   <213> Artificial
<220>
   <223> Primer
<400> 43
   gcgtaatacg actcactata ggccctctac gagtgcatca atgg 44
<210> 44
   <211> 20
   <212> DNA
   <213> Artificial
<220>
   <223> Primer
<400> 44
   cccatcgtgg atttgacgtg 20
<210> 45
   <211> 22
   <212> DNA
   <213> Artificial
<220>
   <223> Primer
<400> 45
   ccctctacga gtgcatcaat gg 22
<210> 46
   <211> 42
   <212> DNA
   <213> Artificial
<220>
   <223> Primer
<400> 46
   gcgtaatacg actcactata ggcccatcgt ggatttgacg tg 42
<210> 47
   <211> 47
   <212> DNA
   <213> Artificial
<220>
   <223> Primer
<400> 47
   gcgtaatacg actcactata ggccaataaa gatcaacttt cccagag 47
<210> 48
   <211> 25
   <212> DNA
   <213> Artificial
<220>
   <223> Primer
<400> 48
   ttagacagac tcttgaactg gaggc 25
<210> 49
   <211> 25
   <212> DNA
   <213> Artificial
<220>
   <223> Primer
<400> 49
   ccaataaaga tcaactttcc cagag 25
<210> 50
   <211> 47
   <212> DNA
   <213> Artificial
<220>
   <223> Primer
<400> 50
   gcgtaatacg actcactata ggttagacag actcttgaac tggaggc 47
<210> 51
   <211> 44
   <212> DNA
   <213> Artificial
<220>
   <223> Primer
<400> 51
   gcgtaatacg actcactata gggaaaggtg atgttcgcca tgac 44
<210> 52
   <211> 20
   <212> DNA
   <213> Artificial
<220>
   <223> Primer
<400> 52
   ttgaccacgc accctcaaac 20
<210> 53
   <211> 22
   <212> DNA
   <213> Artificial
<220>
   <223> Primer
<400> 53
   gaaaggtgat gttcgccatg ac 22
<210> 54
   <211> 42
   <212> DNA
   <213> Artificial
<220>
   <223> Primer
<400> 54
   gcgtaatacg actcactata ggttgaccac gcaccctcaa ac 42
<210> 55
   <211> 47
   <212> DNA
   <213> Artificial
<220>
   <223> Primer
<400> 55
   gcgtaatacg actcactata ggcttccctg aaattatttc gttgaag 47
<210> 56
   <211> 28
   <212> DNA
   <213> Artificial
<220>
   <223> Primer
<400> 56
   caccaagaat aataatgttg atttctcc 28
<210> 57
   <211> 25
   <212> DNA
   <213> Artificial
<220>
   <223> Primer
<400> 57
   cttccctgaa attatttcgt tgaag 25
<210> 58
   <211> 50
   <212> DNA
   <213> Artificial
<220>
   <223> Primer
<400> 58
   gcgtaatacg actcactata ggcaccaaga ataataatgt tgatttctcc 50
<210> 59
   <211> 47
   <212> DNA
   <213> Artificial
<220>
   <223> Primer
<400> 59
   gcgtaatacg actcactata gggttcaaga gagttaaagc caagagg 47
<210> 60
   <211> 22
   <212> DNA
   <213> Artificial
<220>
   <223> Primer
<400> 60
   catacggtgg ggatattgac tg 22
<210> 61
   <211> 25
   <212> DNA
   <213> Artificial
<220>
   <223> Primer
<400> 61
   gttcaagaga gttaaagcca agagg 25
<210> 62
   <211> 44
   <212> DNA
   <213> Artificial
<220>
   <223> Primer
<400> 62
   gcgtaatacg actcactata ggcatacggt ggggatattg actg 44
<210> 63
   <211> 42
   <212> DNA
   <213> Artificial
<220>
   <223> Primer
<400> 63
   gcgtaatacg actcactata gggggtcgta tgcacgcacc tg 42
<210> 64
   <211> 23
   <212> DNA
   <213> Artificial
<220>
   <223> Primer
<400> 64
   tattcaagcc accagagcag agg 23
<210> 65
   <211> 20
   <212> DNA
   <213> Artificial
<220>
   <223> Primer
<400> 65
   gggtcgtatg cacgcacctg 20
<210> 66
   <211> 45
   <212> DNA
   <213> Artificial
<220>
   <223> Primer
<400> 66
   gcgtaatacg actcactata ggtattcaag ccaccagagc agagg 45
<210> 67
   <211> 42
   <212> DNA
   <213> Artificial
<220>
   <223> Primer
<400> 67
   gcgtaatacg actcactata ggaccgtttg cctcacaatc ca 42
<210> 68
   <211> 20
   <212> DNA
   <213> Artificial
<220>
   <223> Primer
<400> 68
   tcgccgttgt tgatggagtc 20
<210> 69
   <211> 20
   <212> DNA
   <213> Artificial
<220>
   <223> Primer
<400> 69
   accgtttgcc tcacaatcca 20
<210> 70
   <211> 42
   <212> DNA
   <213> Artificial
<220>
   <223> Primer
<400> 70
   gcgtaatacg actcactata ggtcgccgtt gttgatggag tc 42
<210> 71
   <211> 45
   <212> DNA
   <213> Artificial
<220>
   <223> Primer
<400> 71
   gcgtaatacg actcactata ggggtatcaa cgcagagtac atggg 45
<210> 72
   <211> 20
   <212> DNA
   <213> Artificial
<220>
   <223> Primer
<400> 72
   gtccttgacg ccgtcttgaa 20
<210> 73
   <211> 23
   <212> DNA
   <213> Artificial
<220>
   <223> Primer
<400> 73
   ggtatcaacg cagagtacat ggg 23
<210> 74
   <211> 42
   <212> DNA
   <213> Artificial
<220>
   <223> Primer
<400> 74
   gcgtaatacg actcactata gggtccttga cgccgtcttg aa 42
<210> 75
   <211> 44
   <212> DNA
   <213> Artificial
<220>
   <223> Primer
<400> 75
   gcgtaatacg actcactata ggtgcccata tattagtcct ggtc 44
<210> 76
   <211> 20
   <212> DNA
   <213> Artificial
<220>
   <223> Primer
<400> 76
   aacgcagagt acatgggatc 20
<210> 77
   <211> 22
   <212> DNA
   <213> Artificial
<220>
   <223> Primer
<400> 77
   tgcccatata ttagtcctgg tc 22
<210> 78
   <211> 42
   <212> DNA
   <213> Artificial
<220>
   <223> Primer
<400> 78
   gcgtaatacg actcactata ggaacgcaga gtacatggga tc 42
<210> 79
   <211> 197
   <212> PRT
   <213> Lygus hesperus
<400> 79
   Met Ala Asp Asp Glu Ala Lys Lys Ala Lys Gln Ala Glu Ile Glu Arg
   1               5                   10                  15
   Lys Arg Ala Glu Val Arg Lys Arg Met Glu Glu Ala Ser Lys Ala Lys
               20                  25                  30
   Lys Ala Lys Lys Gly Phe Met Thr Pro Glu Arg Lys Lys Lys Leu Arg
           35                  40                  45
   Leu Leu Leu Arg Lys Lys Ala Ala Glu Glu Leu Lys Lys Glu Gln Glu
       50                  55                  60
   Arg Lys Ala Ala Glu Arg Arg Arg Thr Ile Glu Glu Arg Cys Gly Gln
   65                  70                  75                  80
   Ile Ala Asp Val Asp Asn Ala Asn Glu Ala Thr Leu Lys Lys Leu Cys
                   85                  90                  95
   Thr Asp Tyr His Lys Arg Ile Asp Ala Leu Glu Arg Ser Lys Ile Asp
               100                 105                 110
   Ile Glu Phe Glu Val Glu Arg Arg Asp Leu Glu Ile Ala Asp Leu Asn
           115                 120                 125
   Ser Gln Val Asn Asp Leu Arg Gly Lys Phe Val Lys Pro Thr Leu Lys
       130                 135                 140
   Lys Val Ser Lys Tyr Glu Asn Lys Phe Ala Lys Leu Gln Lys Lys Ala
   145                 150                 155                 160
   Ala Glu Phe Asn Phe Arg Asn Gln Leu Lys Val Val Lys Lys Lys Glu
                   165                 170                 175
   Phe Thr Leu Glu Glu Glu Asp Lys Glu Pro Lys Lys Ser Glu Lys Ala
               180                 185                 190
   Glu Trp Gln Lys Lys
           195
<210> 80
   <211> 115
   <212> PRT
   <213> Lygus hesperus
<400> 80
   Met Gly Ile Met Ser Lys Ala Glu Leu Ala Cys Val Tyr Ser Ala Leu
   1               5                   10                  15
   Ile Leu Ile Asp Asp Asp Val Ala Val Thr Gly Glu Lys Ile Gln Thr
               20                  25                  30
   Ile Leu Lys Ala Ala Ser Val Asp Ile Glu Pro Tyr Trp Pro Gly Leu
           35                  40                  45
   Phe Ala Lys Ala Leu Glu Gly Ile Asn Pro Lys Asp Leu Ile Ser Ser
       50                  55                  60
   Ile Gly Ser Gly Val Gly Ala Gly Ala Pro Ala Val Gly Gly Ala Ala
   65                  70                  75                  80
   Pro Ala Ala Ala Ala Ala Pro Ala Ala Glu Ala Lys Lys Glu Glu Lys
                   85                  90                  95
   Lys Lys Val Glu Ser Asp Pro Glu Ser Asp Asp Asp Met Gly Phe Gly
               100                 105                 110
   Leu Phe Asp
           115
<210> 81
   <211> 121
   <212> PRT
   <213> Lygus hesperus
<400> 81
   Met Ile Pro Pro Thr Ser Arg Pro Gln Val Thr Val Tyr Ser Asp Lys
   1               5                   10                  15
   Asn Glu Ala Thr Gly Thr Leu Leu Asn Leu Pro Ala Val Phe Asn Ala
               20                  25                  30
   Pro Ile Arg Pro Asp Val Val Asn Phe Val His Gln Asn Val Ala Lys
           35                  40                  45
   Asn His Arg Gln Pro Tyr Cys Val Ser Ala Gln Ala Gly His Gln Thr
       50                  55                  60
   Ser Ala Glu Ser Trp Gly Thr Gly Arg Ala Val Ala Arg Ile Pro Arg
   65                  70                  75                  80
   Val Arg Gly Gly Gly Thr His Arg Ser Gly Gln Gly Ala Phe Gly Asn
                   85                  90                  95
   Met Cys Arg Gly Gly Arg Met Phe Ala Pro Thr Arg Pro Trp Arg Arg
               100                 105                 110
   Trp His Arg Lys Ile Asn Val Asn Gln
           115                 120
<210> 82
   <211> 133
   <212> PRT
   <213> Lygus hesperus
<400> 82
   Trp Asp Leu Tyr Ala Glu Lys Val Ala Thr Arg Gly Leu Cys Ala Ile
   1               5                   10                  15
   Ala Gln Ala Glu Ser Leu Arg Tyr Lys Leu Ile Gly Gly Leu Ala Val
               20                  25                  30
   Arg Gly Ala Cys Tyr Gly Val Leu Arg Phe Ile Met Glu Asn Gly Ala
           35                  40                  45
   Lys Gly Cys Glu Val Val Val Ser Gly Lys Leu Arg Gly Gln Arg Ala
       50                  55                  60
   Lys Ser Met Lys Phe Val Asp Gly Leu Met Ile His Ser Gly Asp Pro
   65                  70                  75                  80
   Cys Asn Glu Tyr Val Asp Thr Ala Thr Arg His Val Leu Leu Arg Gln
                   85                  90                  95
   Gly Val Leu Gly Ile Lys Val Lys Ile Met Leu Pro Trp Asp Val Thr
               100                 105                 110
   Gly Lys Asn Gly Pro Lys Asn Pro Leu Pro Asp His Val Ser Val Leu
           115                 120                 125
   Leu Pro Lys Glu Glu
       130
<210> 83
   <211> 217
   <212> PRT
   <213> Lygus hesperus
<400> 83
   Met Thr Ser Lys Val Ser Arg Glu Thr Leu Tyr Glu Cys Ile Asn Gly
   1 5 10 15
   Val Ile Gln Ser Ser Gln Glu Lys Lys Arg Asn Phe Val Glu Thr Val
   20 25 30
   Glu Ile Gln Ile Gly Leu Lys Asn Tyr Asp Pro Gln Lys Asp Lys Arg
   35 40 45
   Phe Ser Gly Thr Val Lys Leu Lys His Ile Pro Arg Pro Lys Met Gln
   50 55 60
   Val Cys Ile Leu Gly Asp Gln Gln His Cys Asp Glu Ala Lys Ala Asn
   65 70 75 80
   Asn Val Pro Tyr Met Asp Val Glu Ala Leu Lys Lys Leu Asn Lys Asn
   85 90 95
   Lys Lys Leu Val Lys Lys Leu Ala Lys Lys Tyr Asp Ala Phe Leu Ala
   100 105 110
   Ser Glu Ala Leu Ile Lys Gln Ile Pro Arg Leu Leu Gly Pro Gly Leu
   115 120 125
   Asn Lys Ala Gly Lys Phe Pro Gly Leu Leu Ser His Gln Glu Ser Met
   130 135 140
   Met Met Lys Ile Asp Glu Val Lys Ala Thr Ile Lys Phe Gln Met Lys
   145 150 155 160
   Lys Val Leu Cys Leu Ser Val Ala Val Gly His Val Gly Met Thr Ala
   165 170 175
   Asp Glu Leu Val Gln Asn Val His Leu Ser Val Asn Phe Leu Val Ser
   180 185 190
   Leu Leu Lys Lys His Trp Gln Asn Val Arg Ser Leu His Val Lys Ser
   195 200 205
   Thr Met Gly Pro Pro Gln Arg Leu Tyr
   210 215
<210> 84
   <211> 118
   <212> PRT
   <213> Lygus hesperus
<400> 84
   Gly Thr Asn Lys Asp Gln Leu Ser Gln Arg Lys Thr Cys Tyr Ala Gln
   1 5 10 15
   His Asn Gln Val Arg Glu Ile Arg Lys Lys Met Val Lys Asn Ile Ser
   20 25 30
   Asp Ser Ile Ser Ser Cys Asp Leu Arg Ser Val Val Asn Lys Leu Ile
   35 40 45
   Pro Asp Ser Ile Ala Lys Asp Ile Glu Lys Asn Cys Gln Gly Ile Tyr
   50 55 60
   Pro Leu His Asp Val Tyr Ile Arg Lys Val Lys Val Leu Lys Lys Pro
   65 70 75 80
   Arg Phe Glu Leu Ser Lys Leu Leu Glu Leu His Val Asp Gly Lys Gly
   85 90 95
   Ile Asp Glu Pro Gly Ala Lys Val Thr Arg Thr Asp Ala Tyr Glu Pro
   100 105 110
   Pro Val Gln Glu Ser Val
   115
<210> 85
   <211> 128
   <212> PRT
   <213> Lygus hesperus
<400> 85
   Lys Val Met Phe Ala Met Thr Ala Ile Lys Gly Val Gly Arg Arg Tyr
   1 5 10 15
   Ala Asn Ile Val Leu Lys Lys Ala Asp Val Asn Leu Asp Lys Arg Ala
   20 25 30
   Gly Glu Cys Ser Glu Glu Glu Val Glu Lys Ile Val Thr Ile Met Gln
   35 40 45
   Asn Pro Arg Gln Tyr Lys Ile Pro Asn Trp Phe Leu Asn Arg Gln Lys
   50 55 60
   Asp Thr Val Glu Gly Lys Tyr Ser Gln Leu Thr Ser Ser Leu Leu Asp
   65 70 75 80
   Ser Lys Leu Arg Asp Asp Leu Glu Arg Leu Lys Lys Ile Arg Ala His
                   85                  90                  95
   Arg Gly Met Arg His Tyr Trp Gly Leu Arg Val Arg Gly Gln His Thr
               100                 105                 110
   Lys Thr Thr Gly Arg Arg Gly Arg Thr Val Gly Val Ser Lys Lys Lys
           115                 120                 125
<210> 86
   <211> 122
   <212> PRT
   <213> Lygus hesperus
<400> 86
   Val Leu Leu Leu Ile Ser Phe Ser Phe Pro Glu Ile Ile Ser Leu Lys
   1               5                   10                  15
   Leu Ile Trp Ile Thr Leu Lys Glu Ser Ala Ala Phe Ser Leu Ala Lys
               20                  25                  30
   Asn Leu Leu His Pro Ser Pro Arg Pro Pro Val Gly Arg His Lys Leu
           35                  40                  45
   Lys His Leu Pro Val His Pro Asn Ser His Phe Met Asp Val Asn Cys
       50                  55                  60
   Pro Gly Cys Tyr Lys Ile Pro Thr Val Phe Ser Pro Ala Gln Asn Asp
   65                  70                  75                  80
   Phe Gly Cys Trp Thr Cys Ser Thr Ile Leu Cys Leu Pro Thr Gly Gly
                   85                  90                  95
   Arg Ala Asp Leu Thr Lys Arg Cys Ser Phe Arg Arg Asn Gln His Tyr
               100                 105                 110
   Tyr Ser Trp Trp Glu His Leu Phe Phe Leu
           115                 120
<210> 87
   <211> 77
   <212> PRT
   <213> Lygus hesperus
<400> 87
   Gly Phe Lys Arg Val Lys Ala Lys Arg Ala Lys Lys Asp Asp Gly Glu
   1               5                   10                  15
   Ile Phe Ala Ala Lys Lys Glu Val Tyr Lys Pro Ser Glu Gln Arg Lys
               20                  25                  30
   Ala Asp Gln Lys Asn Ile Asp Lys Gln Thr Leu Lys Ala Ile Lys Arg
           35                  40                  45
   Leu Lys Gly Asp Ala Cys Leu Met Arg Lys Tyr Leu Cys Thr Met Phe
       50                  55                  60
   Gly Phe Arg Ser Ser Gln Tyr Pro His Arg Met Lys Phe
   65                  70                  75
<210> 88
   <211> 151
   <212> PRT
   <213> Lygus hesperus
<400> 88
   Met Gly Arg Met His Ala Pro Gly Lys Gly Ile Ser Gln Ser Ala Leu
   1               5                   10                  15
   Pro Tyr Arg Arg Ser Val Pro Thr Trp Leu Lys Leu Thr Pro Asp Asp
               20                  25                  30
   Val Lys Asp Gln Ile Phe Lys Leu Thr Lys Lys Gly Leu Thr Pro Ser
           35                  40                  45
   Gln Ile Gly Val Ile Leu Arg Asp Ser His Gly Val Ala Gln Val Arg
       50                  55                  60
   Phe Val Thr Gly Ser Lys Ile Leu Arg Ile Met Lys Ala Ile Gly Leu
   65                  70                  75                  80
   Ala Pro Asp Leu Pro Glu Asp Leu Tyr Phe Leu Ile Lys Lys Ala Val
                   85                  90                  95
   Ala Ile Arg Lys His Leu Glu Arg Asn Arg Lys Asp Lys Asp Ser Lys
               100                 105                 110
   Phe Gly Leu Ile Pro Val Glu Ser Arg Ile His Arg Leu Ala Arg Tyr
           115                 120                 125
   Tyr Lys Thr Lys Gly Thr Leu Pro Pro Thr Trp Lys Tyr Glu Ser Ser
       130                 135                 140
   Thr Ala Ser Ala Leu Val Ala
   145                 150
<210> 89
   <211> 108
   <212> PRT
   <213> Lygus hesperus
<400> 89
   Gly Thr Val Cys Leu Thr Ile Gln Asn Arg Gln Ala Ala Ile Ser Val
   1               5                   10                  15
   Val Pro Ser Ala Ala Ser Leu Val Ile Lys Ala Leu Lys Glu Pro Pro
               20                  25                  30
   Arg Asp Arg Lys Lys Asn Lys Asn Ile Lys His Asp Gly Asn Leu Ser
           35                  40                  45
   Met Asp Asp Ile Leu Gly Ile Ala Lys Thr Met Arg Pro Arg Ser Met
       50                  55                  60
   Ser Arg Lys Leu Glu Gly Thr Val Lys Glu Ile Leu Gly Thr Ala Gln
   65                  70                  75                  80
   Ser Val Gly Cys Thr Ile Glu Gly Arg Ala Pro His Asp Val Ile Asp
                   85                  90                  95
   Ser Ile Asn Asn Gly Glu Met Glu Ile Pro Asp Glu
               100                 105
<210> 90
   <211> 133
   <212> PRT
   <213> Lygus hesperus
<400> 90
   His Gly Glu Ser Ile Trp Ile Tyr Arg Gln Met Lys Met Ser Pro Ala
   1               5                   10                  15
   Val Phe Ala Val Leu Leu Val Leu Ser Ala Ser Gln Val Leu Gly Asp
               20                  25                  30
   Asp Ala Ser Lys Phe Gln His Glu Glu Ile Met Glu Val Leu Ser Ser
           35                  40                  45
   Val Asn Lys Thr Val Asn Lys Leu Tyr Asp Leu Met Ser Thr Gln Lys
       50                  55                  60
   Glu Arg Asp Ile Asp Phe Ile Glu Lys Lys Met Asp Glu Thr Tyr Gln
   65                  70                  75                  80
   Gln Leu Arg Asn Lys Arg Glu Ala Pro Ala Glu Asn Pro Glu Ala Ile
                   85                  90                  95
   Asp Lys Ile Gln Asn Ala Phe Lys Ser Phe Gln Asp Gly Val Lys Asp
               100                 105                 110
   Phe Val Lys Ser Ala Ser Ser Ser Asp Leu Tyr Lys Lys Val Gln Glu
   115 120 125
   Ile Gly Glu Asp Leu
   130
<210> 91
   <211> 118
   <212> PRT
   <213> Lygus hesperus
<400> 91
   Thr Tyr Glu Tyr Ser Asp Ile Lys Asn Ile Glu Ile Glu Ser Tyr Ile
   1 5 10 15
   Lys Pro Thr Asn Ala Leu Glu Asn Asn Glu Phe Arg Leu Leu Glu Val
   20 25 30
   Asp Asn Arg Ile Val Leu Pro Ile Lys Ser Thr Ile Arg Ile Leu Val
   35 40 45
   Thr Ser Ser Asp Val Ile His Ser Thr Ile Pro Ser Leu Gly Ile Lys
   50 55 60
   Ile Asp Gly Thr Pro Gly Arg Leu Asn Gln Gly Arg Ile Asn Ile Asn
   65 70 75 80
   Arg Pro Gly Leu Ile Tyr Gly Gln Cys Ser Glu Ile Cys Gly Ala Asn
   85 90 95
   His Arg Phe Ile Pro Ile Val Ile Glu Arg Val Ser Ile Asn Gln Phe
   100 105 110
   Ile Asn Leu Asn Ser Lys
   115
<210> 92
   <211> 20
   <212> DNA
   <213> Artificial
<220>
   <223> Adaptor
<400> 92
   aagcagtggt atcaacgcag 20
<210> 93
   <211> 20
   <212> DNA
   <213> Artificial
<220>
   <223> Primer
<400> 93
   aagcagtggt atcaacgcag 20
<210> 94
   <211> 42
   <212> DNA
   <213> Artificial
<220>
   <223> Primer
<400> 94
   gcgtaatacg actcactata ggaagcagtg gtatcaacgc ag 42
<210> 95
   <211> 717
   <212> DNA
   <213> Lygus hesperus
<400> 95
   aaagtgtggt tctcttcgtc cgaccatgag ttcgctcaaa ctgcagaaga ggctcgccgc 60
   ctcggtgatg agatgcggca agaagaaagt gtggttggac cctaatgaaa tcaacgaaat 120
   cgccaacacc aactctaggc aaaacatccg taagctgatc aaggatggtt tgatcatcaa 180
   aaagcctgtg gctgtccact ccagagcccg cgtccgtaaa aacacagaag ccagacggaa 240
   gggtcgtcat tgtggcttcg gtaagaggaa gggtaccgcc aacgccagaa tgcctgtgaa 300
   ggtcctgtgg gtcaacagaa tgagagtcct gcgacggctc cttaaaaaat acagagaagc 360
   caagaagatc gataggcaaa tgtaccacga cctttacatg aaagccaaag gtaacgtctt 420
   caaaaacaag agggtactga tggacttcat tcacaagaag aaggctgaaa aggcgagatc 480
   aaagatgttg aaggaccagg cagaggcgag acgtttcaag gtcaaggagg cgaagaagag 540
   gcgcgaggag aggatcgcca ccaagaagca agagatcatg caggcgtacg cccgagaaga 600
   cgaggctgcc gtcaaaaagt gatctcgccc cctccgtttt taaattttaa acaaaaaacg 660
   tattttgtac aaaaatttac aaaaaaaaaa aaaaaaaaaa aaaaaaaaaa aaaaaaa 717
<210> 96
   <211> 2304
   <212> DNA
   <213> Lygus hesperus
<400> 96
   atgacgacct acgaggagtt cattcaacag agcgaggagc gcgacggtat caggttcact      60
   tggaacgtct ggccatcaag tcgcatcgaa gccaccaggt tggtcgtacc cgtaggatgt     120
   ctctatcaac cactaaaaga acgcacggat cttccagcta ttcaatacga tcccgttcta     180
   tgcactagga atacctgtag agccatactc aacccgatgt gccaagtaaa ctatagggca     240
   aagttgtggg tgtgtaactt ctgtttccag aggaatccgt tcccaccaca atacgccgca     300
   atttccgagc agcatcagcc tgctgagttg attccatcat tctcaactat agagtatact 360
   atatctagag ctcaattttt gcctcctata ttcctattgg tggtggatac gtgtttggat 420
   gatgacgagc taggagctct gaaagattcg ttacaaacgt ctctatcttt gctaccaacc 480
   aactccctag ttggtctgat cacgtttggt aaaatggtcc aagttcacga acttgggtgt 540
   gaaggttgtt cccggagcta cgtgttcaga ggcaccaagg atttgacgtc caagcaagta 600
   caggacatgc ttgggatcgg aaaggtttcc gcttctcctc agcaacagca gcaaagggca 660
   atgggcggtc agcagccatt ccccaccaat cggttcattc agccgattca aagttgtgac 720
   atgagcctca ccgacttgtt gggcgaaatg cagcgtgatc catggccagt gggtcagggt 780
   aagcgacctc ttagatcaac gggtgctgct ctagctattg ccattgggtt gttggagtgc 840
   tcctacccca acacgggagc aaaagtcatg ttgttccttg gtggcccttg ttcccaaggg 900
   cctggtcaag ttgtcaatga tgacctgagg gaacctatcc gctctcatca tgacatccag 960
   aaagataatg cccgctacat gaaaaaagcc attaaacatt acgattcttt ggcattgaga 1020
   gcagccacta atgggcattc agtagacatt tattcctgtg ctttagatca gacaggtttg 1080
   gcggaaatga agcaatgttg caattctact gggggtcata tggtgatggg tgacaccttc 1140
   aactccactt tgttcaaaca gacgttccag agggtgctct cccgtgatca aaaaggcgaa 1200
   ttcaaaatgg ctttcaatgg cgtagttgaa gtcaaaacct cccgagagct aaaagttatg 1260
   ggagccattg ggccttgcgt ttcattgaat acgaaaggtc cgtgtgttag tgaaactgac 1320
   atagggcttg gaggaacttg ccagtggaag ttctgcacat ttaaccaaaa taccactgct 1380
   gccatgttct ttgaggtagt aaaccaacac gctgctccta tccctcaagg tggaagagga 1440
   tgtatacagt tcataactca ataccagcat gcgtcgggcc aaaggcgcat ccgagtaacc 1500
   actgtagcca ggaattgggc tgatgcgact accaacatgc accatgttag tgcaggattt 1560
   gatcaggaag ctggagcggt actcatggcc aggatggtcg ttcacagagc tgaaactgat 1620
   gatggacctg atgtcatgag atgggctgat cgcatgttga ttcgtctttg ccagaaattc 1680
   ggcgagtaca acaaggatga tccaaatagt ttccgcctcc cagaaaactt ctcgctttac 1740
   ccacagttca tgtatcactt gagaaggtcc caattcttgc aggtattcaa caacagccca 1800
   gacgaaacgt cgtactatcg tcacatcttg atgcgggaag atttgtcgca gagcttgatc 1860
   atgattcagc cgatcctgta cagttacagt ttcaacggtc cagaaccagt ccttttggac 1920
   acttccagca ttcaacctga tcggatcctg ctgatggaca ccttcttcca aatcctcatc 1980
   ttccacggcg agaccatcgc ccagtggcgt gcccaaaggt accaggacct acctgaatat 2040
   gagaacttca agcagctcct acaggctcct gtagacgatg ctaaggaaat cctgcacact 2100
   cggttcccca tgccgaggta cattgacacc gaacagggcg gatcacaagc tagattcctt 2160
   ctctccaaag tcaacccatc ccaaactcac aacaacatgt acggctatgg aggggaattt 2220
   ggagcccctg tgctcactga tgatgtttcc ctccaagtct tcatggaaca ccttaaaaag 2280
   ctagccgttt catttactgc ctag 2304
<210> 97
   <211> 311
   <212> DNA
   <213> Artificial
<220>
   <223> GUS
<400> 97
   ccagcgtatc gtgctgcgtt tcgatgcggt cactcattac ggcaaagtgt gatggagcat 60
   cagggcggct atacgccatt tgaagccgat gtcacgccgt atgttattgc cgggaaaagt 120
   gtacgtatct gaaatcaaaa aactcgacgg cctgtgggca ttcagtctgg atcgcgaaaa 180
   ctgtggaatt gatccagcgc cgtcgtcggt gaacaggtat ggaatttcgc cgattttgcg 240
   acctcgcaag gcatattcgg gtgaaggtta tctctatgaa ctgtgcgtca cagccaaaag 300
   ccagacagag t 311
<210> 98
   <211> 170
   <212> DNA
   <213> Artificial
<220>
   <223> Intron
<400> 98
   ctcgagcctg agagaaaagc atgaagtata cccataacta acccattagt tatgcattta 60
   tgttatatct attcatgctt ctactttaga taatcaatca ccaaacaatg agaatctcaa 120
   cggtcgcaat aatgttcatg aaaatgtagt gtgtacactt accttctaga 170
<210> 99
   <211> 198
   <212> PRT
   <213> Lygus hesperus
<400> 99
   Met Ser Ser Leu Lys Leu Gln Lys Arg Leu Ala Ala Ser Val Met Arg
   1               5                   10                  15
   Cys Gly Lys Lys Lys Val Trp Leu Asp Pro Asn Glu Ile Asn Glu Ile
               20                  25                  30
   Ala Asn Thr Asn Ser Arg Gln Asn Ile Arg Lys Leu Ile Lys Asp Gly
           35                  40                  45
   Leu Ile Ile Lys Lys Pro Val Ala Val His Ser Arg Ala Arg Val Arg
       50                  55                  60
   Lys Asn Thr Glu Ala Arg Arg Lys Gly Arg His Cys Gly Phe Gly Lys
   65                  70                  75                  80
   Arg Lys Gly Thr Ala Asn Ala Arg Met Pro Val Lys Val Leu Trp Val
                   85                  90                  95
   Asn Arg Met Arg Val Leu Arg Arg Leu Leu Lys Lys Tyr Arg Glu Ala
               100                 105                 110
   Lys Lys Ile Asp Arg Gln Met Tyr His Asp Leu Tyr Met Lys Ala Lys
           115                 120                 125
   Gly Asn Val Phe Lys Asn Lys Arg Val Leu Met Asp Phe Ile His Lys
       130                 135                 140
   Lys Lys Ala Glu Lys Ala Arg Ser Lys Met Leu Lys Asp Gln Ala Glu
   145                 150                 155                 160
   Ala Arg Arg Phe Lys Val Lys Glu Ala Lys Lys Arg Arg Glu Glu Arg
                   165                 170                 175
   Ile Ala Thr Lys Lys Gln Glu Ile Met Gln Ala Tyr Ala Arg Glu Asp
               180                 185                 190
   Glu Ala Ala Val Lys Lys
           195
<210> 100
   <211> 767
   <212> PRT
   <213> Lygus hesperus
<400> 100
   Met Thr Thr Tyr Glu Glu Phe Ile Gln Gln Ser Glu Glu Arg Asp Gly
   1               5                   10                  15
   Ile Arg Phe Thr Trp Asn Val Trp Pro Ser Ser Arg Ile Glu Ala Thr
               20                  25                  30
   Arg Leu Val Val Pro Val Gly Cys Leu Tyr Gln Pro Leu Lys Glu Arg
           35                  40                  45
   Thr Asp Leu Pro Ala Ile Gln Tyr Asp Pro Val Leu Cys Thr Arg Asn
       50                  55                  60
   Thr Cys Arg Ala Ile Leu Asn Pro Met Cys Gln Val Asn Tyr Arg Ala
   65                  70                  75                  80
   Lys Leu Trp Val Cys Asn Phe Cys Phe Gln Arg Asn Pro Phe Pro Pro
   85 90 95
   Gln Tyr Ala Ala Ile Ser Glu Gln His Gln Pro Ala Glu Leu Ile Pro
   100 105 110
   Ser Phe Ser Thr Ile Glu Tyr Thr Ile Ser Arg Ala Gln Phe Leu Pro
   115 120 125
   Pro Ile Phe Leu Leu Val Val Asp Thr Cys Leu Asp Asp Asp Glu Leu
   130 135 140
   Gly Ala Leu Lys Asp Ser Leu Gln Thr Ser Leu Ser Leu Leu Pro Thr
   145 150 155 160
   Asn Ser Leu Val Gly Leu Ile Thr Phe Gly Lys Met Val Gln Val His
   165 170 175
   Glu Leu Gly Cys Glu Gly Cys Ser Arg Ser Tyr Val Phe Arg Gly Thr
   180 185 190
   Lys Asp Leu Thr Ser Lys Gln Val Gln Asp Met Leu Gly Ile Gly Lys
   195 200 205
   Val Ser Ala Ser Pro Gln Gln Gln Gln Gln Arg Ala Met Gly Gly Gln
   210 215 220
   Gln Pro Phe Pro Thr Asn Arg Phe Ile Gln Pro Ile Gln Ser Cys Asp
   225 230 235 240
   Met Ser Leu Thr Asp Leu Leu Gly Glu Met Gln Arg Asp Pro Trp Pro
   245 250 255
   Val Gly Gln Gly Lys Arg Pro Leu Arg Ser Thr Gly Ala Ala Leu Ala
   260 265 270
   Ile Ala Ile Gly Leu Leu Glu Cys Ser Tyr Pro Asn Thr Gly Ala Lys
   275 280 285
   Val Met Leu Phe Leu Gly Gly Pro Cys Ser Gln Gly Pro Gly Gln Val
   290 295 300
   Val Asn Asp Asp Leu Arg Glu Pro Ile Arg Ser His His Asp Ile Gln
   305 310 315 320
   Lys Asp Asn Ala Arg Tyr Met Lys Lys Ala Ile Lys His Tyr Asp Ser
   325 330 335
   Leu Ala Leu Arg Ala Ala Thr Asn Gly His Ser Val Asp Ile Tyr Ser
   340 345 350
   Cys Ala Leu Asp Gln Thr Gly Leu Ala Glu Met Lys Gln Cys Cys Asn
           355                 360                 365
   Ser Thr Gly Gly His Met Val Met Gly Asp Thr Phe Asn Ser Thr Leu
       370                 375                 380
   Phe Lys Gln Thr Phe Gln Arg Val Leu Ser Arg Asp Gln Lys Gly Glu
   385                 390                 395                 400
   Phe Lys Met Ala Phe Asn Gly Val Val Glu Val Lys Thr Ser Arg Glu
                   405                 410                 415
   Leu Lys Val Met Gly Ala Ile Gly Pro Cys Val Ser Leu Asn Thr Lys
               420                 425                 430
   Gly Pro Cys Val Ser Glu Thr Asp Ile Gly Leu Gly Gly Thr Cys Gln
           435                 440                 445
   Trp Lys Phe Cys Thr Phe Asn Gln Asn Thr Thr Ala Ala Met Phe Phe
       450                 455                 460
   Glu Val Val Asn Gln His Ala Ala Pro Ile Pro Gln Gly Gly Arg Gly
   465                 470                 475                 480
   Cys Ile Gln Phe Ile Thr Gln Tyr Gln His Ala Ser Gly Gln Arg Arg
                   485                 490                 495
   Ile Arg Val Thr Thr Val Ala Arg Asn Trp Ala Asp Ala Thr Thr Asn
               500                 505                 510
   Met His His Val Ser Ala Gly Phe Asp Gln Glu Ala Gly Ala Val Leu
           515                 520                 525
   Met Ala Arg Met Val Val His Arg Ala Glu Thr Asp Asp Gly Pro Asp
       530                 535                 540
   Val Met Arg Trp Ala Asp Arg Met Leu Ile Arg Leu Cys Gln Lys Phe
   545                 550                 555                 560
   Gly Glu Tyr Asn Lys Asp Asp Pro Asn Ser Phe Arg Leu Pro Glu Asn
                   565                 570                 575
   Phe Ser Leu Tyr Pro Gln Phe Met Tyr His Leu Arg Arg Ser Gln Phe
               580                 585                 590
   Leu Gln Val Phe Asn Asn Ser Pro Asp Glu Thr Ser Tyr Tyr Arg His
           595                 600                 605
   Ile Leu Met Arg Glu Asp Leu Ser Gln Ser Leu Ile Met Ile Gln Pro
       610                 615                 620
   Ile Leu Tyr Ser Tyr Ser Phe Asn Gly Pro Glu Pro Val Leu Leu Asp
   625                 630                 635                 640
   Thr Ser Ser Ile Gln Pro Asp Arg Ile Leu Leu Met Asp Thr Phe Phe
                   645                 650                 655
   Gln Ile Leu Ile Phe His Gly Glu Thr Ile Ala Gln Trp Arg Ala Gln
               660                 665                 670
   Arg Tyr Gln Asp Leu Pro Glu Tyr Glu Asn Phe Lys Gln Leu Leu Gln
           675                 680                 685
   Ala Pro Val Asp Asp Ala Lys Glu Ile Leu His Thr Arg Phe Pro Met
       690                 695                 700
   Pro Arg Tyr Ile Asp Thr Glu Gln Gly Gly Ser Gln Ala Arg Phe Leu
   705                 710                 715                 720
   Leu Ser Lys Val Asn Pro Ser Gln Thr His Asn Asn Met Tyr Gly Tyr
                   725                 730                 735
   Gly Gly Glu Phe Gly Ala Pro Val Leu Thr Asp Asp Val Ser Leu Gln
               740                 745                 750
   Val Phe Met Glu His Leu Lys Lys Leu Ala Val Ser Phe Thr Ala
           755                 760                 765
<210> 101
   <211> 511
   <212> DNA
   <213> Lygus hesperus
<400> 101
   ggtgatgaga tgcggcaaga agaaagtgtg gttggaccct aatgaaatca acgaaatcgc     60
   caacaccaac tctaggcaaa acatccgtaa gctgatcaag gatggtttga tcatcaaaaa    120
   gcctgtggct gtccactcca gagcccgcgt ccgtaaaaac acagaagcca gacggaaggg    180
   tcgtcactgt ggcttcggta agaggaaggg taccgccaac gccagaatgc ctgtgaaggt    240
   cctgtgggtc aacagaatga gagtcctgcg acggctcctt aaaaaataca gagaagccaa    300
   gaagatcgat aggcaaatgt accacgacct ttacatgaaa gccaaaggta acgtcttcaa    360
   aaacaagagg gtactgatgg acttcattca caagaagaag gctgaaaagg cgagatcaaa    420
   gatgttgaag gaccaggcag aggcgagacg tctcaaggtc aaggaggcga agaagaggcg    480
   cgaggagagg atcgccacca agaagcaaga g                                   511
<210> 102
   <211> 1145
   <212> DNA
   <213> Lygus hesperus
<400> 102
   tgggttgttg gagtgctcct accccaacac gggagcaaaa gtcatgttgt tccttggtgg 60
   cccttgttcc caagggcctg gtcaagttgt caatgatgac ctgagggaac ctatccgctc 120
   tcatcatgac atccagaaag ataatgcccg ctacatgaaa aaagccatta aacattacga 180
   ttctttggca ttgagagcag ccactaatgg gcattcagta gacatttatt cctgtgcttt 240
   agatcagaca ggtttggcgg aaatgaagca atgttgcaat tctactgggg gtcatatggt 300
   gatgggtgac accttcaact ccactttgtt caaacagacg ttccagaggg tgctctcccg 360
   tgatcaaaaa ggcgaattca aaatggcttt caatggcgta gttgaagtca aaacctcccg 420
   agagctaaaa gttatgggag ccattgggcc ttgcgtttca ttgaatacga aaggtccgtg 480
   tgttagtgaa actgacatag ggcttggagg aacttgccag tggaagttct gcacatttaa 540
   ccaaaatacc actgctgcca tgttctttga ggtagtaaac caacacgctg ctcctatccc 600
   tcaaggtgga agaggatgta tacagttcat aactcaatac cagcatgcgt cgggccaaag 660
   gcgcatccga gtaaccactg tagccaggaa ttgggctgat gcgactacca acatgcacca 720
   tgttagtgca ggatttgatc aggaagctgg agcggtactc atggccagga tggtcgttca 780
   cagagctgaa actgatgatg gacctgatgt catgagatgg gctgatcgca tgttgattcg 840
   tctttgccag aaattcggcg agtacaacaa ggatgatcca aatagtttcc gcctcccaga 900
   aaacttctcg ctttacccac agttcatgta tcacttgaga aggtcccaat tcttgcaggt 960
   attcaacaac agcccagacg aaacgtcgta ctatcgtcac atcttgatgc gggaagattt 1020
   gtcgcagagc ttgatcatga ttcagccgat cctgtacagt tacagtttca acggtccaga 1080
   accagtcctt ttggacactt ccagcattca acctgatcgg atcctgctga tggacacctt 1140
   cttcc 1145
<210> 103
   <211> 258
   <212> DNA
   <213> Artificial
<220>
   <223> GFP
<400> 103
   agatacccag atcatatgaa acggcatgac tttttcaaga gtgccatgcc cgaaggttat 60
   gtacaggaaa gaactatatt tttcaaagat gacgggaact acaagacacg taagtttaaa 120
   cagttcggta ctaactaacc atacatattt aaattttcag gtgctgaagt caagtttgaa 180
   ggtgataccc ttgttaatag aatcgagtta aaaggtattg attttaaaga agatggaaac 240
   attcttggac acaaattg 258
<210> 104
   <211> 745
   <212> DNA
   <213> Artificial
<220>
   <223> Pt coral fluorescent protein
<400> 104
   agtgtaataa cttactttga gtctaccgtc atgagtgcaa ttaaaccagt catgaagatt 60
   gaattggtca tggaaggaga ggtgaacggg cacaagttca cgatcacggg agagggacaa 120
   ggcaagcctt acgagggaac acagactcta aaccttacag tcactaaagg cgtgcccctt 180
   cctttcgctt tcgatatctt gtcaacagca ttccagtatg gcaacagggt atttaccaaa 240
   tacccagatg atataccgga ctatttcaag cagacctttc cggaaggata ttcgtgggaa 300
   agaactttca aatatgaaga gggcgtttgc accacaaaga gtgacataag cctcaagaaa 360
   ggccaaccag actgctttca atataaaatt aactttaaag gggagaagct tgaccccaac 420
   ggcccaatta tgcagaagaa gaccctgaaa tgggagccat ccactgagag gatgtacatg 480
   gacgtggata aagacggtgc aaaggtgctg aagggcgatg ttaatgcggc cctgttgctt 540
   gaaggaggtg gccattatcg ttgtgacttt aacagtactt acaaggcgaa gaaaactgtg 600
   tccttcccag catatcactt tgtggaccac cgcattgaga ttttgagcca caatacggat 660
   tacagcaagg ttacactgta tgaagttgcc gtggctcgca attctcctct tcagattatg 720
   gcgccccagt aaaggcttaa cgaaa 745
<210> 105
   <211> 42
   <212> DNA
   <213> Artificial
<220>
   <223> Primer
<400> 105
   gcgtaatacg actcactata ggtgatgaga tgcggcaaga ag 42
<210> 106
   <211> 22
   <212> DNA
   <213> Artificial
<220>
   <223> Primer
<400> 106
   ctcttgcttc ttggtggcga tc 22
<210> 107
   <211> 22
   <212> DNA
   <213> Artificial
<220>
   <223> Primer
<400> 107
   ggtgatgaga tgcggcaaga ag 22
<210> 108
   <211> 44
   <212> DNA
   <213> Artificial
<220>
   <223> Primer
<400> 108
   gcgtaatacg actcactata ggctcttgct tcttggtggc gatc 44
<210> 109
   <211> 44
   <212> DNA
   <213> Artificial
<220>
   <223> Primer
<400> 109
   gcgtaatacg actcactata ggtgggttgt tggagtgctc ctac 44
<210> 110
   <211> 22
   <212> DNA
   <213> Artificial
<220>
   <223> Primer
<400> 110
   ggaagaaggt gtccatcagc ag 22
<210> 111
   <211> 22
   <212> DNA
   <213> Artificial
<220>
   <223> Primer
<400> 111
   tgggttgttg gagtgctcct ac 22
<210> 112
   <211> 44
   <212> DNA
   <213> Artificial
<220>
   <223> Primer
<400> 112
   gcgtaatacg actcactata ggggaagaag gtgtccatca gcag 44
<210> 113
   <211> 46
   <212> DNA
   <213> Artificial
<220>
   <223> Primer
<400> 113
   gcgtaatacg actcactata ggagataccc agatcatatg aaacgg 46
<210> 114
   <211> 24
   <212> DNA
   <213> Artificial
<220>
   <223> Primer
<400> 114
   caatttgtgt ccaagaatgt ttcc 24
<210> 115
   <211> 24
   <212> DNA
   <213> Artificial
<220>
   <223> Primer
<400> 115
   agatacccag atcatatgaa acgg 24
<210> 116
   <211> 46
   <212> DNA
   <213> Artificial
<220>
   <223> Primer
<400> 116
   gcgtaatacg actcactata ggcaatttgt gtccaagaat gtttcc 46
<210> 117
   <211> 43
   <212> DNA
   <213> Artificial
<220>
   <223> Primer
<400> 117
   gcgtaatacg actcactata ggagtgtaat aacttacttt gag 43
<210> 118
   <211> 20
   <212> DNA
   <213> Artificial
<220>
   <223> Primer
<400> 118
   tttcgttaag cctttactgg 20
<210> 119
   <211> 21
   <212> DNA
   <213> Artificial
<220>
   <223> Primer
<400> 119
   agtgtaataa cttactttga g 21
<210> 120
   <211> 42
   <212> DNA
   <213> Artificial
<220>
   <223> Primer
<400> 120
   gcgtaatacg actcactata ggtttcgtta agcctttact gg 42
<210> 121
   <211> 473
   <212> DNA
   <213> Lygus hesperus
<400> 121
   tgccgggccg ctcgccgaac catctgggaa gcttggaatg ggctcgactg ccgaactgat 60
   caactttttc ggtccacacc ttttctatca actccttata ccgctccagg atgccgcctt 120
   caaacagttt tttcttgtcg tcataagatc tggtgtcaac tcttcgttca tatttggagg 180
   cgacttggat tttgggtggg tacttgccgg tgagggcctc agggtccaag ccttttttca 240
   aggctttgtg ccgaagttgt tgcttctgtc tttccttcag ttctttaaga tcgtagtctt 300
   gcctcttttg cctttcctca agatcgtatt tctcggtctc aagtttgaca atggcttccc 360
   agagttcctg agctttgatg cgtagcctgt ctatgctcat attttctatc gccaggggct 420
   tgagcctaat gctgagggag atacgtttct cttcctccag ctgctccttg gtc 473
<210> 122
   <211> 773
   <212> DNA
   <213> Lygus hesperus
<400> 122
   gctgctcgcc gtccagttcg ttttcgagtt ccctgacacg ttgttccagc ttggcgatgg      60
   ccttcttgcc tcccttgagg gcgttgtttt cggcttcgtc caacctgact tggagttcct     120
   tgatttgcgt ttccagagcc ttgcggagct tctcctgggt ctgagcgtgg tcctgttctg     180
   ccctgagttc atcagctaac ctagcggcat caaccattgc cttcttggcc ttctcttcgg     240
   agttcttggc ttcgttgaga agttcgtcga ggtcagcatg aagtgtctgc aactctccct     300
   caagcttgcg tttggcggct gaggcgctgg tagcttgggc agccaactcg ttgatctgtt     360
   cgtgggcatc tccaagttct tgttcggctt ggcgcctgcc cctgtcggcc tgttcgagga     420
   gagtgcgcga ctcctcgagc tcgtttccga gagcgttggc cctcctttcg gcgattccga     480
   gttgttcacg agcatcgtcg cgtgcccttt gttcttcctc aagagcggtc tgtacgtcct     540
   tgagttgttg ttggtatttc ttgatggtct tctgggcttc ggcgttagcc ttgttggcgt     600
   ggtcgagagc gatttcgagt tcgttgatgt cggcttcaag cttcttcttc atgcgaagag     660
   cctcagcctt acccttggct tcagcctcca agctggcttg catggagtcg agtgcccgtt     720
   ggtggttctt cctggtgttc tcgaactcct cctccttttc ctggatccgc cgg            773
<210> 123
   <211> 771
   <212> DNA
   <213> Lygus hesperus
<400> 123
   tggacgccat caagaagaaa atgcaggcga tgaagatgga gaaggacacg gccatggaca 60
   aggccgacac ctgcgagggg caggccaagg acgctaacac ccgcgccgac aaaatccttg 120
   aagatgtgag ggacctccaa aagaaactca accaggtaga aagtgatctc gaaaggacca 180
   agagggaact cgagacgaaa accaccgaac tcgaagagaa ggagaaggcc aacaccaacg 240
   ctgagagcga ggtcgcctcc ctcaacagga aagtccagat ggttgaagag gacttggaaa 300
   gatctgaaga aaggtccggc accgcacaac aaaaactgtc cgaagcctcc cacgccgctg 360
   atgaagcctc tcgtatgtgc aaagtattgg agaacaggtc acaacaggat gaggagagga 420
   tggaccagct caccaaccag ctgaaagaag cccgactcct cgctgaagac gccgacggca 480
   aatcggatga ggtatcaagg aagctggcct tcgttgaaga cgaactggaa gtagctgaag 540
   atcgtgtcaa atctggagac tcgaagatca tggagcttga ggaggagttg aaagttgtcg 600
   gtaacagctt gaaatctctc gaagtttcag aggagaaggc caaccagcga gtcgaagagt 660
   acaaacgtca aatcaagcaa ctgactgtca agttgaagga ggctgaagct cgcgctgagt 720
   tcgccgaaaa gacagtcaag aagttgcaga aagaggtgga ccggctggag g 771
<210> 124
   <211> 257
   <212> DNA
   <213> Lygus hesperus
<400> 124
   tgcgggccct ggggcagaat cccacagaat ctgacgtgaa gaagttcacc caccagcaca 60
   aaccagatga aagaatcagc ttcgaggtgt ttctcccgat ataccaagcc atatcgaagg 120
   gtaggacgtc agacacagct gaagacttca tcgagggtct cagacacttt gacaaagatg 180
   gaaatggctt catttcaaca gctgagcttc gccacttgct cacaactttg ggcgaaaaac 240
   tgaccgacga cgaggtg 257
<210> 125
   <211> 410
   <212> DNA
   <213> Lygus hesperus
<400> 125
   gccacctcca acgtgtttgc catgttcgat caggctcaga ttcaagaatt caaggaggca        60
   ttcaacatga tcgaccagaa cagggacggc ttcgtggata aggaagacct ccatgacatg       120
   ctcgcttccc taggtaagaa cccctcagac gagtatctcg aggggatgat gaacgaggcg       180
   cctggtccca tcaacttcac aatgttcctc accctcttcg gtgagcggct tcagggaact       240
   gatccggagg aggttatcaa gaacgcattt gggtgttttg acgaagacaa caacggattc       300
   atcaacgagg aaagactgcg cgagctgctc acctccatgg gggacaggtt cactgatgaa       360
   gacgtggacg aaatgtaccg agaggccccc atcaagaacg gcatgttcga                  410
<210> 126
   <211> 1021
   <212> DNA
   <213> Lygus hesperus
<400> 126
   tgttcatcct ggagcaggag gagtatcaga gagaaggtat tgaatggaag ttcatcgact        60
   tcggacttga tcttcagccg accattgatc tcattgataa gccaatggga gtcatggctc       120
   tcctggatga agaatgttgg ttccccaaag ccactgacaa gaccttcgtt gagaagctgg       180
   tcggtgctca cagcgttcac cccaaattca tcaaaactga tttccgtgga gtcgccgact       240
   ttgctgtcgt ccattatgcc ggaaaagtcg attattcggc ggcgcagtgg ctgatgaaga       300
   acatggaccc tctgaacgaa aacgtcgtgc agctcctcca gaactcgcaa gatccgttcg       360
   tcatccacat ctggaaggac gcagagatcg tcggcatggc tcaccaagct ctcagcgaca       420
   ctcagtttgg agctcgtacc aggaagggta tgttccgaac cgtgtctcaa ctctacaaag       480
   accagctgtc caaactcatg atcacacttc gcaacacgaa ccccaacttc gtccgttgca       540
   tcctccccaa ccacgagaag agagctggca agatcgatgc tcctttggtg ctggatcagc       600
   tcagatgcaa cggtgtgttg gaaggcatca gaatttgcag acaaggtttc ccgaatagaa       660
   tcccattcca ggaattccgg caaagatacg agctcttaac tcccaatgtc atccccaaag       720
   ggttcatgga cggtaaaaag gcttgcgaga agatgatcaa cgctctcgaa ctggacccta       780
   atctctacag agttggtcag tccaagatat tcttcagagc tggagtctta gctcatctag       840
   aagaagagcg cgactataag attactgatc tgatagccaa tttccgggct ttctgtaggg       900
   gatatcttgc ccgaaggaac taccaaaagc gtcttcagca gctcaacgcc attcgtatta       960
   tccagcgaaa ttgctcagct tacttgaagt tgaggaactg gcaatggtgg cggctgtaca      1020
   c                                                                      1021
<210> 127
   <211> 325
   <212> DNA
   <213> Lygus hesperus
<400> 127
   cggtcatcat ctccatgaac tcgtcgaagt caacagttcc ggaaccgtca gaatcaattt      60
   cagcaatcat catgtcaagt tcttgggagg tgattttgtc gtcgagttcc ttcaggattt     120
   ccctcaagac gtcagtggta atgtaaccgt tcccttcctt gtcgtagagc ctgaaggcct     180
   ccctcagttc ttgctgcatg gcctcagcat cttgtgtctc atcttctgtc aggaaaccgg     240
   cagccaaggc tacgaactcc tcaaattcaa gttgtccaga gccatcagcg tcgacctccg     300
   caatgatctc ctccaggatc ttctt                                           325
<210> 128
   <211> 463
   <212> DNA
   <213> Lygus hesperus
<400> 128
   cggtcatcat ctccatgaac tcgtcgaagt cgacagttcc ggatccgtca gagtcgatct      60
   cctcgatgat catgtccagc tcctcgttgg tcagctgctc gtccaattca tgaaggattt     120
   ctttgaggca ggaggtcggg atgtagccat taccttcttt gtcgtagaga cggaaggctt     180
   ctcgcagctc tttctgcatg gcttcatcgt cttcctcaac aatgaacttg gctgccaacg     240
   tgatgaactc ttcaaactcc agccttcccg atttgtcagc gtcaacttct tcgatgagtt     300
   catcgagaat cttcttgttg aagggttgac ccatgagtct gaggatgtcg gccaccatgt     360
   ccgtcgggat ggaacccgag tgatcccggt cgaaagcgtt caacgcgatg gtcatgatgg     420
   ggataattcg gttaattctg ttagaccagt ccgattagtg acg                       463
<210> 129
   <211> 413
   <212> DNA
   <213> Lygus hesperus
<400> 129
   atgggtgaag gagggtgcct gctcagagca gtcctccagg atgacggcta tggacaacgc      60
   ctcgaagaac gccgctgaga tgatcgacaa gctgaccttg acgttcaaca ggactcggca     120
   agccgtcatc accagggagc tcatcgaaat catctccggt gcctctgctt tggagtaacg     180
   tctcagctca cccagccacc tcccgtagat ccactagtgc tgcgagagac cgagtacctc 240
   gttctattca ccctgtacat ttcttaatca atattattgg aattcgattc gatagtcgta 300
   tgctgggaaa tatcttgttc atattcatga tacttgttca acattgttct ggtaaataat 360
   ttatgtaata caggttgagt taccaaaaaa aaaaaaaaaa aaaaaaaaaa aaa 413
<210> 130
   <211> 449
   <212> DNA
   <213> Lygus hesperus
<400> 130
   gcagctggag gaagagaaac gtatctccct cagcattagg ctcaagcccc tggcgataga 60
   aaatatgagc atagacaggc tacgcatcaa agctcaggaa ctctgggaag ccattgtcaa 120
   acttgagacc gagaaatacg atcttgagga aaggcaaaag aggcaagact acgatcttaa 180
   agaactgaag gaaagacaga agcaacaact tcggcacaaa gccttgaaaa aaggcttgga 240
   ccctgaggcc ctcaccggca agtacccacc caaaatccaa gtcgcctcca aatatgaacg 300
   aagagttgac accagatctt atgacgacaa gaaaaaactg tttgaaggcg gcatcctgga 360
   gcggtataag gagttgatag aaaaggtgtg gaccgaaaaa gttgatcagt tcggcagtcg 420
   agcccattcc aagcttccca gatggttcg 449
<210> 131
   <211> 719
   <212> DNA
   <213> Lygus hesperus
<400> 131
   aggagttcga gaacaccagg aagaaccacc aacgggcact cgactccatg caagccagct        60
   tggaggctga agccaagggt aaggctgagg ctcttcgcat gaagaagaag cttgaagccg       120
   acatcaacga actcgaaatc gctctcgacc acgccaacaa ggctaacgcc gaagcccaga       180
   agaccatcaa gaaataccaa caacaactca aggacgtaca gaccgctctt gaggaagaac       240
   aaagggcacg cgacgatgct cgtgaacaac tcggaatcgc cgaaaggagg gccaacgctc       300
   tcggaaacga gctcgaggag tcgcgcactc tcctcgaaca ggccgacagg ggcaggcgcc       360
   aagccgaaca agaacttgga gatgcccacg aacagatcaa cgagttggct gcccaagcta       420
   ccagcgcctc agccgccaaa cgcaagcttg agggagagtt gcagacactt catgctgacc       480
   tcgacgaact tctcaacgaa gccaagaact ccgaagagaa ggccaagaag gcaatggttg       540
   atgccgctag gttagctgat gaactcaggg cagaacagga ccacgctcag acccaggaga       600
   agctccgcaa ggctctggaa acgcaaatca aggaactcca agtcaggttg gacgaagccg       660
   aaaacaacgc cctcaaggga ggcaagaagg ccatcgccaa gctggaacaa cgtgtcagg        719
<210> 132
   <211> 737
   <212> DNA
   <213> Lygus hesperus
<400> 132
   gcaggcgatg aagatggaga aggacacggc catggacaag gccgacacct gcgaggggca        60
   ggccaaggac gctaacaccc gcgccgacaa aatccttgaa gatgtgaggg acctccaaaa       120
   gaaactcaac caggtagaaa gtgatctcga aaggaccaag agggaactcg agacgaaaac       180
   caccgaactc gaagagaagg agaaggccaa caccaacgct gagagcgagg tcgcctccct       240
   caacaggaaa gtccagatgg ttgaagagga cttggaaaga tctgaagaaa ggtccggcac       300
   cgcacaacaa aaactgtccg aagcctccca cgccgctgat gaagcctctc gtatgtgcaa       360
   agtattggag aacaggtcac aacaggatga ggagaggatg gaccagctca ccaaccagct       420
   gaaagaagcc cgactcctcg ctgaagacgc cgacggcaaa tcggatgagg tatcaaggaa       480
   gctggccttc gttgaagacg aactggaagt agctgaagat cgtgtcaaat ctggagactc       540
   gaagatcatg gagcttgagg aggagttgaa agttgtcggt aacagcttga aatctctcga       600
   agtttcagag gagaaggcca accagcgagt cgaagagtac aaacgtcaaa tcaagcaact       660
   gactgtcaag ttgaaggagg ctgaagctcg cgctgagttc gccgaaaaga cagtcaagaa       720
   gttgcagaaa gaggtgg                                                      737
<210> 133
   <211> 205
   <212> DNA
   <213> Lygus hesperus
<400> 133
   cagaatccca cagaatctga cgtgaagaag ttcacccacc agcacaaacc agatgaaaga      60
   atcagcttcg aggtgtttct cccgatatac caagccatat cgaagggtag gacgtcagac     120
   acagctgaag acttcatcga gggtctcaga cactttgaca aagatggaaa tggcttcatt     180
   tcaacagctg agcttcgcca cttgc                                           205
<210> 134
   <211> 326
   <212> DNA
   <213> Lygus hesperus
<400> 134
   ggaggcattc aacatgatcg accagaacag ggacggcttc gtggataagg aagacctcca      60
   tgacatgctc gcttccctag gtaagaaccc ctcagacgag tatctcgagg ggatgatgaa     120
   cgaggcgcct ggtcccatca acttcacaat gttcctcacc ctcttcggtg agcggcttca     180
   gggaactgat ccggaggagg ttatcaagaa cgcatttggg tgttttgacg aagacaacaa     240
   cggattcatc aacgaggaaa gactgcgcga gctgctcacc tccatggggg acaggttcac     300
   tgatgaagac gtggacgaaa tgtacc                                          326
<210> 135
   <211> 944
   <212> DNA
   <213> Lygus hesperus
<400> 135
   gacttgatct tcagccgacc attgatctca ttgataagcc aatgggagtc atggctctcc 60
   tggatgaaga atgttggttc cccaaagcca ctgacaagac cttcgttgag aagctggtcg 120
   gtgctcacag cgttcacccc aaattcatca aaactgattt ccgtggagtc gccgactttg 180
   ctgtcgtcca ttatgccgga aaagtcgatt attcggcggc gcagtggctg atgaagaaca 240
   tggaccctct gaacgaaaac gtcgtgcagc tcctccagaa ctcgcaagat ccgttcgtca 300
   tccacatctg gaaggacgca gagatcgtcg gcatggctca ccaagctctc agcgacactc 360
   agtttggagc tcgtaccagg aagggtatgt tccgaaccgt gtctcaactc tacaaagacc 420
   agctgtccaa actcatgatc acacttcgca acacgaaccc caacttcgtc cgttgcatcc 480
   tccccaacca cgagaagaga gctggcaaga tcgatgctcc tttggtgctg gatcagctca 540
   gatgcaacgg tgtgttggaa ggcatcagaa tttgcagaca aggtttcccg aatagaatcc 600
   cattccagga attccggcaa agatacgagc tcttaactcc caatgtcatc cccaaagggt 660
   tcatggacgg taaaaaggct tgcgagaaga tgatcaacgc tctcgaactg gaccctaatc 720
   tctacagagt tggtcagtcc aagatattct tcagagctgg agtcttagct catctagaag 780
   aagagcgcga ctataagatt actgatctga tagccaattt ccgggctttc tgtaggggat 840
   atcttgcccg aaggaactac caaaagcgtc ttcagcagct caacgccatt cgtattatcc 900
   agcgaaattg ctcagcttac ttgaagttga ggaactggca atgg 944
<210> 136
   <211> 318
   <212> DNA
   <213> Lygus hesperus
<400> 136
   atcctggagg agatcattgc ggaggtcgac gctgatggct ctggacaact tgaatttgag 60
   gagttcgtag ccttggctgc cggtttcctg acagaagatg agacacaaga tgctgaggcc 120
   atgcagcaag aactgaggga ggccttcagg ctctacgaca aggaagggaa cggttacatt 180
   accactgacg tcttgaggga aatcctgaag gaactcgacg acaaaatcac ctcccaagaa 240
   cttgacatga tgattgctga aattgattct gacggttccg gaactgttga cttcgacgag 300
   ttcatggaga tgatgacc 318
<210> 137
   <211> 423
   <212> DNA
   <213> Lygus hesperus
<400> 137
   atccccatca tgaccatcgc gttgaacgct ttcgaccggg atcactcggg ttccatcccg 60
   acggacatgg tggccgacat cctcagactc atgggtcaac ccttcaacaa gaagattctc 120
   gatgaactca tcgaagaagt tgacgctgac aaatcgggaa ggctggagtt tgaagagttc 180
   atcacgttgg cagccaagtt cattgttgag gaagacgatg aagccatgca gaaagagctg 240
   cgagaagcct tccgtctcta cgacaaagaa ggtaatggct acatcccgac ctcctgcctc 300
   aaagaaatcc ttcatgaatt ggacgagcag ctgaccaacg aggagctgga catgatcatc 360
   gaggagatcg actctgacgg atccggaact gtcgacttcg acgagttcat ggagatgatg 420
   acc 423
<210> 138
   <211> 252
   <212> DNA
   <213> Lygus hesperus
<400> 138
   ggtgaaggag ggtgcctgct cagagcagtc ctccaggatg acggctatgg acaacgcctc 60
   gaagaacgcc gctgagatga tcgacaagct gaccttgacg ttcaacagga ctcggcaagc 120
   cgtcatcacc agggagctca tcgaaatcat ctccggtgcc tctgctttgg agtaacgtct 180
   cagctcaccc agccacctcc cgtagatcca ctagtgctgc gagagaccga gtacctcgtt 240
   ctattcaccc tg 252
<210> 139
   <211> 1110
   <212> DNA
   <213> Lygus hesperus
<400> 139
   gtctccgctc aagctggtca tcagacttca gctgagtcct ggggtaccgg tcgtgctgtg      60
   gctcgtatcc cccgtgttcg cggaggtggt actcaccgct caggtcaggg tgcttttggc     120
   aacatgtgtc gcggcggtag gatgttcgct cccactcgcc catggcgtcg ttggcaccgc     180
   aagatcaacg ttaaccaaaa acgttatgcc gtcgtgtccg ccatcgctgc atccggcgtc     240
   ccagccctcg tcatgtccaa aggacacatg gtgcaaagcg tccctgaatt cccccttgtt     300
   gtgtctgaca aagttcagga atacactaaa accaaacagg ctgtcatctt ccttcaccgc     360
   atcaaagcct ggcaagacat ccagaaagtg tacaagtcga agaggttccg tgctggtaag     420
   ggtaaaatga ggaaccgcag gaggatccag aggcgtggac ccctcatcat ctacgaccag     480
   gatcagggtc tgaacagggc tttccgtaac attcccggcg tcgatttgat cgaagtgagc     540
   cgcctcaact tgctgaagct cgctccagga ggtcacatcg gccggttcgt catctggact     600
   cagtcggcct tcgagaagtt ggacgccctc tacggcacct ggaagaagaa gtccaccctc     660
   aaggctggat acaatctccc catgcccaag atggccaaca ccgacctttc ccgcctcttc     720
   aaggccccgg agatcaaggc tgtcctcagg aatcccaaga agaccatcgt acgacgagtg     780
   cgcaaactga accctctccg caacaccagg gctatgctgc gtctcaaccc atacgctgct     840
   gtcctcaaga ggaaggccat ccttgatcaa aggaagttga aactccagaa gctcgtagaa     900
   gctgccaaga agggagatac caagctgtcg ccccgcgtcg agcgtcacct gaagatgatc     960
   gagagaagga aagccctgat caagaaagcc aaggctgcca agcccaagaa gcccaaaacg    1020
   gccaagaaac ccaagaccgc cgagaaggca ccagcacccg ccaagaaggc ggcagcgccc    1080
   aaaaaggcca ccacccctgc caagaaatga                                     1110
<210> 140
   <211> 729
   <212> DNA
   <213> Lygus hesperus
<400> 140
   atggccaatg ctaagcctat ttctaagaag aagaagtttg tgtctgacgg tgtcttcaaa 60
   gccgaattga acgaatttct taccagagaa ctcgctgaag aggggtactc aggtgttgag 120
   gtccgagtga cccccaacaa gacagaaatt atcatcatgg cgacaaggac acaaagcgtt 180
   cttggtgata agggccgccg aatcagggag ctcacgtctg tagttcagaa aagattcaat 240
   ttcaagcctc agactttgga tctctatgct gaaaaggtcg ccaccagagg tttgtgtgct 300
   attgcacaag ctgaatccct ccgttacaaa ctcattggcg gtcttgctgt ccgaggggct 360
   tgctatggtg tccttcgctt catcatggaa aatggtgcca agggttgcga agtcgtagta 420
   tctggaaaac tgcgtggtca gagagccaag tcaatgaagt tcgtggatgg tttgatgatc 480
   cacagtgggg atccctgtaa cgaatatgtt gatactgcta cccgacatgt gctccttaga 540
   caaggtgtcc tgggaataaa ggtgaagatt atgttgccgt gggacgttac cggcaaaaat 600
   gggccgaaga accctcttcc cgaccacgtc agcgttctct tacctaagga ggagctacca 660
   aatttggccg ttagtgtgcc tggatccgac atcaaaccaa agcctgaagt accagcaccc 720
   gctttgtga 729
<210> 141
   <211> 789
   <212> DNA
   <213> Lygus hesperus
<400> 141
   atggctgttg gtaaaaataa gggtctatcg aaaggaggaa agaagggagt taaaaaaaag 60
   gtagtggacc ctttcaccag gaaggattgg tacgatgtta aggctccttc catgttcaaa 120
   aagcgtcaag ttggcaaaac tttggtcaac cgaactcagg gaaccaagat tgcttctgaa 180
   gggttgaaag gacgagtttt cgaagtttcg ctcgctgata tccaggagga cactgatgcc 240
   gagcgctcct tcaggaaatt caggctcatc gctgaagatg tccaagccag aaacgtcctt 300
   accaatttcc acggtatgga tttgaccact gacaaactcc ggagcatggt caagaagtgg 360
   cagactctca tcgaagccaa cgttgacgtc aagaccaccg acggctacct cctgcgcgtc 420
   ttctgcatag gattcaccaa taaagatcaa ctttcccaga gaaagacttg ctatgcccag      480
   cataatcagg tccgagaaat ccgcaaaaag atggttaaaa acatcagtga cagcatttcc      540
   agctgtgatt tgaggagtgt tgtgaacaag ctgatcccag actccatcgc taaagatata      600
   gaaaagaatt gccaaggaat ctacccactc cacgatgtgt acattcggaa ggtgaaggtg      660
   ttgaagaagc cgaggttcga gctcagcaag ctccttgagc ttcacgtcga tggcaaaggg      720
   atcgacgaac ccggcgcgaa agtgacgagg actgacgctt acgagcctcc agttcaagag      780
   tctgtctaa                                                              789
<210> 142
   <211> 473
   <212> DNA
   <213> Lygus hesperus
<400> 142
   gaccaaggag cagctggagg aagagaaacg tatctccctc agcattaggc tcaagcccct       60
   ggcgatagaa aatatgagca tagacaggct acgcatcaaa gctcaggaac tctgggaagc      120
   cattgtcaaa cttgagaccg agaaatacga tcttgaggaa aggcaaaaga ggcaagacta      180
   cgatcttaaa gaactgaagg aaagacagaa gcaacaactt cggcacaaag ccttgaaaaa      240
   aggcttggac cctgaggccc tcaccggcaa gtacccaccc aaaatccaag tcgcctccaa      300
   atatgaacga agagttgaca ccagatctta tgacgacaag aaaaaactgt ttgaaggcgg      360
   catcctggag cggtataagg agttgataga aaaggtgtgg accgaaaaag ttgatcagtt      420
   cggcagtcga gcccattcca agcttcccag atggttcggc gagcggcccg gca             473
<210> 143
   <211> 1463
   <212> DNA
   <213> Lygus hesperus
<400> 143
   gggtctcagc tgaggcacat tccatctcgt cgcaaatctt tcctgcatct ctcctgggtg 60
   acctttaggt gaccaatcac atccatcatg tcggacgagg agtattcgga gtcggaggaa 120
   gagacccagc cggaaccaca gaaaaaacca gaggctgaag gaggcggcga cccagaattc 180
   gtcaagcgta aggaagccca gacctcagcc ttagacgagc agcttaaaga ctatatcgca 240
   gaatggagga aacaaagagc tcgcgaagaa gaagacctca agaagctgaa ggagaagcaa 300
   gccaagcgca aggtcgctcg ggcagaagaa gaaaagagat tggcggaaaa gaagaagcag 360
   gaagaagaac gacgtgtgag ggaagcagaa gagaagaaac agagggaaat cgaagagaag 420
   aggcgaaggc ttgaagaggc cgagaagaag agacaagcca tgatggctgc tctcaaggac 480
   cagagcaaaa cgaagggacc caattttgtc gttaataaga aagccgaaac ccttggcatg 540
   tcctccgctc aaattgagcg caacaagact aaggaacagc ttgaggaaga aaaacgtatc 600
   tccctcagca ttaggctcaa gcccctggcg atagaaaata tgagcataga caggctacgc 660
   ataaaagctc aggaactctg ggaagccatt gtcaaacttg agaccgagaa atacgatctt 720
   gaggaaaggc aaaagaggca agactacgat cttaaagaac tgaaggaaag acagaagcaa 780
   caacttcggc acaaagcctt gaaaaaaggc ttggaccctg aggccctcac cggcaagtac 840
   ccacccaaaa tccaagtcgc ctccaaatat gaacgaagag ttgacaccag atcttatgac 900
   gacaagaaaa aactgtttga aggcggcatc ctggagcggt ataaggagtt gatagaaaag 960
   gtgtggaccg aaaaagttga tcagttcggc agtcgagccc attccaagct tcccagatgg 1020
   ttcggcgagc ggcccggcaa gaagaaggat gcccctgaaa gcccggaaga agaggaagtg 1080
   aaggtagaag atgaacctga agctgaacca agcttcatgc tcgacgaaga agaagaagaa 1140
   gcggaagaag aggaggcgga agaggaagag gaagccgagg aagaggagga agaagaagag 1200
   gaagaggaag aggaggagga ggaagaagaa taggtctttt tcaacatttc actgcaccca 1260
   cagttccacg gtctttccgc ccacaaactc aatctgtgct cacgagatct tagcaggaaa 1320
   agtattgcga cccgataaga acaaattaaa ttatttttgg aatatctcgt tcagttattt 1380
   cgtgagaaac aattttattc atgtaaacga ttaaaagatc ccatacattt ccaaaaaaaa 1440
   aaaaaaaaaa aaaaaaaaaa aaa 1463
<210> 144
   <211> 773
   <212> DNA
   <213> Lygus hesperus
<400> 144
   ccggcggatc caggaaaagg aggaggagtt cgagaacacc aggaagaacc accaacgggc 60
   actcgactcc atgcaagcca gcttggaggc tgaagccaag ggtaaggctg aggctcttcg 120
   catgaagaag aagcttgaag ccgacatcaa cgaactcgaa atcgctctcg accacgccaa 180
   caaggctaac gccgaagccc agaagaccat caagaaatac caacaacaac tcaaggacgt 240
   acagaccgct cttgaggaag aacaaagggc acgcgacgat gctcgtgaac aactcggaat 300
   cgccgaaagg agggccaacg ctctcggaaa cgagctcgag gagtcgcgca ctctcctcga 360
   acaggccgac aggggcaggc gccaagccga acaagaactt ggagatgccc acgaacagat 420
   caacgagttg gctgcccaag ctaccagcgc ctcagccgcc aaacgcaagc ttgagggaga 480
   gttgcagaca cttcatgctg acctcgacga acttctcaac gaagccaaga actccgaaga 540
   gaaggccaag aaggcaatgg ttgatgccgc taggttagct gatgaactca gggcagaaca 600
   ggaccacgct cagacccagg agaagctccg caaggctctg gaaacgcaaa tcaaggaact 660
   ccaagtcagg ttggacgaag ccgaaaacaa cgccctcaag ggaggcaaga aggccatcgc 720
   caagctggaa caacgtgtca gggaactcga aaacgaactg gacggcgagc agc 773
<210> 145
   <211> 5446
   <212> DNA
   <213> Lygus hesperus
<400> 145
   tcaggaaaac tggctggtgc tgatattgag acctatctgc tggagaaggc tcgtgtcatc 60
   tcccaacaaa cactcgagag atcctaccac attttctacc agatgatgtc tggagctgtc 120
   aagggcgtca aggaaatgtg cttgctggtc gacgatatct atacgtacaa cttcatatcc 180
   cagggtaaag tcagcattgc aggcgttgat gacggagagg aaatggttct gaccgatcaa 240
   gccttcgaca tcttgggttt caccaagcaa gagaaggaag acatctacaa gatcaccgcc 300
   gctgtcattc acatgggtac catgaagttc aagcaaaggg gtcgtgaaga gcaggctgaa 360
   gccgatggaa ctgaggaagg cggtaaggtc ggtgtgctcc tcggtatcga cggtgacgac 420
   ttgtacaaga atatgtgcaa gcccagaatc aaggtcggaa ctgagttcgt gacccaggga 480
   aagaacgtca accaggtctc atactctctc ggtgccatgt ccaagggtat gttcgatcgt 540
   ctcttcaaat tcttggtcaa gaaatgtaac gaaactctgg acaccaaaca gaagagacag 600
   cacttcattg gtgtactgga tattgccggg ttcgaaattt tcgacttcaa cggttttgag 660
   caactgtgta tcaacttcac caacgagaaa ttgcaacaat tcttcaacca ccacatgttc 720
   gtactcgagc aagaagagta caagagggaa ggcattaact gggctttcat tgatttcgga 780
   atggacttgc tcgcttgtat tgaactgatt gagaagccca tgggtatctt gtccatcctt 840
   gaagaagagt ctatgttccc caaggctact gacaagacct ttgaggacaa actcatcacc 900
   aaccacttgg gcaaatctcc caacttcagg aagcccgccg ttccaaagcc tggccaacaa 960
   gctggtcact tcgccatcgc tcactacgct ggttgcgtgt catacaacat caccggctgg 1020
   cttgagaaga acaaggatcc gttgaacgac actgttgtcg atcagtacaa gaagggaacc 1080
   aacaaactgt tgtgcgagat cttcgctgat catcctggcc aatctggtgc ccctggtggt 1140
   gatgctggtg gcaagggtgg tcgtggcaag aaaggtggtg gcttcgccac tgtgtcatct 1200
   tcctacaagg aacaattgaa caacttgatg accactttga agagcacaca gcctcacttc 1260
   gtccgttgta tcatccccaa cgaattgaaa cagcccggtg ttattgattc tcacttggtc 1320
   atgcaccagc tgacttgtaa cggtgtactt gaaggcatcc gtatttgccg taaaggcttc 1380
   cccaacagga tgaactaccc tgacttcaag ctccgataca agatccttaa ccccgctgcc 1440
   gtggacagag agagtgatat cctcaaggct gctggtctcg tccttgagtc aactgggctc 1500
   gaccctgata tgtaccgtct cggccacacc aaggtgttct tcagggccgg agttttgggt 1560
   caacttgaag aattgcgtga cgacaggctt agcaagatca tcggatggat gcaggccttc 1620
   atgcgcggtt acctcgtcag gaaggagtac aagaagctcc aggaacagag gttagccctc 1680
   caagttgtcc agcgcaactt gagaaggtac ctccaactga ggacctggcc ctggtggaag 1740
   atgtggtcca gggtcaagcc cctcctcaac gtcgccaacg tcgaagagga gatgcggaaa 1800
   ctcgaagagt tggtcgccga gacccaggcc gctttggaga aggaggagaa gctgaggaag    1860
   gaggccgaag cccttaacgc caagcttctc caagagaaga ccgaccttct caggaacttg    1920
   gaaggagaga agggatccat cagcggtatc caggaacgat gtgccaagct gcaagcccaa    1980
   aaggccgatc ttgagtctca actcatggac acccaagaaa ggctgcagaa cgaagaagat    2040
   gccaggaacc agctcttcca acagaagaag aaattggaac aagaagccgc tgccctcaag    2100
   aaggacatcg aagatctcga actctccaac caaaagaccg accaagataa ggccagcaag    2160
   gaacaccaaa tcagaaacct caatgacgag atcgctcacc aagatgactt gatcaacaag    2220
   ctcaacaagg agaagaaaat ccagagcgaa ctcaaccaaa agactgctga agaacttcag    2280
   gccgctgaag acaaaatcaa ccacctcacc aaggttaagg tcaagcttga acagaccttg    2340
   gatgaactcg aagacaccct cgaacgtgaa aagaaactcc gaggagatgt cgaaaaggcc    2400
   aagaggaaga ctgaaggcga cctcaagctc actcaggaag ccgttgccga tcttgaaagg    2460
   aacaagaaag aactcgaaca gaccatccag aggaaagaca aggaaattgc ttccctcacc    2520
   gccaagctcg aagacgaaca atccatcgtc aacaagactg gcaaacagat caaggaactc    2580
   cagagccgca ttgaagagct cgaggaggaa gtcgaggctg agaggcaagc ccgcggaaag    2640
   gctgagaagc aacgtgctga cctcgcccgc gaacttgagg aactcggcga gaggttagag    2700
   gaagctggtg gtgccacctc tgcccagatc gagctcaaca agaagcgtga agctgagatg    2760
   agcaaactca ggagggacct ggaagaagcc aacatccagc acgaaggcac gctcgccaac    2820
   ctccgcaaga agcacaacga tgctgtcagt gagatgggag accaaatcga ccagctcaac    2880
   aaacttaaga ccaaggttga aaaggagaag tctcaatacc tcggtgaact caacgacgtc    2940
   cgcgcctcca ttgaccactt gaccaacgag aaggctgcca ctgaaaaggt tgccaagcaa    3000
   ctgcaacacc aaatcaatga agttcaaggc aaacttgatg aagctaacag gacgctcaac    3060
   gacttcgatg ctgccaagaa gaagttgtct attgagaact ctgacctcct cagacagttg    3120
   gaggaagctg agagccaagt ttctcaactt agcaagatca agatctccct caccactcaa    3180
   ctcgaggaca ctaagcgtct cgccgatgag gaagctaggg aacgcgcaac ccttcttggc    3240
   aagttccgca acttggaaca cgaccttgac aacctgaggg aacaggtgga ggaagaagcc    3300
   gaagctaagg ctgatatcca acgtcaactc agcaaggcca acgctgaagc tcagttgtgg    3360
   cgcagcaagt acgaaagcga gggtgttgcc cgcgctgagg agcttgagga ggccaagagg    3420
   aaactccagg cccgtttggc tgaggctgag gagaccattg agtccctcaa ccagaaggtt    3480
   atcgcccttg agaagacgaa gcagcgcctt gccactgaag tcgaggatct gcagctcgag    3540
   gtcgaccgtg ccaacgccat tgccaatgcc gctgaaaaga aggctaaggc tattgacaag    3600
   atcattggtg aatggaaact caaggttgat gaccttgctg ctgagcttga tgctagtcaa    3660
   aaggaatgca gaaactactc cactgagctc ttcaggctca agggagctta tgaagaagga    3720
   caggaacaac ttgaagctgt ccgcagggag aacaagaacc ttgctgatga agtcaaggac    3780
   ttgctcgacc agatcggtga gggtggccgc aacatccacg aaattgagaa gcagcgcaag 3840
   aggctcgaag ttgagaagga cgaacttcag gccgctcttg aggaggctga agccgctctt 3900
   gaacaggagg agaacaaagt actcagggct caacttgagc tcagccaggt gcgtcaagaa 3960
   attgaccgcc gcatccagga gaaggaagag gagttcgaga acaccaggaa gaaccaccaa 4020
   cgggcactcg actccatgca agccagcttg gaggctgaag ccaagggtaa ggctgaggct 4080
   cttcgcatga agaagaagct tgaagccgac atcaacgaac tcgaaatcgc tctcgaccac 4140
   gccaacaagg ctaacgccga agcccagaag accatcaaga aataccaaca acaactcaag 4200
   gacgtacaga ccgctcttga ggaagaacaa agggcacgcg acgatgctcg tgaacaactc 4260
   ggaatcgccg aaaggagggc caacgctctc ggaaacgagc tcgaggagtc gcgcactctc 4320
   ctcgaacagg ccgacagggg caggcgccaa gccgaacaag aacttggaga tgcccacgaa 4380
   cagatcaacg agttggctgc ccaagctacc agcgcctcag ccgccaaacg caagcttgag 4440
   ggagagttgc agacacttca tgctgacctc gacgaacttc tcaacgaagc caagaactcc 4500
   gaagagaagg ccaagaaggc aatggttgat gccgctaggt tagctgatga actcagggca 4560
   gaacaggacc acgctcagac ccaggagaag ctccgcaagg ctctggaaac gcaaatcaag 4620
   gaactccaag tcaggttgga cgaagccgaa aacaacgccc tcaagggagg caagaaggcc 4680
   atcgccaagc tggaacaacg tgtcagggaa ctcgaaaacg aactggacgg cgagcagagg 4740
   agacacgccg acgcacaaaa gaacctccgt aaatccgagc gtagaattaa ggagctcagt 4800
   ttccagtccg acgaggaccg taagaaccac gaacgcatgc aagacctcgt agacaaactg 4860
   caacagaaga tcaagactta caagaggcag attgaagaag ccgaagaaat cgcggccctt 4920
   aacctcgcca aattccgcaa agcacaacaa gaactcgaag aagctgaaga acgcgctgat 4980
   ctcgctgaac aggctgtttc caaattcaga acaaagggtg gacgcgcagg atctgctgcc 5040
   agagcgatga gccctgtcgg ccagaagtga aggaacgaat aagcggacgt ataagctatc 5100
   aatacctcgc acacaaacct gccaggcctc aatttgacgg caatgccttc ccaccacgat 5160
   tcgatctaca tcccgacgac ttttaagatc tttgatagca acgcaaaaca tcaaatgaaa 5220
   atcttttaaa ttttatgtat ttattttgac ctattttatt aagttattgt taatacaaac 5280
   ataattccat gagctagata tctagccaac gaaccatcac aatcacgatt attcgaactg 5340
   tacgatagaa gcattatttg tacagctgga ccatttacaa aatatttttg cttcgaataa 5400
   taaagagttt atatcgcgaa aaaaaaaaaa aaaaaaaaaa aaaaaa 5446
<210> 146
   <211> 964
   <212> DNA
   <213> Lygus hesperus
<400> 146
   tcctcctctg gtgcccgact cttcaaatac ccaaatccag tcatgtcttc ccgtaaaacc     60
   gctggccgca gggcgaccac caagaagcgc gctcagcgtg cgacgtcaaa cgtattcgcc    120
   atgttcgatc aggctcagat tcaagaattc aaggaggcat tcaacatgat cgaccagaac    180
   agggacggct tcgtggataa ggaagacctc catgacatgc tcgcttccct aggtaagaac    240
   ccctcagacg agtatctcga ggggatgatg aacgaggcgc ctggtcccat caacttcaca    300
   atgttcctca ccctcttcgg tgagcggctt cagggaactg atccggagga ggttatcaag    360
   aacgcatttg ggtgttttga cgaagacaac aacggattca tcaacgagga aagactgcgc    420
   gagctgctca cctccatggg ggacaggttc actgatgaag acgtggacga aatgtaccga    480
   gaggccccca tcaagaacgg catgttcgac tacatcgaat tcactcggat cctcaagcac    540
   ggagccaaag acaaagacga gcagtgacct atcaaatcct cgtcaacctc ccttcagtaa    600
   tttgaaacca atccatcaaa ttttgtttaa aactcttact taaaatccga tcatctacgt    660
   cactttgcca ccaatcggta ttattttttg agccgttcct acataaatcg aattaatttt    720
   atacctacga atcatattgt tggaaatttc tctcttgtac ttatactttc tgttatttcc    780
   taatttttct aactaaccaa gttagtcgtt agtttttatt cattccttta taaattatta    840
   gttatccatt tttaatcatc ttgaagttat ttgtttttcg agtggtagaa tatttataca    900
   ttttccaata tataatggtt tattcattct taaaaaacga aaaaaaagaa aaaaaaaaaa    960
   aaaa                                                                 964
<210> 147
   <211> 5872
   <212> DNA
   <213> Lygus hesperus
<400> 147
   gatcttacct gcctgaacga ggcgtccgtt cttcacaaca tcaaggacag atattactcc      60
   ggattgattt atacgtattc gggactcttc tgcgtggtgg tcaaccctta caagaaactg     120
   ccaatctaca cagagagaat catggagaaa tacaaaggcg tcaaaagaca cgacctccct     180
   ccacacgtat tcgccatcac agacacagct taccgttcta tgctgcaaga tagggaagat     240
   caatcgatac tctgcaccgg cgaatcgggt gcggggaaaa ccgaaaacac gaaaaaagta     300
   atccagtact tggcctacgt tgcagcctcg aaacccaaat cttccgcatc cccacatacg     360
   gcccagagtc aagctctgat cattggagaa ctcgaacaac agctgcttca agctaaccca     420
   attttggaag cattcggaaa cgccaagact gttaaaaacg ataattcttc tcgattcggt     480
   aaattcattc gtatcaattt cgacgcatca ggctacatcg caggagccaa catagaaacg     540
   tatcttctag agaaatctag ggccatcaga caagcgaaag atgagcgaac gttccacatc     600
   ttttaccaac ttctggccgg agcatctgca gaacaaagaa aggagttcat cctcgaagat     660
   ccgaaaaact accctttcct cagcagcggg atggtgtctg tgcctggagt tgacgatggt     720
   gttgatttcc aagcaactat cgcctccatg tccatcatgg gcatgaccaa cgacgatctt     780
   tccgctctct tccgcatcgt cagtgccgtc atgctgttcg gcagcatgca gttcaagcag 840
   gagcgaaaca gcgaccaggc gacgctccca gacaacactg tagcgcaaaa aatcgcccac 900
   ctccttggtc tctcaatcac agagatgacc aaagcgttcc tcaggcctag aatcaaagta 960
   ggacgggatt tcgtcaccaa ggctcaaact aaggaacaag ttgagttcgc agtggaagcc 1020
   atttcgaaag cctgctacga acgtatgttc cgatggctcg tcaacagaat caaccgctcc 1080
   ctggatcgta ccaaaaggca gggagcatct ttcattggta ttcttgatat ggctggtttc 1140
   gaaatctttg agatcaactc cttcgagcag ctttgtatca attacaccaa tgagaaactt 1200
   caacaactct tcaaccacac catgttcatt ttggagcaag aggagtacca gagagaaggt 1260
   attgaatgga agttcatcga cttcggactt gatcttcagc cgaccattga tctcattgat 1320
   aagccaatgg gagtcatggc tctcctggat gaagaatgtt ggttccccaa agccactgac 1380
   aagaccttcg ttgagaagct ggtcggtgct cacagcgttc accccaaatt catcaaaact 1440
   gatttccgtg gagtcgccga ctttgctgtc gtccattatg ccggaaaagt cgattattcg 1500
   gcggcgcagt ggctgatgaa gaacatggac cctctgaacg aaaacgtcgt gcagctcctc 1560
   cagaactcgc aagatccgtt cgtcatccac atctggaagg acgcagagat cgtcggcatg 1620
   gctcaccaag ctctcagcga cactcagttt ggagctcgta ccaggaaggg tatgttccga 1680
   accgtgtctc aactctacaa agaccagctg tccaaactca tgatcacact tcgcaacacg 1740
   aaccccaact tcgtccgttg catcctcccc aaccacgaga agagagctgg caagatcgat 1800
   gctcctttgg tgctggatca gctcagatgc aacggtgtgt tggaaggcat cagaatttgc 1860
   agacaaggtt tcccgaatag aatcccattc caggaattcc ggcaaagata cgagctctta 1920
   actcccaatg tcatccccaa agggttcatg gacggtaaaa aggcttgcga gaagatgatc 1980
   aacgctctcg aactggaccc taatctctac agagttggtc agtccaagat attcttcaga 2040
   gctggagtct tagctcatct agaagaagag cgcgactata agattactga tctgatagcc 2100
   aatttccggg ctttctgtag ggatatcttg cccgaaggga actaccaaaa gcgtcttcag 2160
   cagctcaacg ccattcgtat tatccagcga aattgctcag cttacttgaa gttgaggaac 2220
   tggcaatggt ggcggctgta caccaaggtc aaacctctgc ttgaagtgac gaaacaagaa 2280
   gagaagctga cgcaaaagga agacgaactg aagcaggtcc gcgagaaact ggacaaccag 2340
   gtgaggtcca aggaagagta tgaaaagagg cttcaggacg ctttggagga gaaagctgct 2400
   ctggcagagc aacttcaggc agaagtagag ctgtgtgcgg aagccgaaga aatgagagcc 2460
   aggctcgctg tgaggaagca agaactagag gaaattctcc acgatctaga agccagaata 2520
   gaggaagaag agcaacgaaa cacggtcctc atcaacgaaa agaagaagtt gaccctcaac 2580
   atcgccgacc tcgaagaaca actggaagag gaagaaggag ctcgacagaa actccaactc 2640
   gaaaaagtcc agatcgaagc tcggctgaag aaaatggaag aggacctcgc tctggccgaa 2700
   gacaccaaca ccaaagtcgt aaaggagaag aaagtgttgg aagagagggc tagtgacttg 2760
   gcccagaccc tcgctgagga agaagaaaaa gctaaacacc tcgcgaagct caagaccaag 2820
   cacgagacga cgatagcgga attggaagag aggttgctca aagacaatca gcagaggcag 2880
   gaaatggata ggaacaagag gaagatcgaa tcagaggtga atgatttgaa agaacaaatt 2940
   aacgagaaga aggtccaagt agaggagctt cagttgcaac tcgggaagag ggaagaggaa 3000
   atcgctcaag ctctgatgag aattgacgag gaaggagcag gcaaagctca gactcaaaag 3060
   gctctcaggg aattggagtc tcagctggct gagctacaag aggatctaga ggctgaaaag 3120
   gccgctcgcg ccaaggccga aaagcagaag cgcgacctca acgaagaact cgagtccctc 3180
   aagaatgaac ttcttgactc actggacacg acagcagctc aacaggaatt gaggaccaag 3240
   agagaacacg aactggcaac gctcaagaaa acattagaag aggaaacgca cattcacgaa 3300
   gtatctctca ccgaaatgag gcacaaacac actcaagaag tcgctgcact caacgaacag 3360
   ttggagcaac tcaaaaaggc caaatctgca ctcgaaaaat cgaaagcaca acttgaaggg 3420
   gaagctgctg agctcgccaa cgaactggaa acagcaggaa cgagcaaggg cgagagtgaa 3480
   aggaaacgga agcaggccga atcgtctctg caggagctct cgtcgcgact cttggaaatg 3540
   gagagaacca aagccgagct ccaagagagg gtccagaaac tgtctgcaga agccgactct 3600
   gtcaatcagc agttggaagc agcggaactg aaagcatcag cagccctcaa ggcatctggt 3660
   accttggaga ctcagctcca ggaggcgcaa gtgctcctgg aagaggaaac tcggcagaag 3720
   ctgtcgttga ccaccaaact gaaaggcctc gaaagcgaaa gagatgctct caaagagcaa 3780
   ctctacgaag aggacgaggg taggaagaac ctagaaaaac agatggcgat actcaatcaa 3840
   caagtagctg aaagcaagaa gaagtctgaa gaagaaacgg aaaaaataac tgaactcgaa 3900
   gaaagtcgca aaaaattgct caaagacata gaaattcttc aaaggcaagt cgaagaactt 3960
   caagttacca acgacaaatt agagaaaggc aagaagaagc tgcagtcaga actggaagac 4020
   ctcaccatcg acctggagtc tcagagaaca aaggtggtcg agctcgagaa gaaacaaaga 4080
   aatttcgaca aagttttggc cgaagaaaaa gcgttgtcgc aacaaatcac gcacgagagg 4140
   gatgcggctg aaagagaagc ccgtgaaaag gaaactagag tactgtcgct gacgcgagaa 4200
   ctcgatgaat tcatggagaa aatcgaggaa ctggagagaa gcaaacggca actccaggct 4260
   gaactagacg agctggtcaa caaccaaggc accaccgaca aaagcgtgca cgaattggaa 4320
   agggcgaaac gagttctgga gtcacaactt gcagagcaga aagcacaaaa tgaagagctt 4380
   gaagatgaac tccaaatgac ggaagacgcc aaattgaggc tcgaagtcaa catgcaagct 4440
   ctgagagctc aattcgaaag agatctacag ggcaaagaag agtcgggaga agaaaagagg 4500
   agaggattgc tgaaacagct gagggacatt gaggctgaac ttgaagacga gagaaaacaa 4560
   aggaccgctg ctgttgcctc tagaaagaag attgaagcgg atttcaaaga tgtagaacag 4620
   caactggaaa tgcacactaa ggtaaaggaa gatcttcaga agcaactgaa gaaatgccag 4680
   gtccaactga aggacgcaat cagagacgcg gaagaggctc ggctcggtcg ggaagagctg     4740
   caggctgccg ctaaagaggc cgaaaggaag tggaagggtt tggaaacgga gctcattcaa     4800
   gtgcaagagg atttgatggc gagcgaaagg cagcggcggg cagcggaagc cgaaagggat     4860
   gaagtcgttg aagaagccaa caagaatgtc aagagcttat cgaatcttct cgacgaaaag     4920
   aagaggctcg aagcccaatg ctcaggcctg gaagaggaac tcgaagaaga acttagcaac     4980
   aatgaggccc tccaagacaa agcgagaaaa gcacaactca gcgttgagca acttaatgca     5040
   gaacttgctg ccgaacggag taatgtgcag aaacttgagg gaacgagatt gtcgatggaa     5100
   aggcaaaaca aggaactgaa ggccaaactg aacgaactgg aaacgttaca acgcaacaag     5160
   ttcaaggcca atgcgtctct ggaggctaag attaccaatc ttgaagagca actggaaaat     5220
   gaagccaagg aaaagctact tctccagaaa ggcaacagga agctcgacaa gaaaatcaaa     5280
   gacctcctcg ttcaattgga ggatgaaagg aggcatgccg accagtataa agaacaagtc     5340
   gagaagatca acgtcagggt gaagacgcta aagcgaactt tggacgacgc cgaagaagaa     5400
   atgagtaggg agaagaccca gaagaggaaa gcacttcgcg aattggaaga cctcagggag     5460
   aactacgatt ccctactccg agagaacgat aacctcaaaa acaaactcag gcggggcggc     5520
   ggtatttccg ggatctcgag caggctcgga ggctccaagc gaggttccat ccccggagag     5580
   gattcccagg gtctcaacaa caccacagac gaatcagtcg atggtgacga tatctcgaat     5640
   ccttaaacgc tacttggatt taccagccag catccaactt tccactgaag acgtctccca     5700
   taaacgttga aagagacccg tcgaggaaga aaaaaaggct ctttaagaaa aactattctg     5760
   cctttttcaa aactttgtac ttaaaagtac tttcgcttaa caatgaaaga agaataaaaa     5820
   tgtaaagttt tcatttatac aaaaaaaaaa aaaaaaaaaa aaaaaaaaaa aa             5872
<210> 148
   <211> 325
   <212> DNA
   <213> Lygus hesperus
<400> 148
   aagaagatcc tggaggagat cattgcggag gtcgacgctg atggctctgg acaacttgaa       60
   tttgaggagt tcgtagcctt ggctgccggt ttcctgacag aagatgagac acaagatgct      120
   gaggccatgc agcaagaact gagggaggcc ttcaggctct acgacaagga agggaacggt      180
   tacattacca ctgacgtctt gagggaaatc ctgaaggaac tcgacgacaa aatcacctcc      240
   caagaacttg acatgatgat tgctgaaatt gattctgacg gttccggaac tgttgacttc      300
   gacgagttca tggagatgat gaccg                                            325
<210> 149
   <211> 428
   <212> DNA
   <213> Lygus hesperus
<400> 149
   aattatcccc atcatgacca tcgcgttgaa cgctttcgac cgggatcact cgggttccat        60
   cccgacggac atggtggccg acatcctcag actcatgggt caacccttca acaagaagat       120
   tctcgatgaa ctcatcgaag aagttgacgc tgacaaatcg ggaaggctgg agtttgaaga       180
   gttcatcacg ttggcagcca agttcattgt tgaggaagac gatgaagcca tgcagaaaga       240
   gctgcgagaa gccttccgtc tctacgacaa agaaggtaat ggctacatcc cgacctcctg       300
   cctcaaagaa atccttcatg aattggacga gcagctgacc aacgaggagc tggacatgat       360
   catcgaggag atcgactctg acggatccgg aactgtcgac ttcgacgagt tcatggagat       420
   gatgaccg                                                                428
<210> 150
   <211> 538
   <212> DNA
   <213> Lygus hesperus
<400> 150
   gcttctttta caaatcgcac cacgccgact taattcattc ccggagggtt taaattttat        60
   cgaagcagca tggtgcggat gaatgtgctg agcgatgctc tgaaaagcat caacaatgct       120
   gagaagaggg gcaaaaggca ggtgctcctg aggccttgtt ccaaagtcat cattaaattc       180
   cttacagtga tgatgaagaa aggttatatc ggcgaattcg aaatagtaga tgatcacaga       240
   tctggtaaaa tcgtcgtcaa cctcaacggc agattgaaca aatgtggagt tatatcgccc       300
   agattcgacg tacccatcac acaaatcgaa aaatggacga acaacctcct gccttcccga       360
   cagttcggtt atgtcgtact caccactagt ggagggatca tggatcacga agaagccagg       420
   cgaaaacatc ttgggggtaa aatattaggg tttttctttt aataaaaaaa gacgagatgt       480
   aaattaataa aactctttta cgtttcgcta aaaaaaaaaa aaaaaaaaaa aaaaaaaa         538
<210> 151
   <211> 405
   <212> DNA
   <213> Lygus hesperus
<400> 151
   ccacgccgac ttaattcatt cccggagggt ttaaatttta tcgaagcagc atggtgcgga 60
   tgaatgtgct gagcgatgct ctgaaaagca tcaacaatgc tgagaagagg ggcaaaaggc 120
   aggtgctcct gaggccttgt tccaaagtca tcattaaatt ccttacagtg atgatgaaga 180
   aaggttatat cggcgaattc gaaatagtag atgatcacag atctggtaaa atcgtcgtca 240
   acctcaacgg cagattgaac aaatgtggag ttatatcgcc cagattcgac gtacccatca 300
   cacaaatcga aaaatggacg aacaacctcc tgccttcccg acagttcggt tatgtcgtac 360
   tcaccactag tggagggatc atggatcacg aagaagccag gcgaa 405
<210> 152
   <211> 470
   <212> DNA
   <213> Lygus hesperus
<400> 152
   tgtcgatggc ggtcttaaca tcccccattc caccaagagg ttccctgggt acgacagtga 60
   gtctaaggaa ttcaacgctg aggtccacag gaagcacatt ttcggcattc acgtcgctga 120
   ctacatgcgt cagctggctg aagaggatga cgatgcttac aagaagcagt tctcgcagta 180
   tgtcaagaac ggagtcactg ctgacagcat tgaaagtatc tacaagaagg ctcacgaagc 240
   aatccgagct gatccaactc gcaaaccact tgagaagaag gaagtcaaga agaagaggtg 300
   gaaccgcgcc aagctttcct tgtctgaaag gaagaacacc atcaaccaaa agaaggcaac 360
   ttatctcaag aaagtggaag ctggagaaat cgaataagtt tttatattcc tgacattacc 420
   cattaaaggt ttcgttttaa cctaaaaaaa aaaaaaaaaa aaaaaaaaaa 470
<210> 153
   <211> 387
   <212> DNA
   <213> Lygus hesperus
<400> 153
   tgtcgatggc ggtcttaaca tcccccattc caccaagagg ttccctgggt acgacagtga 60
   gtctaaggaa ttcaacgctg aggtccacag gaagcacatt ttcggcattc acgtcgctga 120
   ctacatgcgt cagctggctg aagaggatga cgatgcttac aagaagcagt tctcgcagta 180
   tgtcaagaac ggagtcactg ctgacagcat tgaaagtatc tacaagaagg ctcacgaagc 240
   aatccgagct gatccaactc gcaaaccact tgagaagaag gaagtcaaga agaagaggtg 300
   gaaccgcgcc aagctttcct tgtctgaaag gaagaacacc atcaaccaaa agaaggcaac 360
   ttatctcaag aaagtggaag ctggaga 387
<210> 154
   <211> 745
   <212> DNA
   <213> Lygus hesperus
<400> 154
   gtcctacgtg tttccggaaa aacgtgcatt tcgcgtaccc ctcgtggtga tccgttttca      60
   tagaaataat ccaaaatggc tcccaagggg aataatatga ttcccaatgg ccatttccac     120
   aaggattggc agaggttcat caaaacctgg ttcaaccagc ctgcccgcaa gttgaggagg     180
   agaaacaaga ggttggagaa ggcccaacgg ctcgcgcccc gccccgcggg acctcttcgc     240
   cccgctgtca gatgtcccac cgtcaggtac cacaccaagc tacgacctgg acgtggcttc     300
   accttggaag aaatcaagag agccggtctg tgcaaaggat tcgcgatgtc catcggaatc     360
   gctgtcgacc ccagaagaag gaataaatcc atcgagtccc tccaactcaa tgtacagaga     420
   ctcaaggagt acagggctaa gcttatcctc ttcccacaca agaatgccaa gaaactgaag     480
   aagggagaag ctactgagga agagaggaag gtggccaccc aacagcccct gccagttatg     540
   cccatcaagc aaccagtcat caaattcaag gctcgcgtca ttacagacga tgagaagaaa     600
   tactctgcct tcaccgccct ccgcaaggga cgagcagacc aaaggttggt cggtatccgt     660
   gctaagcgcg caaaggaagc cgcagaaaac gccgaagacc cctctaaagc tcctaaaaaa     720
   aaaaaaaaaa aaaaaaaaaa aaaaa                                           745
<210> 155
   <211> 527
   <212> DNA
   <213> Lygus hesperus
<400> 155
   aacgtgcatt tcgcgtaccc ctcgtggtga tccgttttca tagaaataat ccaaaatggc      60
   tcccaagggg aataatatga ttcccaatgg ccatttccac aaggattggc agaggttcat     120
   caaaacctgg ttcaaccagc ctgcccgcaa gttgaggagg agaaacaaga ggttggagaa     180
   ggcccaacgg ctcgcgcccc gccccgcggg acctcttcgc cccgctgtca gatgtcccac     240
   cgtcaggtac cacaccaagc tacgacctgg acgtggcttc accttggaag aaatcaagag     300
   agccggtctg tgcaaaggat tcgcgatgtc catcggaatc gctgtcgacc ccagaagaag     360
   gaataaatcc atcgagtccc tccaactcaa tgtacagaga ctcaaggagt acagggctaa     420
   gcttatcctc ttcccacaca agaatgccaa gaaactgaag aagggagaag ctactgagga     480
   agagaggaag gtggccaccc aacagcccct gccagttatg cccatca                   527
<210> 156
   <211> 576
   <212> DNA
   <213> Lygus hesperus
<400> 156
   gccttattga acgtggtcga cagaaaactc ggtttctgag ctcatctcaa catggatatc      60
   gaagaaccgg ccgcggcccc tacggagccc tcggacgtca acaccgccct tcaagaggtc     120
   ctcaaggccg cccttcaaca cggagtcgtc gtccacggta tccacgagtc cgccaaggcc     180
   ctcgacaaga ggcaagcttt gttgtgcgtc ctcgctgaga actgcgacga gccgatgtac     240
   aagaagctgg tacaagccct ctgctcagag caccacatcc ccctcgtcaa agtagattcc     300
   aataagaaac tcggcgaatg gacgggcctt tgcaagatcg acaagaccgg caaatctagg     360
   aaaatcgtcg gctgctcttg tgtcgtcatc aaggactggg gtgaggacac gccccacttg     420
   gacctcctca aggactacat cagggacgtc ttctaagaag tttctcctca atttcctttt     480
   tataatgatt taacaactga gaattaataa taaaaatgtt aaattaaaca aaaaaatctc     540
   aaaactgtaa aaaaaaagaa gaaaaaaaaa aaaaaa                               576
<210> 157
   <211> 442
   <212> DNA
   <213> Lygus hesperus
<400> 157
   ccttattgaa cgtggtcgac agaaaactcg gtttctgagc tcatctcaac atggatatcg      60
   aagaaccggc cgcggcccct acggagccct cggacgtcaa caccgccctt caagaggtcc     120
   tcaaggccgc ccttcaacac ggagtcgtcg tccacggtat ccacgagtcc gccaaggccc     180
   tcgacaagag gcaagctttg ttgtgcgtcc tcgctgagaa ctgcgacgag ccgatgtaca     240
   agaagctggt acaagccctc tgctcagagc accacatccc cctcgtcaaa gtagattcca     300
   ataagaaact cggcgaatgg acgggccttt gcaagatcga caagaccggc aaatctagga     360
   aaatcgtcgg ctgctcttgt gtcgtcatca aggactgggg tgaggacacg ccccacttgg     420
   acctcctcaa ggactacatc ag                                              442
<210> 158
   <211> 601
   <212> DNA
   <213> Lygus hesperus
<400> 158
   ctttcatttg tatagtacgg acgggtagtt tagttgtgtc ggttcatcgt aattcatcgg 60
   ctgaatcatg aagatgaata aattggtcac ttcctcgagg aggaagaaca ggaagaggca 120
   cttcaccgcc ccatcccaca tccgtagaaa gttgatgtcg gcaccactgt ccaaagaact 180
   taggcagaag tacaacgtcc gaactatgcc tgtgaggaag gacgatgaag tccaggttgt 240
   acgaggacac tacaaaggcc aacaggttgg caaagtcctc caggtgtaca ggaagaagtt 300
   cattatttac attgagcgga tccaaagaga aaaagccaat ggtgccagcg tttacgttgg 360
   cattcacccc tcaaagtgtg tgatcgtcaa attgaaggtc gacaaggata ggaaagaaat 420
   ccttgacaga agatccaaag gacgtgactt ggcacttggc aaggacaagg gcaaatacac 480
   cgaagacagt acgactgcta tggacacgtc ttaaattaat ttggtttatt tggttcctta 540
   actccgttct tctttaataa tgactttttt aaagcaaaaa aaaaaaaaaa aaaaaaaaaa 600
   a 601
<210> 159
   <211> 448
   <212> DNA
   <213> Lygus hesperus
<400> 159
   gtacggacgg gtagtttagt tgtgtcggtt catcgtaatt catcggctga atcatgaaga 60
   tgaataaatt ggtcacttcc tcgaggagga agaacaggaa gaggcacttc accgccccat 120
   cccacatccg tagaaagttg atgtcggcac cactgtccaa agaacttagg cagaagtaca 180
   acgtccgaac tatgcctgtg aggaaggacg atgaagtcca ggttgtacga ggacactaca 240
   aaggccaaca ggttggcaaa gtcctccagg tgtacaggaa gaagttcatt atttacattg 300
   agcggatcca aagagaaaaa gccaatggtg ccagcgttta cgttggcatt cacccctcaa 360
   agtgtgtgat cgtcaaattg aaggtcgaca aggataggaa agaaatcctt gacagaagat 420
   ccaaaggacg tgacttggca cttggcaa 448
<210> 160
   <211> 456
   <212> DNA
   <213> Lygus hesperus
<400> 160
   ggctgttgtc ggctggtcat atcccgtttt ccacgtggtg tgtcgagtta tttttcttgt 60
   aaattcgcat ttaaaatcgg atttataacc gaaattcatt atggaaaagc cagtagtttt 120
   ggcccgtgtc atcaaaatcc tcggacgtac cggctcacag ggccaatgta cgcaagtgaa 180
   ggtggagttc attggtgagc agaaccgaca gatcatcagg aacgtgaaag gaccagttag 240
   agaaggcgac atcctcacac tcctagagtc tgaaagagaa gcgagaagac tgaggtagtg 300
   ggaggtggcg atgcgttacg ttattttact tcattcaaca tttgaaaaaa accatcttcg 360
   tgacaaaaaa catcttcacg caactatttg tattacctat gtttcgtaaa taaagtaacc 420
   tcgttactta aaaaaaaaaa aaaaaaaaaa aaaaaa 456
<210> 161
   <211> 321
   <212> DNA
   <213> Lygus hesperus
<400> 161
   ctgttgtcgg ctggtcatat cccgttttcc acgtggtgtg tcgagttatt tttcttgtaa 60
   attcgcattt aaaatcggat ttataaccga aattcattat ggaaaagcca gtagttttgg 120
   cccgtgtcat caaaatcctc ggacgtaccg gctcacaggg ccaatgtacg caagtgaagg 180
   tggagttcat tggtgagcag aaccgacaga tcatcaggaa cgtgaaagga ccagttagag 240
   aaggcgacat cctcacactc ctagagtctg aaagagaagc gagaagactg aggtagtggg 300
   aggtggcgat gcgttacgtt a 321
<210> 162
   <211> 865
   <212> DNA
   <213> Lygus hesperus
<400> 162
   aatcccggat tcatcgtttt attgaattgt ttttcgaagt ttctggtatt atcgttaaat 60
   tagtctgtta agccctcatc cgtgatttgg caagttgttg attgttctat tttccttttt 120
   ccagaaaatg gggagacgtc cagcgaggtg ttatcggtac tgtaaaaaca agccataccc 180
   ccaaatcccg gttctgtcgt ggtgtccccg accccaagat caggatcttc gatctgggaa 240
   agaagaaggc ccgcgtggaa gacttccccc tctgcgttca cctcgtctcc gatgagtacg 300
   agcagctgtc ctccgaagcc ctcgaggcag gacgtatctg ctgcaacaag tacctcgtca 360
   agaactgcgg caaggaccag ttccacatca ggatgaggct ccaccccttc cacgtcatta 420
   ggatcaacaa aatgttatcg tgcgctggag ctgataggct ccagacaggg atgagaggag 480
   cgttcggaaa gccgcaagga accgtcgctc gcgtccgcat cggtcagccc atcatgagcg 540
   tccgctcgtc cgacaggtac aaggccgccg tcatcaaggc tctgaggaga gccaaattca 600
   agttccctgg tcgccagaag atctacgttt ccaagaaatg gggcttcacc aagttcgacc 660
   gcgaagagta cgagggcctt aggaacgaca acaaactagc gaatgacggc tgcaacgtca 720
   aattgaggcc ggatcacgga cctttgcagg cgtggaggaa ggctcagctt gacatcgctg 780
   ctggcctcta aattactttc caatggtttt ataaatcaac aaataaaact cgttttatgt 840
   aaaaaaaaaa aaaaaaaaaa aaaaa 865
<210> 163
   <211> 792
   <212> DNA
   <213> Lygus hesperus
<400> 163
   ggttcctttc tcagattttg actttgccgt gttgtctctc ccaattttcc aaaatgggga 60
   gacgtccagc gaggtgttat cggtactgta aaaacaagcc ataccccaaa tcccggttct 120
   gtcgtggtgt ccccgacccc aagatcagga tcttcgatct gggaaagaag aaggcccgcg 180
   tggaagactt ccccctctgc gttcacctcg tctccgatga gtacgagcag ctgtcctccg 240
   aagccctcga ggcaggacgt atctgctgca acaagtacct cgtcaagaac tgcggcaagg 300
   accagttcca catcaggatg aggctccacc ccttccacgt cattaggatc aacaaaatgt 360
   tatcgtgcgc tggagctgat aggctccaga cagggatgag aggagcattc ggaaagccgc 420
   aaggaaccgt cgctcgcgtc cgcatcggtc agcccatcat gagcgtccgc tcgtccgaca 480
   ggtacaaggc cgccgtcatc gaggctctga ggagagccaa attcaagttc cctggtcgcc 540
   agaagatcta cgtttccaag aaatggggct tcaccaagtt cgaccgcgaa gagtacgagg 600
   gccttaggaa cgacaacaaa ctagcgaatg gcggctgcaa cgtcaaattg aggccggatc 660
   acggaccttt gcaggcgtgg aggaaggctc agcttgacat cgctgctggc ctctaaatta 720
   ctttccaatg gttttataaa tcaacaaata aaactcgttt tatctaaaaa aaaaaaaaaa 780
   aaaaaaaaaa aa 792
<210> 164
   <211> 645
   <212> DNA
   <213> Lygus hesperus
<400> 164
   agccctcatc cgtgatttgg caagttgttg attgttctat tttccttttt ccagaaaatg      60
   gggagacgtc cagcgaggtg ttatcggtac tgtaaaaaca agccataccc ccaaatcccg     120
   gttctgtcgt ggtgtccccg accccaagat caggatcttc gatctgggaa agaagaaggc     180
   ccgcgtggaa gacttccccc tctgcgttca cctcgtctcc gatgagtacg agcagctgtc     240
   ctccgaagcc ctcgaggcag gacgtatctg ctgcaacaag tacctcgtca agaactgcgg     300
   caaggaccag ttccacatca ggatgaggct ccaccccttc cacgtcatta ggatcaacaa     360
   aatgttatcg tgcgctggag ctgataggct ccagacaggg atgagaggag cgttcggaaa     420
   gccgcaagga accgtcgctc gcgtccgcat cggtcagccc atcatgagcg tccgctcgtc     480
   cgacaggtac aaggccgccg tcatcaaggc tctgaggaga gccaaattca agttccctgg     540
   tcgccagaag atctacgttt ccaagaaatg gggcttcacc aagttcgacc gcgaagagta     600
   cgagggcctt aggaacgaca acaaactagc gaatgacggc tgcaa                     645
<210> 165
   <211> 619
   <212> DNA
   <213> Lygus hesperus
<400> 165
   gctttaccga ttccgttctt gtttagtcca cgtttctctg ctcattcgtg cagattttaa      60
   aacatgacca actccaaagg ttatcgtcgc ggaacgaggg atctcttctc gaggcccttc     120
   cgtcaccatg gtgtcatccc actctcaacg tacatgaaag tataccgagt aggagacatc     180
   gtatctatca aaggtaatgg agcagtgcaa aaaggtatgc cccacaaagt ttaccacggc     240
   aagaccggac gagtctacaa tgttacacct cgcgcccttg gtgttattgt caacaagagg     300
   gttcgtggaa aaatccttcc caagaggatc aacatcagga ttgaacacgt caaccacagt     360
   aaatgcagag aagatttctt gaagcgagtg cgagaaaatg aaaggctccg caaattcgcc     420
   aaagaaactg gcaccagggt tgaactcaaa agacagcctg ctcagccacg ccctgcacac     480
   tttgtacaag ctaaagaagt cccagagctg ctggccccca taccttacga gttcatcgct     540
   taaaaaattt tcaattccat cttaacttta tatatttgaa taaaattgtg ttctcaaaaa     600
   aaaaaaaaaa aaaaaaaaa                                                  619
<210> 166
   <211> 461
   <212> DNA
   <213> Lygus hesperus
<400> 166
   acgtttctct gctcattcgt gcagatttta aaacatgacc aactccaaag gttatcgtcg      60
   cggaacgagg gatctcttct cgaggccctt ccgtcaccat ggtgtcatcc cactctcaac     120
   gtacatgaaa gtataccgag taggagacat cgtatctatc aaaggtaatg gagcagtgca     180
   aaaaggtatg ccccacaaag tttaccacgg caagaccgga cgagtctaca atgttacacc     240
   tcgcgccctt ggtgttattg tcaacaagag ggttcgtgga aaaatccttc ccaagaggat     300
   caacatcagg attgaacacg tcaaccacag taaatgcaga gaagatttct tgaagcgagt     360
   gcgagaaaat gaaaggctcc gcaaattcgc caaagaaact ggcaccaggg ttgaactcaa     420
   aagacagcct gctcagccac gccctgcaca ctttgtacaa g                          461
<210> 167
   <211> 481
   <212> DNA
   <213> Lygus hesperus
<400> 167
   caacgtacat gaaagtatac cgagtaggag acatcgtatc tatcaaaggt aatggagcag      60
   tgcaaaaagg tatgccccac aaagtttacc acggcaagac cggacgagtc tataatgtta     120
   cacctcgcgc ccttggtgtt attgtcaaca agagggttcg tggaaaaatc cttcccaaga     180
   ggatcaacat caggattgaa cacgtcaacc acagtaaatg cagagaagat ttcttgaagc     240
   gagtgcgaga aaatgagagg ctccgcaaat tcgccaaaga aactggcacc agggttgaac     300
   tcaaaagaca gcctgctcag ccacgccctg cacactttgt acaagctaaa gaagtcccag     360
   agctgctggc ccccatacct tacgagttca tcgcttaaac aattttcaat tccatcttaa     420
   ctttatatat ttgaataaaa ttgtgttccc taaaaaaaaa aaaaaaaaaa aaaaaaaaaa     480
   a                                                                      481
<210> 168
   <211> 747
   <212> DNA
   <213> Lygus hesperus
<400> 168
   gcataaatat atagggcgat tgatttagcg gccgcgaatt cgcccttaag cagtggtatc        60
   aacgcagagg gggggtcttc tctcccggtt ttcttcttgc ccgaatcgtc catcctgatg       120
   ttggggtcac tgtcaccacg accatacccc aatttggggt atggcttggt tgtcccctac       180
   ccataaatcc tgattggaca tctccccatt atgaaagact gcgagaaaca cccctgcccc       240
   cggctttaaa cccacggcta aggggggatt cgcgggcggc aaatttcatt cggcccatag       300
   tgagtcgtat tacaattcac tgggcgtcct ttttacacct tcggaccggg aaaaacctgg       360
   cggttaccca aaatccgtta tttgccacat ccccctttac tccactgggt tatataacaa       420
   agaggcccct tccaatgtcc tttcccaaaa gtgcgcagcc ctatactaat ggcctttaaa       480
   ggaaccccta ttaaaaaaaa aacccttaac cacaggttgg tgatgtaacc aaggaaaata        540
   atgaacacac cgggccaaag aaggtgatac ccctggtctt ggcgaccgcc tgtcaaatct       600
   tcctcccgga acgaaacccg tagtggcatc gaggaataac cttgcgcatc atagactcca       660
   aatggccact gtggccgctc tcgattcatg gaagaaatga gatgacccct accccgcgca       720
   aaaggattca gaaccaatac cagaatc                                           747
<210> 169
   <211> 1052
   <212> DNA
   <213> Lygus hesperus
<220>
   <221> misc_feature
   <222> (662)..(665)
   <223> n is a, c, g, or t
<400> 169
   ggttttcttc ttgcccgaat cgtccatcct gatgttgggg tcactgtcac cacgaccata 60
   ccccaatttg gggtatggct tggttgtccc ctacccataa atcctgattg gacatctccc 120
   cattatgaaa gactgcgaga aacacccctg cccccggctt taaacccacg gctaaggggg 180
   gattcgcggg cggcaaattt cattcggccc atagtgagtc gtattacaat tcactgggcg 240
   tcctttttac accttcggac cgggaaaaac ctggcggtta cccaaaatcc gttatttgcc 300
   acatccccct ttactccact gggttatata acaaagaggc cccttccaat gtcctttccc 360
   aaaagtgcgc agccctatac taatggcctt taaaggaacc cctattaaaa aaaaaaccct 420
   taaccacagg ttggtgatgt aaccaaggaa aataatgaac acaccgggcc aaagaaggtg 480
   atacccctgg tcttggcgac cgcctgtcaa atcttcctcc cggaacgaaa cccgtagtgg 540
   catcgaggaa taaccttgcg catcatagac tccaaatggc cactgtggcc gctctcgatt 600
   catggaagaa atgagatgac ccctaccccg cgcaaaagga ttcagaacca ataccagaat 660
   cnnnntagca aaacggctat ttcccggttc tttgtcggat tcttttgcca gggccatgcc 720
   ttttcccgga atggaaggcg ggctgtttga gaaacgcatt aaatgggatt agtccattca 780
   taggccaccc aaggaaacca ctttaatttc gggttggtag gttgagagaa atggtgaggg 840
   gtaacaattt tacaccggga accgtttatg cccagaatta ccccagcttc gaattaaccc 900
   cccctaaagg ggatagttcc gccgggttaa aagaaattcg ccttaaacca gtgttttaaa 960
   gcaggagaca gaagtgtttc tcgcaagctt tcaaaatggg gagatgtcca atcaggattt 1020
   atgggtaggg tacaaccaag ccgaacccca aa 1052
<210> 170
   <211> 555
   <212> DNA
   <213> Lygus hesperus
<400> 170
   tagcaaaacg gctatttccc ggttctttgt cggattcttt tgccagggcc atgccttttc 60
   ccggaatgga aggcgggctg tttgagaaac gcattaaatg ggattagtcc attcataggc 120
   cacccaagga aaccacttta atttcgggtt ggtaggttga gagaaatggt gaggggtaac 180
   aattttacac cgggaaccgt ttatgcccag aattacccca gcttcgaatt aaccccccct 240
   aaaggggata gttccgccgg gttaaaagaa attcgcctta aaccagtgtt ttaaagcagg 300
   agacagaagt gtttctcgca agctttcaaa atggggagat gtccaatcag gatttatggg 360
   tagggtacaa ccaagccgaa ccccaaatcc ctgttctgtc gtggtgacag tgaccccaag 420
   atctggatgt tcgttttggg aaagaagaaa accgggaggg accacttcct cctctgcgtt 480
   gataccactg cttaagggcg aattcgttta aacctgcagg actagtccct tagtgagggt 540
   aatctagcag cccac 555
<210> 171
   <211> 1052
   <212> DNA
   <213> Lygus hesperus
<220>
   <221> misc_feature
   <222> (662)..(665)
   <223> n is a, c, g, or t
<400> 171
   ggttttcttc ttgcccgaat cgtccatcct gatgttgggg tcactgtcac cacgaccata 60
   ccccaatttg gggtatggct tggttgtccc ctacccataa atcctgattg gacatctccc 120
   cattatgaaa gactgcgaga aacacccctg cccccggctt taaacccacg gctaaggggg 180
   gattcgcggg cggcaaattt cattcggccc atagtgagtc gtattacaat tcactgggcg 240
   tcctttttac accttcggac cgggaaaaac ctggcggtta cccaaaatcc gttatttgcc 300
   acatccccct ttactccact gggttatata acaaagaggc cccttccaat gtcctttccc 360
   aaaagtgcgc agccctatac taatggcctt taaaggaacc cctattaaaa aaaaaaccct 420
   taaccacagg ttggtgatgt aaccaaggaa aataatgaac acaccgggcc aaagaaggtg 480
   atacccctgg tcttggcgac cgcctgtcaa atcttcctcc cggaacgaaa cccgtagtgg 540
   catcgaggaa taaccttgcg catcatagac tccaaatggc cactgtggcc gctctcgatt 600
   catggaagaa atgagatgac ccctaccccg cgcaaaagga ttcagaacca ataccagaat 660
   cnnnntagca aaacggctat ttcccggttc tttgtcggat tcttttgcca gggccatgcc 720
   ttttcccgga atggaaggcg ggctgtttga gaaacgcatt aaatgggatt agtccattca 780
   taggccaccc aaggaaacca ctttaatttc gggttggtag gttgagagaa atggtgaggg 840
   gtaacaattt tacaccggga accgtttatg cccagaatta ccccagcttc gaattaaccc 900
   cccctaaagg ggatagttcc gccgggttaa aagaaattcg ccttaaacca gtgttttaaa 960
   gcaggagaca gaagtgtttc tcgcaagctt tcaaaatggg gagatgtcca atcaggattt 1020
   atgggtaggg tacaaccaag ccgaacccca aa 1052
<210> 172
   <211> 1175
   <212> DNA
   <213> Lygus hesperus
<400> 172
   ctcagcgaga tccctaagac aacgcctgcc acgtgggaga atatcggaca cgcctcccca      60
   gagtgcggaa aggggaacgg cgttccgtat cggtcaaggt gcaagcttcg gaaccggagg     120
   acgaccgttg caaggtgcaa ggggcaggta tcttgtattt tcattgtgcg tgtcgacatc     180
   taccaaactg agacttggag ttcgatattt tgacgatggg gccgggggcc ggaggcaaaa     240
   cgacaaacac aggcaccgtg accgtgttcc ggtccctggc ctgcgttgcc ttacgttcac     300
   atcttgttct tgcgctttct ctggttttac gataacccta ctacgagttt agtagagccg     360
   atcccgtagc cgaagccaaa gcccaagcgc tccgtatccg agaacgcgga agagcacgaa     420
   ctccccaaac ccctccgccc ctcccccgcg cgtatccgaa acacaaatgc agcgggcagt     480
   acaggttttg gaaggggacg cgggcagtga gcgcaatgca agtaaatgtg attagctcat     540
   ggctacgcag ccctgctttt tcagtttcgg ttcggatcgt tagggggtgt gggattggga      600
   gcggattcaa tctggacagg aaacagctat gaccaaggtc acgccaagct ctgaattaac     660
   cctcaggaaa gggactagtc cggcaggttg aaacgaactc gcccctaagc agtggtatca     720
   gagcacagtg gttttttttt tttgtttttt ttcgtagaaa aaaatatgta ttaagtcaat      780
   taattaaatc attggttttc tggcttcaca acaggtggca cgtgctgtgc tcggagaaat     840
   ttatgaacta tgttctgttc ttcaatgagg aaagatgaga tgatccattc tcagacacat     900
   tcagacagag gacaccaccg taagccctat ccacagtctg tccacgtaag gggatcgtgt     960
   ccccttccat gggcagagca gggagagggc cgtaagcttg ttcttgcgtc atcaacatgt    1020
   gggggtaatg ttggtcatag cgatgttcgg tacacaagag aaccacctgg tgtaatcatt    1080
   acagcacagc aatactctgt gttttgtaag ataacaaaaa aggtacttaa gacgctgaac    1140
   cattttctac gatcggaaaa caaaaaaaaa gaaaa                               1175
<210> 173
   <211> 1023
   <212> DNA
   <213> Lygus hesperus
<400> 173
   tcagcgagat ccctaagaca acgcctgcca cgtgggagaa tatcggacac gcctccccag 60
   agtgcggaaa ggggaacggc gttccgtatc ggtcaaggtg caagcttcgg aaccggagga 120
   cgaccgttgc aaggtgcaag gggcaggtat cttgtatttt cattgtgcgt gtcgacatct 180
   accaaactga gacttggagt tcgatatttt gacgatgggg ccgggggccg gaggcaaaac 240
   gacaaacaca ggcaccgtga ccgtgttccg gtccctggcc tgcgttgcct tacgttcaca 300
   tcttgttctt gcgctttctc tggttttacg ataaccctac tacgagttta gtagagccga 360
   tcccgtagcc gaagccaaag cccaagcgct ccgtatccga gaacgcggaa gagcacgaac 420
   tccccaaacc cctccgcccc tcccccgcgc gtatccgaaa cacaaatgca gcgggcagta 480
   caggttttgg aaggggacgc gggcagtgag cgcaatgcaa gtaaatgtga ttagctcatg 540
   gctacgcagc cctgcttttt cagtttcggt tcggatcgtt agggggtgtg ggattgggag 600
   cggattcaat ctggacagga aacagctatg accaaggtca cgccaagctc tgaattaacc 660
   ctcaggaaag ggactagtcc ggcaggttga aacgaactcg cccctaagca gtggtatcag 720
   agcacagtgg tttttttttt ttgttttttt tcgtagaaaa aaatatgtat taagtcaatt 780
   aattaaatca ttggttttct ggcttcacaa caggtggcac gtgctgtgct cggagaaatt 840
   tatgaactat gttctgttct tcaatgagga aagatgagat gatccattct cagacacatt 900
   cagacagagg acaccaccgt aagccctatc cacagtctgt ccacgtaagg ggatcgtgtc 960
   cccttccatg ggcagagcag ggagagggcc gtaagcttgt tcttgcgtca tcaacatgtg 1020
   ggg 1023
<210> 174
   <211> 454
   <212> DNA
   <213> leptinotarsa decemlineata
<400> 174
   ccaagaaggc caagaagggg tttatgaccc ctgagaggaa gaagaaactt aggttattgc 60
   tgagaaagaa agcagcagaa gaactgaaaa aagaacaaga acgcaaagct gccgaaagga 120
   gacgtattat tgaagagaga tgcggaaaac caaaactcat tgatgaggca aatgaagagc 180
   aggtgaggaa ctattgcaag ttatatcacg gtagaatagc taaactggag gaccagaaat 240
   ttgatttgga ataccttgtc aaaaagaaag acatggagat cgccgaattg aacagtcaag 300
   tcaacgacct caggggtaaa ttcgtcaaac ccactctcaa gaaagtatcc aaatacgaga 360
   acaaatttgc taaactccaa aagaaagcag cagaattcaa tttccgtaat caactgaaag 420
   ttgtaaagaa gaaggagttc accctggagg agga 454
<210> 175
   <211> 431
   <212> DNA
   <213> leptinotarsa decemlineata
<400> 175
   ggtttatgac ccctgagagg aagaagaaac ttaggttatt gctgagaaag aaagcagcag      60
   aagaactgaa aaaagaacaa gaacgcaaag ctgccgaaag gagacgtatt attgaagaga     120
   gatgcggaaa accaaaactc attgatgagg caaatgaaga gcaggtgagg aactattgca     180
   agttatatca cggtagaata gctaaactgg aggaccagaa atttgatttg gaataccttg     240
   tcaaaaagaa agacatggag atcgccgaat tgaacagtca agtcaacgac ctcaggggta     300
   aattcgtcaa acccactctc aagaaagtat ccaaatacga gaacaaattt gctaaactcc     360
   aaaagaaagc agcagaattc aatttccgta atcaactgaa agttgtaaag aagaaggagt     420
   tcaccctgga g                                                          431
<210> 176
   <211> 888
   <212> DNA
   <213> leptinotarsa decemlineata
<400> 176
   agcagtggta tcaacgcaga gtacgcgggg acatcgagga gaagaggcaa cgcctcgaag 60
   aggctgaaaa gaaacgccag gccatgatgc aggccctcaa ggaccagaac aagaacaagg 120
   ggcccaactt caccatcacc aagagggatg cttcatctaa cctttctgcc gctcagttgg 180
   aacgcaacaa gaccaaggag caactcgagg aagagaagaa aatttccctt tccatccgca 240
   tcaagccctt ggtcgttgat ggtctgggcg tagataaact ccgtctgaaa gcacaagaac 300
   tttgggaatg catcgtcaag ttggagactg aaaagtacga cttggaagag aggcagaaac 360
   gtcaagacta cgatctcaaa gagctgaaag aaagacagaa acaacagctg agacacaaag 420
   ccttgaagaa gggtctagac ccagaagccc taaccggcaa atacccgcct aaaatccaag 480
   tagcctccaa atatgaacgt cgtgttgaca cgaggtcgta tggagacaaa aagaagctat 540
   tcgaaggggg attagaagaa atcattaaag agaccaatga aaagagctgg aaagagaaat 600
   ttggacagtt cgattccaga caaaaggcaa gacttcccaa gtggttcggt gaacgtcctg 660
   gcaaaaaacc tggagatccc gaaactccag aaggcgagga ggagggcaaa caagtcattg 720
   atgaggatga cgacctcaag gagcctgtaa tcgaagctga aattgaagaa gaggaggaag 780
   aagaggaagt cgaggtcgat gaagaagaag aggatgacga agaagaagaa gaagaagagt 840
   gaatgccaaa ggcagaagat aatcatgaaa tcaacattag ataacgtc 888
<210> 177
   <211> 404
   <212> DNA
   <213> leptinotarsa decemlineata
<400> 177
   caaggaccag aacaagaaca aggggcccaa cttcaccatc accaagaggg atgcttcatc 60
   taacctttct gccgctcagt tggaacgcaa caagaccaag gagcaactcg aggaagagaa 120
   gaaaatttcc ctttccatcc gcatcaagcc cttggtcgtt gatggtctgg gcgtagataa 180
   actccgtctg aaagcacaag aactttggga atgcatcgtc aagttggaga ctgaaaagta 240
   cgacttggaa gagaggcaga aacgtcaaga ctacgatctc aaagagctga aagaaagaca 300
   gaaacaacag ctgagacaca aagccttgaa gaagggtcta gacccagaag ccctaaccgg 360
   caaatacccg cctaaaatcc aagtagcctc caaatatgaa cgtc 404
<210> 178
   <211> 1155
   <212> DNA
   <213> leptinotarsa decemlineata
<400> 178
   gctcttcaga atgaacttga agaatctcgt acactgttgg aacaagctga ccgtgcccgt        60
   cgccaagcag aacaagaatt gggagatgct cacgaacaat tgaatgatct tggtgcacag       120
   aatggttctc tgtctgccgc caagaggaaa ctggaaactg aactccaaac tctccattcc       180
   gatcttgatg aacttctcaa tgaagccaag aactctgagg agaaggctaa gaaagccatg       240
   gtcgatgcag ctcgtcttgc agatgaactg agagcagaac aagatcatgc acaaactcag       300
   gagaaacttc gtaaagcctt agaatcacaa atcaaggacc ttcaagttcg tctcgacgag       360
   gctgaagcta acgccctcaa aggaggtaag aaagcaatcg ctaaacttga acaacgcgtc       420
   agggaattgg agaatgagtt agatggtgaa caaagacgac acgccgatgc tcaaaagaat       480
   ttgagaaagt ccgaacgtcg catcaaggag ctcagcctcc aagctgaaga agaccgtaag       540
   aaccacgaaa aaatgcaaga cttagtcgac aaacttcaac agaaaatcaa gacccacaag       600
   aggcaaatag aagaagctga agaaatagcg gctctcaatt tggccaaatt ccgtaaagca       660
   caacaggaat tggaagaagc agaagagcgt gcagaccttg ctgaacaagc aattgtcaaa       720
   ttccgtacca agggacgttc tggatcagca gctaggggag ccagccctgc gcctcagcga       780
   cagcgtccca cattcggaat gggagattca cttggaggtg ccttccctcc aaggttcgat       840
   cttgcacccg actttgaatg aatctgacat tgtgttataa gtgtaaggtg aacattctat       900
   cgcagtgtaa atatcatccc aatgcgaatc aattctacat tcagtttaag tcattctatc       960
   tctcaaaata ataatagtgt catccattct cactatcaaa tcaagacaag agatgatgat      1020
   cagagaacac gtatcacatc tacagcaaac cctcagtcct cggcatctct gataatattt      1080
   tcaattatcg agattgatga tatcgggtgt tgaatgctga tgaatagaag gcgccctatg      1140
   gaaataagag agaag                                                       1155
<210> 179
   <211> 523
   <212> DNA
   <213> leptinotarsa decemlineata
<400> 179
   gaatctcgta cactgttgga acaagctgac cgtgcccgtc gccaagcaga acaagaattg 60
   ggagatgctc acgaacaatt gaatgatctt ggtgcacaga atggttctct gtctgccgcc 120
   aagaggaaac tggaaactga actccaaact ctccattccg atcttgatga acttctcaat 180
   gaagccaaga actctgagga gaaggctaag aaagccatgg tcgatgcagc tcgtcttgca 240
   gatgaactga gagcagaaca agatcatgca caaactcagg agaaacttcg taaagcctta 300
   gaatcacaaa tcaaggacct tcaagttcgt ctcgacgagg ctgaagctaa cgccctcaaa 360
   ggaggtaaga aagcaatcgc taaacttgaa caacgcgtca gggaattgga gaatgagtta 420
   gatggtgaac aaagacgaca cgccgatgct caaaagaatt tgagaaagtc cgaacgtcgc 480
   atcaaggagc tcagcctcca agctgaagaa gaccgtaaga acc 523
<210> 180
   <211> 865
   <212> DNA
   <213> nilaparvata lugens
<400> 180
   ctaggagtat ctcctacgta attcggtgct tgagccaact gcagctactc acttttttcc 60
   aggttcagtg gtagggacgc aaacacagct aaaatggcgg acgatgaggc aaagaaggca 120
   aagcaggcgg aaatcgaccg caagagagcc gaggtccgca agcggatgga ggaagcctcc 180
   aaggccaaga aggccaagaa aggtttcatg acgcctgaca gaaagaagaa gctcaggttg 240
   ttgctgagga aaaaggctgc tgaggaattg aagaaggaac aggagaggaa agccgcggaa 300
   aggagaagga tcatcgagga gaggtgtggc aaggctgttg atctcgatga cggaagtgaa 360
   gagaaagtca aggcaacttt aaaaacctat cacgacagaa ttggaaaatt ggaggatgaa 420
   aaatttgacc tggaatatat tgtaaaaaag aaagacttcg agatcgctga cctcaacagc 480
   caggtgaatg acctccgtgg taaatttgtc aagccaacct tgaaaaaagt ctccaaatat 540
   gagaacaaat tcgccaagct ccagaagaaa gcagctgaat tcaatttcag aaatcagctc 600
   aaagttgtca agaagaagga attcaccttg gaagaagaag acaaggagcc gaagaaatcg 660
   gagaaagccg aatggcagaa gaaatgaact cacatcacct cttcataata ttgtcccaca 720
   cttctacaac cttcatcaaa taacttttat tcgagtaaac ttactgttac taacaaaatt 780
   acaaaaccaa actcttatca tcaacgtagg caatgtgctc aacttatttc ttaaacatat 840
   tgtccagcta tttattgaaa ttaaa 865
<210> 181
   <211> 269
   <212> DNA
   <213> nilaparvata lugens
<400> 181
   aagaagaagc tcaggttgtt gctgaggaaa aaggctgctg aggaattgaa gaaggaacag        60
   gagaggaaag ccgcggaaag gagaaggatc atcgaggaga ggtgtggcaa ggctgttgat       120
   ctcgatgacg gaagtgaaga gaaagtcaag gcaactttaa aaacctatca cgacagaatt       180
   ggaaaattgg aggatgaaaa atttgacctg gaatatattg taaaaaagaa agacttcgag       240
   atcgctgacc tcaacagcca ggtgaatga                                         269
<210> 182
   <211> 553
   <212> DNA
   <213> nilaparvata lugens
<400> 182
   aatgatggcg gctctcaagg accagagcaa atcgaaagga cccaacttca ccgtaaacaa        60
   gaaaacagac ttgaacatga cgtcagctca aatggaaagg aacaagacta aggagcagct       120
   ggaggaggag aagaagatct ctctgtcgtt ccgcatcaag ccgttggcca tcgagaacat       180
   gagcatcaac gcactgcgcg ccaaggccca ggaactgtgg gactgcatcg tcaagctcga       240
   aactgagaag tacgatctgg aggaacgcca gaagaggcag gactacgatc tcaaagaatt       300
   gaaagaaaga caaaagcaac agctgaggca taaagccctc aaaaaaggtc tagaccctga       360
   ggctctcaca ggaaagtacc caccaaaaat ccaagttgcc tccaaatatg aaagacgtgt       420
   agatacaagg tcatacgacg acaagaagaa gctcttcgaa ggtggctggg acacattaac       480
   atcagaaacc aatgagaaaa tatggaagag cagaaacgat cagttttcaa atcgtagcaa       540
   ggctaaactg cca                                                          553
<210> 183
   <211> 470
   <212> DNA
   <213> nilaparvata lugens
<400> 183
   atgatggcgg ctctcaagga ccagagcaaa tcgaaaggac ccaacttcac cgtaaacaag 60
   aaaacagact tgaacatgac gtcagctcaa atggaaagga acaagactaa ggagcagctg 120
   gaggaggaga agaagatctc tctgtcgttc cgcatcaagc cgttggccat cgagaacatg 180
   agcatcaacg cactgcgcgc caaggcccag gaactgtggg actgcatcgt caagctcgaa 240
   actgagaagt acgatctgga ggaacgccag aagaggcagg actacgatct caaagaattg 300
   aaagaaagac aaaagcaaca gctgaggcat aaagccctca aaaaaggtct agaccctgag 360
   gctctcacag gaaagtaccc accaaaaatc caagttgcct ccaaatatga aagacgtgta 420
   gatacaaggt catacgacga caagaagaag ctcttcgaag gtggctggga 470
<210> 184
   <211> 367
   <212> DNA
   <213> nilaparvata lugens
<400> 184
   tgccttcgac cgtgaaaggt ctggaagtat cccaacagac atggtcgccg acatcctcag 60
   gctcatggga cagcctttca acaagaagat cctcgacgaa ctcattgagg aagttgatgc 120
   tgacaaatct ggccgtcttg agtttgacga attcgtgact ctggccgcca aattcattgt 180
   tgaggaagac gatgaggcaa tgcagaagga attgaaggaa gctttcagat tatacgacaa 240
   ggaaggtaac ggctacatcc ccacatcatg tctgaaggaa atcttaaggg aacttgacga 300
   tcagctgaca aacgaggaac tcaacatgat gattgatgag atcgactctg acggatcagg 360
   aactgtt 367
<210> 185
   <211> 204
   <212> DNA
   <213> nilaparvata lugens
<400> 185
   acatcctcag gctcatggga cagcctttca acaagaagat cctcgacgaa cttattgagg 60
   aggttgatgc tgacaagtct ggccgtctag agtttgacga attcgtgact ctggccgcca 120
   aattcattgt tgaggaagac gatgaggcaa tgcagaagga attgaaggaa gctttcagat 180
   tatacgacaa ggaaggtaac ggct 204
<210> 186
   <211> 221
   <212> DNA
   <213> nilaparvata lugens
<400> 186
   cgtaaaaact ctgaccggca agaccatcac cttggaagtg gagccttccg ataccattga 60
   aaacgtgaag gccaagatcc aagacaagga gggaattcct cccgaccagc agagacttat 120
   cttcgctgga aagcaactgg aggatggcag aaccctgtcc gactacaaca tccaaaaaga 180
   atctacactc cacttggttc tcagacttcg tggtggaact a 221
<210> 187
   <211> 221
   <212> DNA
   <213> nilaparvata lugens
<400> 187
   cgtaaaaact ctgaccggca agaccatcac cttggaagtg gagccttccg ataccattga 60
   aaacgtgaag gccaagatcc aagacaagga gggaattcct cccgaccagc agagacttat 120
   cttcgctgga aagcaactgg aggatggcag aaccctgtcc gactacaaca tccaaaaaga 180
   atctacactc cacttggttc tcagacttcg tggtggaact a 221
<210> 188
   <211> 759
   <212> DNA
   <213> Acyrthosiphon pisum
<400> 188
   atggccgacg atgaagctaa gaaagcaaaa caggcggaaa tcgaccgcaa gagggccgaa 60
   gtgcgcaagc gtatggaaga ggcgtccaag gccaagaagg ccaagaaggg tttcatgacc 120
   ccagacagaa agaagaaact ccgtctgttg ttgaaaaaaa aggcggccga agagttgaag 180
   aaagaacaag aacgcaaagc tgccgaacga aggcggatca tcgaagagcg gtgcggacaa 240
   ccgaagaaca tcgacgacgc cggcgaagag gagcttgcgg aaatctgcga agaactatgg 300
   aaacgggttt acaccgtaga gggcataaaa tttgacttgg aaagggatat caggatgaaa 360
   gttttcgaga tcagcgaatt gaacagccaa gtcaatgact tacgaggaaa attcgtcaaa 420
   ccaacattga agaaggtttc caaatacgaa aacaaattcg caaaactcca aaagaaagcg 480
   gcggagttca acttcagaaa ccaactgaaa gtagtgaaga aaaaggagtt caccttggaa 540
   gaagaagaca aagagaaaaa acccgattgg tccaaaaagg gagacgaaaa gaagggcgaa 600
   ggagaagacg gcgacggtac cgaagacgaa aagaccgacg acggtttgac caccgaaggc 660
   gaatcggtcg cgggcgatct aacggacgcg acggaagacg cgcagagcga caacgagata 720
   ctcgaaccag aacccgtggt tgaacccgaa ccagaacca 759
<210> 189
   <211> 759
   <212> DNA
   <213> Acyrthosiphon pisum
<400> 189
   atggccgacg atgaagctaa gaaagcaaaa caggcggaaa tcgaccgcaa gagggccgaa        60
   gtgcgcaagc gtatggaaga ggcgtccaag gccaagaagg ccaagaaggg tttcatgacc       120
   ccagacagaa agaagaaact ccgtctgttg ttgaaaaaaa aggcggccga agagttgaag       180
   aaagaacaag aacgcaaagc tgccgaacga aggcggatca tcgaagagcg gtgcggacaa       240
   ccgaagaaca tcgacgacgc cggcgaagag gagcttgcgg aaatctgcga agaactatgg       300
   aaacgggttt acaccgtaga gggcataaaa tttgacttgg aaagggatat caggatgaaa       360
   gttttcgaga tcagcgaatt gaacagccaa gtcaatgact tacgaggaaa attcgtcaaa       420
   ccaacattga agaaggtttc caaatacgaa aacaaattcg caaaactcca aaagaaagcg       480
   gcggagttca acttcagaaa ccaactgaaa gtagtgaaga aaaaggagtt caccttggaa       540
   gaagaagaca aagagaaaaa acccgattgg tccaaaaagg gagacgaaaa gaagggcgaa       600
   ggagaagacg gcgacggtac cgaagacgaa aagaccgacg acggtttgac caccgaaggc       660
   gaatcggtcg cgggcgatct aacggacgcg acggaagacg cgcagagcga caacgagata       720
   ctcgaaccag aacccgtggt tgaacccgaa ccagaacca                              759
<210> 190
   <211> 759
   <212> DNA
   <213> Acyrthosiphon pisum
<400> 190
   atggccgacg atgaagctaa gaaagcaaaa caggcggaaa tcgaccgcaa gagggccgaa        60
   gtgcgcaagc gtatggaaga ggcgtccaag gccaagaagg ccaagaaggg tttcatgacc       120
   ccagacagaa agaagaaact ccgtctgttg ttgaaaaaaa aggcggccga agagttgaag       180
   aaagaacaag aacgcaaagc tgccgaacga aggcggatca tcgaagagcg gtgcggacaa       240
   ccgaagaaca tcgacgacgc cggcgaagag gagcttgcgg aaatctgcga agaactatgg       300
   aaacgggttt acaccgtaga gggcataaaa tttgacttgg aaagggatat caggatgaaa       360
   gttttcgaga tcagcgaatt gaacagccaa gtcaatgact tacgaggaaa attcgtcaaa       420
   ccaacattga agaaggtttc caaatacgaa aacaaattcg caaaactcca aaagaaagcg       480
   gcggagttca acttcagaaa ccaactgaaa gtagtgaaga aaaaggagtt caccttggaa 540
   gaagaagaca aagagaaaaa acccgattgg tccaaaaagg gagacgaaaa gaagggcgaa 600
   ggagaagacg gcgacggtac cgaagacgaa aagaccgacg acggtttgac caccgaaggc 660
   gaatcggtcg cgggcgatct aacggacgcg acggaagacg cgcagagcga caacgagata 720
   ctcgaaccag aacccgtggt tgaacccgaa ccagaacca 759
<210> 191
   <211> 759
   <212> DNA
   <213> Acyrthosiphon pisum
<400> 191
   atggccgacg atgaagctaa gaaagcaaaa caggcggaaa tcgaccgcaa gagggccgaa 60
   gtgcgcaagc gtatggaaga ggcgtccaag gccaagaagg ccaagaaggg tttcatgacc 120
   ccagacagaa agaagaaact ccgtctgttg ttgaaaaaaa aggcggccga agagttgaag 180
   aaagaacaag aacgcaaagc tgccgaacga aggcggatca tcgaagagcg gtgcggacaa 240
   ccgaagaaca tcgacgacgc cggcgaagag gagcttgcgg aaatctgcga agaactatgg 300
   aaacgggttt acaccgtaga gggcataaaa tttgacttgg aaagggatat caggatgaaa 360
   gttttcgaga tcagcgaatt gaacagccaa gtcaatgact tacgaggaaa attcgtcaaa 420
   ccaacattga agaaggtttc caaatacgaa aacaaattcg caaaactcca aaagaaagcg 480
   gcggagttca acttcagaaa ccaactgaaa gtagtgaaga aaaaggagtt caccttggaa 540
   gaagaagaca aagagaaaaa acccgattgg tccaaaaagg gagacgaaaa gaagggcgaa 600
   ggagaagacg gcgacggtac cgaagacgaa aagaccgacg acggtttgac caccgaaggc 660
   gaatcggtcg cgggcgatct aacggacgcg acggaagacg cgcagagcga caacgagata 720
   ctcgaaccag aacccgtggt tgaacccgaa ccagaacca 759
<210> 192
   <211> 759
   <212> DNA
   <213> Acyrthosiphon pisum
<400> 192
   atggccgacg atgaagctaa gaaagcaaaa caggcggaaa tcgaccgcaa gagggccgaa    60
   gtgcgcaagc gtatggaaga ggcgtccaag gccaagaagg ccaagaaggg tttcatgacc   120
   ccagacagaa agaagaaact ccgtctgttg ttgaaaaaaa aggcggccga agagttgaag   180
   aaagaacaag aacgcaaagc tgccgaacga aggcggatca tcgaagagcg gtgcggacaa   240
   ccgaagaaca tcgacgacgc cggcgaagag gagcttgcgg aaatctgcga agaactatgg   300
   aaacgggttt acaccgtaga gggcataaaa tttgacttgg aaagggatat caggatgaaa   360
   gttttcgaga tcagcgaatt gaacagccaa gtcaatgact tacgaggaaa attcgtcaaa   420
   ccaacattga agaaggtttc caaatacgaa aacaaattcg caaaactcca aaagaaagcg   480
   gcggagttca acttcagaaa ccaactgaaa gtagtgaaga aaaaggagtt caccttggaa   540
   gaagaagaca aagagaaaaa acccgattgg tccaaaaagg gagacgaaaa gaagggcgaa   600
   ggagaagacg gcgacggtac cgaagacgaa aagaccgacg acggtttgac caccgaaggc   660
   gaatcggtcg cgggcgatct aacggacgcg acggaagacg cgcagagcga caacgagata   720
   ctcgaaccag aacccgtggt tgaacccgaa ccagaacca                          759
<210> 193
   <211> 759
   <212> DNA
   <213> Acyrthosiphon pisum
<400> 193
   atggccgacg atgaagctaa gaaagcaaaa caggcggaaa tcgaccgcaa gagggccgaa    60
   gtgcgcaagc gtatggaaga ggcgtccaag gccaagaagg ccaagaaggg tttcatgacc   120
   ccagacagaa agaagaaact ccgtctgttg ttgaaaaaaa aggcggccga agagttgaag   180
   aaagaacaag aacgcaaagc tgccgaacga aggcggatca tcgaagagcg gtgcggacaa   240
   ccgaagaaca tcgacgacgc cggcgaagag gagcttgcgg aaatctgcga agaactatgg   300
   aaacgggttt acaccgtaga gggcataaaa tttgacttgg aaagggatat caggatgaaa   360
   gttttcgaga tcagcgaatt gaacagccaa gtcaatgact tacgaggaaa attcgtcaaa   420
   ccaacattga agaaggtttc caaatacgaa aacaaattcg caaaactcca aaagaaagcg   480
   gcggagttca acttcagaaa ccaactgaaa gtagtgaaga aaaaggagtt caccttggaa   540
   gaagaagaca aagagaaaaa acccgattgg tccaaaaagg gagacgaaaa gaagggcgaa   600
   ggagaagacg gcgacggtac cgaagacgaa aagaccgacg acggtttgac caccgaaggc   660
   gaatcggtcg cgggcgatct aacggacgcg acggaagacg cgcagagcga caacgagata   720
   ctcgaaccag aacccgtggt tgaacccgaa ccagaacca                          759
<210> 194
   <211> 759
   <212> DNA
   <213> Acyrthosiphon pisum
<400> 194
   atggccgacg atgaagctaa gaaagcaaaa caggcggaaa tcgaccgcaa gagggccgaa 60
   gtgcgcaagc gtatggaaga ggcgtccaag gccaagaagg ccaagaaggg tttcatgacc 120
   ccagacagaa agaagaaact ccgtctgttg ttgaaaaaaa aggcggccga agagttgaag 180
   aaagaacaag aacgcaaagc tgccgaacga aggcggatca tcgaagagcg gtgcggacaa 240
   ccgaagaaca tcgacgacgc cggcgaagag gagcttgcgg aaatctgcga agaactatgg 300
   aaacgggttt acaccgtaga gggcataaaa tttgacttgg aaagggatat caggatgaaa 360
   gttttcgaga tcagcgaatt gaacagccaa gtcaatgact tacgaggaaa attcgtcaaa 420
   ccaacattga agaaggtttc caaatacgaa aacaaattcg caaaactcca aaagaaagcg 480
   gcggagttca acttcagaaa ccaactgaaa gtagtgaaga aaaaggagtt caccttggaa 540
   gaagaagaca aagagaaaaa acccgattgg tccaaaaagg gagacgaaaa gaagggcgaa 600
   ggagaagacg gcgacggtac cgaagacgaa aagaccgacg acggtttgac caccgaaggc 660
   gaatcggtcg cgggcgatct aacggacgcg acggaagacg cgcagagcga caacgagata 720
   ctcgaaccag aacccgtggt tgaacccgaa ccagaacca 759
<210> 195
   <211> 759
   <212> DNA
   <213> Acyrthosiphon pisum
<400> 195
   atggccgacg atgaagctaa gaaagcaaaa caggcggaaa tcgaccgcaa gagggccgaa        60
   gtgcgcaagc gtatggaaga ggcgtccaag gccaagaagg ccaagaaggg tttcatgacc       120
   ccagacagaa agaagaaact ccgtctgttg ttgaaaaaaa aggcggccga agagttgaag       180
   aaagaacaag aacgcaaagc tgccgaacga aggcggatca tcgaagagcg gtgcggacaa       240
   ccgaagaaca tcgacgacgc cggcgaagag gagcttgcgg aaatctgcga agaactatgg       300
   aaacgggttt acaccgtaga gggcataaaa tttgacttgg aaagggatat caggatgaaa       360
   gttttcgaga tcagcgaatt gaacagccaa gtcaatgact tacgaggaaa attcgtcaaa       420
   ccaacattga agaaggtttc caaatacgaa aacaaattcg caaaactcca aaagaaagcg       480
   gcggagttca acttcagaaa ccaactgaaa gtagtgaaga aaaaggagtt caccttggaa       540
   gaagaagaca aagagaaaaa acccgattgg tccaaaaagg gagacgaaaa gaagggcgaa       600
   ggagaagacg gcgacggtac cgaagacgaa aagaccgacg acggtttgac caccgaaggc       660
   gaatcggtcg cgggcgatct aacggacgcg acggaagacg cgcagagcga caacgagata       720
   ctcgaaccag aacccgtggt tgaacccgaa ccagaacca                              759
<210> 196
   <211> 759
   <212> DNA
   <213> Acyrthosiphon pisum
<400> 196
   atggccgacg atgaagctaa gaaagcaaaa caggcggaaa tcgaccgcaa gagggccgaa        60
   gtgcgcaagc gtatggaaga ggcgtccaag gccaagaagg ccaagaaggg tttcatgacc       120
   ccagacagaa agaagaaact ccgtctgttg ttgaaaaaaa aggcggccga agagttgaag       180
   aaagaacaag aacgcaaagc tgccgaacga aggcggatca tcgaagagcg gtgcggacaa       240
   ccgaagaaca tcgacgacgc cggcgaagag gagcttgcgg aaatctgcga agaactatgg       300
   aaacgggttt acaccgtaga gggcataaaa tttgacttgg aaagggatat caggatgaaa       360
   gttttcgaga tcagcgaatt gaacagccaa gtcaatgact tacgaggaaa attcgtcaaa       420
   ccaacattga agaaggtttc caaatacgaa aacaaattcg caaaactcca aaagaaagcg       480
   gcggagttca acttcagaaa ccaactgaaa gtagtgaaga aaaaggagtt caccttggaa       540
   gaagaagaca aagagaaaaa acccgattgg tccaaaaagg gagacgaaaa gaagggcgaa       600
   ggagaagacg gcgacggtac cgaagacgaa aagaccgacg acggtttgac caccgaaggc       660
   gaatcggtcg cgggcgatct aacggacgcg acggaagacg cgcagagcga caacgagata       720
   ctcgaaccag aacccgtggt tgaacccgaa ccagaacca                              759
<210> 197
   <211> 759
   <212> DNA
   <213> Acyrthosiphon pisum
<400> 197
   atggccgacg atgaagctaa gaaagcaaaa caggcggaaa tcgaccgcaa gagggccgaa      60
   gtgcgcaagc gtatggaaga ggcgtccaag gccaagaagg ccaagaaggg tttcatgacc     120
   ccagacagaa agaagaaact ccgtctgttg ttgaaaaaaa aggcggccga agagttgaag     180
   aaagaacaag aacgcaaagc tgccgaacga aggcggatca tcgaagagcg gtgcggacaa     240
   ccgaagaaca tcgacgacgc cggcgaagag gagcttgcgg aaatctgcga agaactatgg     300
   aaacgggttt acaccgtaga gggcataaaa tttgacttgg aaagggatat caggatgaaa     360
   gttttcgaga tcagcgaatt gaacagccaa gtcaatgact tacgaggaaa attcgtcaaa     420
   ccaacattga agaaggtttc caaatacgaa aacaaattcg caaaactcca aaagaaagcg     480
   gcggagttca acttcagaaa ccaactgaaa gtagtgaaga aaaaggagtt caccttggaa     540
   gaagaagaca aagagaaaaa acccgattgg tccaaaaagg gagacgaaaa gaagggcgaa     600
   ggagaagacg gcgacggtac cgaagacgaa aagaccgacg acggtttgac caccgaaggc     660
   gaatcggtcg cgggcgatct aacggacgcg acggaagacg cgcagagcga caacgagata     720
   ctcgaaccag aacccgtggt tgaacccgaa ccagaacca                           759
<210> 198
   <211> 759
   <212> DNA
   <213> Acyrthosiphon pisum
<400> 198
   atggccgacg atgaagctaa gaaagcaaaa caggcggaaa tcgaccgcaa gagggccgaa      60
   gtgcgcaagc gtatggaaga ggcgtccaag gccaagaagg ccaagaaggg tttcatgacc     120
   ccagacagaa agaagaaact ccgtctgttg ttgaaaaaaa aggcggccga agagttgaag     180
   aaagaacaag aacgcaaagc tgccgaacga aggcggatca tcgaagagcg gtgcggacaa     240
   ccgaagaaca tcgacgacgc cggcgaagag gagcttgcgg aaatctgcga agaactatgg     300
   aaacgggttt acaccgtaga gggcataaaa tttgacttgg aaagggatat caggatgaaa     360
   gttttcgaga tcagcgaatt gaacagccaa gtcaatgact tacgaggaaa attcgtcaaa      420
   ccaacattga agaaggtttc caaatacgaa aacaaattcg caaaactcca aaagaaagcg      480
   gcggagttca acttcagaaa ccaactgaaa gtagtgaaga aaaaggagtt caccttggaa      540
   gaagaagaca aagagaaaaa acccgattgg tccaaaaagg gagacgaaaa gaagggcgaa      600
   ggagaagacg gcgacggtac cgaagacgaa aagaccgacg acggtttgac caccgaaggc      660
   gaatcggtcg cgggcgatct aacggacgcg acggaagacg cgcagagcga caacgagata      720
   ctcgaaccag aacccgtggt tgaacccgaa ccagaacca                             759
<210> 199
   <211> 759
   <212> DNA
   <213> Acyrthosiphon pisum
<400> 199
   atggccgacg atgaagctaa gaaagcaaaa caggcggaaa tcgaccgcaa gagggccgaa       60
   gtgcgcaagc gtatggaaga ggcgtccaag gccaagaagg ccaagaaggg tttcatgacc      120
   ccagacagaa agaagaaact ccgtctgttg ttgaaaaaaa aggcggccga agagttgaag      180
   aaagaacaag aacgcaaagc tgccgaacga aggcggatca tcgaagagcg gtgcggacaa      240
   ccgaagaaca tcgacgacgc cggcgaagag gagcttgcgg aaatctgcga agaactatgg      300
   aaacgggttt acaccgtaga gggcataaaa tttgacttgg aaagggatat caggatgaaa      360
   gttttcgaga tcagcgaatt gaacagccaa gtcaatgact tacgaggaaa attcgtcaaa      420
   ccaacattga agaaggtttc caaatacgaa aacaaattcg caaaactcca aaagaaagcg      480
   gcggagttca acttcagaaa ccaactgaaa gtagtgaaga aaaaggagtt caccttggaa      540
   gaagaagaca aagagaaaaa acccgattgg tccaaaaagg gagacgaaaa gaagggcgaa      600
   ggagaagacg gcgacggtac cgaagacgaa aagaccgacg acggtttgac caccgaaggc      660
   gaatcggtcg cgggcgatct aacggacgcg acggaagacg cgcagagcga caacgagata      720
   ctcgaaccag aacccgtggt tgaacccgaa ccagaacca                             759
<210> 200
   <211> 541
   <212> DNA
   <213> Acyrthosiphon pisum
<400> 200
   cggtaatgcg atgcggtaag aagaaggtat ggttggatcc aaacgaaata aatgaaattg      60
   ccaacaccaa ttccagacaa aatattcgta agttgatcaa agatggtttg atcattaaaa     120
   agccagtagc tgtacactct agggctcgtg cacgtaaaaa tgcagatgcc agaagaaaag     180
   gtcgtcattg tggttttggt aaaaggaagg gtactgctaa tgctcgaaca cctcaaaaag     240
   acctttgggt gaaaagaatg cgagtattaa ggcggttgct taaaaaatac cgtgaagcaa     300
   agaaaattga caaccatctt taccatcagt tatacatgaa ggctaagggt aatgttttca     360
   agaacaaacg tgtattgatg gagttcatcc acaaaaagaa ggcagagaag gcccgtgcca     420
   agatgttgag tgatcaagct gaagctagac gtcaaaaggt taaggaagct aggaaacgta     480
   aagaagcaag atttttacaa aataggaagg aacttttggc tgcatacgcc cgagaagatg     540
   a                                                                     541
<210> 201
   <211> 541
   <212> DNA
   <213> Acyrthosiphon pisum
<400> 201
   cggtaatgcg atgcggtaag aagaaggtat ggttggatcc aaacgaaata aatgaaattg      60
   ccaacaccaa ttccagacaa aatattcgta agttgatcaa agatggtttg atcattaaaa     120
   agccagtagc tgtacactct agggctcgtg cacgtaaaaa tgcagatgcc agaagaaaag     180
   gtcgtcattg tggttttggt aaaaggaagg gtactgctaa tgctcgaaca cctcaaaaag     240
   acctttgggt gaaaagaatg cgagtattaa ggcggttgct taaaaaatac cgtgaagcaa     300
   agaaaattga caaccatctt taccatcagt tatacatgaa ggctaagggt aatgttttca     360
   agaacaaacg tgtattgatg gagttcatcc acaaaaagaa ggcagagaag gcccgtgcca     420
   agatgttgag tgatcaagct gaagctagac gtcaaaaggt taaggaagct aggaaacgta     480
   aagaagcaag atttttacaa aataggaagg aacttttggc tgcatacgcc cgagaagatg     540
   a                                                                     541
<210> 202
   <211> 823
   <212> DNA
   <213> Acyrthosiphon pisum
<400> 202
   gttgtagtcg gaaagggtac gtccgtcttc aagttgtttt ccggcaaaga tcaaacgttg      60
   ttggtctggt gggatacctt ctttgtcttg gatcttggct tttacatttt caatggaatc     120
   agatgattcc acctccaatg taatggtctt tccagtgagg gtctttacaa agatttgcat     180
   accaccacgg agacgcaaca ctaagtgaag ggtagattct ttctggatgt tgtagtcaga     240
   aagtgtgcgt ccgtcttcaa gttgctttcc ggcaaagatc aaacgttgtt ggtcaggtgg     300
   aataccttct ttgtcttgga tcttagcttt tacattttca atggaatctg atgactcaac     360
   ttccaatgta atggtctttc cagtgagggt ctttacaaag atttgcatac caccacggag     420
   acgcaacact aagtgaaggg tagattcttt ctggatgttg tagtcggaaa gggtacgtcc     480
   gtcttcaagt tgctttccgg caaagatcaa acgttgttgg tctggtggga taccttcttt     540
   gtcttggatc ttggctttta cattttcaat ggaatcagat gattccacct ccaatgtaat     600
   ggtctttcca gtgagggtct ttacaaagat ttgcatacca ccacggagac gcaacactaa     660
   gtgaagggta gattctttct ggatgttgta gtcggaaagg gtacgtccgt cttcaagttg     720
   ctttccagca aagatcaaac gttgctggtc tggtgggata ccttccttgt cttggatctt     780
   ggccttaaca ttttcaatgg aatctgatga ctcaacttcc aaa                       823
<210> 203
   <211> 823
   <212> DNA
   <213> Acyrthosiphon pisum
<400> 203
   gttgtagtcg gaaagggtac gtccgtcttc aagttgtttt ccggcaaaga tcaaacgttg    60
   ttggtctggt gggatacctt ctttgtcttg gatcttggct tttacatttt caatggaatc   120
   agatgattcc acctccaatg taatggtctt tccagtgagg gtctttacaa agatttgcat   180
   accaccacgg agacgcaaca ctaagtgaag ggtagattct ttctggatgt tgtagtcaga   240
   aagtgtgcgt ccgtcttcaa gttgctttcc ggcaaagatc aaacgttgtt ggtcaggtgg   300
   aataccttct ttgtcttgga tcttagcttt tacattttca atggaatctg atgactcaac   360
   ttccaatgta atggtctttc cagtgagggt ctttacaaag atttgcatac caccacggag   420
   acgcaacact aagtgaaggg tagattcttt ctggatgttg tagtcggaaa gggtacgtcc   480
   gtcttcaagt tgctttccgg caaagatcaa acgttgttgg tctggtggga taccttcttt   540
   gtcttggatc ttggctttta cattttcaat ggaatcagat gattccacct ccaatgtaat   600
   ggtctttcca gtgagggtct ttacaaagat ttgcatacca ccacggagac gcaacactaa   660
   gtgaagggta gattctttct ggatgttgta gtcggaaagg gtacgtccgt cttcaagttg   720
   ctttccagca aagatcaaac gttgctggtc tggtgggata ccttccttgt cttggatctt   780
   ggccttaaca ttttcaatgg aatctgatga ctcaacttcc aaa                      823
<210> 204
   <211> 172
   <212> DNA
   <213> Acyrthosiphon pisum
<400> 204
   aagacttgct tcatcctact gcaattgaag aacgcaggaa acacaaatta aagcgccttg    60
   ttcaacaccc aaactctttt ttcatggatg tcaaatgccc tggatgttat aaaattacaa   120
   ctgtattcag tcacgctcag agtgtagtta tatgtaccgg atgttccaca at           172
<210> 205
   <211> 172
   <212> DNA
   <213> Acyrthosiphon pisum
<400> 205
   aagacttgct tcatcctact gcaattgaag aacgcaggaa acacaaatta aagcgccttg    60
   ttcaacaccc aaactctttt ttcatggatg tcaaatgccc tggatgttat aaaattacaa   120
   ctgtattcag tcacgctcag agtgtagtta tatgtaccgg atgttccaca at           172
<210> 206
   <211> 25
   <212> DNA
   <213> Artificial
<220>
   <223> Primer
<400> 206
   cgaaccatct gggaagcttg gaatg 25
<210> 207
   <211> 25
   <212> DNA
   <213> Artificial
<220>
   <223> Primer
<400> 207
   gcagctggag gaagagaaac gtatc 25
<210> 208
   <211> 47
   <212> DNA
   <213> Artificial
<220>
   <223> Primer
<400> 208
   gcgtaatacg actcactata ggcgaaccat ctgggaagct tggaatg 47
<210> 209
   <211> 46
   <212> DNA
   <213> Artificial
<220>
   <223> Primer
<400> 209
   gcgtaatacg actcactata ggcagctgga ggaagagaaa cgtatc 46
<210> 210
   <211> 20
   <212> DNA
   <213> Artificial
<220>
   <223> Primer
<400> 210
   agttcgagaa caccaggaag 20
<210> 211
   <211> 22
   <212> DNA
   <213> Artificial
<220>
   <223> Primer
<400> 211
   cctgacacgt tgttccagct tg 22
<210> 212
   <211> 45
   <212> DNA
   <213> Artificial
<220>
   <223> Primer
<400> 212
   gcgtaatacg actcactata ggaggagttc gagaacacca ggaag 45
<210> 213
   <211> 44
   <212> DNA
   <213> Artificial
<220>
   <223> Primer
<400> 213
   gcgtaatacg actcactata ggcctgacac gttgttccag cttg 44
<210> 214
   <211> 20
   <212> DNA
   <213> Artificial
<220>
   <223> Primer
<400> 214
   gcaggcgatg aagatggaga 20
<210> 215
   <211> 24
   <212> DNA
   <213> Artificial
<220>
   <223> Primer
<400> 215
   ccacctcttt ctgcaacttc ttga 24
<210> 216
   <211> 42
   <212> DNA
   <213> Artificial
<220>
   <223> Primer
<400> 216
   gcgtaatacg actcactata gggcaggcga tgaagatgga ga 42
<210> 217
   <211> 46
   <212> DNA
   <213> Artificial
<220>
   <223> Primer
<400> 217
   gcgtaatacg actcactata ggccacctct ttctgcaact tcttga 46
<210> 218
   <211> 25
   <212> DNA
   <213> Artificial
<220>
   <223> Primer
<400> 218
   cagaatccca cagaatctga cgtga 25
<210> 219
   <211> 20
   <212> DNA
   <213> Artificial
<220>
   <223> Primer
<400> 219
   gcaagtggcg aagctcagct 20
<210> 220
   <211> 47
   <212> DNA
   <213> Artificial
<220>
   <223> Primer
<400> 220
   gcgtaatacg actcactata ggcagaatcc cacagaatct gacgtga 47
<210> 221
   <211> 41
   <212> DNA
   <213> Artificial
<220>
   <223> Primer
<400> 221
   gcgtaatacg actcactata ggcaagtggc gaagctcagc t 41
<210> 222
   <211> 21
   <212> DNA
   <213> Artificial
<220>
   <223> Primer
<400> 222
   cgtgtttgcc atgttcgatc a 21
<210> 223
   <211> 23
   <212> DNA
   <213> Artificial
<220>
   <223> Primer
<400> 223
   ggtacatttc gtccacgtct tca 23
<210> 224
   <211> 43
   <212> DNA
   <213> Artificial
<220>
   <223> Primer
<400> 224
   gcgtaatacg actcactata ggcgtgtttg ccatgttcga tca 43
<210> 225
   <211> 43
   <212> DNA
   <213> Artificial
<220>
   <223> Primer
<400> 225
   gcgtaatacg actcactata ggtacatttc gtccacgtct tca 43
<210> 226
   <211> 23
   <212> DNA
   <213> Artificial
<220>
   <223> Primer
<400> 226
   gacttgatct tcagccgacc att 23
<210> 227
   <211> 23
   <212> DNA
   <213> Artificial
<220>
   <223> Primer
<400> 227
   ccattgccag ttcctcaact tca 23
<210> 228
   <211> 44
   <212> DNA
   <213> Artificial
<220>
   <223> Primer
<400> 228
   gcgtaatacg actcactata ggacttgatc ttcagccgac catt 44
<210> 229
   <211> 45
   <212> DNA
   <213> Artificial
<220>
   <223> Primer
<400> 229
   gcgtaatacg actcactata ggccattgcc agttcctcaa cttca 45
<210> 230
   <211> 21
   <212> DNA
   <213> Artificial
<220>
   <223> Primer
<400> 230
   cgcaatgatc tcctccagga t 21
<210> 231
   <211> 24
   <212> DNA
   <213> Artificial
<220>
   <223> Primer
<400> 231
   ggtcatcatc tccatgaact cgtc 24
<210> 232
   <211> 43
   <212> DNA
   <213> Artificial
<220>
   <223> Primer
<400> 232
   gcgtaatacg actcactata ggcgcaatga tctcctccag gat 43
<210> 233
   <211> 45
   <212> DNA
   <213> Artificial
<220>
   <223> Primer
<400> 233
   gcgtaatacg actcactata gggtcatcat ctccatgaac tcgtc 45
<210> 234
   <211> 26
   <212> DNA
   <213> Artificial
<220>
   <223> Primer
<400> 234
   cgtcactaat cggactggtc taacag 26
<210> 235
   <211> 24
   <212> DNA
   <213> Artificial
<220>
   <223> Primer
<400> 235
   ggtcatcatc tccatgaact cgtc 24
<210> 236
   <211> 48
   <212> DNA
   <213> Artificial
<220>
   <223> Primer
<400> 236
   gcgtaatacg actcactata ggcgtcacta atcggactgg tctaacag 48
<210> 237
   <211> 45
   <212> DNA
   <213> Artificial
<220>
   <223> Primer
<400> 237
   gcgtaatacg actcactata gggtcatcat ctccatgaac tcgtc 45
<210> 238
   <211> 23
   <212> DNA
   <213> Artificial
<220>
   <223> Primer
<400> 238
   ggtgaaggag ggtgcctgct cag 23
<210> 239
   <211> 25
   <212> DNA
   <213> Artificial
<220>
   <223> Primer
<400> 239
   cagggtgaat agaacgaggt actcg 25
<210> 240
   <211> 40
   <212> DNA
   <213> Artificial
<220>
   <223> Primer
<400> 240
   aatacgactc actatagggc gctatgaaat tccaagcaca 40
<210> 241
   <211> 47
   <212> DNA
   <213> Artificial
<220>
   <223> Primer
<400> 241
   gcgtaatacg actcactata ggcagggtga atagaacgag gtactcg 47
<210> 242
   <211> 29
   <212> DNA
   <213> Artificial
<220>
   <223> Primer
<400> 242
   ctcaacgaag gtcttgtcag tggctttgg 29
<210> 243
   <211> 20
   <212> DNA
   <213> Artificial
<220>
   <223> Primer
<400> 243
   ttcgcctggc ttcttcgtga 20
<210> 244
   <211> 44
   <212> DNA
   <213> Artificial
<220>
   <223> Primer
<400> 244
   gcgtaatacg actcactata ggccacgccg acttaattca ttcc 44
<210> 245
   <211> 42
   <212> DNA
   <213> Artificial
<220>
   <223> Primer
<400> 245
   gcgtaatacg actcactata ggttcgcctg gcttcttcgt ga 42
<210> 246
   <211> 22
   <212> DNA
   <213> Artificial
<220>
   <223> Primer
<400> 246
   tgtcgatggc ggtcttaaca tc 22
<210> 247
   <211> 24
   <212> DNA
   <213> Artificial
<220>
   <223> Primer
<400> 247
   tctccagctt ccactttctt gaga 24
<210> 248
   <211> 44
   <212> DNA
   <213> Artificial
<220>
   <223> Primer
<400> 248
   gcgtaatacg actcactata ggtgtcgatg gcggtcttaa catc 44
<210> 249
   <211> 46
   <212> DNA
   <213> Artificial
<220>
   <223> Primer
<400> 249
   gcgtaatacg actcactata ggtctccagc ttccactttc ttgaga 46
<210> 250
   <211> 20
   <212> DNA
   <213> Artificial
<220>
   <223> Primer
<400> 250
   aacgtgcatt tcgcgtaccc 20
<210> 251
   <211> 20
   <212> DNA
   <213> Artificial
<220>
   <223> Primer
<400> 251
   tgatgggcat aactggcagg 20
<210> 252
   <211> 42
   <212> DNA
   <213> Artificial
<220>
   <223> Primer
<400> 252
   gcgtaatacg actcactata ggaacgtgca tttcgcgtac cc 42
<210> 253
   <211> 42
   <212> DNA
   <213> Artificial
<220>
   <223> Primer
<400> 253
   gcgtaatacg actcactata ggtgatgggc ataactggca gg 42
<210> 254
   <211> 22
   <212> DNA
   <213> Artificial
<220>
   <223> Primer
<400> 254
   ccttattgaa cgtggtcgac ag 22
<210> 255
   <211> 20
   <212> DNA
   <213> Artificial
<220>
   <223> Primer
<400> 255
   ctgatgtagt ccttgaggag 20
<210> 256
   <211> 44
   <212> DNA
   <213> Artificial
<220>
   <223> Primer
<400> 256
   gcgtaatacg actcactata ggccttattg aacgtggtcg acag 44
<210> 257
   <211> 42
   <212> DNA
   <213> Artificial
<220>
   <223> Primer
<400> 257
   gcgtaatacg actcactata ggctgatgta gtccttgagg ag 42
<210> 258
   <211> 26
   <212> DNA
   <213> Artificial
<220>
   <223> Primer
<400> 258
   gtacggacgg gtagtttagt tgtgtc 26
<210> 259
   <211> 20
   <212> DNA
   <213> Artificial
<220>
   <223> Primer
<400> 259
   ttgccaagtg ccaagtcacg 20
<210> 260
   <211> 48
   <212> DNA
   <213> Artificial
<220>
   <223> Primer
<400> 260
   gcgtaatacg actcactata gggtacggac gggtagttta gttgtgtc 48
<210> 261
   <211> 42
   <212> DNA
   <213> Artificial
<220>
   <223> Primer
<400> 261
   gcgtaatacg actcactata ggttgccaag tgccaagtca cg 42
<210> 262
   <211> 22
   <212> DNA
   <213> Artificial
<220>
   <223> Primer
<400> 262
   ctgttgtcgg ctggtcatat cc 22
<210> 263
   <211> 20
   <212> DNA
   <213> Artificial
<220>
   <223> Primer
<400> 263
   taacgtaacg catcgccacc 20
<210> 264
   <211> 44
   <212> DNA
   <213> Artificial
<220>
   <223> Primer
<400> 264
   gcgtaatacg actcactata ggctgttgtc ggctggtcat atcc 44
<210> 265
   <211> 42
   <212> DNA
   <213> Artificial
<220>
   <223> Primer
<400> 265
   gcgtaatacg actcactata ggtaacgtaa cgcatcgcca cc 42
<210> 266
   <211> 20
   <212> DNA
   <213> Artificial
<220>
   <223> Primer
<400> 266
   agccctcatc cgtgatttgg 20
<210> 267
   <211> 20
   <212> DNA
   <213> Artificial
<220>
   <223> Primer
<400> 267
   gatccggcct caatttgacg 20
<210> 268
   <211> 42
   <212> DNA
   <213> Artificial
<220>
   <223> Primer
<400> 268
   gcgtaatacg actcactata ggagccctca tccgtgattt gg 42
<210> 269
   <211> 42
   <212> DNA
   <213> Artificial
<220>
   <223> Primer
<400> 269
   gcgtaatacg actcactata gggatccggc ctcaatttga cg 42
<210> 270
   <211> 22
   <212> DNA
   <213> Artificial
<220>
   <223> Primer
<400> 270
   acgtttctct gctcattcgt gc 22
<210> 271
   <211> 20
   <212> DNA
   <213> Artificial
<220>
   <223> Primer
<400> 271
   cttgtacaaa gtgtgcaggg 20
<210> 272
   <211> 44
   <212> DNA
   <213> Artificial
<220>
   <223> Primer
<400> 272
   gcgtaatacg actcactata ggacgtttct ctgctcattc gtgc 44
<210> 273
   <211> 42
   <212> DNA
   <213> Artificial
<220>
   <223> Primer
<400> 273
   gcgtaatacg actcactata ggcttgtaca aagtgtgcag gg 42
<210> 274
   <211> 22
   <212> DNA
   <213> Artificial
<220>
   <223> Primer
<400> 274
   ggttttcttc ttgcccgaat cg 22
<210> 275
   <211> 20
   <212> DNA
   <213> Artificial
<220>
   <223> Primer
<400> 275
   tttggggttc ggcttggttg 20
<210> 276
   <211> 44
   <212> DNA
   <213> Artificial
<220>
   <223> Primer
<400> 276
   gcgtaatacg actcactata ggggttttct tcttgcccga atcg 44
<210> 277
   <211> 42
   <212> DNA
   <213> Artificial
<220>
   <223> Primer
<400> 277
   gcgtaatacg actcactata ggtttggggt tcggcttggt tg 42
<210> 278
   <211> 23
   <212> DNA
   <213> Artificial
<220>
   <223> Primer
<400> 278
   tcagcgagat ccctaagaca acg 23
<210> 279
   <211> 20
   <212> DNA
   <213> Artificial
<220>
   <223> Primer
<400> 279
   ccccacatgt tgatgacgca 20
<210> 280
   <211> 45
   <212> DNA
   <213> Artificial
<220>
   <223> Primer
<400> 280
   gcgtaatacg actcactata ggtcagcgag atccctaaga caacg 45
<210> 281
   <211> 42
   <212> DNA
   <213> Artificial
<220>
   <223> Primer
<400> 281
   gcgtaatacg actcactata ggccccacat gttgatgacg ca 42
<210> 282
   <211> 23
   <212> DNA
   <213> Artificial
<220>
   <223> Primer
<400> 282
   ggtttatgac ccctgagagg aag 23
<210> 283
   <211> 22
   <212> DNA
   <213> Artificial
<220>
   <223> Primer
<400> 283
   ctccagggtg aactccttct tc 22
<210> 284
   <211> 43
   <212> DNA
   <213> Artificial
<220>
   <223> Primer
<400> 284
   gcgtaatacg actcactata ggtttatgac ccctgagagg aag 43
<210> 285
   <211> 44
   <212> DNA
   <213> Artificial
<220>
   <223> Primer
<400> 285
   gcgtaatacg actcactata ggctccaggg tgaactcctt cttc 44
<210> 286
   <211> 24
   <212> DNA
   <213> Artificial
<220>
   <223> Primer
<400> 286
   caaggaccag aacaagaaca aggg 24
<210> 287
   <211> 26
   <212> DNA
   <213> Artificial
<220>
   <223> Primer
<400> 287
   gacgttcata tttggaggct acttgg 26
<210> 288
   <211> 46
   <212> DNA
   <213> Artificial
<220>
   <223> Primer
<400> 288
   gcgtaatacg actcactata ggcaaggacc agaacaagaa caaggg 46
<210> 289
   <211> 47
   <212> DNA
   <213> Artificial
<220>
   <223> Primer
<400> 289
   gcgtaatacg actcactata ggacgttcat atttggaggc tacttgg 47
<210> 290
   <211> 25
   <212> DNA
   <213> Artificial
<220>
   <223> Primer
<400> 290
   aatctcgtac actgttggaa caagc 25
<210> 291
   <211> 25
   <212> DNA
   <213> Artificial
<220>
   <223> Primer
<400> 291
   ggttcttacg gtcttcttca gcttg 25
<210> 292
   <211> 47
   <212> DNA
   <213> Artificial
<220>
   <223> Primer
<400> 292
   gcgtaatacg actcactata ggaatctcgt acactgttgg aacaagc 47
<210> 293
   <211> 45
   <212> DNA
   <213> Artificial
<220>
   <223> Primer
<400> 293
   gcgtaatacg actcactata ggttcttacg gtcttcttca gcttg 45
<210> 294
   <211> 22
   <212> DNA
   <213> Artificial
<220>
   <223> Primer
<400> 294
   aagaagaagc tcaggttgtt gc 22
<210> 295
   <211> 20
   <212> DNA
   <213> Artificial
<220>
   <223> Primer
<400> 295
   tcattcacct ggctgttgag 20
<210> 296
   <211> 44
   <212> DNA
   <213> Artificial
<220>
   <223> Primer
<400> 296
   gcgtaatacg actcactata ggaagaagaa gctcaggttg ttgc 44
<210> 297
   <211> 42
   <212> DNA
   <213> Artificial
<220>
   <223> Primer
<400> 297
   gcgtaatacg actcactata ggtcattcac ctggctgttg ag 42
<210> 298
   <211> 20
   <212> DNA
   <213> Artificial
<220>
   <223> Primer
<400> 298
   acatcctcag gctcatggga 20
<210> 299
   <211> 20
   <212> DNA
   <213> Artificial
<220>
   <223> Primer
<400> 299
   agccgttacc ttccttgtcg 20
<210> 300
   <211> 42
   <212> DNA
   <213> Artificial
<220>
   <223> Primer
<400> 300
   gcgtaatacg actcactata ggacatcctc aggctcatgg ga 42
<210> 301
   <211> 42
   <212> DNA
   <213> Artificial
<220>
   <223> Primer
<400> 301
   gcgtaatacg actcactata ggagccgtta ccttccttgt cg 42
<210> 302
   <211> 20
   <212> DNA
   <213> Artificial
<220>
   <223> Primer
<400> 302
   acatcctcag gctcatggga 20
<210> 303
   <211> 20
   <212> DNA
   <213> Artificial
<220>
   <223> Primer
<400> 303
   agccgttacc ttccttgtcg 20
<210> 304
   <211> 42
   <212> DNA
   <213> Artificial
<220>
   <223> Primer
<400> 304
   gcgtaatacg actcactata ggacatcctc aggctcatgg ga 42
<210> 305
   <211> 42
   <212> DNA
   <213> Artificial
<220>
   <223> Primer
<400> 305
   gcgtaatacg actcactata ggagccgtta ccttccttgt cg 42
<210> 306
   <211> 24
   <212> DNA
   <213> Artificial
<220>
   <223> Primer
<400> 306
   cgtaaaaact ctgaccggca agac 24
<210> 307
   <211> 26
   <212> DNA
   <213> Artificial
<220>
   <223> Primer
<400> 307
   tagttccacc acgaagtctg agaacc 26
<210> 308
   <211> 46
   <212> DNA
   <213> Artificial
<220>
   <223> Primer
<400> 308
   gcgtaatacg actcactata ggcgtaaaaa ctctgaccgg caagac 46
<210> 309
   <211> 48
   <212> DNA
   <213> Artificial
<220>
   <223> Primer
<400> 309
   gcgtaatacg actcactata ggtagttcca ccacgaagtc tgagaacc 48
<210> 310
   <211> 21
   <212> DNA
   <213> Artificial
<220>
   <223> Primer
<400> 310
   atggccgacg atgaagctaa g 21
<210> 311
   <211> 20
   <212> DNA
   <213> Artificial
<220>
   <223> Primer
<400> 311
   tggttgtggt tctggttcgg 20
<210> 312
   <211> 43
   <212> DNA
   <213> Artificial
<220>
   <223> Primer
<400> 312
   gcgtaatacg actcactata ggatggccga cgatgaagct aag 43
<210> 313
   <211> 42
   <212> DNA
   <213> Artificial
<220>
   <223> Primer
<400> 313
   gcgtaatacg actcactata ggtggttctg gttcgggttc aa 42
<210> 314
   <211> 20
   <212> DNA
   <213> Artificial
<220>
   <223> Primer
<400> 314
   cggtaatgcg atgcggtaag 20
<210> 315
   <211> 20
   <212> DNA
   <213> Artificial
<220>
   <223> Primer
<400> 315
   tcatcttctc gggcgtatgc 20
<210> 316
   <211> 42
   <212> DNA
   <213> Artificial
<220>
   <223> Primer
<400> 316
   gcgtaatacg actcactata ggcggtaatg cgatgcggta ag 42
<210> 317
   <211> 42
   <212> DNA
   <213> Artificial
<220>
   <223> Primer
<400> 317
   gcgtaatacg actcactata ggtcatcttc tcgggcgtat gc 42
<210> 318
   <211> 25
   <212> DNA
   <213> Artificial
<220>
   <223> Primer
<400> 318
   tttggaagtt gagtcatcag attcc 25
<210> 319
   <211> 24
   <212> DNA
   <213> Artificial
<220>
   <223> Primer
<400> 319
   gttgtagtcg gaaagggtac gtcc 24
<210> 320
   <211> 47
   <212> DNA
   <213> Artificial
<220>
   <223> Primer
<400> 320
   gcgtaatacg actcactata ggtttggaag ttgagtcatc agattcc 47
<210> 321
   <211> 46
   <212> DNA
   <213> Artificial
<220>
   <223> Primer
<400> 321
   gcgtaatacg actcactata gggttgtagt cggaaagggt acgtcc 46
<210> 322
   <211> 23
   <212> DNA
   <213> Artificial
<220>
   <223> Primer
<400> 322
   aagacttgct tcatcctact gca 23
<210> 323
   <211> 20
   <212> DNA
   <213> Artificial
<220>
   <223> Primer
<400> 323
   attgtggaac atccggtaca 20
<210> 324
   <211> 45
   <212> DNA
   <213> Artificial
<220>
   <223> Primer
<400> 324
   gcgtaatacg actcactata ggaagacttg cttcatccta ctgca 45
<210> 325
   <211> 42
   <212> DNA
   <213> Artificial
<220>
   <223> Primer
<400> 325
   gcgtaatacg actcactata ggattgtgga acatccggta ca 42
<210> 326
   <211> 424
   <212> PRT
   <213> Lygus hesperus
<400> 326
   Met Ile Pro Pro Thr Ser Arg Pro Gln Val Thr Val Tyr Ser Asp Lys
   1               5                   10                  15
   Asn Glu Ala Thr Gly Thr Leu Leu Asn Leu Pro Ala Val Phe Asn Ala
               20                  25                  30
   Pro Ile Arg Pro Asp Val Val Asn Phe Val His Gln Asn Val Ala Lys
           35                  40                  45
   Asn His Arg Gln Pro Tyr Cys Val Ser Ala Gln Ala Gly His Gln Thr
       50                  55                  60
   Ser Ala Glu Ser Trp Gly Thr Gly Arg Ala Val Ala Arg Ile Pro Arg
   65                  70                  75                  80
   Val Arg Gly Gly Gly Thr His Arg Ser Gly Gln Gly Ala Phe Gly Asn
                   85                  90                  95
   Met Cys Arg Gly Gly Arg Met Phe Ala Pro Thr Arg Pro Trp Arg Arg
               100                 105                 110
   Trp His Arg Lys Ile Asn Val Asn Gln Lys Arg Tyr Ala Val Val Ser
           115                 120                 125
   Ala Ile Ala Ala Ser Gly Val Pro Ala Leu Val Met Ser Lys Gly His
       130                 135                 140
   Met Val Gln Ser Val Pro Glu Phe Pro Leu Val Val Ser Asp Lys Val
   145                 150                 155                 160
   Gln Glu Tyr Thr Lys Thr Lys Gln Ala Val Ile Phe Leu His Arg Ile
                   165                 170                 175
   Lys Ala Trp Gln Asp Ile Gln Lys Val Tyr Lys Ser Lys Arg Phe Arg
   180 185 190
   Ala Gly Lys Gly Lys Met Arg Asn Arg Arg Arg Ile Gln Arg Arg Gly
   195 200 205
   Pro Leu Ile Ile Tyr Asp Gln Asp Gln Gly Leu Asn Arg Ala Phe Arg
   210 215 220
   Asn Ile Pro Gly Val Asp Leu Ile Glu Val Ser Arg Leu Asn Leu Leu
   225 230 235 240
   Lys Leu Ala Pro Gly Gly His Ile Gly Arg Phe Val Ile Trp Thr Gln
   245 250 255
   Ser Ala Phe Glu Lys Leu Asp Ala Leu Tyr Gly Thr Trp Lys Lys Lys
   260 265 270
   Ser Thr Leu Lys Ala Gly Tyr Asn Leu Pro Met Pro Lys Met Ala Asn
   275 280 285
   Thr Asp Leu Ser Arg Leu Phe Lys Ala Pro Glu Ile Lys Ala Val Leu
   290 295 300
   Arg Asn Pro Lys Lys Thr Ile Val Arg Arg Val Arg Lys Leu Asn Pro
   305 310 315 320
   Leu Arg Asn Thr Arg Ala Met Leu Arg Leu Asn Pro Tyr Ala Ala Val
   325 330 335
   Leu Lys Arg Lys Ala Ile Leu Asp Gln Arg Lys Leu Lys Leu Gln Lys
   340 345 350
   Leu Val Glu Ala Ala Lys Lys Gly Asp Thr Lys Leu Ser Pro Arg Val
   355 360 365
   Glu Arg His Leu Lys Met Ile Glu Arg Arg Lys Ala Leu Ile Lys Lys
   370 375 380
   Ala Lys Ala Ala Lys Pro Lys Lys Pro Lys Thr Ala Lys Lys Pro Lys
   385 390 395 400
   Thr Ala Glu Lys Ala Pro Ala Pro Ala Lys Lys Ala Ala Ala Pro Lys
   405 410 415
   Lys Ala Thr Thr Pro Ala Lys Lys
   420
<210> 327
   <211> 242
   <212> PRT
   <213> Lygus hesperus
<400> 327
   Met Ala Asn Ala Lys Pro Ile Ser Lys Lys Lys Lys Phe Val Ser Asp
   1               5                   10                  15
   Gly Val Phe Lys Ala Glu Leu Asn Glu Phe Leu Thr Arg Glu Leu Ala
               20                  25                  30
   Glu Glu Gly Tyr Ser Gly Val Glu Val Arg Val Thr Pro Asn Lys Thr
           35                  40                  45
   Glu Ile Ile Ile Met Ala Thr Arg Thr Gln Ser Val Leu Gly Asp Lys
       50                  55                  60
   Gly Arg Arg Ile Arg Glu Leu Thr Ser Val Val Gln Lys Arg Phe Asn
   65                  70                  75                  80
   Phe Lys Pro Gln Thr Leu Asp Leu Tyr Ala Glu Lys Val Ala Thr Arg
                   85                  90                  95
   Gly Leu Cys Ala Ile Ala Gln Ala Glu Ser Leu Arg Tyr Lys Leu Ile
               100                 105                 110
   Gly Gly Leu Ala Val Arg Gly Ala Cys Tyr Gly Val Leu Arg Phe Ile
           115                 120                 125
   Met Glu Asn Gly Ala Lys Gly Cys Glu Val Val Val Ser Gly Lys Leu
       130                 135                 140
   Arg Gly Gln Arg Ala Lys Ser Met Lys Phe Val Asp Gly Leu Met Ile
   145                 150                 155                 160
   His Ser Gly Asp Pro Cys Asn Glu Tyr Val Asp Thr Ala Thr Arg His
                   165                 170                 175
   Val Leu Leu Arg Gln Gly Val Leu Gly Ile Lys Val Lys Ile Met Leu
               180                 185                 190
   Pro Trp Asp Val Thr Gly Lys Asn Gly Pro Lys Asn Pro Leu Pro Asp
           195                 200                 205
   His Val Ser Val Leu Leu Pro Lys Glu Glu Leu Pro Asn Leu Ala Val
       210                 215                 220
   Ser Val Pro Gly Ser Asp Ile Lys Pro Lys Pro Glu Val Pro Ala Pro
   225                 230                 235                 240
   Ala Leu
<210> 328
   <211> 262
   <212> PRT
   <213> Lygus hesperus
<400> 328
   Met Ala Val Gly Lys Asn Lys Gly Leu Ser Lys Gly Gly Lys Lys Gly
   1               5                   10                  15
   Val Lys Lys Lys Val Val Asp Pro Phe Thr Arg Lys Asp Trp Tyr Asp
               20                  25                  30
   Val Lys Ala Pro Ser Met Phe Lys Lys Arg Gln Val Gly Lys Thr Leu
           35                  40                  45
   Val Asn Arg Thr Gln Gly Thr Lys Ile Ala Ser Glu Gly Leu Lys Gly
       50                  55                  60
   Arg Val Phe Glu Val Ser Leu Ala Asp Ile Gln Glu Asp Thr Asp Ala
   65                  70                  75                  80
   Glu Arg Ser Phe Arg Lys Phe Arg Leu Ile Ala Glu Asp Val Gln Ala
                   85                  90                  95
   Arg Asn Val Leu Thr Asn Phe His Gly Met Asp Leu Thr Thr Asp Lys
               100                 105                 110
   Leu Arg Ser Met Val Lys Lys Trp Gln Thr Leu Ile Glu Ala Asn Val
           115                 120                 125
   Asp Val Lys Thr Thr Asp Gly Tyr Leu Leu Arg Val Phe Cys Ile Gly
       130                 135                 140
   Phe Thr Asn Lys Asp Gln Leu Ser Gln Arg Lys Thr Cys Tyr Ala Gln
   145                 150                 155                 160
   His Asn Gln Val Arg Glu Ile Arg Lys Lys Met Val Lys Asn Ile Ser
                   165                 170                 175
   Asp Ser Ile Ser Ser Cys Asp Leu Arg Ser Val Val Asn Lys Leu Ile
               180                 185                 190
   Pro Asp Ser Ile Ala Lys Asp Ile Glu Lys Asn Cys Gln Gly Ile Tyr
           195                 200                 205
   Pro Leu His Asp Val Tyr Ile Arg Lys Val Lys Val Leu Lys Lys Pro
       210                 215                 220
   Arg Phe Glu Leu Ser Lys Leu Leu Glu Leu His Val Asp Gly Lys Gly
   225                 230                 235                 240
   Ile Asp Glu Pro Gly Ala Lys Val Thr Arg Thr Asp Ala Tyr Glu Pro
                   245                 250                 255
   Pro Val Gln Glu Ser Val
               260
<210> 329
   <211> 152
   <212> PRT
   <213> Lygus hesperus
<400> 329
   Met Ser Leu Met Leu Pro Glu Lys Phe Gln His Ile Leu Arg Ile Met
   1               5                   10                  15
   Gly Thr Asn Ile Asp Gly Lys Arg Lys Val Met Phe Ala Met Thr Ala
               20                  25                  30
   Ile Lys Gly Val Gly Arg Arg Tyr Ala Asn Ile Val Leu Lys Lys Ala
           35                  40                  45
   Asp Val Asn Leu Asp Lys Arg Ala Gly Glu Cys Ser Glu Glu Glu Val
       50                  55                  60
   Glu Lys Ile Val Thr Ile Met Gln Asn Pro Arg Gln Tyr Lys Ile Pro
   65                  70                  75                  80
   Asn Trp Phe Leu Asn Arg Gln Lys Asp Thr Val Glu Gly Lys Tyr Ser
                   85                  90                  95
   Gln Leu Thr Ser Ser Leu Leu Asp Ser Lys Leu Arg Asp Asp Leu Glu
               100                 105                 110
   Arg Leu Lys Lys Ile Arg Ala His Arg Gly Met Arg His Tyr Trp Gly
           115                 120                 125
   Leu Arg Val Arg Gly Gln His Thr Lys Thr Thr Gly Arg Arg Gly Arg
       130                 135                 140
   Thr Val Gly Val Ser Lys Lys Lys
   145                 150
<210> 330
   <211> 381
   <212> PRT
   <213> Lygus hesperus
<400> 330
   Met Ser Asp Glu Glu Tyr Ser Glu Ser Glu Glu Glu Thr Gln Pro Glu
   1               5                   10                  15
   Pro Gln Lys Lys Pro Glu Ala Glu Gly Gly Gly Asp Pro Glu Phe Val
               20                  25                  30
   Lys Arg Lys Glu Ala Gln Thr Ser Ala Leu Asp Glu Gln Leu Lys Asp
           35                  40                  45
   Tyr Ile Ala Glu Trp Arg Lys Gln Arg Ala Arg Glu Glu Glu Asp Leu
       50                  55                  60
   Lys Lys Leu Lys Glu Lys Gln Ala Lys Arg Lys Val Ala Arg Ala Glu
   65                  70                  75                  80
   Glu Glu Lys Arg Leu Ala Glu Lys Lys Lys Gln Glu Glu Glu Arg Arg
                   85                  90                  95
   Val Arg Glu Ala Glu Glu Lys Lys Gln Arg Glu Ile Glu Glu Lys Arg
               100                 105                 110
   Arg Arg Leu Glu Glu Ala Glu Lys Lys Arg Gln Ala Met Met Ala Ala
           115                 120                 125
   Leu Lys Asp Gln Ser Lys Thr Lys Gly Pro Asn Phe Val Val Asn Lys
       130                 135                 140
   Lys Ala Glu Thr Leu Gly Met Ser Ser Ala Gln Ile Glu Arg Asn Lys
   145                 150                 155                 160
   Thr Lys Glu Gln Leu Glu Glu Glu Lys Arg Ile Ser Leu Ser Ile Arg
                   165                 170                 175
   Leu Lys Pro Leu Ala Ile Glu Asn Met Ser Ile Asp Arg Leu Arg Ile
               180                 185                 190
   Lys Ala Gln Glu Leu Trp Glu Ala Ile Val Lys Leu Glu Thr Glu Lys
           195                 200                 205
   Tyr Asp Leu Glu Glu Arg Gln Lys Arg Gln Asp Tyr Asp Leu Lys Glu
       210                 215                 220
   Leu Lys Glu Arg Gln Lys Gln Gln Leu Arg His Lys Ala Leu Lys Lys
   225                 230                 235                 240
   Gly Leu Asp Pro Glu Ala Leu Thr Gly Lys Tyr Pro Pro Lys Ile Gln
   245 250 255
   Val Ala Ser Lys Tyr Glu Arg Arg Val Asp Thr Arg Ser Tyr Asp Asp
   260 265 270
   Lys Lys Lys Leu Phe Glu Gly Gly Ile Leu Glu Arg Tyr Lys Glu Leu
   275 280 285
   Ile Glu Lys Val Trp Thr Glu Lys Val Asp Gln Phe Gly Ser Arg Ala
   290 295 300
   His Ser Lys Leu Pro Arg Trp Phe Gly Glu Arg Pro Gly Lys Lys Lys
   305 310 315 320
   Asp Ala Pro Glu Ser Pro Glu Glu Glu Glu Val Lys Val Glu Asp Glu
   325 330 335
   Pro Glu Ala Glu Pro Ser Phe Met Leu Asp Glu Glu Glu Glu Glu Ala
   340 345 350
   Glu Glu Glu Glu Ala Glu Glu Glu Glu Glu Ala Glu Glu Glu Glu Glu
   355 360 365
   Glu Glu Glu Glu Glu Glu Glu Glu Glu Glu Glu Glu Glu
   370 375 380
<210> 331
   <211> 1689
   <212> PRT
   <213> Lygus hesperus
<400> 331
   Ser Gly Lys Leu Ala Gly Ala Asp Ile Glu Thr Tyr Leu Leu Glu Lys
   1               5                   10                  15
   Ala Arg Val Ile Ser Gln Gln Thr Leu Glu Arg Ser Tyr His Ile Phe
               20                  25                  30
   Tyr Gln Met Met Ser Gly Ala Val Lys Gly Val Lys Glu Met Cys Leu
           35                  40                  45
   Leu Val Asp Asp Ile Tyr Thr Tyr Asn Phe Ile Ser Gln Gly Lys Val
       50                  55                  60
   Ser Ile Ala Gly Val Asp Asp Gly Glu Glu Met Val Leu Thr Asp Gln
   65                  70                  75                  80
   Ala Phe Asp Ile Leu Gly Phe Thr Lys Gln Glu Lys Glu Asp Ile Tyr
                   85                  90                  95
   Lys Ile Thr Ala Ala Val Ile His Met Gly Thr Met Lys Phe Lys Gln
               100                 105                 110
   Arg Gly Arg Glu Glu Gln Ala Glu Ala Asp Gly Thr Glu Glu Gly Gly
           115                 120                 125
   Lys Val Gly Val Leu Leu Gly Ile Asp Gly Asp Asp Leu Tyr Lys Asn
       130                 135                 140
   Met Cys Lys Pro Arg Ile Lys Val Gly Thr Glu Phe Val Thr Gln Gly
   145                 150                 155                 160
   Lys Asn Val Asn Gln Val Ser Tyr Ser Leu Gly Ala Met Ser Lys Gly
                   165                 170                 175
   Met Phe Asp Arg Leu Phe Lys Phe Leu Val Lys Lys Cys Asn Glu Thr
               180                 185                 190
   Leu Asp Thr Lys Gln Lys Arg Gln His Phe Ile Gly Val Leu Asp Ile
           195                 200                 205
   Ala Gly Phe Glu Ile Phe Asp Phe Asn Gly Phe Glu Gln Leu Cys Ile
       210                 215                 220
   Asn Phe Thr Asn Glu Lys Leu Gln Gln Phe Phe Asn His His Met Phe
   225                 230                 235                 240
   Val Leu Glu Gln Glu Glu Tyr Lys Arg Glu Gly Ile Asn Trp Ala Phe
                   245                 250                 255
   Ile Asp Phe Gly Met Asp Leu Leu Ala Cys Ile Glu Leu Ile Glu Lys
               260                 265                 270
   Pro Met Gly Ile Leu Ser Ile Leu Glu Glu Glu Ser Met Phe Pro Lys
           275                 280                 285
   Ala Thr Asp Lys Thr Phe Glu Asp Lys Leu Ile Thr Asn His Leu Gly
       290                 295                 300
   Lys Ser Pro Asn Phe Arg Lys Pro Ala Val Pro Lys Pro Gly Gln Gln
   305                 310                 315                 320
   Ala Gly His Phe Ala Ile Ala His Tyr Ala Gly Cys Val Ser Tyr Asn
                   325                 330                 335
   Ile Thr Gly Trp Leu Glu Lys Asn Lys Asp Pro Leu Asn Asp Thr Val
               340                 345                 350
   Val Asp Gln Tyr Lys Lys Gly Thr Asn Lys Leu Leu Cys Glu Ile Phe
           355                 360                 365
   Ala Asp His Pro Gly Gln Ser Gly Ala Pro Gly Gly Asp Ala Gly Gly
       370                 375                 380
   Lys Gly Gly Arg Gly Lys Lys Gly Gly Gly Phe Ala Thr Val Ser Ser
   385                 390                 395                 400
   Ser Tyr Lys Glu Gln Leu Asn Asn Leu Met Thr Thr Leu Lys Ser Thr
                   405                 410                 415
   Gln Pro His Phe Val Arg Cys Ile Ile Pro Asn Glu Leu Lys Gln Pro
               420                 425                 430
   Gly Val Ile Asp Ser His Leu Val Met His Gln Leu Thr Cys Asn Gly
           435                 440                 445
   Val Leu Glu Gly Ile Arg Ile Cys Arg Lys Gly Phe Pro Asn Arg Met
       450                 455                 460
   Asn Tyr Pro Asp Phe Lys Leu Arg Tyr Lys Ile Leu Asn Pro Ala Ala
   465                 470                 475                 480
   Val Asp Arg Glu Ser Asp Ile Leu Lys Ala Ala Gly Leu Val Leu Glu
                   485                 490                 495
   Ser Thr Gly Leu Asp Pro Asp Met Tyr Arg Leu Gly His Thr Lys Val
               500                 505                 510
   Phe Phe Arg Ala Gly Val Leu Gly Gln Leu Glu Glu Leu Arg Asp Asp
           515                 520                 525
   Arg Leu Ser Lys Ile Ile Gly Trp Met Gln Ala Phe Met Arg Gly Tyr
       530                 535                 540
   Leu Val Arg Lys Glu Tyr Lys Lys Leu Gln Glu Gln Arg Leu Ala Leu
   545                 550                 555                 560
   Gln Val Val Gln Arg Asn Leu Arg Arg Tyr Leu Gln Leu Arg Thr Trp
                   565                 570                 575
   Pro Trp Trp Lys Met Trp Ser Arg Val Lys Pro Leu Leu Asn Val Ala
               580                 585                 590
   Asn Val Glu Glu Glu Met Arg Lys Leu Glu Glu Leu Val Ala Glu Thr
           595                 600                 605
   Gln Ala Ala Leu Glu Lys Glu Glu Lys Leu Arg Lys Glu Ala Glu Ala
   610 615 620
   Leu Asn Ala Lys Leu Leu Gln Glu Lys Thr Asp Leu Leu Arg Asn Leu
   625 630 635 640
   Glu Gly Glu Lys Gly Ser Ile Ser Gly Ile Gln Glu Arg Cys Ala Lys
   645 650 655
   Leu Gln Ala Gln Lys Ala Asp Leu Glu Ser Gln Leu Met Asp Thr Gln
   660 665 670
   Glu Arg Leu Gln Asn Glu Glu Asp Ala Arg Asn Gln Leu Phe Gln Gln
   675 680 685
   Lys Lys Lys Leu Glu Gln Glu Ala Ala Ala Leu Lys Lys Asp Ile Glu
   690 695 700
   Asp Leu Glu Leu Ser Asn Gln Lys Thr Asp Gln Asp Lys Ala Ser Lys
   705 710 715 720
   Glu His Gln Ile Arg Asn Leu Asn Asp Glu Ile Ala His Gln Asp Asp
   725 730 735
   Leu Ile Asn Lys Leu Asn Lys Glu Lys Lys Ile Gln Ser Glu Leu Asn
   740 745 750
   Gln Lys Thr Ala Glu Glu Leu Gln Ala Ala Glu Asp Lys Ile Asn His
   755 760 765
   Leu Thr Lys Val Lys Val Lys Leu Glu Gln Thr Leu Asp Glu Leu Glu
   770 775 780
   Asp Thr Leu Glu Arg Glu Lys Lys Leu Arg Gly Asp Val Glu Lys Ala
   785 790 795 800
   Lys Arg Lys Thr Glu Gly Asp Leu Lys Leu Thr Gln Glu Ala Val Ala
   805 810 815
   Asp Leu Glu Arg Asn Lys Lys Glu Leu Glu Gln Thr Ile Gln Arg Lys
   820 825 830
   Asp Lys Glu Ile Ala Ser Leu Thr Ala Lys Leu Glu Asp Glu Gln Ser
   835 840 845
   Ile Val Asn Lys Thr Gly Lys Gln Ile Lys Glu Leu Gln Ser Arg Ile
   850 855 860
   Glu Glu Leu Glu Glu Glu Val Glu Ala Glu Arg Gln Ala Arg Gly Lys
   865 870 875 880
   Ala Glu Lys Gln Arg Ala Asp Leu Ala Arg Glu Leu Glu Glu Leu Gly
   885 890 895
   Glu Arg Leu Glu Glu Ala Gly Gly Ala Thr Ser Ala Gln Ile Glu Leu
   900 905 910
   Asn Lys Lys Arg Glu Ala Glu Met Ser Lys Leu Arg Arg Asp Leu Glu
   915 920 925
   Glu Ala Asn Ile Gln His Glu Gly Thr Leu Ala Asn Leu Arg Lys Lys
   930 935 940
   His Asn Asp Ala Val Ser Glu Met Gly Asp Gln Ile Asp Gln Leu Asn
   945 950 955 960
   Lys Leu Lys Thr Lys Val Glu Lys Glu Lys Ser Gln Tyr Leu Gly Glu
   965 970 975
   Leu Asn Asp Val Arg Ala Ser Ile Asp His Leu Thr Asn Glu Lys Ala
   980 985 990
   Ala Thr Glu Lys Val Ala Lys Gln Leu Gln His Gln Ile Asn Glu Val
   995 1000 1005
   Gln Gly Lys Leu Asp Glu Ala Asn Arg Thr Leu Asn Asp Phe Asp
   1010 1015 1020
   Ala Ala Lys Lys Lys Leu Ser Ile Glu Asn Ser Asp Leu Leu Arg
   1025 1030 1035
   Gln Leu Glu Glu Ala Glu Ser Gln Val Ser Gln Leu Ser Lys Ile
   1040 1045 1050
   Lys Ile Ser Leu Thr Thr Gln Leu Glu Asp Thr Lys Arg Leu Ala
   1055 1060 1065
   Asp Glu Glu Ala Arg Glu Arg Ala Thr Leu Leu Gly Lys Phe Arg
   1070 1075 1080
   Asn Leu Glu His Asp Leu Asp Asn Leu Arg Glu Gln Val Glu Glu
   1085 1090 1095
   Glu Ala Glu Ala Lys Ala Asp Ile Gln Arg Gln Leu Ser Lys Ala
   1100 1105 1110
   Asn Ala Glu Ala Gln Leu Trp Arg Ser Lys Tyr Glu Ser Glu Gly
   1115 1120 1125
   Val Ala  Arg Ala Glu Glu Leu  Glu Glu Ala Lys Arg  Lys Leu Gln
       1130                 1135                 1140
   Ala Arg  Leu Ala Glu Ala Glu  Glu Thr Ile Glu Ser  Leu Asn Gln
       1145                 1150                 1155
   Lys Val  Ile Ala Leu Glu Lys  Thr Lys Gln Arg Leu  Ala Thr Glu
       1160                 1165                 1170
   Val Glu  Asp Leu Gln Leu Glu  Val Asp Arg Ala Asn  Ala Ile Ala
       1175                 1180                 1185
   Asn Ala  Ala Glu Lys Lys Ala  Lys Ala Ile Asp Lys  Ile Ile Gly
       1190                 1195                 1200
   Glu Trp  Lys Leu Lys Val Asp  Asp Leu Ala Ala Glu  Leu Asp Ala
       1205                 1210                 1215
   Ser Gln  Lys Glu Cys Arg Asn  Tyr Ser Thr Glu Leu  Phe Arg Leu
       1220                 1225                 1230
   Lys Gly  Ala Tyr Glu Glu Gly  Gln Glu Gln Leu Glu  Ala Val Arg
       1235                 1240                 1245
   Arg Glu  Asn Lys Asn Leu Ala  Asp Glu Val Lys Asp  Leu Leu Asp
       1250                 1255                 1260
   Gln Ile  Gly Glu Gly Gly Arg  Asn Ile His Glu Ile  Glu Lys Gln
       1265                 1270                 1275
   Arg Lys  Arg Leu Glu Val Glu  Lys Asp Glu Leu Gln  Ala Ala Leu
       1280                 1285                 1290
   Glu Glu  Ala Glu Ala Ala Leu  Glu Gln Glu Glu Asn  Lys Val Leu
       1295                 1300                 1305
   Arg Ala  Gln Leu Glu Leu Ser  Gln Val Arg Gln Glu  Ile Asp Arg
       1310                 1315                 1320
   Arg Ile  Gln Glu Lys Glu Glu  Glu Phe Glu Asn Thr  Arg Lys Asn
       1325                 1330                 1335
   His Gln  Arg Ala Leu Asp Ser  Met Gln Ala Ser Leu  Glu Ala Glu
       1340                 1345                 1350
   Ala Lys  Gly Lys Ala Glu Ala  Leu Arg Met Lys Lys  Lys Leu Glu
       1355                 1360                 1365
   Ala Asp Ile Asn Glu Leu Glu Ile Ala Leu Asp His Ala Asn Lys
   1370 1375 1380
   Ala Asn Ala Glu Ala Gln Lys Thr Ile Lys Lys Tyr Gln Gln Gln
   1385 1390 1395
   Leu Lys Asp Val Gln Thr Ala Leu Glu Glu Glu Gln Arg Ala Arg
   1400 1405 1410
   Asp Asp Ala Arg Glu Gln Leu Gly Ile Ala Glu Arg Arg Ala Asn
   1415 1420 1425
   Ala Leu Gly Asn Glu Leu Glu Glu Ser Arg Thr Leu Leu Glu Gln
   1430 1435 1440
   Ala Asp Arg Gly Arg Arg Gln Ala Glu Gln Glu Leu Gly Asp Ala
   1445 1450 1455
   His Glu Gln Ile Asn Glu Leu Ala Ala Gln Ala Thr Ser Ala Ser
   1460 1465 1470
   Ala Ala Lys Arg Lys Leu Glu Gly Glu Leu Gln Thr Leu His Ala
   1475 1480 1485
   Asp Leu Asp Glu Leu Leu Asn Glu Ala Lys Asn Ser Glu Glu Lys
   1490 1495 1500
   Ala Lys Lys Ala Met Val Asp Ala Ala Arg Leu Ala Asp Glu Leu
   1505 1510 1515
   Arg Ala Glu Gln Asp His Ala Gln Thr Gln Glu Lys Leu Arg Lys
   1520 1525 1530
   Ala Leu Glu Thr Gln Ile Lys Glu Leu Gln Val Arg Leu Asp Glu
   1535 1540 1545
   Ala Glu Asn Asn Ala Leu Lys Gly Gly Lys Lys Ala Ile Ala Lys
   1550 1555 1560
   Leu Glu Gln Arg Val Arg Glu Leu Glu Asn Glu Leu Asp Gly Glu
   1565 1570 1575
   Gln Arg Arg His Ala Asp Ala Gln Lys Asn Leu Arg Lys Ser Glu
   1580 1585 1590
   Arg Arg Ile Lys Glu Leu Ser Phe Gln Ser Asp Glu Asp Arg Lys
   1595 1600 1605
   Asn His Glu Arg Met Gln Asp Leu Val Asp Lys Leu Gln Gln Lys
   1610 1615 1620
   Ile Lys Thr Tyr Lys Arg Gln Ile Glu Glu Ala Glu Glu Ile Ala
   1625 1630 1635
   Ala Leu Asn Leu Ala Lys Phe Arg Lys Ala Gln Gln Glu Leu Glu
   1640 1645 1650
   Glu Ala Glu Glu Arg Ala Asp Leu Ala Glu Gln Ala Val Ser Lys
   1655 1660 1665
   Phe Arg Thr Lys Gly Gly Arg Ala Gly Ser Ala Ala Arg Ala Met
   1670 1675 1680
   Ser Pro Val Gly Gln Lys
   1685
<210> 332
   <211> 256
   <212> PRT
   <213> Lygus hesperus
<400> 332
   Asp Ala Ile Lys Lys Lys Met Gln Ala Met Lys Met Glu Lys Asp Thr
   1               5                   10                  15
   Ala Met Asp Lys Ala Asp Thr Cys Glu Gly Gln Ala Lys Asp Ala Asn
               20                  25                  30
   Thr Arg Ala Asp Lys Ile Leu Glu Asp Val Arg Asp Leu Gln Lys Lys
           35                  40                  45
   Leu Asn Gln Val Glu Ser Asp Leu Glu Arg Thr Lys Arg Glu Leu Glu
       50                  55                  60
   Thr Lys Thr Thr Glu Leu Glu Glu Lys Glu Lys Ala Asn Thr Asn Ala
   65                  70                  75                  80
   Glu Ser Glu Val Ala Ser Leu Asn Arg Lys Val Gln Met Val Glu Glu
                   85                  90                  95
   Asp Leu Glu Arg Ser Glu Glu Arg Ser Gly Thr Ala Gln Gln Lys Leu
               100                 105                 110
   Ser Glu Ala Ser His Ala Ala Asp Glu Ala Ser Arg Met Cys Lys Val
           115                 120                 125
   Leu Glu Asn Arg Ser Gln Gln Asp Glu Glu Arg Met Asp Gln Leu Thr
       130                 135                 140
   Asn Gln Leu Lys Glu Ala Arg Leu Leu Ala Glu Asp Ala Asp Gly Lys
   145                 150                 155                 160
   Ser Asp Glu Val Ser Arg Lys Leu Ala Phe Val Glu Asp Glu Leu Glu
                   165                 170                 175
   Val Ala Glu Asp Arg Val Lys Ser Gly Asp Ser Lys Ile Met Glu Leu
               180                 185                 190
   Glu Glu Glu Leu Lys Val Val Gly Asn Ser Leu Lys Ser Leu Glu Val
           195                 200                 205
   Ser Glu Glu Lys Ala Asn Gln Arg Val Glu Glu Tyr Lys Arg Gln Ile
       210                 215                 220
   Lys Gln Leu Thr Val Lys Leu Lys Glu Ala Glu Ala Arg Ala Glu Phe
   225                 230                 235                 240
   Ala Glu Lys Thr Val Lys Lys Leu Gln Lys Glu Val Asp Arg Leu Glu
                   245                 250                 255
<210> 333
   <211> 85
   <212> PRT
   <213> Lygus hesperus
<400> 333
   Arg Ala Leu Gly Gln Asn Pro Thr Glu Ser Asp Val Lys Lys Phe Thr
   1               5                   10                  15
   His Gln His Lys Pro Asp Glu Arg Ile Ser Phe Glu Val Phe Leu Pro
               20                  25                  30
   Ile Tyr Gln Ala Ile Ser Lys Gly Arg Thr Ser Asp Thr Ala Glu Asp
           35                  40                  45
   Phe Ile Glu Gly Leu Arg His Phe Asp Lys Asp Gly Asn Gly Phe Ile
       50                  55                  60
   Ser Thr Ala Glu Leu Arg His Leu Leu Thr Thr Leu Gly Glu Lys Leu
   65                  70                  75                  80
   Thr Asp Asp Glu Val
                   85
<210> 334
   <211> 174
   <212> PRT
   <213> Lygus hesperus
<400> 334
   Met Ser Ser Arg Lys Thr Ala Gly Arg Arg Ala Thr Thr Lys Lys Arg
   1               5                   10                  15
   Ala Gln Arg Ala Thr Ser Asn Val Phe Ala Met Phe Asp Gln Ala Gln
               20                  25                  30
   Ile Gln Glu Phe Lys Glu Ala Phe Asn Met Ile Asp Gln Asn Arg Asp
           35                  40                  45
   Gly Phe Val Asp Lys Glu Asp Leu His Asp Met Leu Ala Ser Leu Gly
       50                  55                  60
   Lys Asn Pro Ser Asp Glu Tyr Leu Glu Gly Met Met Asn Glu Ala Pro
   65                  70                  75                  80
   Gly Pro Ile Asn Phe Thr Met Phe Leu Thr Leu Phe Gly Glu Arg Leu
                   85                  90                  95
   Gln Gly Thr Asp Pro Glu Glu Val Ile Lys Asn Ala Phe Gly Cys Phe
               100                 105                 110
   Asp Glu Asp Asn Asn Gly Phe Ile Asn Glu Glu Arg Leu Arg Glu Leu
           115                 120                 125
   Leu Thr Ser Met Gly Asp Arg Phe Thr Asp Glu Asp Val Asp Glu Met
       130                 135                 140
   Tyr Arg Glu Ala Pro Ile Lys Asn Gly Met Phe Asp Tyr Ile Glu Phe
   145                 150                 155                 160
   Thr Arg Ile Leu Lys His Gly Ala Lys Asp Lys Asp Glu Gln
                   165                 170
<210> 335
   <211> 1881
   <212> PRT
   <213> Lygus hesperus
<400> 335
   Asp Leu Thr Cys Leu Asn Glu Ala Ser Val Leu His Asn Ile Lys Asp
   1               5                   10                  15
   Arg Tyr Tyr Ser Gly Leu Ile Tyr Thr Tyr Ser Gly Leu Phe Cys Val
               20                  25                  30
   Val Val Asn Pro Tyr Lys Lys Leu Pro Ile Tyr Thr Glu Arg Ile Met
           35                  40                  45
   Glu Lys Tyr Lys Gly Val Lys Arg His Asp Leu Pro Pro His Val Phe
       50                  55                  60
   Ala Ile Thr Asp Thr Ala Tyr Arg Ser Met Leu Gln Asp Arg Glu Asp
   65                  70                  75                  80
   Gln Ser Ile Leu Cys Thr Gly Glu Ser Gly Ala Gly Lys Thr Glu Asn
                   85                  90                  95
   Thr Lys Lys Val Ile Gln Tyr Leu Ala Tyr Val Ala Ala Ser Lys Pro
               100                 105                 110
   Lys Ser Ser Ala Ser Pro His Thr Ala Gln Ser Gln Ala Leu Ile Ile
           115                 120                 125
   Gly Glu Leu Glu Gln Gln Leu Leu Gln Ala Asn Pro Ile Leu Glu Ala
       130                 135                 140
   Phe Gly Asn Ala Lys Thr Val Lys Asn Asp Asn Ser Ser Arg Phe Gly
   145                 150                 155                 160
   Lys Phe Ile Arg Ile Asn Phe Asp Ala Ser Gly Tyr Ile Ala Gly Ala
                   165                 170                 175
   Asn Ile Glu Thr Tyr Leu Leu Glu Lys Ser Arg Ala Ile Arg Gln Ala
               180                 185                 190
   Lys Asp Glu Arg Thr Phe His Ile Phe Tyr Gln Leu Leu Ala Gly Ala
           195                 200                 205
   Ser Ala Glu Gln Arg Lys Glu Phe Ile Leu Glu Asp Pro Lys Asn Tyr
       210                 215                 220
   Pro Phe Leu Ser Ser Gly Met Val Ser Val Pro Gly Val Asp Asp Gly
   225                 230                 235                 240
   Val Asp Phe Gln Ala Thr Ile Ala Ser Met Ser Ile Met Gly Met Thr
                   245                 250                 255
   Asn Asp Asp Leu Ser Ala Leu Phe Arg Ile Val Ser Ala Val Met Leu
               260                 265                 270
   Phe Gly Ser Met Gln Phe Lys Gln Glu Arg Asn Ser Asp Gln Ala Thr
           275                 280                 285
   Leu Pro Asp Asn Thr Val Ala Gln Lys Ile Ala His Leu Leu Gly Leu
       290                 295                 300
   Ser Ile Thr Glu Met Thr Lys Ala Phe Leu Arg Pro Arg Ile Lys Val
   305                 310                 315                 320
   Gly Arg Asp Phe Val Thr Lys Ala Gln Thr Lys Glu Gln Val Glu Phe
                   325                 330                 335
   Ala Val Glu Ala Ile Ser Lys Ala Cys Tyr Glu Arg Met Phe Arg Trp
               340                 345                 350
   Leu Val Asn Arg Ile Asn Arg Ser Leu Asp Arg Thr Lys Arg Gln Gly
           355                 360                 365
   Ala Ser Phe Ile Gly Ile Leu Asp Met Ala Gly Phe Glu Ile Phe Glu
       370                 375                 380
   Ile Asn Ser Phe Glu Gln Leu Cys Ile Asn Tyr Thr Asn Glu Lys Leu
   385                 390                 395                 400
   Gln Gln Leu Phe Asn His Thr Met Phe Ile Leu Glu Gln Glu Glu Tyr
                   405                 410                 415
   Gln Arg Glu Gly Ile Glu Trp Lys Phe Ile Asp Phe Gly Leu Asp Leu
               420                 425                 430
   Gln Pro Thr Ile Asp Leu Ile Asp Lys Pro Met Gly Val Met Ala Leu
           435                 440                 445
   Leu Asp Glu Glu Cys Trp Phe Pro Lys Ala Thr Asp Lys Thr Phe Val
       450                 455                 460
   Glu Lys Leu Val Gly Ala His Ser Val His Pro Lys Phe Ile Lys Thr
   465                 470                 475                 480
   Asp Phe Arg Gly Val Ala Asp Phe Ala Val Val His Tyr Ala Gly Lys
                   485                 490                 495
   Val Asp Tyr Ser Ala Ala Gln Trp Leu Met Lys Asn Met Asp Pro Leu
               500                 505                 510
   Asn Glu Asn Val Val Gln Leu Leu Gln Asn Ser Gln Asp Pro Phe Val
           515                 520                 525
   Ile His Ile Trp Lys Asp Ala Glu Ile Val Gly Met Ala His Gln Ala
       530                 535                 540
   Leu Ser Asp Thr Gln Phe Gly Ala Arg Thr Arg Lys Gly Met Phe Arg
   545                 550                 555                 560
   Thr Val Ser Gln Leu Tyr Lys Asp Gln Leu Ser Lys Leu Met Ile Thr
                   565                 570                 575
   Leu Arg Asn Thr Asn Pro Asn Phe Val Arg Cys Ile Leu Pro Asn His
               580                 585                 590
   Glu Lys Arg Ala Gly Lys Ile Asp Ala Pro Leu Val Leu Asp Gln Leu
           595                 600                 605
   Arg Cys Asn Gly Val Leu Glu Gly Ile Arg Ile Cys Arg Gln Gly Phe
       610                 615                 620
   Pro Asn Arg Ile Pro Phe Gln Glu Phe Arg Gln Arg Tyr Glu Leu Leu
   625                 630                 635                 640
   Thr Pro Asn Val Ile Pro Lys Gly Phe Met Asp Gly Lys Lys Ala Cys
                   645                 650                 655
   Glu Lys Met Ile Asn Ala Leu Glu Leu Asp Pro Asn Leu Tyr Arg Val
               660                 665                 670
   Gly Gln Ser Lys Ile Phe Phe Arg Ala Gly Val Leu Ala His Leu Glu
           675                 680                 685
   Glu Glu Arg Asp Tyr Lys Ile Thr Asp Leu Ile Ala Asn Phe Arg Ala
       690                 695                 700
   Phe Cys Arg Gly Tyr Leu Ala Arg Arg Asn Tyr Gln Lys Arg Leu Gln
   705                 710                 715                 720
   Gln Leu Asn Ala Ile Arg Ile Ile Gln Arg Asn Cys Ser Ala Tyr Leu
                   725                 730                 735
   Lys Leu Arg Asn Trp Gln Trp Trp Arg Leu Tyr Thr Lys Val Lys Pro
               740                 745                 750
   Leu Leu Glu Val Thr Lys Gln Glu Glu Lys Leu Thr Gln Lys Glu Asp
           755                 760                 765
   Glu Leu Lys Gln Val Arg Glu Lys Leu Asp Asn Gln Val Arg Ser Lys
       770                 775                 780
   Glu Glu Tyr Glu Lys Arg Leu Gln Asp Ala Leu Glu Glu Lys Ala Ala
   785                 790                 795                 800
   Leu Ala Glu Gln Leu Gln Ala Glu Val Glu Leu Cys Ala Glu Ala Glu
                   805                 810                 815
   Glu Met Arg Ala Arg Leu Ala Val Arg Lys Gln Glu Leu Glu Glu Ile
               820                 825                 830
   Leu His Asp Leu Glu Ala Arg Ile Glu Glu Glu Glu Gln Arg Asn Thr
   835 840 845
   Val Leu Ile Asn Glu Lys Lys Lys Leu Thr Leu Asn Ile Ala Asp Leu
   850 855 860
   Glu Glu Gln Leu Glu Glu Glu Glu Gly Ala Arg Gln Lys Leu Gln Leu
   865 870 875 880
   Glu Lys Val Gln Ile Glu Ala Arg Leu Lys Lys Met Glu Glu Asp Leu
   885 890 895
   Ala Leu Ala Glu Asp Thr Asn Thr Lys Val Val Lys Glu Lys Lys Val
   900 905 910
   Leu Glu Glu Arg Ala Ser Asp Leu Ala Gln Thr Leu Ala Glu Glu Glu
   915 920 925
   Glu Lys Ala Lys His Leu Ala Lys Leu Lys Thr Lys His Glu Thr Thr
   930 935 940
   Ile Ala Glu Leu Glu Glu Arg Leu Leu Lys Asp Asn Gln Gln Arg Gln
   945 950 955 960
   Glu Met Asp Arg Asn Lys Arg Lys Ile Glu Ser Glu Val Asn Asp Leu
   965 970 975
   Lys Glu Gln Ile Asn Glu Lys Lys Val Gln Val Glu Glu Leu Gln Leu
   980 985 990
   Gln Leu Gly Lys Arg Glu Glu Glu Ile Ala Gln Ala Leu Met Arg Ile
   995 1000 1005
   Asp Glu Glu Gly Ala Gly Lys Ala Gln Thr Gln Lys Ala Leu Arg
   1010 1015 1020
   Glu Leu Glu Ser Gln Leu Ala Glu Leu Gln Glu Asp Leu Glu Ala
   1025 1030 1035
   Glu Lys Ala Ala Arg Ala Lys Ala Glu Lys Gln Lys Arg Asp Leu
   1040 1045 1050
   Asn Glu Glu Leu Glu Ser Leu Lys Asn Glu Leu Leu Asp Ser Leu
   1055 1060 1065
   Asp Thr Thr Ala Ala Gln Gln Glu Leu Arg Thr Lys Arg Glu His
   1070 1075 1080
   Glu Leu  Ala Thr Leu Lys Lys  Thr Leu Glu Glu Glu  Thr His Ile
       1085                 1090                 1095
   His Glu  Val Ser Leu Thr Glu  Met Arg His Lys His  Thr Gln Glu
       1100                 1105                 1110
   Val Ala  Ala Leu Asn Glu Gln  Leu Glu Gln Leu Lys  Lys Ala Lys
       1115                 1120                 1125
   Ser Ala  Leu Glu Lys Ser Lys  Ala Gln Leu Glu Gly  Glu Ala Ala
       1130                 1135                 1140
   Glu Leu  Ala Asn Glu Leu Glu  Thr Ala Gly Thr Ser  Lys Gly Glu
       1145                 1150                 1155
   Ser Glu  Arg Lys Arg Lys Gln  Ala Glu Ser Ser Leu  Gln Glu Leu
       1160                 1165                 1170
   Ser Ser  Arg Leu Leu Glu Met  Glu Arg Thr Lys Ala  Glu Leu Gln
       1175                 1180                 1185
   Glu Arg  Val Gln Lys Leu Ser  Ala Glu Ala Asp Ser  Val Asn Gln
       1190                 1195                 1200
   Gln Leu  Glu Ala Ala Glu Leu  Lys Ala Ser Ala Ala  Leu Lys Ala
       1205                 1210                 1215
   Ser Gly  Thr Leu Glu Thr Gln  Leu Gln Glu Ala Gln  Val Leu Leu
       1220                 1225                 1230
   Glu Glu  Glu Thr Arg Gln Lys  Leu Ser Leu Thr Thr  Lys Leu Lys
       1235                 1240                 1245
   Gly Leu  Glu Ser Glu Arg Asp  Ala Leu Lys Glu Gln  Leu Tyr Glu
       1250                 1255                 1260
   Glu Asp  Glu Gly Arg Lys Asn  Leu Glu Lys Gln Met  Ala Ile Leu
       1265                 1270                 1275
   Asn Gln  Gln Val Ala Glu Ser  Lys Lys Lys Ser Glu  Glu Glu Thr
       1280                 1285                 1290
   Glu Lys  Ile Thr Glu Leu Glu  Glu Ser Arg Lys Lys  Leu Leu Lys
       1295                 1300                 1305
   Asp Ile  Glu Ile Leu Gln Arg  Gln Val Glu Glu Leu  Gln Val Thr
       1310                 1315                 1320
   Asn Asp  Lys Leu Glu Lys Gly  Lys Lys Lys Leu Gln  Ser Glu Leu
       1325                1330                1335
   Glu Asp  Leu Thr Ile Asp Leu  Glu Ser Gln Arg Thr  Lys Val Val
       1340                1345                1350
   Glu Leu  Glu Lys Lys Gln Arg  Asn Phe Asp Lys Val  Leu Ala Glu
       1355                1360                1365
   Glu Lys  Ala Leu Ser Gln Gln  Ile Thr His Glu Arg  Asp Ala Ala
       1370                1375                1380
   Glu Arg  Glu Ala Arg Glu Lys  Glu Thr Arg Val Leu  Ser Leu Thr
       1385                1390                1395
   Arg Glu  Leu Asp Glu Phe Met  Glu Lys Ile Glu Glu  Leu Glu Arg
       1400                1405                1410
   Ser Lys  Arg Gln Leu Gln Ala  Glu Leu Asp Glu Leu  Val Asn Asn
       1415                1420                1425
   Gln Gly  Thr Thr Asp Lys Ser  Val His Glu Leu Glu  Arg Ala Lys
       1430                1435                1440
   Arg Val  Leu Glu Ser Gln Leu  Ala Glu Gln Lys Ala  Gln Asn Glu
       1445                1450                1455
   Glu Leu  Glu Asp Glu Leu Gln  Met Thr Glu Asp Ala  Lys Leu Arg
       1460                1465                1470
   Leu Glu  Val Asn Met Gln Ala  Leu Arg Ala Gln Phe  Glu Arg Asp
       1475                1480                1485
   Leu Gln  Gly Lys Glu Glu Ser  Gly Glu Glu Lys Arg  Arg Gly Leu
       1490                1495                1500
   Leu Lys  Gln Leu Arg Asp Ile  Glu Ala Glu Leu Glu  Asp Glu Arg
       1505                1510                1515
   Lys Gln  Arg Thr Ala Ala Val  Ala Ser Arg Lys Lys  Ile Glu Ala
       1520                1525                1530
   Asp Phe  Lys Asp Val Glu Gln  Gln Leu Glu Met His  Thr Lys Val
       1535                1540                1545
   Lys Glu  Asp Leu Gln Lys Gln  Leu Lys Lys Cys Gln  Val Gln Leu
       1550                1555                1560
   Lys Asp  Ala Ile Arg Asp Ala  Glu Glu Ala Arg Leu  Gly Arg Glu
       1565                1570                1575
   Glu Leu  Gln Ala Ala Ala Lys  Glu Ala Glu Arg Lys  Trp Lys Gly
       1580                 1585                 1590
   Leu Glu  Thr Glu Leu Ile Gln  Val Gln Glu Asp Leu  Met Ala Ser
       1595                 1600                 1605
   Glu Arg  Gln Arg Arg Ala Ala  Glu Ala Glu Arg Asp  Glu Val Val
       1610                 1615                 1620
   Glu Glu  Ala Asn Lys Asn Val  Lys Ser Leu Ser Asn  Leu Leu Asp
       1625                 1630                 1635
   Glu Lys  Lys Arg Leu Glu Ala  Gln Cys Ser Gly Leu  Glu Glu Glu
       1640                 1645                 1650
   Leu Glu  Glu Glu Leu Ser Asn  Asn Glu Ala Leu Gln  Asp Lys Ala
       1655                 1660                 1665
   Arg Lys  Ala Gln Leu Ser Val  Glu Gln Leu Asn Ala  Glu Leu Ala
       1670                 1675                 1680
   Ala Glu  Arg Ser Asn Val Gln  Lys Leu Glu Gly Thr  Arg Leu Ser
       1685                 1690                 1695
   Met Glu  Arg Gln Asn Lys Glu  Leu Lys Ala Lys Leu  Asn Glu Leu
       1700                 1705                 1710
   Glu Thr  Leu Gln Arg Asn Lys  Phe Lys Ala Asn Ala  Ser Leu Glu
       1715                 1720                 1725
   Ala Lys  Ile Thr Asn Leu Glu  Glu Gln Leu Glu Asn  Glu Ala Lys
       1730                 1735                 1740
   Glu Lys  Leu Leu Leu Gln Lys  Gly Asn Arg Lys Leu  Asp Lys Lys
       1745                 1750                 1755
   Ile Lys  Asp Leu Leu Val Gln  Leu Glu Asp Glu Arg  Arg His Ala
       1760                 1765                 1770
   Asp Gln  Tyr Lys Glu Gln Val  Glu Lys Ile Asn Val  Arg Val Lys
       1775                 1780                 1785
   Thr Leu  Lys Arg Thr Leu Asp  Asp Ala Glu Glu Glu  Met Ser Arg
       1790                 1795                 1800
   Glu Lys  Thr Gln Lys Arg Lys  Ala Leu Arg Glu Leu  Glu Asp Leu
       1805                 1810                 1815
   Arg Glu Asn Tyr Asp Ser Leu Leu Arg Glu Asn Asp Asn Leu Lys
   1820 1825 1830
   Asn Lys Leu Arg Arg Gly Gly Gly Ile Ser Gly Ile Ser Ser Arg
   1835 1840 1845
   Leu Gly Gly Ser Lys Arg Gly Ser Ile Pro Gly Glu Asp Ser Gln
   1850 1855 1860
   Gly Leu Asn Asn Thr Thr Asp Glu Ser Val Asp Gly Asp Asp Ile
   1865 1870 1875
   Ser Asn Pro
   1880
<210> 336
   <211> 108
   <212> PRT
   <213> Lygus hesperus
<400> 336
   Lys Lys Ile Leu Glu Glu Ile Ile Ala Glu Val Asp Ala Asp Gly Ser
   1 5 10 15
   Gly Gln Leu Glu Phe Glu Glu Phe Val Ala Leu Ala Ala Gly Phe Leu
   20 25 30
   Thr Glu Asp Glu Thr Gln Asp Ala Glu Ala Met Gln Gln Glu Leu Arg
   35 40 45
   Glu Ala Phe Arg Leu Tyr Asp Lys Glu Gly Asn Gly Tyr Ile Thr Thr
   50 55 60
   Asp Val Leu Arg Glu Ile Leu Lys Glu Leu Asp Asp Lys Ile Thr Ser
   65 70 75 80
   Gln Glu Leu Asp Met Met Ile Ala Glu Ile Asp Ser Asp Gly Ser Gly
   85 90 95
   Thr Val Asp Phe Asp Glu Phe Met Glu Met Met Thr
   100 105
<210> 337
   <211> 141
   <212> PRT
   <213> Lygus hesperus
<400> 337
   Ile Pro Ile Met Thr Ile Ala Leu Asn Ala Phe Asp Arg Asp His Ser
   1 5 10 15
   Gly Ser Ile Pro Thr Asp Met Val Ala Asp Ile Leu Arg Leu Met Gly
   20 25 30
   Gln Pro Phe Asn Lys Lys Ile Leu Asp Glu Leu Ile Glu Glu Val Asp
   35 40 45
   Ala Asp Lys Ser Gly Arg Leu Glu Phe Glu Glu Phe Ile Thr Leu Ala
   50 55 60
   Ala Lys Phe Ile Val Glu Glu Asp Asp Glu Ala Met Gln Lys Glu Leu
   65 70 75 80
   Arg Glu Ala Phe Arg Leu Tyr Asp Lys Glu Gly Asn Gly Tyr Ile Pro
   85 90 95
   Thr Ser Cys Leu Lys Glu Ile Leu His Glu Leu Asp Glu Gln Leu Thr
   100 105 110
   Asn Glu Glu Leu Asp Met Ile Ile Glu Glu Ile Asp Ser Asp Gly Ser
   115 120 125
   Gly Thr Val Asp Phe Asp Glu Phe Met Glu Met Met Thr
   130 135 140
<210> 338
   <211> 58
   <212> PRT
   <213> Lygus hesperus
<400> 338
   Trp Val Lys Glu Gly Ala Cys Ser Glu Gln Ser Ser Arg Met Thr Ala
   1 5 10 15
   Met Asp Asn Ala Ser Lys Asn Ala Ala Glu Met Ile Asp Lys Leu Thr
   20 25 30
   Leu Thr Phe Asn Arg Thr Arg Gln Ala Val Ile Thr Arg Glu Leu Ile
   35 40 45
   Glu Ile Ile Ser Gly Ala Ser Ala Leu Glu
   50 55
<210> 339
   <211> 130
   <212> PRT
   <213> Lygus hesperus
<400> 339
   Met Val Arg Met Asn Val Leu Ser Asp Ala Leu Lys Ser Ile Asn Asn
   1               5                   10                  15
   Ala Glu Lys Arg Gly Lys Arg Gln Val Leu Leu Arg Pro Cys Ser Lys
               20                  25                  30
   Val Ile Ile Lys Phe Leu Thr Val Met Met Lys Lys Gly Tyr Ile Gly
           35                  40                  45
   Glu Phe Glu Ile Val Asp Asp His Arg Ser Gly Lys Ile Val Val Asn
       50                  55                  60
   Leu Asn Gly Arg Leu Asn Lys Cys Gly Val Ile Ser Pro Arg Phe Asp
   65                  70                  75                  80
   Val Pro Ile Thr Gln Ile Glu Lys Trp Thr Asn Asn Leu Leu Pro Ser
                   85                  90                  95
   Arg Gln Phe Gly Tyr Val Val Leu Thr Thr Ser Gly Gly Ile Met Asp
               100                 105                 110
   His Glu Glu Ala Arg Arg Lys His Leu Gly Gly Lys Ile Leu Gly Phe
           115                 120                 125
   Phe Phe
       130
<210> 340
   <211> 131
   <212> PRT
   <213> Lygus hesperus
<400> 340
   Val Asp Gly Gly Leu Asn Ile Pro His Ser Thr Lys Arg Phe Pro Gly
   1               5                   10                  15
   Tyr Asp Ser Glu Ser Lys Glu Phe Asn Ala Glu Val His Arg Lys His
               20                  25                  30
   Ile Phe Gly Ile His Val Ala Asp Tyr Met Arg Gln Leu Ala Glu Glu
           35                  40                  45
   Asp Asp Asp Ala Tyr Lys Lys Gln Phe Ser Gln Tyr Val Lys Asn Gly
       50                  55                  60
   Val Thr Ala Asp Ser Ile Glu Ser Ile Tyr Lys Lys Ala His Glu Ala
   65                  70                  75                  80
   Ile Arg Ala Asp Pro Thr Arg Lys Pro Leu Glu Lys Lys Glu Val Lys
                   85                  90                  95
   Lys Lys Arg Trp Asn Arg Ala Lys Leu Ser Leu Ser Glu Arg Lys Asn
               100                 105                 110
   Thr Ile Asn Gln Lys Lys Ala Thr Tyr Leu Lys Lys Val Glu Ala Gly
           115                 120                 125
   Glu Ile Glu
       130
<210> 341
   <211> 214
   <212> PRT
   <213> Lygus hesperus
<400> 341
   Met Ala Pro Lys Gly Asn Asn Met Ile Pro Asn Gly His Phe His Lys
   1               5                   10                  15
   Asp Trp Gln Arg Phe Ile Lys Thr Trp Phe Asn Gln Pro Ala Arg Lys
               20                  25                  30
   Leu Arg Arg Arg Asn Lys Arg Leu Glu Lys Ala Gln Arg Leu Ala Pro
           35                  40                  45
   Arg Pro Ala Gly Pro Leu Arg Pro Ala Val Arg Cys Pro Thr Val Arg
       50                  55                  60
   Tyr His Thr Lys Leu Arg Pro Gly Arg Gly Phe Thr Leu Glu Glu Ile
   65                  70                  75                  80
   Lys Arg Ala Gly Leu Cys Lys Gly Phe Ala Met Ser Ile Gly Ile Ala
                   85                  90                  95
   Val Asp Pro Arg Arg Arg Asn Lys Ser Ile Glu Ser Leu Gln Leu Asn
               100                 105                 110
   Val Gln Arg Leu Lys Glu Tyr Arg Ala Lys Leu Ile Leu Phe Pro His
           115                 120                 125
   Lys Asn Ala Lys Lys Leu Lys Lys Gly Glu Ala Thr Glu Glu Glu Arg
       130                 135                 140
   Lys Val Ala Thr Gln Gln Pro Leu Pro Val Met Pro Ile Lys Gln Pro
   145                 150                 155                 160
   Val Ile Lys Phe Lys Ala Arg Val Ile Thr Asp Asp Glu Lys Lys Tyr
                   165                 170                 175
   Ser Ala Phe Thr Ala Leu Arg Lys Gly Arg Ala Asp Gln Arg Leu Val
               180                 185                 190
   Gly Ile Arg Ala Lys Arg Ala Lys Glu Ala Ala Glu Asn Ala Glu Asp
           195                 200                 205
   Pro Ser Lys Ala Pro Lys
       210
<210> 342
   <211> 134
   <212> PRT
   <213> Lygus hesperus
<400> 342
   Met Asp Ile Glu Glu Pro Ala Ala Ala Pro Thr Glu Pro Ser Asp Val
   1               5                   10                  15
   Asn Thr Ala Leu Gln Glu Val Leu Lys Ala Ala Leu Gln His Gly Val
               20                  25                  30
   Val Val His Gly Ile His Glu Ser Ala Lys Ala Leu Asp Lys Arg Gln
           35                  40                  45
   Ala Leu Leu Cys Val Leu Ala Glu Asn Cys Asp Glu Pro Met Tyr Lys
       50                  55                  60
   Lys Leu Val Gln Ala Leu Cys Ser Glu His His Ile Pro Leu Val Lys
   65                  70                  75                  80
   Val Asp Ser Asn Lys Lys Leu Gly Glu Trp Thr Gly Leu Cys Lys Ile
                   85                  90                  95
   Asp Lys Thr Gly Lys Ser Arg Lys Ile Val Gly Cys Ser Cys Val Val
               100                 105                 110
   Ile Lys Asp Trp Gly Glu Asp Thr Pro His Leu Asp Leu Leu Lys Asp
           115                 120                 125
   Tyr Ile Arg Asp Val Phe
       130
<210> 343
   <211> 148
   <212> PRT
   <213> Lygus hesperus
<400> 343
   Met Lys Met Asn Lys Leu Val Thr Ser Ser Arg Arg Lys Asn Arg Lys
   1               5                   10                  15
   Arg His Phe Thr Ala Pro Ser His Ile Arg Arg Lys Leu Met Ser Ala
               20                  25                  30
   Pro Leu Ser Lys Glu Leu Arg Gln Lys Tyr Asn Val Arg Thr Met Pro
           35                  40                  45
   Val Arg Lys Asp Asp Glu Val Gln Val Val Arg Gly His Tyr Lys Gly
       50                  55                  60
   Gln Gln Val Gly Lys Val Leu Gln Val Tyr Arg Lys Lys Phe Ile Ile
   65                  70                  75                  80
   Tyr Ile Glu Arg Ile Gln Arg Glu Lys Ala Asn Gly Ala Ser Val Tyr
                   85                  90                  95
   Val Gly Ile His Pro Ser Lys Cys Val Ile Val Lys Leu Lys Val Asp
               100                 105                 110
   Lys Asp Arg Lys Glu Ile Leu Asp Arg Arg Ser Lys Gly Arg Asp Leu
           115                 120                 125
   Ala Leu Gly Lys Asp Lys Gly Lys Tyr Thr Glu Asp Ser Thr Thr Ala
       130                 135                 140
   Met Asp Thr Ser
   145
<210> 344
   <211> 65
   <212> PRT
   <213> Lygus hesperus
<400> 344
   Met Glu Lys Pro Val Val Leu Ala Arg Val Ile Lys Ile Leu Gly Arg
   1               5                   10                  15
   Thr Gly Ser Gln Gly Gln Cys Thr Gln Val Lys Val Glu Phe Ile Gly
               20                  25                  30
   Glu Gln Asn Arg Gln Ile Ile Arg Asn Val Lys Gly Pro Val Arg Glu
           35                  40                  45
   Gly Asp Ile Leu Thr Leu Leu Glu Ser Glu Arg Glu Ala Arg Arg Leu
       50                  55                  60
   Arg
   65
<210> 345
   <211> 229
   <212> PRT
   <213> Lygus hesperus
<400> 345
   Leu Phe Tyr Phe Pro Phe Ser Arg Lys Trp Gly Asp Val Gln Arg Gly
   1               5                   10                  15
   Val Ile Gly Thr Val Lys Thr Ser His Thr Pro Lys Ser Arg Phe Cys
               20                  25                  30
   Arg Gly Val Pro Asp Pro Lys Ile Arg Ile Phe Asp Leu Gly Lys Lys
           35                  40                  45
   Lys Ala Arg Val Glu Asp Phe Pro Leu Cys Val His Leu Val Ser Asp
       50                  55                  60
   Glu Tyr Glu Gln Leu Ser Ser Glu Ala Leu Glu Ala Gly Arg Ile Cys
   65                  70                  75                  80
   Cys Asn Lys Tyr Leu Val Lys Asn Cys Gly Lys Asp Gln Phe His Ile
                   85                  90                  95
   Arg Met Arg Leu His Pro Phe His Val Ile Arg Ile Asn Lys Met Leu
               100                 105                 110
   Ser Cys Ala Gly Ala Asp Arg Leu Gln Thr Gly Met Arg Gly Ala Phe
           115                 120                 125
   Gly Lys Pro Gln Gly Thr Val Ala Arg Val Arg Ile Gly Gln Pro Ile
       130                 135                 140
   Met Ser Val Arg Ser Ser Asp Arg Tyr Lys Ala Ala Val Ile Lys Ala
   145                 150                 155                 160
   Leu Arg Arg Ala Lys Phe Lys Phe Pro Gly Arg Gln Lys Ile Tyr Val
                   165                 170                 175
   Ser Lys Lys Trp Gly Phe Thr Lys Phe Asp Arg Glu Glu Tyr Glu Gly
               180                 185                 190
   Leu Arg Asn Asp Asn Lys Leu Ala Asn Asp Gly Cys Asn Val Lys Leu
           195                 200                 205
   Arg Pro Asp His Gly Pro Leu Gln Ala Trp Arg Lys Ala Gln Leu Asp
       210                 215                 220
   Ile Ala Ala Gly Leu
   225
<210> 346
   <211> 220
   <212> PRT
   <213> Lygus hesperus
<400> 346
   Met Gly Arg Arg Pro Ala Arg Cys Tyr Arg Tyr Cys Lys Asn Lys Pro
   1               5                   10                  15
   Tyr Pro Lys Ser Arg Phe Cys Arg Gly Val Pro Asp Pro Lys Ile Arg
               20                  25                  30
   Ile Phe Asp Leu Gly Lys Lys Lys Ala Arg Val Glu Asp Phe Pro Leu
           35                  40                  45
   Cys Val His Leu Val Ser Asp Glu Tyr Glu Gln Leu Ser Ser Glu Ala
       50                  55                  60
   Leu Glu Ala Gly Arg Ile Cys Cys Asn Lys Tyr Leu Val Lys Asn Cys
   65                  70                  75                  80
   Gly Lys Asp Gln Phe His Ile Arg Met Arg Leu His Pro Phe His Val
                   85                  90                  95
   Ile Arg Ile Asn Lys Met Leu Ser Cys Ala Gly Ala Asp Arg Leu Gln
               100                 105                 110
   Thr Gly Met Arg Gly Ala Phe Gly Lys Pro Gln Gly Thr Val Ala Arg
           115                 120                 125
   Val Arg Ile Gly Gln Pro Ile Met Ser Val Arg Ser Ser Asp Arg Tyr
       130                 135                 140
   Lys Ala Ala Val Ile Glu Ala Leu Arg Arg Ala Lys Phe Lys Phe Pro
   145                 150                 155                 160
   Gly Arg Gln Lys Ile Tyr Val Ser Lys Lys Trp Gly Phe Thr Lys Phe
                   165                 170                 175
   Asp Arg Glu Glu Tyr Glu Gly Leu Arg Asn Asp Asn Lys Leu Ala Asn
               180                 185                 190
   Gly Gly Cys Asn Val Lys Leu Arg Pro Asp His Gly Pro Leu Gln Ala
           195                 200                 205
   Trp Arg Lys Ala Gln Leu Asp Ile Ala Ala Gly Leu
       210                 215                 220
<210> 347
   <211> 159
   <212> PRT
   <213> Lygus hesperus
<400> 347
   Met Thr Asn Ser Lys Gly Tyr Arg Arg Gly Thr Arg Asp Leu Phe Ser
   1               5                   10                  15
   Arg Pro Phe Arg His His Gly Val Ile Pro Leu Ser Thr Tyr Met Lys
               20                  25                  30
   Val Tyr Arg Val Gly Asp Ile Val Ser Ile Lys Gly Asn Gly Ala Val
           35                  40                  45
   Gln Lys Gly Met Pro His Lys Val Tyr His Gly Lys Thr Gly Arg Val
       50                  55                  60
   Tyr Asn Val Thr Pro Arg Ala Leu Gly Val Ile Val Asn Lys Arg Val
   65                  70                  75                  80
   Arg Gly Lys Ile Leu Pro Lys Arg Ile Asn Ile Arg Ile Glu His Val
                   85                  90                  95
   Asn His Ser Lys Cys Arg Glu Asp Phe Leu Lys Arg Val Arg Glu Asn
               100                 105                 110
   Glu Arg Leu Arg Lys Phe Ala Lys Glu Thr Gly Thr Arg Val Glu Leu
           115                 120                 125
   Lys Arg Gln Pro Ala Gln Pro Arg Pro Ala His Phe Val Gln Ala Lys
       130                 135                 140
   Glu Val Pro Glu Leu Leu Ala Pro Ile Pro Tyr Glu Phe Ile Ala
   145                 150                 155
<210> 348
   <211> 131
   <212> PRT
   <213> Lygus hesperus
<400> 348
   Thr Tyr Met Lys Val Tyr Arg Val Gly Asp Ile Val Ser Ile Lys Gly
   1               5                   10                  15
   Asn Gly Ala Val Gln Lys Gly Met Pro His Lys Val Tyr His Gly Lys
               20                  25                  30
   Thr Gly Arg Val Tyr Asn Val Thr Pro Arg Ala Leu Gly Val Ile Val
           35                  40                  45
   Asn Lys Arg Val Arg Gly Lys Ile Leu Pro Lys Arg Ile Asn Ile Arg
       50                  55                  60
   Ile Glu His Val Asn His Ser Lys Cys Arg Glu Asp Phe Leu Lys Arg
   65                  70                  75                  80
   Val Arg Glu Asn Glu Arg Leu Arg Lys Phe Ala Lys Glu Thr Gly Thr
                   85                  90                  95
   Arg Val Glu Leu Lys Arg Gln Pro Ala Gln Pro Arg Pro Ala His Phe
               100                 105                 110
   Val Gln Ala Lys Glu Val Pro Glu Leu Leu Ala Pro Ile Pro Tyr Glu
           115                 120                 125
   Phe Ile Ala
       130
<210> 349
   <211> 150
   <212> PRT
   <213> leptinotarsa decemlineata
<400> 349
   Lys Lys Ala Lys Lys Gly Phe Met Thr Pro Glu Arg Lys Lys Lys Leu
   1               5                   10                  15
   Arg Leu Leu Leu Arg Lys Lys Ala Ala Glu Glu Leu Lys Lys Glu Gln
               20                  25                  30
   Glu Arg Lys Ala Ala Glu Arg Arg Arg Ile Ile Glu Glu Arg Cys Gly
           35                  40                  45
   Lys Pro Lys Leu Ile Asp Glu Ala Asn Glu Glu Gln Val Arg Asn Tyr
       50                  55                  60
   Cys Lys Leu Tyr His Gly Arg Ile Ala Lys Leu Glu Asp Gln Lys Phe
   65                  70                  75                  80
   Asp Leu Glu Tyr Leu Val Lys Lys Lys Asp Met Glu Ile Ala Glu Leu
                   85                  90                  95
   Asn Ser Gln Val Asn Asp Leu Arg Gly Lys Phe Val Lys Pro Thr Leu
               100                 105                 110
   Lys Lys Val Ser Lys Tyr Glu Asn Lys Phe Ala Lys Leu Gln Lys Lys
           115                 120                 125
   Ala Ala Glu Phe Asn Phe Arg Asn Gln Leu Lys Val Val Lys Lys Lys
       130                 135                 140
   Glu Phe Thr Leu Glu Glu
   145                 150
<210> 350
   <211> 279
   <212> PRT
   <213> leptinotarsa decemlineata
<400> 350
   Gln Trp Tyr Gln Arg Arg Val Arg Gly Asp Ile Glu Glu Lys Arg Gln
   1               5                   10                  15
   Arg Leu Glu Glu Ala Glu Lys Lys Arg Gln Ala Met Met Gln Ala Leu
               20                  25                  30
   Lys Asp Gln Asn Lys Asn Lys Gly Pro Asn Phe Thr Ile Thr Lys Arg
           35                  40                  45
   Asp Ala Ser Ser Asn Leu Ser Ala Ala Gln Leu Glu Arg Asn Lys Thr
       50                  55                  60
   Lys Glu Gln Leu Glu Glu Glu Lys Lys Ile Ser Leu Ser Ile Arg Ile
   65                  70                  75                  80
   Lys Pro Leu Val Val Asp Gly Leu Gly Val Asp Lys Leu Arg Leu Lys
                   85                  90                  95
   Ala Gln Glu Leu Trp Glu Cys Ile Val Lys Leu Glu Thr Glu Lys Tyr
               100                 105                 110
   Asp Leu Glu Glu Arg Gln Lys Arg Gln Asp Tyr Asp Leu Lys Glu Leu
           115                 120                 125
   Lys Glu Arg Gln Lys Gln Gln Leu Arg His Lys Ala Leu Lys Lys Gly
       130                 135                 140
   Leu Asp Pro Glu Ala Leu Thr Gly Lys Tyr Pro Pro Lys Ile Gln Val
   145                 150                 155                 160
   Ala Ser Lys Tyr Glu Arg Arg Val Asp Thr Arg Ser Tyr Gly Asp Lys
                   165                 170                 175
   Lys Lys Leu Phe Glu Gly Gly Leu Glu Glu Ile Ile Lys Glu Thr Asn
               180                 185                 190
   Glu Lys Ser Trp Lys Glu Lys Phe Gly Gln Phe Asp Ser Arg Gln Lys
           195                 200                 205
   Ala Arg Leu Pro Lys Trp Phe Gly Glu Arg Pro Gly Lys Lys Pro Gly
   210 215 220
   Asp Pro Glu Thr Pro Glu Gly Glu Glu Glu Gly Lys Gln Val Ile Asp
   225 230 235 240
   Glu Asp Asp Asp Leu Lys Glu Pro Val Ile Glu Ala Glu Ile Glu Glu
   245 250 255
   Glu Glu Glu Glu Glu Glu Val Glu Val Asp Glu Glu Glu Glu Asp Asp
   260 265 270
   Glu Glu Glu Glu Glu Glu Glu
   275
<210> 351
   <211> 286
   <212> PRT
   <213> leptinotarsa decemlineata
<400> 351
   Ala Leu Gln Asn Glu Leu Glu Glu Ser Arg Thr Leu Leu Glu Gln Ala
   1               5                   10                  15
   Asp Arg Ala Arg Arg Gln Ala Glu Gln Glu Leu Gly Asp Ala His Glu
               20                  25                  30
   Gln Leu Asn Asp Leu Gly Ala Gln Asn Gly Ser Leu Ser Ala Ala Lys
           35                  40                  45
   Arg Lys Leu Glu Thr Glu Leu Gln Thr Leu His Ser Asp Leu Asp Glu
       50                  55                  60
   Leu Leu Asn Glu Ala Lys Asn Ser Glu Glu Lys Ala Lys Lys Ala Met
   65                  70                  75                  80
   Val Asp Ala Ala Arg Leu Ala Asp Glu Leu Arg Ala Glu Gln Asp His
                   85                  90                  95
   Ala Gln Thr Gln Glu Lys Leu Arg Lys Ala Leu Glu Ser Gln Ile Lys
               100                 105                 110
   Asp Leu Gln Val Arg Leu Asp Glu Ala Glu Ala Asn Ala Leu Lys Gly
           115                 120                 125
   Gly Lys Lys Ala Ile Ala Lys Leu Glu Gln Arg Val Arg Glu Leu Glu
       130                 135                 140
   Asn Glu Leu Asp Gly Glu Gln Arg Arg His Ala Asp Ala Gln Lys Asn
   145                 150                 155                 160
   Leu Arg Lys Ser Glu Arg Arg Ile Lys Glu Leu Ser Leu Gln Ala Glu
                   165                 170                 175
   Glu Asp Arg Lys Asn His Glu Lys Met Gln Asp Leu Val Asp Lys Leu
               180                 185                 190
   Gln Gln Lys Ile Lys Thr His Lys Arg Gln Ile Glu Glu Ala Glu Glu
           195                 200                 205
   Ile Ala Ala Leu Asn Leu Ala Lys Phe Arg Lys Ala Gln Gln Glu Leu
       210                 215                 220
   Glu Glu Ala Glu Glu Arg Ala Asp Leu Ala Glu Gln Ala Ile Val Lys
   225                 230                 235                 240
   Phe Arg Thr Lys Gly Arg Ser Gly Ser Ala Ala Arg Gly Ala Ser Pro
                   245                 250                 255
   Ala Pro Gln Arg Gln Arg Pro Thr Phe Gly Met Gly Asp Ser Leu Gly
               260                 265                 270
   Gly Ala Phe Pro Pro Arg Phe Asp Leu Ala Pro Asp Phe Glu
           275                 280                 285
<210> 352
   <211> 197
   <212> PRT
   <213> nilaparvata lugens
<400> 352
   Met Ala Asp Asp Glu Ala Lys Lys Ala Lys Gln Ala Glu Ile Asp Arg
   1               5                   10                  15
   Lys Arg Ala Glu Val Arg Lys Arg Met Glu Glu Ala Ser Lys Ala Lys
               20                  25                  30
   Lys Ala Lys Lys Gly Phe Met Thr Pro Asp Arg Lys Lys Lys Leu Arg
           35                  40                  45
   Leu Leu Leu Arg Lys Lys Ala Ala Glu Glu Leu Lys Lys Glu Gln Glu
       50                  55                  60
   Arg Lys Ala Ala Glu Arg Arg Arg Ile Ile Glu Glu Arg Cys Gly Lys
   65                  70                  75                  80
   Ala Val Asp Leu Asp Asp Gly Ser Glu Glu Lys Val Lys Ala Thr Leu
                   85                  90                  95
   Lys Thr Tyr His Asp Arg Ile Gly Lys Leu Glu Asp Glu Lys Phe Asp
               100                 105                 110
   Leu Glu Tyr Ile Val Lys Lys Lys Asp Phe Glu Ile Ala Asp Leu Asn
           115                 120                 125
   Ser Gln Val Asn Asp Leu Arg Gly Lys Phe Val Lys Pro Thr Leu Lys
       130                 135                 140
   Lys Val Ser Lys Tyr Glu Asn Lys Phe Ala Lys Leu Gln Lys Lys Ala
   145                 150                 155                 160
   Ala Glu Phe Asn Phe Arg Asn Gln Leu Lys Val Val Lys Lys Lys Glu
                   165                 170                 175
   Phe Thr Leu Glu Glu Glu Asp Lys Glu Pro Lys Lys Ser Glu Lys Ala
               180                 185                 190
   Glu Trp Gln Lys Lys
           195
<210> 353
   <211> 184
   <212> PRT
   <213> nilaparvata lugens
<400> 353
   Met Met Ala Ala Leu Lys Asp Gln Ser Lys Ser Lys Gly Pro Asn Phe
   1               5                   10                  15
   Thr Val Asn Lys Lys Thr Asp Leu Asn Met Thr Ser Ala Gln Met Glu
               20                  25                  30
   Arg Asn Lys Thr Lys Glu Gln Leu Glu Glu Glu Lys Lys Ile Ser Leu
           35                  40                  45
   Ser Phe Arg Ile Lys Pro Leu Ala Ile Glu Asn Met Ser Ile Asn Ala
       50                  55                  60
   Leu Arg Ala Lys Ala Gln Glu Leu Trp Asp Cys Ile Val Lys Leu Glu
   65                  70                  75                  80
   Thr Glu Lys Tyr Asp Leu Glu Glu Arg Gln Lys Arg Gln Asp Tyr Asp
                   85                  90                  95
   Leu Lys Glu Leu Lys Glu Arg Gln Lys Gln Gln Leu Arg His Lys Ala
               100                 105                 110
   Leu Lys Lys Gly Leu Asp Pro Glu Ala Leu Thr Gly Lys Tyr Pro Pro
           115                 120                 125
   Lys Ile Gln Val Ala Ser Lys Tyr Glu Arg Arg Val Asp Thr Arg Ser
       130                 135                 140
   Tyr Asp Asp Lys Lys Lys Leu Phe Glu Gly Gly Trp Asp Thr Leu Thr
   145                 150                 155                 160
   Ser Glu Thr Asn Glu Lys Ile Trp Lys Ser Arg Asn Asp Gln Phe Ser
                   165                 170                 175
   Asn Arg Ser Lys Ala Lys Leu Pro
               180
<210> 354
   <211> 122
   <212> PRT
   <213> nilaparvata lugens
<400> 354
   Ala Phe Asp Arg Glu Arg Ser Gly Ser Ile Pro Thr Asp Met Val Ala
   1               5                   10                  15
   Asp Ile Leu Arg Leu Met Gly Gln Pro Phe Asn Lys Lys Ile Leu Asp
               20                  25                  30
   Glu Leu Ile Glu Glu Val Asp Ala Asp Lys Ser Gly Arg Leu Glu Phe
           35                  40                  45
   Asp Glu Phe Val Thr Leu Ala Ala Lys Phe Ile Val Glu Glu Asp Asp
       50                  55                  60
   Glu Ala Met Gln Lys Glu Leu Lys Glu Ala Phe Arg Leu Tyr Asp Lys
   65                  70                  75                  80
   Glu Gly Asn Gly Tyr Ile Pro Thr Ser Cys Leu Lys Glu Ile Leu Arg
                   85                  90                  95
   Glu Leu Asp Asp Gln Leu Thr Asn Glu Glu Leu Asn Met Met Ile Asp
               100                 105                 110
   Glu Ile Asp Ser Asp Gly Ser Gly Thr Val
           115                 120
<210> 355
   <211> 73
   <212> PRT
   <213> nilaparvata lugens
<400> 355
   Val Lys Thr Leu Thr Gly Lys Thr Ile Thr Leu Glu Val Glu Pro Ser
   1               5                   10                  15
   Asp Thr Ile Glu Asn Val Lys Ala Lys Ile Gln Asp Lys Glu Gly Ile
               20                  25                  30
   Pro Pro Asp Gln Gln Arg Leu Ile Phe Ala Gly Lys Gln Leu Glu Asp
           35                  40                  45
   Gly Arg Thr Leu Ser Asp Tyr Asn Ile Gln Lys Glu Ser Thr Leu His
       50                  55                  60
   Leu Val Leu Arg Leu Arg Gly Gly Thr
   65                  70
<210> 356
   <211> 253
   <212> PRT
   <213> Acyrthosiphon pisum
<400> 356
   Met Ala Asp Asp Glu Ala Lys Lys Ala Lys Gln Ala Glu Ile Asp Arg
   1               5                   10                  15
   Lys Arg Ala Glu Val Arg Lys Arg Met Glu Glu Ala Ser Lys Ala Lys
               20                  25                  30
   Lys Ala Lys Lys Gly Phe Met Thr Pro Asp Arg Lys Lys Lys Leu Arg
           35                  40                  45
   Leu Leu Leu Lys Lys Lys Ala Ala Glu Glu Leu Lys Lys Glu Gln Glu
       50                  55                  60
   Arg Lys Ala Ala Glu Arg Arg Arg Ile Ile Glu Glu Arg Cys Gly Gln
   65                  70                  75                  80
   Pro Lys Asn Ile Asp Asp Ala Gly Glu Glu Glu Leu Ala Glu Ile Cys
                   85                  90                  95
   Glu Glu Leu Trp Lys Arg Val Tyr Thr Val Glu Gly Ile Lys Phe Asp
               100                 105                 110
   Leu Glu Arg Asp Ile Arg Met Lys Val Phe Glu Ile Ser Glu Leu Asn
           115                 120                 125
   Ser Gln Val Asn Asp Leu Arg Gly Lys Phe Val Lys Pro Thr Leu Lys
       130                 135                 140
   Lys Val Ser Lys Tyr Glu Asn Lys Phe Ala Lys Leu Gln Lys Lys Ala
   145                 150                 155                 160
   Ala Glu Phe Asn Phe Arg Asn Gln Leu Lys Val Val Lys Lys Lys Glu
                   165                 170                 175
   Phe Thr Leu Glu Glu Glu Asp Lys Glu Lys Lys Pro Asp Trp Ser Lys
               180                 185                 190
   Lys Gly Asp Glu Lys Lys Gly Glu Gly Glu Asp Gly Asp Gly Thr Glu
           195                 200                 205
   Asp Glu Lys Thr Asp Asp Gly Leu Thr Thr Glu Gly Glu Ser Val Ala
       210                 215                 220
   Gly Asp Leu Thr Asp Ala Thr Glu Asp Ala Gln Ser Asp Asn Glu Ile
   225                 230                 235                 240
   Leu Glu Pro Glu Pro Val Val Glu Pro Glu Pro Glu Pro
                   245                 250
<210> 357
   <211> 179
   <212> PRT
   <213> Acyrthosiphon pisum
<400> 357
   Val Met Arg Cys Gly Lys Lys Lys Val Trp Leu Asp Pro Asn Glu Ile
   1               5                   10                  15
   Asn Glu Ile Ala Asn Thr Asn Ser Arg Gln Asn Ile Arg Lys Leu Ile
               20                  25                  30
   Lys Asp Gly Leu Ile Ile Lys Lys Pro Val Ala Val His Ser Arg Ala
           35                  40                  45
   Arg Ala Arg Lys Asn Ala Asp Ala Arg Arg Lys Gly Arg His Cys Gly
       50                  55                  60
   Phe Gly Lys Arg Lys Gly Thr Ala Asn Ala Arg Thr Pro Gln Lys Asp
   65                  70                  75                  80
   Leu Trp Val Lys Arg Met Arg Val Leu Arg Arg Leu Leu Lys Lys Tyr
                   85                  90                  95
   Arg Glu Ala Lys Lys Ile Asp Asn His Leu Tyr His Gln Leu Tyr Met
               100                 105                 110
   Lys Ala Lys Gly Asn Val Phe Lys Asn Lys Arg Val Leu Met Glu Phe
           115                 120                 125
   Ile His Lys Lys Lys Ala Glu Lys Ala Arg Ala Lys Met Leu Ser Asp
       130                 135                 140
   Gln Ala Glu Ala Arg Arg Gln Lys Val Lys Glu Ala Arg Lys Arg Lys
   145                 150                 155                 160
   Glu Ala Arg Phe Leu Gln Asn Arg Lys Glu Leu Leu Ala Ala Tyr Ala
                   165                 170                 175
   Arg Glu Asp
<210> 358
   <211> 275
   <212> PRT
   <213> Acyrthosiphon pisum
<400> 358
   Gly Leu Glu Val Glu Ser Ser Asp Ser Ile Glu Asn Val Lys Ala Lys
   1               5                   10                  15
   Ile Gln Asp Lys Glu Gly Ile Pro Pro Asp Gln Gln Arg Leu Ile Phe
               20                  25                  30
   Ala Gly Lys Gln Leu Glu Asp Gly Arg Thr Leu Ser Asp Tyr Asn Ile
           35                  40                  45
   Gln Lys Glu Ser Thr Leu His Leu Val Leu Arg Leu Arg Gly Gly Met
       50                  55                  60
   Gln Ile Phe Val Lys Thr Leu Thr Gly Lys Thr Ile Thr Leu Glu Val
   65                  70                  75                  80
   Glu Ser Ser Asp Ser Ile Glu Asn Val Lys Ala Lys Ile Gln Asp Lys
                   85                  90                  95
   Glu Gly Ile Pro Pro Asp Gln Gln Arg Leu Ile Phe Ala Gly Lys Gln
               100                 105                 110
   Leu Glu Asp Gly Arg Thr Leu Ser Asp Tyr Asn Ile Gln Lys Glu Ser
           115                 120                 125
   Thr Leu His Leu Val Leu Arg Leu Arg Gly Gly Met Gln Ile Phe Val
       130                 135                 140
   Lys Thr Leu Thr Gly Lys Thr Ile Thr Leu Glu Val Glu Ser Ser Asp
   145                 150                 155                 160
   Ser Ile Glu Asn Val Lys Ala Lys Ile Gln Asp Lys Glu Gly Ile Pro
                   165                 170                 175
   Pro Asp Gln Gln Arg Leu Ile Phe Ala Gly Lys Gln Leu Glu Asp Gly
               180                 185                 190
   Arg Thr Leu Ser Asp Tyr Asn Ile Gln Lys Glu Ser Thr Leu His Leu
           195                 200                 205
   Val Leu Arg Leu Arg Gly Gly Met Gln Ile Phe Val Lys Thr Leu Thr
       210                 215                 220
   Gly Lys Thr Ile Thr Leu Glu Val Glu Ser Ser Asp Ser Ile Glu Asn
   225                 230                 235                 240
   Val Lys Ala Lys Ile Gln Asp Lys Glu Gly Ile Pro Pro Asp Gln Gln
                   245                 250                 255
   Arg Leu Ile Phe Ala Gly Lys Gln Leu Glu Asp Gly Arg Thr Leu Ser
               260                 265                 270
   Asp Tyr Asn
           275
<210> 359
   <211> 56
   <212> PRT
   <213> Acyrthosiphon pisum
<400> 359
   Asp Leu Leu His Pro Thr Ala Ile Glu Glu Arg Arg Lys His Lys Leu
   1               5                   10                  15
   Lys Arg Leu Val Gln His Pro Asn Ser Phe Phe Met Asp Val Lys Cys
               20                  25                  30
   Pro Gly Cys Tyr Lys Ile Thr Thr Val Phe Ser His Ala Gln Ser Val
           35                  40                  45
   Val Ile Cys Thr Gly Cys Ser Thr
       50                  55
<210> 360
   <211> 20
   <212> DNA
   <213> Artificial
<220>
   <223> Primer
<400> 360
   atcatgcagg cgtacgcccg 20
<210> 361
   <211> 19
   <212> DNA
   <213> Artificial
<220>
   <223> Primer
<400> 361
   cggagggggc gagatcact 19
<210> 362
   <211> 62
   <212> DNA
   <213> Artificial
<220>
   <223> Amplicon
<400> 362
   atcatgcagg cgtacgcccg agaagacgag gctgccgtca aaaagtgatc tcgccccctc 60
   cg 62
<210> 363
   <211> 22
   <212> DNA
   <213> Artificial
<220>
   <223> Primer
<400> 363
   tgtgttggct actggtggct ac 22
<210> 364
   <211> 25
   <212> DNA
   <213> Artificial
<220>
   <223> Primer
<400> 364
   tcggatggaa ctggacaaat tcaag 25
<210> 365
   <211> 137
   <212> DNA
   <213> Artificial
<220>
   <223> Amplicon
<400> 365
   tgtgttggct actggtggct acggcagagc ttacttttca tgcacttcag ctcacacttg 60
   cacgggagat ggccaagcaa tggtttcacg agctgggctt cccaacgaag atcttgaatt 120
   tgtccagttc catccga 137
<210> 366
   <211> 21
   <212> DNA
   <213> Artificial
<220>
   <223> Primer
<400> 366
   gcaacccgtg ttctccaaag c 21
<210> 367
   <211> 28
   <212> DNA
   <213> Artificial
<220>
   <223> Primer
<400> 367
   tcaactcgta ttctcgtact ttcaaacc 28
<210> 368
   <211> 146
   <212> DNA
   <213> Artificial
<220>
   <223> Amplicon
<400> 368
   gcaacccgtg ttctccaaag ccagatacac tgtgcgatcc ttcggtatca ggcgtaacga 60
   aaaaatcgcc gttcactgca ctgtcagggg cgccaaagca gaggaaattc tggagcgtgg 120
   tttgaaagta cgagaatacg agttga 146
<210> 369
   <211> 21
   <212> DNA
   <213> Artificial
<220>
   <223> Primer
<400> 369
   atggccgacg atgaagctaa g 21
<210> 370
   <211> 20
   <212> DNA
   <213> Artificial
<220>
   <223> Primer
<400> 370
   tggttctggt tcgggttcaa 20
<210> 371
   <211> 20
   <212> DNA
   <213> Artificial
<220>
   <223> Primer
<400> 371
   cggtaatgcg atgcggtaag 20
<210> 372
   <211> 20
   <212> DNA
   <213> Artificial
<220>
   <223> Primer
<400> 372
   tcatcttctc gggcgtatgc 20
<210> 373
   <211> 25
   <212> DNA
   <213> Artificial
<220>
   <223> Primer
<400> 373
   tttggaagtt gagtcatcag attcc 25
<210> 374
   <211> 24
   <212> DNA
   <213> Artificial
<220>
   <223> Primer
<400> 374
   gttgtagtcg gaaagggtac gtcc 24
<210> 375
   <211> 20
   <212> DNA
   <213> Artificial
<220>
   <223> Primer
<400> 375
   attgtggaac atccggtaca 20
<210> 376
   <211> 23
   <212> DNA
   <213> Artificial
<220>
   <223> Primer
<400> 376
   aagacttgct tcatcctact gca 23
<210> 377
   <211> 28
   <212> DNA
   <213> Artificial
<220>
   <223> Primer
<220>
   <221> misc_feature
   <222> (20)..(20)
   <223> n is a, c, g, or t
<400> 377
   ccaagaaggc caagaagggn ttyatgac 28
<210> 378
   <211> 29
   <212> DNA
   <213> Artificial
<220>
   <223> Primer
<400> 378
   tcctcctcca gggtgaactc yttyttytt 29
<210> 379
   <211> 26
   <212> DNA
   <213> Artificial
<220>
   <223> Primer
<220>
   <221> misc_feature
   <222> (21) .. (21)
   <223> n is a, c, g, or t
<220>
   <221> misc_feature
   <222> (24) .. (24)
   <223> n is a, c, g, or t
<400> 379
   gccaagaagg gcttcatgac nccnga 26
<210> 380
   <211> 27
   <212> DNA
   <213> Artificial
<220>
   <223> Primer
<400> 380
   gaagttgaac tcggcggcyt tyttytg 27
<210> 381
   <211> 26
   <212> DNA
   <213> Artificial
<220>
   <223> Primer
<220>
   <221> misc_feature
   <222> (24) .. (24)
   <223> n is a, c, g, or t
<400> 381
   ctggaggagg ccgagaaraa rmgnca 26
<210> 382
   <211> 22
   <212> DNA
   <213> Artificial
<220>
   <223> Primer
<220>
   <221> misc_feature
   <222> (14)..(14)
   <223> n is a, c, g, or t
<400> 382
   tgccgggccg ctcnccraac ca 22
<210> 383
   <211> 28
   <212> DNA
   <213> Artificial
<220>
   <223> Primer
<220>
   <221> misc_feature
   <222> (20)..(20)
   <223> n is a, c, g, or t
<220>
   <221> misc_feature
   <222> (23) .. (23)
   <223> n is a, c, g, or t
<400> 383
   agatcgccat cctgaggaan gcnttyra 28
<210> 384
   <211> 31
   <212> DNA
   <213> Artificial
<220>
   <223> Primer
<400> 384
   cggtcatcat ctccatgaac tcrtcraart c 31
<210> 385
   <211> 170
   <212> DNA
   <213> Artificial
<220>
   <223> Intron
<400> 385
   tctagaaggt aagtgtacac actacatttt catgaacatt attgcgaccg ttgagattct      60
   cattgtttgg tgattgatta tctaaagtag aagcatgaat agatataaca taaactagta     120
   actaatgggt tagttatggg tatacttcat gcttttctct caggctcgag                170
<210> 386
   <211> 521
   <212> DNA
   <213> leptinotarsa decemlineata
<400> 386
   tcgatttttc atttttcttt tattatttgg agtgggcctg ttgtggtcgt tatcaaaatg 60
   ggtaaaataa tgaaatctgg taaagtcgta ttggtccttg gaggccgata cgctggaaga 120
   aaggcagtag tcataaaaaa ttacgatgat gggacgtcag ataaacaata tggacatgcc 180
   gtggtggctg gaatcgatag gtaccctaga aaaatccaca aacgtatggg caaaggaaaa 240
   atgcacaaga ggtccaaaat caagcccttc cttaaggtgc tcaactataa ccatttgatg 300
   cctacaagat attcagtgga tttgacttcg gacttgaaag tggcgcccaa agacctcaag 360
   gatccagtga agaggaagaa gattaggttc caaaccagag ttaaattcga agagagatac 420
   aagcaaggaa aacacaaatg gtttttccag aaattgaggt tctagattct ataaatttaa 480
   ccattttgta atccacccac ctttttgttc aaataaattg t 521
<210> 387
   <211> 475
   <212> DNA
   <213> leptinotarsa decemlineata
<400> 387
   tatcgcgaaa aatatacaac ttacaaaatg aggaacacgt atgagttgag ccctaaagaa 60
   gcagcaaatt tcactcgtcg aaatttagca gatactcttc gaagcaggag tccatatcat 120
   gttaatcttc tcttggctgg atatgacaag aaagacgggg ctcagttgta ttacatggat 180
   tatctagcgt ctgttgctag tgttgattac gctgcccatg gatacggagg atatttctcc 240
   ctttccataa tggatcgcaa ttatttgaaa accctgtcga aagatcaagg atacgaactt 300
   ctgaaggaat gtgttaaaga agttcaaaag agacttgcta taaatttacc aaatttcaaa 360
   gttcaggtta ttgataaaga tggtattaag gatatgccta atataacttc aaaaggtttg 420
   aattgattaa gcaacttcag tttcagattt ttttctaaat aaacatttaa agtgt 475
<210> 388
   <211> 467
   <212> DNA
   <213> leptinotarsa decemlineata
<400> 388
   gcggggactg gatacatctc taaaacacag aaaaatgaaa ttcttcaagt caggaatata      60
   ttctgttgta tttttggcaa ttatattttc tttggtcact gaggaagtgg aaggtcgaag     120
   gactatttta agagggcgta aaacactgac gagaacctat tttcgtgaca atgcagtccc     180
   agcatacgtc atagtgatac tcgttggaat aggagaaatc attttgggag ctatcctgta     240
   tgttataatg aggaaaacga taatagattt tcctttatca gggagttacg cagtggcccc     300
   tactcaagaa gcataaatcc cattgaaatt gtgactgttt actttctttg gaaaaatgtg     360
   tataataaat acaattcatt tataatattt atatttggaa cttaaaatac ttacaaaatt     420
   accatttaca tgatcaaata actaataaag ttctgtctca attataa                   467
<210> 389
   <211> 906
   <212> DNA
   <213> leptinotarsa decemlineata
<400> 389
   ggattggaag taaaaatata caattcatgc tgtagctgta gtgtaaaaac tgaactgaaa      60
   gccataaaat aaagaccttg caagaaacat gtccaagatt aatgaggtgt ctaatttgta     120
   caaacaactg aaatcagaat ggaacacatc caatccaaat ttaagcaaat gtgaaaagct     180
   tttgtcagat ttgaagcttg agctaacaca cttaatgttc cttccaactt caaacgccac     240
   tgcttcaaaa caagaacttc ttctggcaag agatgttctg gaaattgggg tacaatggag     300
   tatagctgca aatgatatac ctgcctttga aagatacatg gcacagttga aatgttatta     360
   tttcgattat aagaatcaac ttcccgaatc ttctttcaaa tatcagttac tgggtctgaa     420
   tttactattt ttgttatcac aaaatagagt ggcagagttc cacacagaat tagaattgtt     480
   gcctgctgac cacattcaga atgatgtata catcaggcac cctccatcta ttgaacagta     540
   ccttatggaa ggaagttata ataagatatt tctggcaaag ggaaatgtcc cagcaacaaa     600
   ttacaatttt tttatggata tacttctaga tactatcaga ggggagattg cagattgtct     660
   agagaaagca tatgaaaaaa tatcaattaa agatgttgct aggatgctat acttgggcag     720
   tgaagaatcg gccaaggcct ttgtaacaaa gagtaagaca tggaaattag aaaaggacaa     780
   cttctttcac ttcacgcccg aggttaaaaa gacacatgag ccaattctat ccaaagaatt     840
   ggcacaacaa gctattgaat atgcaaaaga actggaaatg attgtttaaa gtaataaagt     900
   ttttca                                                                906
<210> 390
   <211> 135
   <212> PRT
   <213> leptinotarsa decemlineata
<400> 390
   Met Gly Lys Ile Met Lys Ser Gly Lys Val Val Leu Val Leu Gly Gly
   1               5                   10                  15
   Arg Tyr Ala Gly Arg Lys Ala Val Val Ile Lys Asn Tyr Asp Asp Gly
               20                  25                  30
   Thr Ser Asp Lys Gln Tyr Gly His Ala Val Val Ala Gly Ile Asp Arg
           35                  40                  45
   Tyr Pro Arg Lys Ile His Lys Arg Met Gly Lys Gly Lys Met His Lys
       50                  55                  60
   Arg Ser Lys Ile Lys Pro Phe Leu Lys Val Leu Asn Tyr Asn His Leu
   65                  70                  75                  80
   Met Pro Thr Arg Tyr Ser Val Asp Leu Thr Ser Asp Leu Lys Val Ala
                   85                  90                  95
   Pro Lys Asp Leu Lys Asp Pro Val Lys Arg Lys Lys Ile Arg Phe Gln
               100                 105                 110
   Thr Arg Val Lys Phe Glu Glu Arg Tyr Lys Gln Gly Lys His Lys Trp
           115                 120                 125
   Phe Phe Gln Lys Leu Arg Phe
       130                 135
<210> 391
   <211> 141
   <212> PRT
   <213> leptinotarsa decemlineata
<400> 391
   Tyr Arg Glu Lys Tyr Thr Thr Tyr Lys Met Arg Asn Thr Tyr Glu Leu
   1               5                   10                  15
   Ser Pro Lys Glu Ala Ala Asn Phe Thr Arg Arg Asn Leu Ala Asp Thr
               20                  25                  30
   Leu Arg Ser Arg Ser Pro Tyr His Val Asn Leu Leu Leu Ala Gly Tyr
   35 40 45
   Asp Lys Lys Asp Gly Ala Gln Leu Tyr Tyr Met Asp Tyr Leu Ala Ser
   50 55 60
   Val Ala Ser Val Asp Tyr Ala Ala His Gly Tyr Gly Gly Tyr Phe Ser
   65 70 75 80
   Leu Ser Ile Met Asp Arg Asn Tyr Leu Lys Thr Leu Ser Lys Asp Gln
   85 90 95
   Gly Tyr Glu Leu Leu Lys Glu Cys Val Lys Glu Val Gln Lys Arg Leu
   100 105 110
   Ala Ile Asn Leu Pro Asn Phe Lys Val Gln Val Ile Asp Lys Asp Gly
   115 120 125
   Ile Lys Asp Met Pro Asn Ile Thr Ser Lys Gly Leu Asn
   130 135 140
<210> 392
   <211> 104
   <212> PRT
   <213> leptinotarsa decemlineata
<400> 392
   Arg Gly Leu Asp Thr Ser Leu Lys His Arg Lys Met Lys Phe Phe Lys
   1 5 10 15
   Ser Gly Ile Tyr Ser Val Val Phe Leu Ala Ile Ile Phe Ser Leu Val
   20 25 30
   Thr Glu Glu Val Glu Gly Arg Arg Thr Ile Leu Arg Gly Arg Lys Thr
   35 40 45
   Leu Thr Arg Thr Tyr Phe Arg Asp Asn Ala Val Pro Ala Tyr Val Ile
   50 55 60
   Val Ile Leu Val Gly Ile Gly Glu Ile Ile Leu Gly Ala Ile Leu Tyr
   65 70 75 80
   Val Ile Met Arg Lys Thr Ile Ile Asp Phe Pro Leu Ser Gly Ser Tyr
   85 90 95
   Ala Val Ala Pro Thr Gln Glu Ala
   100
<210> 393
   <211> 266
   <212> PRT
   <213> leptinotarsa decemlineata
<400> 393
   Met Ser Lys Ile Asn Glu Val Ser Asn Leu Tyr Lys Gln Leu Lys Ser
   1               5                   10                  15
   Glu Trp Asn Thr Ser Asn Pro Asn Leu Ser Lys Cys Glu Lys Leu Leu
               20                  25                  30
   Ser Asp Leu Lys Leu Glu Leu Thr His Leu Met Phe Leu Pro Thr Ser
           35                  40                  45
   Asn Ala Thr Ala Ser Lys Gln Glu Leu Leu Leu Ala Arg Asp Val Leu
       50                  55                  60
   Glu Ile Gly Val Gln Trp Ser Ile Ala Ala Asn Asp Ile Pro Ala Phe
   65                  70                  75                  80
   Glu Arg Tyr Met Ala Gln Leu Lys Cys Tyr Tyr Phe Asp Tyr Lys Asn
                   85                  90                  95
   Gln Leu Pro Glu Ser Ser Phe Lys Tyr Gln Leu Leu Gly Leu Asn Leu
               100                 105                 110
   Leu Phe Leu Leu Ser Gln Asn Arg Val Ala Glu Phe His Thr Glu Leu
           115                 120                 125
   Glu Leu Leu Pro Ala Asp His Ile Gln Asn Asp Val Tyr Ile Arg His
       130                 135                 140
   Pro Pro Ser Ile Glu Gln Tyr Leu Met Glu Gly Ser Tyr Asn Lys Ile
   145                 150                 155                 160
   Phe Leu Ala Lys Gly Asn Val Pro Ala Thr Asn Tyr Asn Phe Phe Met
                   165                 170                 175
   Asp Ile Leu Leu Asp Thr Ile Arg Gly Glu Ile Ala Asp Cys Leu Glu
               180                 185                 190
   Lys Ala Tyr Glu Lys Ile Ser Ile Lys Asp Val Ala Arg Met Leu Tyr
           195                 200                 205
   Leu Gly Ser Glu Glu Ser Ala Lys Ala Phe Val Thr Lys Ser Lys Thr
       210                 215                 220
   Trp Lys Leu Glu Lys Asp Asn Phe Phe His Phe Thr Pro Glu Val Lys
   225                 230                 235                 240
   Lys Thr His Glu Pro Ile Leu Ser Lys Glu Leu Ala Gln Gln Ala Ile
   245 250 255
   Glu Tyr Ala Lys Glu Leu Glu Met Ile Val
   260 265
<210> 394
   <211> 750
   <212> DNA
   <213> Leptinotarsa decemlineata
<400> 394
<210> 395
   <211> 204
   <212> PRT
   <213> Leptinotarsa decemlineata
<400> 395
   Pro Ile Asn Met Cys Asp Glu Glu Val Ala Ala Leu Val Val Asp Asn
   1               5                   10                  15
   Gly Ser Gly Met Cys Lys Ala Gly Phe Ala Gly Asp Asp Ala Pro Arg
               20                  25                  30
   Ala Val Phe Pro Ser Ile Val Gly Arg Pro Arg His Gln Gly Val Met
           35                  40                  45
   Val Gly Met Gly Gln Lys Asp Ser Tyr Val Gly Asp Glu Ala Gln Ser
       50                  55                  60
   Lys Arg Gly Ile Leu Thr Leu Lys Tyr Pro Ile Glu His Gly Ile Val
   65                  70                  75                  80
   Thr Asn Trp Asp Asp Met Glu Lys Ile Trp His His Thr Phe Tyr Asn
                   85                  90                  95
   Glu Leu Arg Val Ala Pro Glu Glu His Pro Val Leu Leu Thr Glu Ala
               100                 105                 110
   Pro Leu Asn Pro Lys Ala Asn Arg Glu Lys Met Thr Gln Ile Met Phe
           115                 120                 125
   Glu Thr Phe Asn Thr Pro Ala Met Tyr Val Ala Ile Gln Ala Val Leu
       130                 135                 140
   Ser Leu Tyr Ala Ser Gly Arg Thr Thr Gly Ile Val Leu Asp Ser Gly
   145                 150                 155                 160
   Asp Gly Val Ser His Thr Val Pro Ile Tyr Glu Gly Tyr Ala Leu Pro
                   165                 170                 175
   His Ala Ile Leu Arg Leu Asp Leu Ala Gly Arg Asp Leu Thr Asp Tyr
               180                 185                 190
   Leu Met Lys Ile Leu Thr Glu Arg Gly Tyr Ser Phe
           195                 200
<210> 396
   <211> 44
   <212> DNA
   <213> Artificial
<220>
   <223> Primer
<400> 396
   gcgtaatacg actcactata ggatgtgtga cgaagaggtt gccg 44
<210> 397
   <211> 24
   <212> DNA
   <213> Artificial
<220>
   <223> Primer
<400> 397
   gtcaacaaaa cagggtgctc ttcg 24
<210> 398
   <211> 22
   <212> DNA
   <213> Artificial
<220>
   <223> Primer
<400> 398
   atgtgtgacg aagaggttgc cg 22
<210> 399
   <211> 46
   <212> DNA
   <213> Artificial
<220>
   <223> Primer
<400> 399
   gcgtaatacg actcactata gggtcaacaa aacagggtgc tcttcg 46
<210> 400
   <211> 320
   <212> DNA
   <213> Leptinotarsa decemlineata
<400> 400
   atgtgtgacg aagaggttgc cgcattagtc gtagacaatg gatctggtat gtgcaaagct 60
   ggatttgctg gggatgatgc cccccgtgca gttttcccat ccattgttgg tcgtccaaga 120
   catcaaggag ttatggtagg aatgggccaa aaggactcgt atgtaggaga tgaagcccaa 180
   agcaaaagag gtatccttac cttgaaatac cccattgaac acggtattgt cacaaactgg 240
   gatgatatgg agaaaatctg gcaccatacc ttctacaatg aacttcgagt tgcccccgaa 300
   gagcaccctg ttttgttgac 320
<210> 401
   <211> 1152
   <212> DNA
   <213> Artificial
<220>
   <223> Hairpin
<400> 401
   tgacgacctt gagttggttt ctgaagttga actcggcagc cttcttctgg agcttggcga 60
   atttgttttc gtacttggaa acctttttca aggtaggttt gacgaattta ccacggaggt 120
   cgttgacctg gctgttgagg tcggcgatct caaggtcacg tctctccact tcgaattcga 180
   tgtcaatttt actcctctcc agagcgtcaa ttcgcttatg gtagtctgtg cagagtttct 240
   tcaaggttgc ttcattggcg ttgtcgacgt cggcaatttg cccgcagcgc tcctcaatcg 300
   ttcgcctcct ctcagctgct ttgcgttcct gctccttctt cagttcctca gcggcttttt 360
   tcctcagcag gagtcggagt ttcttcttcc tttccggggt catgaaaccc ttcttggctt 420
   tcttcgcctt agaggcttcc tccatcctct tgcgcacttc agcgcgcttc ctctcgattt 480
   cggcctgttt gtctagaagg taagtgtaca cactacattt tcatgaacat tattgcgacc 540
   gttgagattc tcattgtttg gtgattgatt atctaaagta gaagcatgaa tagatataac 600
   ataaactagt aactaatggg ttagttatgg gtatacttca tgcttttctc tcaggctcga 660
   gcaaacaggc cgaaatcgag aggaagcgcg ctgaagtgcg caagaggatg gaggaagcct 720
   ctaaggcgaa gaaagccaag aagggtttca tgaccccgga aaggaagaag aaactccgac 780
   tcctgctgag gaaaaaagcc gctgaggaac tgaagaagga gcaggaacgc aaagcagctg 840
   agaggaggcg aacgattgag gagcgctgcg ggcaaattgc cgacgtcgac aacgccaatg 900
   aagcaacctt gaagaaactc tgcacagact accataagcg aattgacgct ctggagagga 960
   gtaaaattga catcgaattc gaagtggaga gacgtgacct tgagatcgcc gacctcaaca 1020
   gccaggtcaa cgacctccgt ggtaaattcg tcaaacctac cttgaaaaag gtttccaagt 1080
   acgaaaacaa attcgccaag ctccagaaga aggctgccga gttcaacttc agaaaccaac 1140
   tcaaggtcgt ca 1152
<210> 402
   <211> 1192
   <212> DNA
   <213> Artificial
<220>
   <223> Hairpin
<400> 402
   ctcttgcttc ttggtggcga tcctctcctc gcgcctcttc ttcgcctcct tgaccttgag      60
   acgtctcgcc tctgcctggt ccttcaacat ctttgatctc gccttttcag ccttcttctt     120
   gtgaatgaag tccatcagta ccctcttgtt tttgaagacg ttacctttgg ctttcatgta     180
   aaggtcgtgg tacatttgcc tatcgatctt cttggcttct ctgtattttt taaggagccg     240
   tcgcaggact ctcattctgt tgacccacag gaccttcaca ggcattctgg cgttggcggt     300
   acccttcctc ttaccgaagc cacagtgacg acccttccgt ctggcttctg tgtttttacg     360
   gacgcgggct ctggagtgga cagccacagg ctttttgatg atcaaaccat ccttgatcag     420
   cttacggatg ttttgcctag agttggtgtt ggcgatttcg ttgatttcat tagggtccaa     480
   ccacactttc ttcttgccgc atctcatcac ctctagaagg taagtgtaca cactacattt     540
   tcatgaacat tattgcgacc gttgagattc tcattgtttg gtgattgatt atctaaagta     600
   gaagcatgaa tagatataac ataaactagt aactaatggg ttagttatgg gtatacttca     660
   tgcttttctc tcaggctcga gggtgatgag atgcggcaag aagaaagtgt ggttggaccc     720
   taatgaaatc aacgaaatcg ccaacaccaa ctctaggcaa aacatccgta agctgatcaa     780
   ggatggtttg atcatcaaaa agcctgtggc tgtccactcc agagcccgcg tccgtaaaaa     840
   cacagaagcc agacggaagg gtcgtcactg tggcttcggt aagaggaagg gtaccgccaa     900
   cgccagaatg cctgtgaagg tcctgtgggt caacagaatg agagtcctgc gacggctcct     960
   taaaaaatac agagaagcca agaagatcga taggcaaatg taccacgacc tttacatgaa    1020
   agccaaaggt aacgtcttca aaaacaagag ggtactgatg gacttcattc acaagaagaa    1080
   ggctgaaaag gcgagatcaa agatgttgaa ggaccaggca gaggcgagac gtctcaaggt    1140
   caaggaggcg aagaagaggc gcgaggagag gatcgccacc aagaagcaag ag            1192
<210> 403
   <211> 792
   <212> DNA
   <213> Artificial
<220>
   <223> Hairpin
<400> 403
   actctgtctg gcttttggct gtgacgcaca gttcatagag ataaccttca cccgaatatg 60
   ccttgcgagg tcgcaaaatc ggcgaaattc catacctgtt caccgacgac ggcgctggat 120
   caattccaca gttttcgcga tccagactga atgcccacag gccgtcgagt tttttgattt 180
   cagatacgta cacttttccc ggcaataaca tacggcgtga catcggcttc aaatggcgta 240
   tagccgccct gatgctccat cacactttgc cgtaatgagt gaccgcatcg aaacgcagca 300
   cgatacgctg gtctagaagg taagtgtaca cactacattt tcatgaacat tattgcgacc 360
   gttgagattc tcattgtttg gtgattgatt atctaaagta gaagcatgaa tagatataac 420
   ataaactagt aactaatggg ttagttatgg gtatacttca tgcttttctc tcaggctcga 480
   gccagcgtat cgtgctgcgt ttcgatgcgg tcactcatta cggcaaagtg tgatggagca 540
   tcagggcggc tatacgccat ttgaagccga tgtcacgccg tatgttattg ccgggaaaag 600
   tgtacgtatc tgaaatcaaa aaactcgacg gcctgtgggc attcagtctg gatcgcgaaa 660
   actgtggaat tgatccagcg ccgtcgtcgg tgaacaggta tggaatttcg ccgattttgc 720
   gacctcgcaa ggcatattcg ggtgaaggtt atctctatga actgtgcgtc acagccaaaa 780
   gccagacaga gt 792
<210> 404
   <211> 839
   <212> DNA
   <213> leptinotarsa decemlineata
<400> 404
   cagtcagcac cgagtccttg ttgactgctc acattttcca tcgtttctac cagaacaaca 60
   gcaacaactt tcatcatggc ggacgacgag gaaaagagga ggaaacaagc ggaaattgaa 120
   cgcaagaggg ctgaggtcag ggctcgcatg gaagaggcct ccaaggccaa aaaagccaag 180
   aaaggtttca tgacccctga gaggaagaag aaacttaggt tattgctgag aaagaaagca 240
   gcagaagaac tgaaaaaaga acaagaacgc aaagctgccg aaaggcgtat tattgaagag 300
   agatgcggaa aaccaaaact cattgatgag gcaaatgaag agcaggtgag gaactattgc 360
   aagttatatc acggtagaat agctaaactg gaggaccaga aatttgattt ggaatacctt 420
   gtcaaaaaga aagacatgga gatcgccgaa ttgaacagtc aagtcaacga cctcaggggt 480
   aaattcgtca aacccactct caagaaagta tccaaatacg agaacaaatt tgctaaactc 540
   caaaagaaag cagcagaatt caatttccgt aatcaactga aagttgtaaa gaagaaggag 600
   ttcaccctgg aggaggaaga caaagaaaag aagcccgatt ggtcgaagaa gggagacgaa 660
   aagaaggtac aagaagtgga agcatgatct gtccctacaa tttaatattt cccttcgtcc 720
   gtggaaattt tacaacttaa gatatattta ttttattcgc ttcttatgag actatgaaag 780
   tgatgtctgc atgtatatta ttcgttttat gtatgtatta aaaaaagaac ttgattgaa 839
<210> 405
   <211> 203
   <212> PRT
   <213> leptinotarsa decemlineata
<400> 405
   Met Ala Asp Asp Glu Glu Lys Arg Arg Lys Gln Ala Glu Ile Glu Arg
   1               5                   10                  15
   Lys Arg Ala Glu Val Arg Ala Arg Met Glu Glu Ala Ser Lys Ala Lys
               20                  25                  30
   Lys Ala Lys Lys Gly Phe Met Thr Pro Glu Arg Lys Lys Lys Leu Arg
           35                  40                  45
   Leu Leu Leu Arg Lys Lys Ala Ala Glu Glu Leu Lys Lys Glu Gln Glu
       50                  55                  60
   Arg Lys Ala Ala Glu Arg Arg Ile Ile Glu Glu Arg Cys Gly Lys Pro
   65                  70                  75                  80
   Lys Leu Ile Asp Glu Ala Asn Glu Glu Gln Val Arg Asn Tyr Cys Lys
                   85                  90                  95
   Leu Tyr His Gly Arg Ile Ala Lys Leu Glu Asp Gln Lys Phe Asp Leu
               100                 105                 110
   Glu Tyr Leu Val Lys Lys Lys Asp Met Glu Ile Ala Glu Leu Asn Ser
           115                 120                 125
   Gln Val Asn Asp Leu Arg Gly Lys Phe Val Lys Pro Thr Leu Lys Lys
       130                 135                 140
   Val Ser Lys Tyr Glu Asn Lys Phe Ala Lys Leu Gln Lys Lys Ala Ala
   145                 150                 155                 160
   Glu Phe Asn Phe Arg Asn Gln Leu Lys Val Val Lys Lys Lys Glu Phe
                   165                 170                 175
   Thr Leu Glu Glu Glu Asp Lys Glu Lys Lys Pro Asp Trp Ser Lys Lys
               180                 185                 190
   Gly Asp Glu Lys Lys Val Gln Glu Val Glu Ala
           195                 200